(19) Europäisches Patentamt
European Patent Office
Office européen des brevets

(11) EP 1 392 641 B1

(12) # EUROPÄISCHE PATENTSCHRIFT

(45) Veröffentlichungstag und Bekanntmachung des Hinweises auf die Patenterteilung:
**11.04.2007 Patentblatt 2007/15**

(21) Anmeldenummer: **02738038.5**

(22) Anmeldetag: **08.05.2002**

(51) Int Cl.:
***C07C 211/36*** (2006.01) ***C07C 233/79*** (2006.01)
***C07D 209/14*** (2006.01) ***C07D 333/58*** (2006.01)
***C07D 277/62*** (2006.01) ***A61K 31/132*** (2006.01)
***A61K 31/165*** (2006.01) ***A61K 31/404*** (2006.01)
***A61K 31/425*** (2006.01) ***A61K 31/38*** (2006.01)
***A61P 25/04*** (2006.01)

(86) Internationale Anmeldenummer:
**PCT/EP2002/005051**

(87) Internationale Veröffentlichungsnummer:
**WO 2002/090317 (14.11.2002 Gazette 2002/46)**

(54) **SUBSTITUIERTE CYCLOHEXAN-1,4-DIAMINDERIVATE**

SUBSTITUTED CYCLOHEXANE-1,4-DIAMINE DERIVATIVES

DERIVES SUBSTITUES DE CYCLOHANE-1,4-DIAMINE

(84) Benannte Vertragsstaaten:
**AT BE CH CY DE DK ES FI FR GB GR IE IT LI LU MC NL PT SE TR**
Benannte Erstreckungsstaaten:
**LT LV RO SI**

(30) Priorität: **09.05.2001 DE 10123163**

(43) Veröffentlichungstag der Anmeldung:
**03.03.2004 Patentblatt 2004/10**

(73) Patentinhaber: **Grünenthal GmbH**
**52078 Aachen (DE)**

(72) Erfinder:
- **SUNDERMANN, Bernd**
**52066 Aachen (DE)**
- **HENNIES, Hagen-Heinrich**
**52152 Simmerath (DE)**
- **ENGLBERGER, Werner**
**52223 Stolberg (DE)**
- **KÖGEL, Babette-Yvonne**
**52379 Langerwehe-Hamaich (DE)**

(56) Entgegenhaltungen:
**WO-A-99/36421** **US-A- 4 113 866**

- **BERTORELLI R ET AL: "Nociceptin/orphanin FQ and its receptor: a potential target for drug discovery" TRENDS IN PHARMACOLOGICAL SCIENCES, ELSEVIER TRENDS JOURNAL, CAMBRIDGE, GB, Bd. 21, Nr. 7, 1. Juli 2000 (2000-07-01), Seiten 233-234, XP004209778 ISSN: 0165-6147**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 392 641 B1

## Beschreibung

Die vorliegende Erfindung betrifft substituierte Cyclohexan-1,4-diaminderivate, Verfahren zu deren Herstellung, Arzneimittel enthaltend diese Verbindungen und die Verwendung von substituierten Cyclohexan-1,4-diaminderivaten zur Herstellung von Arzneimitteln.

Das Heptadekapeptid Nociceptin ist ein endogener Ligand des ORL1 (Opioid-Receptor-Like)-Rezeptors (Meunier et al., Nature 377, 1995, S. 532-535), der zu der Familie der Opioid Rezeptoren gehört und in vielen Regionen des Gehirns und des Rückenmarks zu finden ist (Mollereau et al., FEBS Letters, 341, 1994, S. 33-38, Darland et al., Trends in Neurosciences, 21, 1998, S. 215-221). Das Peptid ist durch eine hohe Affinität, mit einem $K_d$-Wert von annähernd 56 pM (Ardati et al., Mol. Pharmacol. 51, S. 816-824), und durch eine hohe Selektivität für den ORL1-Rezeptor gekennzeichnet. Der ORL1-Rezeptor ist homolog zu den μ, κ und δ Opioid-Rezeptoren und die Aminosäuresequenz des Nociceptin-Peptids weist eine starke Ähnlichkeit mit denen der bekannten Opioidpeptide auf. Die durch das Nociceptin induzierte Aktivierung des Rezeptors führt über die Kopplung mit $G_{i/o}$-Proteinen zu einer Inhibierung der Adenylatcyclase (Meunier et al., Nature 377, 1995, S. 532-535). Auch auf der zellulären Ebene sind funktionelle Ähnlichkeiten der μ, κ und δ Opioid-Rezeptoren mit dem ORL1-Rezeptor in Bezug auf die Aktivierung des Kalium-Kanals (Matthes et al., Mol. Pharmacol. 50, 1996, S. 447-450; Vaughan et al., Br. J. Pharmacol. 117, 1996, S. 1609-1611) und der Inhibierung der L-, N- und P/Q-Typ-Kalzium-Kanäle vorhanden (Conner et al., Br. J. Pharmacol. 118, 1996, S. 205-207; Knoflach et al., J. Neuroscience 16, 1996, S. 6657-6664).

Das Nociceptin-Peptid zeigt nach intercerebroventicularer Applikation eine pronociceptive und hyperalgetische Aktivität in verschiedenen Tiermodellen (Reinscheid et al., Science 270, 1995, S. 792-794; Hara et al,. Br. J. Pharmacol. 121, 1997, S. 401-408). Diese Befunde können als Hemmung der stressinduzierten Analgesie erklärt werden (Mogil et al., Neurosci. Letters 214, 1996, S131-134; sowie Neuroscience 75, 1996, S. 333-337). In diesem Zusammenhang konnte auch eine anxiolytische Aktivität des Nociceptin nachgewiesen werden (Jenck et al., Proc. Natl. Acad. Sci. USA 94, 1997, 14854-14858).

Auf der anderen Seite konnte in verschiedenen Tiermodellen, insbesondere nach intrathekaler Applikation, auch ein antinociceptiver Effekt von Nociceptin gezeigt werden. Nociceptin hemmt die Aktivität Kainat- oder Glutamat-stimulierter Hinterwurzelganglienneuronen (Shu et al., Neuropeptides, 32, 1998, 567-571) oder Glutamat-stimulierter Rückenmarksneuronen (Faber et al., Br. J. Pharmacol., 119, 1996, S. 189-190); es wirkt antinociceptiv im Tail Flick-Test in der Maus (King et al., Neurosci. Lett., 223, 1997, 113-116), im Flexor-Reflex-Modell in der Ratte (Xu et al., NeuroReport, 7, 1996, 2092-2094) und im Formalin-Test an der Ratte (Yamamoto et al., Neuroscience, 81, 1997, S. 249-254). In Modellen für neuropathische Schmerzen konnte ebenfalls eine antinociceptive Wirkung von Nociceptin nachgewiesen werden (Yamamoto und Nozaki-Taguchi, Anesthesiology, 87, 1997), die insofern besonders interessant ist, als das die Wirksamkeit von Nociceptin nach Axotomie von Spinalnerven zunimmt. Dies steht im Gegensatz zu den klassischen Opioiden, deren Wirksamkeit unter diesen Bedingungen abnimmt (Abdulla und Smith, J. Neurosci., 18, 1998, S. 9685-9694).

Der ORL1-Rezeptor ist außerdem noch an der Regulation weiterer physiologischer und pathophysiologischer Prozesse beteiligt. Hierzu gehören unter anderem Lernen und Gedächtnisbildung (Sandin et al., Eur. J. Neurosci., 9, 1997, S. 194-197; Manabe et al., Nature, 394, 1997, S. 577-581), Hörvermögen (Nishi et al., EMBO J., 16, 1997, S. 1858-1864), Nahrungsaufnahme (Pomonis et al., NeuroReport, 8, 1996, S. 369-371), Regulation des Blutdruckes (Gumusel et al., Life Sci., 60, 1997, S. 141-145; Campion und Kadowitz, Biochem. Biophys. Res. Comm., 234, 1997, S. 309-312), Epilepsie (Gutiérrez et al, Abstract 536.18, Society for Neuroscience, Vol 24, 28th Ann. Meeting, Los Angeles, November 7.-12, 1998) und Diurese (Kapista et al., Life Sciences, 60, 1997, PL 15-21). In einem Übersichtsartikel von Calo et al. (Br.J. Pharmacol., 129, 2000, 1261 -1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf μ-Agonisten wie Morphin, Behandlung von Entzugserscheinungen, Reduzierung des Suchtpotentials von Morphinen, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, aterieller Blutdruck, Wasserspeicher-Krankheiten, intestinale Motilität (Diarrhoe), relaxierende Effekte auf die Atemwege, Mikturations Reflex (Haminkontinenz). Weiter wird die Verwendung von Agonisten und Antagonisten als Anoretika, Analgetika (auch in Coadministration mit Opioiden) oder Nootropika diskutiert. Die Bedeutung von Liganden des ORL-1-Rezeptors im Zusammenhang mit Analgesie und Anxiolyse sind in Bertorelli et al., Trends in Pharmaceutical Sciences 2000, Bd. 21, Nr. 7, Seiten 233-234 hervorgehoben.

Entsprechend vielfältig sind die Anwendungsmöglichkeiten von Verbindungen, die an den ORL1-Rezeptor binden und diesen aktivieren oder inhibieren.

WO 99/36421 offenbart ORL-1-Rezeporagonisten, deren Kernstruktur aus einem 2-Keto-benzimidazolinyl-Rest verknüpft mit einem Piperidin-Ring besteht. Die Verbindungen weisen eine analgetische Wirkung auf.

Aufgabe der vorliegenden Erfindung war es, Arzneimittel zur Verfügung zu stellen, die auf das Nociceptin/ORL1-Rezeptor-System wirken und damit für Arzneimittel insbesondere zur Behandlung der verschiedenen mit diesem System nach dem Stand der Technik in Verbindung stehenden Krankeiten bzw. zum Einsatz in den dort genannten Indikationen geeignet sind.

Ein Gegenstand der Erfindung sind daher im folgenden Verbindungsgruppe (**A**) genannte, substituierte Cyclohexan-1,4-diaminderivate der allgemeinen Formel I,

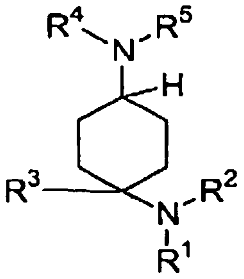

I

, worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen, oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^4$ ausgewählt ist aus H, $C_{1-8}$-Alkyl, gesättigt oder ungesättigt, verzweigt oder unverzweigt, einfach oder mehrfach substituiert oder unsubstituiert; oder $C(X)R^7$, $C(X)NR^7R^8$, $C(X)OR^9$, $C(X)SR^9$, $S(O_2)R^9$

mit X = O oder S.

mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

mit $R^8$ ausgewählt aus H, $C_{1-4}$-Alkyl, gesättigt oder ungesättigt, verzweigt oder unverzweigt, einfach oder mehrfach substituiert oder unsubstituiert oder

die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,

mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach

substituiert;

$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2CH_2R^{12}$ oder $-C(Y)-CH_2-CH_2-CH_2R^{12}$

mit Y = O, S oder $H_2$,

mit $R^{11}$ ausgewählt aus

H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;

und mit $R^{12}$ ausgewählt aus

H; $C_{3-8}$-Cycloalkyl, Aryl oder Heteroaryl, jeweils unsubstituiert oder einfach oder mehrfach substituiert,

oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann,

mit der Maßgabe,

■ daß, wenn $R^3$ substituiertes oder unsubstituiertes Phenyl ist und mindestens einer von $R^1$ oder $R^2$ H oder $C_{1-8}$-Alkyl ist, $R^4$ nicht Alkyl sein darf und $R^4$ und $R^5$ nicht zusammen einen Heterocyclus bilden dürfen

oder

■ daß, wenn $R^3$ unsubstituiertes Phenyl ist und $R^1$ und $R^2$ zusammen $(CH_2)_5$ bedeuten, $R^4$ ausgewählt ist aus H oder $C_{1-8}$-Alkyl, Y nicht O oder S bedeutet und $R^5$ nicht $C_{1-6}$-Alkyl ist,

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;

in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

Ein weiterer die Aufgabe der Erfindung lösender Gegenstand sind im Folgenden als Verbindungsgruppe (**B**) bezeichnete, substituierte Cyclohexan-1,4-diaminderivate der allgemeinen Formel 1,

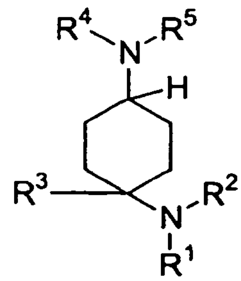

I

, worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen, oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte

$C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^4$ ausgewählt ist aus H, $C(X)R^7$, $C(X)NR^7R^8$, $C(X)OR^9$ oder $C(X)SR^9$, $S(O_2)R^9$

mit X = O oder S,

mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

mit $R^8$ ausgewählt aus H, $C_{1-4}$-Alkyl, gesättigt oder ungesättigt, verzweigt oder unverzweigt, einfach oder mehrfach substituiert oder unsubstituiert oder

die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,

mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$,$-CHR^{11}$- $CH_2R^{12}$, $-CHR^{11}-CH_2-CH_2R^{12}$, $-CHR^{11}-CH_2-CH_2-CH_2R^{12}$,$-C(Y)R^{12}$, $-C(Y)-CH_2R^{12}$, $-C(Y)-CH_2CH_2R^{12}$ oder $-C(Y)-CH_2-CH_2-CH_2R^{12}$

mit Y = O, S oder $H_2$,

mit $R^{11}$ ausgewählt aus

H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;

und mit $R^{12}$ ausgewählt aus

$C_{3-8}$-Cycloalkyl, Aryl oder Heteroaryl, jeweils unsubstituiert oder einfach oder mehrfach substituiert,

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;

in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

Ein weiterer die Aufgabe der Erfindung lösender Gegenstand sind im Folgenden als Verbindungsgruppe (**C**) bezeichnete, substituierte Cyclohexan-1,4-diaminderivate der allgemeinen Formel 1,

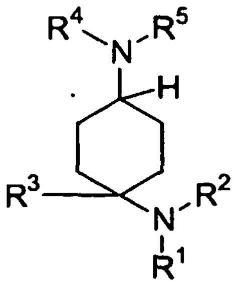

I

, worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl,

jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C(X)R^7$, $C(X)NR^7R^8$, $C(X)OR^9$ oder $C(X)SR^9$, $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
oder die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$,$-CHR^{11}$- $CH_2R^{12}$, $-CHR^{11}-CH_2-CH_2R^{12}$, $-CHR^{11}-CH_2-CH_2-CH_2R^{12}$, - $C(Y)R^{12}$, $-C(Y)-CH_2R^{12}$, -C (Y)-$CH_2$-$CH_2R^{12}$ oder-C(Y)-$CH_2$-$CH_2$-$CH_2R^{12}$
mit Y = O, S oder $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder C(O)O-$C_{1-6}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

Ein weiterer die Aufgabe der Erfindung lösender Gegenstand sind im Folgenden als Verbindungsgruppe (**D**) bezeichnete, substituierte Cyclohexan-1,4-diaminderivate der allgemeinen Formel I,

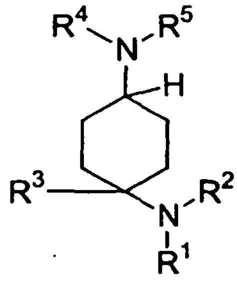

I

, worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,

oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Heteroaryl, unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$, $C(X)OR^9$, $C(X)SR^9$, $S(O_2)R^9$

mit X = O oder S,

mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

mit $R^8$ ausgewählt aus H, $C_{1-4}$-Alkyl, gesättigt oder ungesättigt, verzweigt oder unverzweigt, einfach oder mehrfach substituiert oder unsubstituiert oder

oder die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,

mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2CH_2R^{12}$ oder $-C(Y)-CH_2-CH_2-CH_2R^{12}$

mit Y = O, S oder $H_2$,

mit $R^{11}$ ausgewählt aus

H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert

oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

Ein weiterer die Aufgabe der Erfindung lösender Gegenstand sind im Folgenden als Verbindungsgruppe (**E**) bezeichnete, substituierte Cyclohexan-1,4-diaminderivate der allgemeinen Formel I,

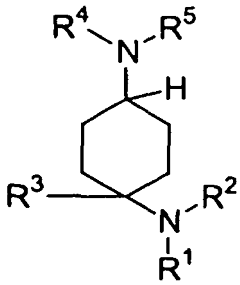

I

, worin
die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$, $C(X)OR^9$, $C(X)SR^9$, $S(O_2)R^9$
mit X=O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}CH_2CH_2$ oder $(CH_2)_3$-$_6$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$,-CHR"- $CH_2R^{12}$, $-CHR^{11}-CH_2-CH_2R^{12}$, $-CHR^{11}-CH_2-CH_2-CH_2R^{12}$, - $C(Y)R^{12}$, $-C(Y)-CH_2R^{12}$, -C (Y)-$CH_2$-$CH_2R^{12}$ oder -C(Y)-$CH_2$-$CH_2$-$CH_2R^{12}$
mit Y = O, S oder $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder C(O)O-$C_{1-6}$-Alkyl, gesättigt oder ungesättigt, verzweigt oder unverzweigt, einfach oder mehrfach substituiert oder unsubstituiert;
und mit $R^{12}$ ausgewählt aus
$C_{3-8}$-Cycloalkyl, Aryl oder Heteroaryl, jeweils unsubstituiert oder einfach oder mehrfach substituiert,
oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

Alle diese erfindungsgemäßen Verbindungen bzw. Verbindungsgruppen zeigen hervorragende Bindung an den ORL1-Rezeptor.

Verbindungen, die eine gewisse Verwandtschaft mit den hier vorgeschlagenen Verbindungen zeigen, sind aus folgenden Schriften bekannt:

- Den zusammenhängenden US-Patenten US 4,460,604, US 4,447,454 und 4,113,866 (Lednicer et al.). Darin werden die genannten Verbindungen als analgetisch wirksam beschrieben, ohne daß Bezug auf den ORL1-Rezeptor genommen wird.
- Der US 5,304,479 (Lin et al.). Die dort beschriebenen Verbindungen sind zum Einsatz in analytischen Testsystemen zur Bestimmung von Phencyclidin (PCP) insbesondere in Körperflüssigkeiten bestimmt, ohne daß Bezug auf den ORL1-Rezeptor genommen wird.
- De Costa et al., J.Chem.Soc., Perkin Trans. 1 (1992), 1671-80. Die genannten Verbindungen werden im Zusammenhang mit der Synthese irreversibler Liganden an der Dopamin-Reuptake-Site erwähnt, ohne daß Bezug auf den ORL1-Rezeptor genommen wird.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht $C_{1-2}$-Alkyl für C1- oder C2-Alkyl, $C_{1-3}$-Alkyl für C1-, C2- oder C3-Alkyl, $C_{1-4}$-Alkyl für C1-, C2-, C3- oder C4-Alkyl, $C_{1-5}$-Alkyl für C1-, C2-, C3-, C4- oder C5-Alkyl, $C_{1-6}$-Alkyl für C1-, C2-, C3-, C4-, C5- oder C6-Alkyl, $C_{1-7}$-Alkyl für C1-, C2-, C3-, C4-, C5-, C6- oder C7-Alkyl, $C_{1-8}$-Alkyl für C1-, C2-, C3-, C4-, C5-, C6-, C7- oder C8-Alkyl, $C_{1-10}$-Alkyl für C1-, C2-, C3-, C4-, C5-, C6-, C7-, C8,- C9- oder C10-Alkyl und $C_{1-18}$-Alkyl für C1-, C2-, C3-, C4-, C5-, C6-, C7-, C8,- C9-, C10-, C11-, C12-C13-, C14-, C15-, C16-, C17- oder C18-Alkyl. Weiter steht $C_{3-4}$-Cycloalkyl für C3- oder C4-Cycloalkyl, $C_{3-5}$-Cycloalkyl für C3-, C4- oder C5-Cycloalkyl, $C_{3-6}$-Cycloalkyl für C3-, C4-, C5- oder C6-Cycloalkyl, $C_{3-7}$-Cycloalkyl für C3-, C4-, C5-, C6- oder C7-Cycloalkyl, $C_{3-8}$-Cycloalkyl für C3-, C4-, C5-, C6-, C7- oder C8-Cycloalkyl, $C_{4-5}$-Cycloalkyl für C4- oder C5-Cycloalkyl, $C_{4-6}$-Cycloalkyl für C4-, C5- oder C6-Cycloalkyl, $C_{4-7}$-Cycloalkyl für C4-, C5-, C6- oder C7-Cycloalkyl, $C_{5-6}$-Cycloalkyl für C5- oder C6-Cycloalkyl und $C_{5-7}$-Cycloalky) für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, $CHF_2$, $CF_3$ oder $CH_2OH$ sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders

definiert ist - unter dem Begriff substituiert im Sinne dieser Erfindung die Substitution mindestens eines (gegebenenfalls auch mehrerer) Wasserstoffreste(s) durch F, Cl, Br, I, $NH_2$, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von $CF_3$ oder an verschiedenen Stellen wie im Falle von -CH(OH)-CH=CH-$CHCl_2$. Besonders bevorzugte Substituenten sind hier F, Cl und OH. In Bezug auf Cycloalkyl kann der Wasserstoffrest auch durch $OC_{1-3}$-Alkyl oder $C_{1-3}$-Alkyl (jeweils ein- oder mehrfach substituiert oder unsubstituiert), insbesondere Methyl, Ethyl, n-Propyl, i-Propyl, $CF_3$, Methoxy oder Ethoxy, ersetzt sein.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$ und $CH_2-CH_2-CH_2-CH_2-CH_2-CH_2-$ zu verstehen, unter $(CH_2)_{1-4}$ ist $-CH_2-$,$-CH_2-CH_2-$, $-CH_2-CH_2-CH_2-$ und $-CH_2-CH_2-CH_2-CH_2-$ zu verstehen, unter $(CH_2)_{4-5}$ ist-$CH_2-CH_2-CH_2-CH_2-$ und $-CH_2-CH_2-CH_2-CH_2-CH_2-$ zu verstehen, etc.

Unter einem Aryl-Rest werden Ringsysteme mit mindestens einem aromatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo[1,2,5] thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert die Substitution des Aryls oder Heteroaryls mit $R^{22}$, $OR^{22}$ einem Halogen, vorzugsweise F und/oder Cl, einem $CF_3$, einem CN, einem $NO_2$, einem $NR^{23}R^{24}$, einem $C_{1-6}$-Alkyl (gesättigt), einem $C_{1-6}$-Alkoxy, einem $C_{3-8}$-Cycloalkoxy, einem $C_{3-8}$-Cycloalkyl oder einem $C_{2-6}$-Alkylen.

Dabei steht der Rest $R^{22}$ für H, einen $C_{1-10}$-Alkyl-, vorzugsweise einen $C_{1-6}$-Alkyl-, einen Aryl- oder Heteroaryl- oder für einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen, die Reste $R^{23}$ und $R^{24}$, gleich oder verschieden, für H, einen $C_{1-10}$-Alkyl-, vorzugsweise einen $C_{1-6}$-Alkyl-, einen Aryl-, einen Heteroaryl- oder einen über $C_{1-3}$-Alkyl, gesättigt oder ungesättigt, oder eine $C_{1-3}$-Alkylen-Gruppe gebundenen Aryl- oder Heteroaryl-Rest bedeuten, wobei diese Aryl und Heteroarylreste nicht selbst mit Aryl- oder Heteroaryl- Resten substituiert sein dürfen,

oder die Reste $R^{23}$ und $R^{24}$ bedeuten zusammen $CH_2CH_2OCH_2CH_2$, $CH_2CH_2NR^{25}CH_2CH_2$ oder $(CH_2)_{3-6}$, und der Rest $R^{25}$ für H, einen $C_{1-10}$-Alkyl-, vorzugsweise einen $C_{1-6}$-Alkyl-, einen Aryl-, oder Heteroaryl- Rest oder für einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, a-Liponsäure, Acetylglycin, Acetylsalicylsäure, Hippursäure und/oder Asparaginsäure. Besonders bevorzugt ist das Hydrochlorid-Salz.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1$\lambda^6$-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, $\alpha$-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit $NH_4^+$, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch $NH_4^+$, insbesondere aber (Mono-) oder (Di-) Natrium-, (Mono-) oder (Di-) Kalium-, Magnesium- oder Calzium-Salze.

In einer bevorzugten Ausführungsform der Verbindungsgruppen (A), (B) oder (D) sind die substituierten Cyclohexan-1,4-diaminderivate so aufgebaut, daß gemäß Formel I

$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,

oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

mit $R^6$ ausgewählt aus H; $C_{1-8}$-Alkyl, gesättigt oder ungesättigt, verzweigt oder unverzweigt, einfach oder mehrfach substituiert oder unsubstituiert,

vorzugsweise

$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,

oder die Reste $R^1$ und $R^2$ zusammen einen Ring bilden und $(CH_2)_{4-5}$ bedeuten,

insbesondere

$R^1$ und $R^2$ unabhängig voneinander ausgewählt sind aus Methyl oder Ethyl oder die Reste $R^1$ und $R^2$ zusammen einen Ring bilden und $(CH_2)_5$ bedeuten.

In einer bevorzugten Ausführungsform der Verbindungsgruppe (E) sind die substituierten Cyclohexan-1,4-diaminderivate so aufgebaut, daß gemäß Formel I

$R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

mit $R^6$ ausgewählt aus H; $C_{1-8}$-Alkyl, gesättigt oder ungesättigt, verzweigt oder unverzweigt, einfach oder mehrfach substituiert oder unsubstituiert,

vorzugsweise

$R^1$ und $R^2$ zusammen einen Ring bilden und $(CH_2)_{4-5}$ bedeuten,

insbesondere

$R^1$ und $R^2$ zusammen einen Ring bilden und $(CH_2)_5$ bedeuten.

In einer bevorzugten Ausführungsform der Verbindungsgruppe (C) sind die substituierten Cyclohexan-1,4-diaminderivate so aufgebaut, daß gemäß Formel I

$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,

vorzugsweise

$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,

insbesondere

$R^1$ und $R^2$ unabhängig voneinander ausgewählt sind aus Methyl oder Ethyl.

In einer bevorzugten Ausführungsform der Verbindungsgruppen (A), (B) oder (C) sind die substituierten Cyclohexan-1,4-diaminderivate so aufgebaut, daß gemäß Formel I

$R^3$ ausgewählt ist aus $C_{3-8}$-Cycloalkyl, Aryl oder Heteroaryl, jeweils unsubstituiert oder einfach oder mehrfach substituiert; über eine gesättigte oder ungesättigte, unverzweigte, substituierte oder unsubstituierte $C_{1-2}$-Alkyl-Gruppe gebundenem Aryl, $C_{3-8}$-Cycloalkyl oder Heteroaryl, jeweils unsubstituiert oder einfach oder mehrfach substituiert;

vorzugsweise

$R^3$ ausgewählt ist aus $C_{5-6}$-Cycloalkyl, Phenyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; über eine gesättigte, unverzweigte $C_{1-2}$-Alkyl-Gruppe gebundenem $C_{5-6}$-Cycloalkyl, Phenyl,

Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Phenyl, Furyl, Thiophenyl, Cyclohexanyl, Naphthyl, Benzofuranyl, Indolyl, Indanyl, Benzodioxanyl, Benzodioxolanyl, Pyrrolyl, Pyrimidyl, Pyrazinyl oder Benzothiophenyl, jeweils unsubstituiert oder einfach oder mehrfach substituiert; über eine gesättigte, unverzweigte $C_{1-2}$-Alkyl-Gruppe gebundenem Phenyl, Furyl oder Thiophenyl, jeweils unsubstituiert oder einfach oder mehrfach substituiert.

In einer bevorzugten Ausführungsform der Verbindungsgruppe (D) sind die substituierten Cyclohexan-1,4-diaminderivate so aufgebaut, daß gemäß Formel I
$R^3$ ausgewählt ist aus $C_{3-8}$-Cycloalkyl oder Heteroaryl, jeweils unsubstituiert oder einfach oder mehrfach substituiert; über eine gesättigte oder ungesättigte, unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
vorzugsweise
$R^3$ ausgewählt ist aus $C_{5-6}$-Cycloalkyl, Thiophenyl, Benzothiophenyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; über eine gesättigte, unverzweigte $C_{1-2}$-AlkylGruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Furyl, Thiophenyl, Cyclohexanyl, Benzofuranyl, Indolyl, Indanyl, Benzodioxanyl, Benzodioxolanyl, Pyrrolyl, Pyrimidyl, Pyrazinyl oder Benzothiophenyl, jeweils unsubstituiert oder einfach oder mehrfach substituiert; über eine gesättigte, unverzweigte $C_{1-2}$-AlkylGruppe gebundenem Phenyl, Furyl oder Thiophenyl, jeweils unsubstituiert oder einfach oder mehrfach substituiert.

In einer bevorzugten Ausführungsform aller vorstehenden erfindungsgemäßen Verbindungen und Verbindungsgruppen sind die substituierten Cyclohexan-1,4-diaminderivate so aufgebaut, daß gemäß Formel I $R^4$ H ist.

In einer bevorzugten Ausführungsform aller vorstehenden erfindungsgemäßen Verbindungen und Verbindungsgruppen sind die substituierten Cyclohexan-1,4-diaminderivate so aufgebaut, daß gemäß Formel I
$R^4$ ausgewählt ist aus H, $C(X)R^7$, $C(X)NR^7R^8$, $C(X)OR^9$, $C(X)SR^9$ oder $S(O_2)R^9$ mit X = O oder S,
vorzugsweise
$R^4$ ausgewählt ist aus H, $C(X)R^7$, $C(X)NR^7R^8$ oder $C(X)OR^9$ mit X = O,
insbesondere
$R^4$ ausgewählt ist aus H oder $C(O)R^7$; vorzugsweise mit $R^7$ ausgewählt aus
H; oder $C_{1-8}$-Alkyl gesättigt oder ungesättigt, verzweigt oder unverzweigt, einfach oder mehrfach substituiert oder unsubstituiert;
vorzugsweise
H; oder $C_{1-3}$-Alkyl gesättigt, unsubstituiert, verzweigt oder unverzweigt;
insbesondere $CH_3$.

In einer bevorzugten Ausführungsform der Verbindungsgruppen (A), (D) oder (E) sind die substituierten Cyclohexan-1,4-diaminderivate so aufgebaut, daß gemäß Formel I
$R^4$ und $R^5$ zusammen einen Heterocyclus mit zwischen 3 und 8 Atomen im Ring bilden, gesättigt oder ungesättigt; einfach oder mehrfach substituiert oder unsubstituiert, vorzugsweise mit zwischen 5 und 7 Atomen im Ring, wovon neben dem obligatorischen N 0 bis 1 weitere Heteroatome, ausgewählt aus N, S oder O, im Ring sind;
wobei der von $R^4$ und $R^5$ zusammen gebildete Heterocyclus gegebenenfalls mit weiteren Ringen kondensiert sein kann, vorzugsweise mit aromatischen und/oder heteroaromatischen Ringen, wobei diese mit weiteren aromatischen und/oder heteroaromatischen Ringen kondensiert sein können,
insbesondere der von $R^4$ und $R^5$ zusammen gebildete Heterocyclus mit ein oder zwei weiteren Ringen kondensiert ist, vorzugsweise der von $R^4$ und $R^5$ zusammen gebildete Heterocyclus so mit zwei weiteren Ringen kondensiert ist, dass $R^4$ und $R^5$ zusammen

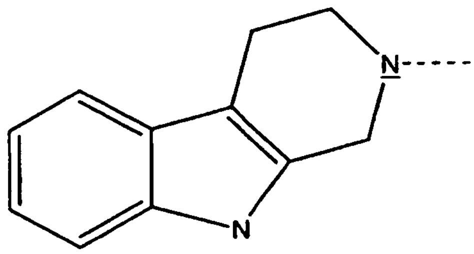

bedeuten.

In einer bevorzugten Ausführungsform der Verbindungsgruppen (A), (D) oder (E) sind die substituierten Cyclohexan-1,4-diaminderivate so aufgebaut, daß gemäß Formel I
$R^4$ ausgewählt ist aus H oder $C_{1-8}$-Alkyl, gesättigt oder ungesättigt, verzweigt oder unverzweigt, einfach oder mehrfach substituiert oder unsubstituiert,
vorzugsweise
H oder $C_{1-6}$-Alkyl, gesättigt oder ungesättigt, verzweigt oder unverzweigt, einfach oder mehrfach substituiert oder unsubstituiert,
insbesondere
H oder $C_{1-3}$-Alkyl, gesättigt, unverzweigt und unsubstituiert.

In einer bevorzugten Ausführungsform aller vorstehenden erfindungsgemäßen Verbindungen und Verbindungsgruppen sind die substituierten Cyclohexan-1,4-diaminderivate so aufgebaut, daß gemäß Formel I
$R^5$ ausgewählt ist aus $C_{3-8}$-Cycloalkyl, Aryl oder Heteroaryl, jeweils unsubstituiert oder einfach oder mehrfach substituiert;
vorzugsweise
$R^5$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^5$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

In einer weiteren besonders bevorzugten Ausführungsform aller vorstehenden erfindungsgemäßen Verbindungen und Verbindungsgruppen sind die substituierten Cyclohexan-1,4-diaminderivate so aufgebaut, daß gemäß Formel I
$R^5$ ausgewählt ist aus $-CHR^{11}R^{12}$, $-CHR^{11}-CH_2R^{12}$, $-CHR^{11}-CH^2-CH_2R^{12}$, $-CHR^{11}-CH_2-CH_2-CH_2R^{12}$, $-C(Y)R^{12}$, $-C(Y)CH_2R^{12}$, $-C(Y)-CH_2-CH_2R^{12}$ oder $-C(Y)-CH_2-CH_2-CH_2R^{12}$
mit Y = O, S oder $H_2$,
vorzugsweise
$R^5$ ausgewählt ist aus $-CHR^{11}R^{12}$, $-CHR^{11}-CH_2R^{12}$, $-CHR^{11}-CH_2-CH_2R^{12}$, $-C(Y)R^{12}$, $-C(Y)-CH_2R^{12}$ oder $-C(Y)-CH_2-CH_2R^{12}$
mit Y = O oder S,
insbesondere
$R^5$ ausgewählt ist aus $-CHR^{11}R^{12}$, $-CHR^{11}-CH_2R^{12}$, $-CHR^{11}-CH_2-CH_2R^{12}$, $-C(Y)R^{12}$ oder $-C(Y)-CH_2R^{12}$
mit Y = O.

Bezüglich dieser Ausführungsform ist es besonders bevorzugt, wenn
$R^{11}$ ausgewählt ist aus
H, $C_{1-4}$-Alkyl, gesättigt oder ungesättigt, verzweigt oder unverzweigt, einfach oder mehrfach substituiert oder unsubstituiert; oder $C(O)O-C_{1-4}$-Alkyl, gesättigt oder ungesättigt, verzweigt oder unverzweigt, einfach oder mehrfach substituiert oder unsubstituiert;
vorzugsweise
H, $C_{1-4}$-Alkyl, gesättigt oder ungesättigt, verzweigt oder unverzweigt, einfach oder mehrfach substituiert oder unsubstituiert; oder $C(O)O-C_{1-2}$-Alkyl, gesättigt, unverzweigt, einfach oder mehrfach substituiert oder unsubstituiert unsubstituiert;
insbesondere

H, $CH_3$, $C_2H_5$ und C(O)O-$CH_3$.
und/oder es ist ebenso besonders bevorzugt, wenn
$R^{12}$ ausgewählt ist aus $C_{3-8}$-Cycloalkyl, Aryl oder Heteroaryl, jeweils unsubstituiert oder einfach oder mehrfach substituiert;
vorzugsweise
$R^{12}$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^{12}$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

Weiter sind besonders bevorzugt die erfindungsgemäßen substituierten Cyclohexan-1,4-diaminderivate insbesondere ausgewählt aus der folgenden Gruppe:

- ■ N'-Benzyl-N,N-dimethyl-1-phenyl-cyclohexan-1,4-diamin Hydrochlorid, unpolareres Diastereomer
- ■ N'-Benzyl-N,N-dimethyl-1-phenyl-cyclohexan-1,4-diamin Hydrochlorid, polareres Diastereomer
- ■ 1,N'-Dibenzyl-N,N-dimethyl-cyclohexan-1,4-diamin Hydrochlorid, unpolareres Diastereomer
- ■ 1,N'-Dibenzyl-N,N-dimethyl-cyclohexan-1,4-diamin Hydrochlorid, polareres Diastereomer
- ■ N-(4-Benzyl-4-dimethylamino-cyclohexyl)-N-propyl-benzamid Hydrochlorid
- ■ N,N-Dimethyl-1-phenyl-N'-propyl-cyclohexan-1,4-diamin Hydrochlorid, unpolareres Diastereomer
- ■ N-(4-Dimethylamino-4-phenyl-cyclohexyl)-N-propyl-benzamid Hydrochlorid unpolareres Diastereomer
- ■ N-(4-Dimethylamino-4-phenyl-cyclohexyl)-N-propyl-benzamid Hydrochlorid, polareres Diastereomer
- ■ 1,N'-Dibenzyl-N,N,N'-trimethyl-cyclohexan-1,4-diamin Hydrochlorid, unpolareres Diastereomer
- ■ 1,N'-Dibenzyl-N,N,N'-trimethyl-cyclohexan-1,4-diamin Hydrochlorid, polareres Diastereomer
- ■ N-(4-Benzyl-4-dimethylamino-cyclohexyl)-N-methyl-benzamid Hydrochlorid, polareres Diastereomer
- ■ N-(4-Benzyl-4-dimethylamino-cyclohexyl)-N-ethyl-benzamid Hydrochlorid, polareres Diastereomer
- ■ 1-Benzyl-N'-(1H-indol-3-ylmethyl)-N,N-dimethyl-cyclohexan-1,4-diamin Dihydrochlorid
- ■ 1-Benzyl-N'-[2-(1 H-indol-3-yl)-1-methyl-ethyl]-N,N-dimethyl-cyclohexan-1,4-diamin, cis/trans-Gemisch
- ■ 1-Benzyl-N'-indan-5-yl-N,N-dimethyl-cyclohexan-1,4-diamin Hydrochlorid
- ■ 1-Benzyl-N'-indan-1-yl-N,N-dimethyl-cyclohexan-1,4-diamin Dihydrochlorid, cis/trans-Gemisch
- ■ N'-Indan-1-yl-N,N-dimethyl-1-phenyl-cyclohexan-1,4-diamin
- ■ N'-(1 H-Indol-5-yl)-N,N-dimethyl-1 -phenyl-cyclohexan-1,4-diamin
- ■ N'-(1 H-Indol-3-ylmethyl)-N,N-dimethyl-1-phenyl-cyclohexan-1,4-diamin, cis/trans-Gemisch
- ■ N'-(1H-Indol-3-ylmethyl)-N,N-dimethyl-1-phenyl-cyclohexan-1,4-diamin, unpolareres Diastereomer

■ N'-[2-(1H-Indol-3-yl)-ethyl]-N,N-dimethyl-1-pheny)-cyclohexan-1,4-diamin, unpolareres Diastereomer

■ N'-[2-(1H-Indol-3-yl)-ethyl]-N,N-dimethyl-1-phenyl-cyclohexan-1,4-diamin, cis/trans-Gemisch

■ N'-Indan-5-yl-N,N-dimethyl-1-phenyl-cyclohexan-1,4-diamin, unpolareres Diastereomer

■ N'-[2-(1H-Indol-3-yl)-1-methyl-ethyl]-N,N-dimethyl-1-phenyl-cyclohexan-1,4-diamin, unpolareres Diastereomer

■ N'-[2-(1H-Indol-3-yl)-1-methyl-ethyl]-N,N-dimethyl-1-phenyl-cyclohexan-1,4-diamin, cis/trans-Gemisch

■ N'-[2-(5-Benzyloxy-1 H-indol-3-yl)-ethyl]-N,N-dimethyl-1-phenyl-cyclohexan-1,4-diamin, cis/trans-Gemisch

■ N'-(9H-Fluoren-1-yl)-N,N-dimethyl-1-phenyl-cyclohexan-1,4-diamin Dihydrochlorid

■ N'-Indan-2-yl-N,N-dimethyl-1-phenyl-cyclohexan-1,4-diamin Dihydrochlorid, cis/trans-Gemisch

■ N'-(9H-Fluoren-9-yl)-N,N-dimethyl-1-phenyl-cyclohexan-1,4-diamin Dihydrochlorid, cis/trans-Gemisch

■ 1-Benzyl-N'-(9H-fluoren-9-yl)-N,N-dimethyl-cyclohexan-1,4-diamin

■ 1-Benzyl-N'-(1H-indol-3-ylmethyl)-N,N-dimethyl-cyclohexan-1,4-diamin, cis/trans-Gemisch

■ N,N-Dimethyl-N'-(1-methyl-1H-indol-3-ylmethyl)-1-phenyl-cyclohexan-1,4-diamin, cis/trans-Gemisch

■ N,N-Dimethyl-N'-(1-methyl-1H-indol-3-ylmethyl)-1-phenyl-cyclohexan-1,4-diamin, polareres Diastereomer

■ N'-(2-Benzo[b]thiophen-3-yl-ethyl)-N,N-dimethyl-1-phenyl-cyclohexan-1,4-diamin Dihydrochlorid, cis/trans-Gemisch

■ N'-(2-Benzo[b]thiophen-3-yl-ethyl)-1-benzyl-N,N-dimethyl-cyclohexan-1,4-diamin Dihydrochlorid, cis/trans-Gemisch

■ N'-Acenaphthen-1-yl-N,N-dimethyl-1-phenyl-cyclohexan-1,4-diamin Dihydrochlorid, polareres Diastereomer

■ N'-Acenaphthen-1-yl-N,N-dimethyl-1-phenyl-cyclohexan-1,4-diamin Dihydrochlorid, unpolareres Diastereomer

■ N'-Benzo[b]thiophen-5-yl-1-benzyl-N,N-dimethyl-cyclohexan-1,4-diamin Dihydrochlorid, unpolareres Diastereomer

■ N'-Benzo[b]thiophen-5-yl-N,N-dimethyl-1-phenyl-cyclohexan-1,4-diamin Hydrochlorid, unpolareres Diastereomer

■ N'-Benzothiazol-6-yl-N,N-dimethyl-1-phenyl-cyclohexan-1,4-diamin Dihydrochlorid, unpolareres Diastereomer

■ N'-Benzo[1,2,5]thiadiazol-4-yl-N,N-dimethyl-1-phenyl-cyclohexan-1,4-diamin Dihydrochlorid, polareres Diastereomer

■ N'-[2-(1 H-Indol-3-yl)-1-methyl-ethyl]-N,N-dimethyl-1-phenyl-cyclohexan-1 ,4-diamin Dihydrochlorid, unpolareres Diastereomer

■ N'-Adamantan-2-yl-N,N-dimethyl-1-phenyl-cyclohexan-1,4-diamin Dihydrochlorid

■ N'-(9-Ethyl-9H-carbazol-3-yl)-N,N-dimethyl-1-phenyl-cyclohexan-1,4-diamin Dihydrochlorid, unpolareres Diastereomer

■ N'-(3H-Benzotriazol-5-yl)-N,N-dimethyl-1-phenyl-cyclohexan-1,4-diamin Hydrochlorid, unpolareres Diastereomer

■ N'-(3H-Benzotriazol-5-yl)-N,N-dimethyl-1-phenyl-cyclohexan-1,4-diamin Hydrochlorid, polareres Diastereomer

■ N'-(9H-Fluoren-9-yl)-N.N-dimethyl-1-thiophen-2-yl-cyclohexan-1,4-diamin Dihydrochlorid, cis/trans-Gemisch

■ N'-Cyclooctyl-N,N-dimethyl-1-phenyl-cyclohexan-1,4-diamin Dihydrochlorid

■ N'-(1 H-Indol-3-ylmethyl)-N,N-dimethyl-1-phenyl-cyclohexan-1,4-diamin Dihydrochlorid, unpolareres Diastereomer

■ N'-(1 H-Indol-3-ylmethyl)-N,N-dimethyl-1-phenyl-cyclohexan-1,4-diamin Dihydrochlorid, polareres Diastereomer

■ N'-Benzo[b]thiophen-3-ylmethyl-N,N-dimethyl-1-phenyl-cyclohexan-1,4-diamin Dihydrochlorid, unpolareres Diastereomer

■ N'-Benzo[b]thiophen-3-ylmethyl-N,N-dimethyl-1-phenyl-cyclohexan-1,4-diamin Dihydrochlorid, polareres Diastereomer

■ N'-Anthracen-2-yl-N,N-dimethyl-1-phenyl-cyclohexan-1,4-diamin Hydrochlorid, unpolareres Diastereomer

■ N'-Benzo[b]thiophen-3-ylmethyl-1-benzyl-N,N-dimethyl-cyclohexan-1,4-diamin Dihydrochlorid, unpolareres Diastereomer

■ N'-Benzo[b]thiophen-3-ylmethyl-1-benzyl-N,N-dimethyl-cyclohexan-1,4-diamin Dihydrochlorid, polareres Diastereomer

■ N'-[2-(1H-Indol-3-yl)-ethyl]-N,N-dimethyl-1-naphthalin-2-yl-cyclohexan-1,4-diamin Dihydrochlorid, unpolareres Diastereomer

■ N'-[2-(1H-Indol-3-yl)-ethyl]-N,N-dimethyl-1-phenyl-cyclohexan-1,4-diamin Dihydrochlorid, unpolareres Diastereomer

■ N'-[2-(1H-Indol-3-yl)-ethyl]-N,N-dimethyl-1-phenyl-cyclohexan-1,4-diamin Dihydrochlorid, polareres Diastereomer

■ N'-[2-(1H-Indol-3-yl)1-methyl-ethyl]-N,N-dimethyl-1-phenyl-cyclohexan-1,4-diamin Dihydrochlorid, polareres Diastereomer

■ Methyl 2-(4-dimethylamino-4-phenyl-cyclohexylamino)-3-(1H-indol-3-yl)-propionat Dihydrochlorid, unpolareres Diastereomer

■ Methyl 2-(4-dimethylamino-4-phenyl-cyclohexylamino)-3-(1 H-indol-3-yl)-propionat Dihydrochlorid, polareres Diastereomer

■ N'-[2-(1H-Indol-3-yl)-1-methyl-ethyl]-N,N-dimethyl-1-naphthaiin-2-yl-cyclohexan-1,4-diamin Dihydrochlorid, unpolareres Diastereomer

■ N'-Benzo[1,3]dioxol-5-ylmethyl-N,N-dimethyt-1-phenyl-cyclohexan-1,4-diamin Dihydrochlorid, cis/trans-Gemisch

■ N'-[2-(6-Fluor-1H-indol-3-yl)-ethyl]-N,N-dimethyl-1-phenyl-cyclohexan-1,4-diamin Dihydrochlorid, unpolareres Diastereomer

■ N'-[2-(6-Fluor-1 H-indol-3-yl)-ethyl]-N,N-dimethyl-1-phenyl-cyclohexan-1,4-diamin Dihydrochlorid, polareres Diastereomer

■ N'-[2-(1H-Indol-3-yl)-ethyl]-N,N,N'-trimethyl-1-phenyl-cyclohexan-1,4-diamin Dihydrochlorid, unpolareres Diastereomer

■ N'-[2-(1 H-Indol-3-yl)-ethyl]-N,N,N-trimethyl-l-phenyl-cyclohexan-1,4-diamin Dihydrochlorid, polareres Diastereomer

■ N,N-Dimethyl-N'-[2-(7-methyl-1 H-indol-3-yl)-ethyl]-1-phenyl-cyclohexan-1,4-diamin Dihydrochlorid, unpolareres Diastereomer

■ N,N-Dimethyl-N'-[2-(7-methyl-1 H-indol-3-yl)-ethyl]-1-phenyl-cyclohexan-1,4-diamin Dihydrochlorid, polareres Diastereomer

■ N'-[2-(5-Fluor-1 H-indol-3-yl)-ethyl]-N,N-dimethyl-1-phenyl-cyclohexan-1,4-diamin Dihydrochlorid, unpolareres Diastereomer

■ N'-[2-(5-Fluor-1H-indol-3-yl)-ethyl]-N,N-dimethyl-1-phenyl-cyclohexan-1,4-diamin Dihydrochlorid, polareres Diastereomer

■ N'-Acenaphthen-5-ylmethyl-N,N-dimethyl-1-phenyl-cyclohexan-1,4-diamin Dihydrochlorid, unpolareres Diastereomer

■ N'-[2-(1 H-Indol-3-yl)-1-methyl-ethyl]-N,N-dimethyl-1-thiophen-2-yl-cyclohexan-1,4-diamin Dihydrochlorid, unpolareres Diastereomer

■ N'-[2-(1 H-Indo)-3-yl)-1-methyl-ethyl]-N,N-dimethyl-1-thiophen-2-yl-cyclohexan-1,4-diamin Dihydrochlorid, cis/trans-Gemisch

■ N'-[2-(7-Benzyloxy-1 H-indol-3-yl)-ethyl]-N,N-dimethyl-1-phenyl-cyclohexan-1,4-diamin Dihydrochlorid, unpolareres Diastereomer

■ N'-Cyclooctyl-N,N-dimethyl-1-thiophen-2-yl-cyclohexan-1,4-diamin Dihydrochlorid, unpolareres Diastereomer

■ N'-Adamantan-2-yl-N,N-dimethyl-1-thiophen-2-yl-cyclohexan-1,4-diamin Dihydrochlorid, unpolareres Diastereomer

■ 3-[2-(4-Dimethylamino-4-phenyl-cyclohexylamino)-ethyl]-1 H-indol-5-ol Dihydrochlorid, unpolareres Diastereomer

■ 3-[2-(4-Dimethylamino-4-phenyl-cyclohexylamino)-ethyl]-1H-indol-5-ol Dihydrochlorid, polareres Diastereomer

■ N'-[2-(5-Methoxy-1H-indol-3-yl)-ethyl]-N,N-dimethyl-1-phenyl-cyclohexan-1,4-diamin Dihydrochlorid, unpolareres Diastereomer

■ N'-[2-(5-Methoxy-1H-indol-3-yl)-ethyl]-N,N-dimethyl-1-phenyl-cyclohexan-1,4-diamin Dihydrochlorid, polareres Diastereomer

■ N,N-Dimethyl-N'-[2-(5-methyl-1H-indol-3-yl)-ethyl]-1-phenyl-cyclohexan-1,4-diamin Dihydrochlorid, unpolareres Diastereomer

■ N,N-Dimethyl-N'-[2-(5-methyl-1 H-indol-3-yl)-ethyl]-1-phenyl-cyclohexan-1,4-diamin Dihydrochlorid, polareres Diastereomer

■ Dimethyl-[1-phenyl-4-(1,3,4,9-tetrahydro-b-carbolin-2-yl)-cyclohexyl]-amin Dihydrochlorid

■ N-(4-Dimethylamino-4-phenyl-cyclohexyl)-N-[2-(4-fluor-phenyl)-ethyl]-acetamid Hydrochlorid, unpolareres Diastereomer

■ 2-(4-Dimethylamino-4-phenyl-cyclohexylamino)-3-(5-fluor-1H-indol-3-yl)-propionsäure-methylester Dihydrochlorid, unpolareres Diastereomer

■ N-(4-Dimethylamino-4-phenyl-cyclohexyl)-N-(3-phenyl-propyl)-acetamid Hydrochlorid, unpolareres Diastereomer

■ 2-(4-Dimethylamino-4-phenyl-cyclohexylamino)-3-(6-fluor-1 H-indol-3-yl)-propionsäure-methylester Dihydrochlorid, unpolareres Diastereomer

- N-(4-Dimethylamino-4-phenyl-cyclohexyl)-2-(1 H-indol-3-yl)-acetamid Hydrochlorid, polareres Diastereomer

- 2-(4-Dimethylamino-4-thiophen-2-yl-cyclohexylamino)-3-(1 H-indol-3-yl)-propionsäure-methylester Dihydrochlorid, unpolareres Diastereomer

- N-(4-Dimethylamino-4-phenyl-cyclohexyl)-2-(5-methoxy-1 H-indol-3-yl)-acetamid Hydrochlorid, unpolareres Diastereomer

gegebenenfalls auch in Form ihrer Racemate, der genannten oder anderen reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
gegebenenfalls auch in Form der Säuren oder Basen oder in Form anderer Salze, insbesondere physiologisch verträglicher Salze oder Salze physiologisch verträglicher Säuren oder Kationen; oder in Form ihrer Solvate, insbesondere der Hydrate.

Die erfindungsgemäßen Substanzen sind toxikologisch unbedenklich, so daß sie sich als pharmazeutischer Wirkstoff in Arzneimittel eignen.

Ein weiterer Gegenstand der Erfindung sind daher Arzneimittel enthaltend wenigstens ein substituiertes Cyclohexan-1,4-diaminderivat aus der im folgenden als (**F**) bezeichneten Verbindungsgruppe gemäß der allgemeinen Formel 1,

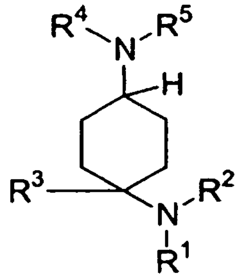

I

, worin
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen, oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$, $C(X)OR^9$, $C(X)SR^9$, $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte

$C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$, - CHR11- $CH_2R^{12}$, $-CHR^{11}-CH_2-CH_2R^{12}$, $-CHR^{11}-CH_2-CH_2CH_2R^{12}$, - $C(Y)R^{12}$, $-C(Y)-CH_2R^{12}$, -C (Y)-$CH_2$-$CH_2R^{12}$ oder $-C(Y)-CH_2-CH_2-CH_2R^{12}$
mit Y = O, S oder $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder C(O)O-$C_{1-6}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann, mit der Maßgabe,

■ daß, wenn $R^3$ substituiertes oder unsubstituiertes Phenyl ist und mindestens einer von $R^1$ oder $R^2$ H oder $C_{1-8}$-Alkyl ist, $R^4$ nicht Alkyl sein darf und $R^4$ und $R^5$ nicht zusammen einen Heterocyclus bilden dürfen,

gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weitere Wirkstoffe.

Ebenfalls ein weiterer Gegenstand der Erfindung sind Arzneimittel enthaltend wenigstens ein substituiertes Cyclohexan-1,4-diaminderivat aus der im folgenden als (**G**) bezeichneten Verbindungsgruppe gemäß der allgemeinen Formel I,

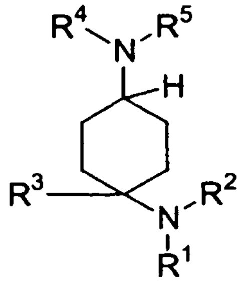

I

, worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen, oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$, $C(X)OR^9$, $C(X)SR^9$, $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2CH_2R^{12}$ oder $-C(Y)-CH_2CH_2CH_2R^{12}$
mit Y = O, S oder $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann, gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weitere Wirkstoffe.

Ebenfalls ein weiterer Gegenstand der Erfindung sind Arzneimittel enthaltend wenigstens ein substituiertes Cyclohexan-1,4-diaminderivat aus der im folgenden als (**H**) bezeichneten Verbindungsgruppe gemäß der allgemeinen Formel 1,

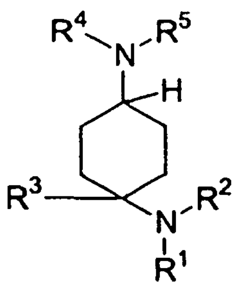

I

, worin

$R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten, mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$, $C(X)OR^9$, $C(X)SR^9$, $S(O_2)R^9$

mit X = O oder S,

mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

mit $R^8$ ausgewählt aus H, $C_{1-4}$-Alkyl, gesättigt oder ungesättigt, verzweigt oder unverzweigt, einfach oder mehrfach substituiert oder unsubstituiert oder

die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,

mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ oder $-C(Y)-CH_2-CH_2CH_2R^{12}$

mit Y = O, S oder $H_2$,

mit $R^{11}$ ausgewählt aus

H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert; und mit $R^{12}$ ausgewählt aus

H; $C_{3-8}$-Cycloalkyl, Aryl oder Heteroaryl, jeweils unsubstituiert oder einfach oder mehrfach substituiert,

oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann,

gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weitere Wirkstoffe.

Ebenfalls ein weiterer Gegenstand der Erfindung sind Arzneimittel enthaltend wenigstens ein substituiertes Cyclohexan-1,4-diaminderivat aus der im folgenden als (**J**) bezeichneten Verbindungsgruppe gemäß der allgemeinen Formel I,

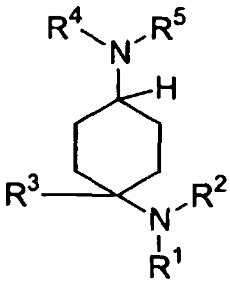

I

, worin
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C(X)R^7$, $C(X)NR^7R^8$, $C(X)OR^9$, $C(X)S_R{}^9$ oder $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach

substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$,-CHR"- $CH_2R^{12}$, $-CHR^{11}-CH_2-CH_2R^{12}$, $-CHR^{11}-CH_2-CH_2-CH_2R^{12}$, - $C(Y)R^{12}$, $-C(Y)-CH_2R^{12}$, -C (Y)-$CH_2$-$CH_2R^{12}$ oder $-C(Y)-CH_2-CH_2-CH_2R^{12}$

mit Y = O, S oder $H_2$,

mit $R^{11}$ ausgewählt aus

H, $C_{1-7}$-Alkyl, gesättigt oder ungesättigt, verzweigt oder unverzweigt, einfach oder mehrfach substituiert oder unsubstituiert; oder C(O)O-$C_{1-6}$-Alkyl, gesättigt oder ungesättigt, verzweigt oder unverzweigt, einfach oder mehrfach substituiert oder unsubstituiert;

und mit $R^{12}$ ausgewählt aus

H; $C_{3-8}$-Cycloalkyl, Aryl oder Heteroaryl, jeweils unsubstituiert oder einfach oder mehrfach substituiert,

gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;

in dargestellter Form oder in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weitere Wirkstoffe.

Bevorzugte erfindungsgemäße Arzneimittel enthalten mindestens ein Cyclohexan-1,4-diaminderivat aus einer der Verbindungsgruppen (**F**) oder (**G**) gemäß Formel I, worin

$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,

oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

mit $R^6$ ausgewählt aus H; $C_{1-8}$-Alkyl, gesättigt oder ungesättigt, verzweigt oder unverzweigt, einfach oder mehrfach substituiert oder unsubstituiert,

vorzugsweise

$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,

oder die Reste $R^1$ und $R^2$ zusammen einen Ring bilden und $(CH_2)_{4-5}$ bedeuten,

insbesondere

$R^1$ und $R^2$ unabhängig voneinander ausgewählt sind aus Methyl oder Ethyl oder die Reste $R^1$ und $R^2$ zusammen einen Ring bilden und $(CH_2)_5$ bedeuten.

Bevorzugte erfindungsgemäße Arzneimittel enthalten mindestens ein Cyclohexan-1,4-diaminderivat aus der Verbindungsgruppe (H) gemäß Formel 1, worin

$R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

mit $R^6$ ausgewählt aus H; $C_{1-8}$-Alkyl, gesättigt oder ungesättigt, verzweigt oder unverzweigt, einfach oder mehrfach substituiert oder unsubstituiert,

vorzugsweise

$R^1$ und $R^2$ zusammen einen Ring bilden und $(CH_2)_{4-5}$ bedeuten,

insbesondere

$R^1$ und $R^2$ zusammen einen Ring bilden und $(CH_2)_5$ bedeuten.

Bevorzugte erfindungsgemäße Arzneimittel enthalten mindestens ein Cyclohexan-1,4-diaminderivat aus der Verbindungsgruppe (J) gemäß Formel 1, worin

$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,

vorzugsweise

$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,

insbesondere

$R^1$ und $R^2$ unabhängig voneinander ausgewählt sind aus Methyl oder Ethyl;

oder

$R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

mit $R^6$ ausgewählt aus H; $C_{1-8}$-Alkyl, gesättigt oder ungesättigt, verzweigt oder unverzweigt, einfach oder mehrfach substituiert oder unsubstituiert,

vorzugsweise

$R^1$ und $R^2$ zusammen einen Ring bilden und $(CH_2)_{4-5}$ bedeuten,
insbesondere
$R^1$ und $R^2$ zusammen einen Ring bilden und $(CH_2)_5$ bedeuten.

Bevorzugte erfindungsgemäße Arzneimittel enthalten mindestens ein Cyclohexan-1,4-diaminderivat aus einer der Verbindungsgruppen (**F**), (**H**) oder (**J**) gemäß Formel I, worin
$R^3$ ausgewählt ist aus $C_{3-8}$-Cycloalkyl, Aryl oder Heteroaryl, jeweils unsubstituiert oder einfach oder mehrfach substituiert; über eine gesättigte oder ungesättigte, unverzweigte, substituierte oder unsubstituierte $C_{1-2}$-Alkyl-Gruppe gebundenem Aryl, $C_{3-8}$-Cycloalkyl oder Heteroaryl, jeweils unsubstituiert oder einfach oder mehrfach substituiert;
vorzugsweise
$R^3$ ausgewählt ist aus $C_{5-6}$-Cycloalkyl, Phenyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; über eine gesättigte, unverzweigte $C_{1-2}$-Alkyl-Gruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Phenyl, Furyl, Thiophenyl, Cyclohexanyl, Naphthyl, Benzofuranyl, Indolyl, Indanyl, Benzodioxanyl, Benzodioxolanyl, Pyrrolyl, Pyrimidyl, Pyrazinyl oder Benzothiophenyl, jeweils unsubstituiert oder einfach oder mehrfach substituiert; über eine gesättigte, unverzweigte $C_{1-2}$-Alkyl-Gruppe gebundenem Phenyl, Furyl oder Thiophenyl, jeweils unsubstituiert oder einfach oder mehrfach substituiert.

Bevorzugte erfindungsgemäße Arzneimittel enthalten mindestens ein Cyclohexan-1,4-diaminderivat aus der Verbindungsgruppe (**G**) gemäß Formel I, worin
$R^3$ ausgewählt ist aus $C_{3-8}$-Cycloalkyl oder Heteroaryl, jeweils unsubstituiert oder einfach oder mehrfach substituiert; über eine gesättigte oder ungesättigte, unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
vorzugsweise
$R^3$ ausgewählt ist aus $C_{5-6}$-Cycloalkyl, Thiophenyl, Benzothiophenyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; über eine gesättigte, unverzweigte $C_{1-2}$-AlkylGruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Furyl, Thiophenyl, Cyclohexanyl, Benzofuranyl, Indolyl, Indanyl, Benzodioxanyl, Benzodioxolanyl, Pyrrolyl, Pyrimidyl, Pyrazinyl oder Benzothiophenyl, jeweils unsubstituiert oder einfach oder mehrfach substituiert; über eine gesättigte, unverzweigte $C_{1-2}$-AlkylGruppe gebundenem Phenyl, Furyl oder Thiophenyl, jeweils unsubstituiert oder einfach oder mehrfach substituiert.

Bevorzugte erfindungsgemäße Arzneimittel enthalten mindestens ein Cyclohexan-1,4-diaminderivat aus den Verbindungsgruppen **(F), (G), (H)** oder **(J)** gemäß Formel I, worin $R^4$ H ist.

Bevorzugte erfindungsgemäße Arzneimittel enthalten mindestens ein Cyclohexan-1,4-diaminderivat aus den Verbindungsgruppen **(F), (G), (H)** oder **(J)** gemäß Formel I, worin
$R^4$ ausgewählt ist aus H, $C(X)R^7$, $C(X)NR^7R^8$, $C(X)OR^9$, $C(X)SR^9$ oder $S(O_2)R^9$ mit X = O oder S,
vorzugsweise
$R^4$ ausgewählt ist aus H, $C(X)R^7$, $C(X)NR^7R^8$ oder $C(X)OR^9$ mit X = O,
insbesondere
$R^4$ ausgewählt ist aus H oder $C(O)R^7$; vorzugsweise mit $R^7$ ausgewählt aus
H; oder $C_{1-8}$-Alkyl gesättigt oder ungesättigt, verzweigt oder unverzweigt, einfach oder mehrfach substituiert oder unsubstituiert;
vorzugsweise
H; oder $C_{1-3}$-Alkyl gesättigt, unsubstituiert, verzweigt oder unverzweigt;
insbesondere $CH_3$.

Weitere bevorzugte erfindungsgemäße Arzneimittel enthalten mindestens ein Cyclohexan-1,4-diaminderivat aus den Verbindungsgruppen **(F), (G)** oder **(H)** gemäß Formel 1, worin
$R^4$ und $R^5$ zusammen einen Heterocyclus mit zwischen 3 und 8 Atomen im Ring bilden, gesättigt oder ungesättigt; einfach oder mehrfach substituiert oder unsubstituiert, vorzugsweise mit zwischen 5 und 7 Atomen im Ring, wovon neben dem obligatorischen N 0 bis 1 weitere Heteroatome, ausgewählt aus N, S oder O, im Ring sind;
wobei der von $R^4$ und $R^5$ zusammen gebildete Heterocyclus gegebenenfalls mit weiteren Ringen kondensiert sein kann, vorzugsweise mit aromatischen und/oder heteroaromatischen Ringen, wobei diese mit weiteren aromatischen und/oder heteroaromatischen Ringen kondensiert sein können,
insbesondere der von $R^4$ und $R^5$ zusammen gebildete Heterocyclus mit ein oder zwei weiteren Ringen kondensiert ist,

vorzugsweise der von $R^4$ und $R^5$ zusammen gebildete Heterocyclus so mit zwei weiteren Ringen kondensiert ist, dass $R^4$ und $R^5$ zusammen

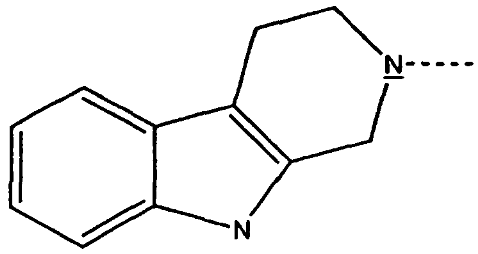

bedeuten.

Bevorzugte erfindungsgemäße Arzneimittel enthalten mindestens ein Cyclohexan-1,4-diaminderivat aus den Verbindungsgruppen **(F), (G)** oder **(H)** gemäß Formel I, worin
$R^4$ ausgewählt ist aus H, $C_{1-8}$-Alkyl, gesättigt oder ungesättigt, verzweigt oder unverzweigt, einfach oder mehrfach substituiert oder unsubstituiert,
vorzugsweise
H, $C_{1-6}$-Alkyl, gesättigt oder ungesättigt, verzweigt oder unverzweigt, einfach oder mehrfach substituiert oder unsubstituiert,
insbesondere
H, $C_{1-3}$-Alkyl, gesättigt, unverzweigt und unsubstituiert.

Bevorzugte erfindungsgemäße Arzneimittel enthalten mindestens ein Cyclohexan-1,4-diaminderivat aus den Verbindungsgruppen **(F), (G), (H)** oder **(J)** gemäß Formel I, worin
$R^5$ ausgewählt ist aus $C_{3-8}$-Cycloalkyl, Aryl oder Heteroaryl, jeweils unsubstituiert oder einfach oder mehrfach substituiert;
vorzugsweise
$R^5$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert; insbesondere
$R^5$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

Ebenfalls bevorzugte erfindungsgemäße Arzneimittel enthalten mindestens ein Cyclohexan-1,4-diaminderivat aus den Verbindungsgruppen **(F), (G), (H)** oder **(J)** gemäß Formel 1, worin
$R^5$ ausgewählt ist aus $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ oder $-C(Y)-CH_2CH_2CH_2R^{12}$
mit Y = O, S oder $H_2$,
vorzugsweise
$R^5$ ausgewählt ist aus -CHR11$R^{12}$, $-CHR^{11}-CH_2R^{12}$, $-CHR^{11}-CH_2CH_2R^{12}$, $-C(Y)R^{12}$, $-C(Y)-CH_2R^{12}$ oder $-C(Y)-CH_2-CH_2R^{12}$
mit Y = O oder S,
insbesondere
$R^5$ ausgewählt ist aus $-CHR^{11}R^{12}$, $-CHR^{11}-CH_2R^{12}$, $-CHR^{11}-CH_2-CH_2R^{12}$, $-C(Y)R^{12}$ oder $-C(Y)-CH_2R^{12}$
mit Y = O.

Dabei ist es in Bezug auf letztere Gruppe bevorzugter Arzneimittel besonders bevorzugt, wenn
$R^{11}$ ausgewählt ist aus
H, $C_{1-4}$-Alkyl, gesättigt oder ungesättigt, verzweigt oder unverzweigt, einfach oder mehrfach substituiert oder unsubstituiert; oder $C(O)O-C_{1-4}$-Alkyl, gesättigt oder ungesättigt, verzweigt oder unverzweigt, einfach oder mehrfach substituiert oder unsubstituiert;

vorzugsweise
H, $C_{1-4}$-Alkyl, gesättigt oder ungesättigt, verzweigt oder unverzweigt, einfach oder mehrfach substituiert oder unsubstituiert; oder C(O)O-$C_{1-2}$-Alkyl, gesättigt, unverzweigt, einfach oder mehrfach substituiert oder unsubstituiert unsubstituiert;
insbesondere
H, $CH_3$, $C_2H_5$ und C(O)O-$CH_3$,
und/oder wenn
$R^{12}$ ausgewählt ist aus $C_{3-8}$-Cycloalkyl, Aryl oder Heteroaryl, jeweils unsubstituiert oder einfach oder mehrfach substituiert;
vorzugsweise
$R^{12}$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^{12}$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

Die erfindungsgemäßen Arzneimittel enthalten neben mindestens einem erfindungsgemäßen substituierten Cyclohexan-1,4-diaminderivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte Cyclohexan-1,4-diaminderivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten Cyclohexan-1,4-diaminderivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens eines erfindungsgemäßen substituierten Cyclohexan-1,4-diaminderivats appliziert.

Für alle vorstehenden Formen der erfindungsgemäßen Arzneimittel ist es besonders bevorzugt, wenn das Arzneimittel neben wenigstens einem substituierten Cyclohexan-1,4-diaminderivat noch ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittel liegt ein enthaltenes erfindungsgemäßes substituiertes Cyclohexan-1,4-diaminderivat als reines Diastereomer und/oder Enantiomer, als Razemat oder als nicht-äquimolare oder äquimolare Mischung der Diastereomere und/oder Enantiomere vor.

Wie in der Einleitung am Stand der Technik abzulesen, wurde der ORL1-Rezeptor insbesondere im Schmerzgeschehen identifiziert. Entsprechend können erfindungsgemäße substituierte Cyclohexan-1,4-diaminderivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung im folgenden als Verbindungsgruppe **(K)** bezeichneter substituierter Cyclohexan-1,4-diaminderivate gemäß der allgemeinen Formel I,

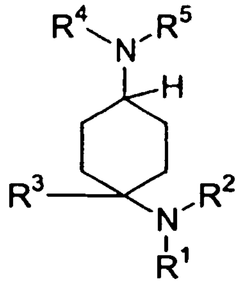

I

, worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen, oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$, $C(X)OR^9$, $C(X)SR^9$, $S(O_2)R^9$

mit X = O oder S,

mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

mit $R^8$ ausgewählt aus H, $C_{1-4}$-Alkyl, gesättigt oder ungesättigt, verzweigt oder unverzweigt, einfach oder mehrfach substituiert oder unsubstituiert oder

die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,

mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$, $- CHR^{11}- CH_2R^{12}$, $-CHR^{11}-CH_2-CH_2R^{12}$, $-CHR^{11}-CH_2-CH_2-CH_2R^{12}$,$-C(Y)R^{12}$, $-C(Y)-CH_2R^{12}$, $-C(Y)-CH_2-CH_2R^{12}$ oder $-C(Y)-CH_2-CH_2-CH_2R^{12}$

mit Y = O, S oder $H_2$,

mit $R^{11}$ ausgewählt aus

H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert

oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann, mit der Maßgabe,

- daß, wenn $R^3$ substituiertes oder unsubstituiertes Phenyl ist und mindestens einer von $R^1$ oder $R^2$ H oder $C_{1-8}$-Alkyl ist, $R^4$ nicht Alkyl sein darf und $R^4$ und $R^5$ nicht zusammen einen Heterocyclus bilden dürfen

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate; zur Herstellung eines Arzneimittels zur Behandlung von Schmerz, insbesondere von akutem, neuropathischem oder chronischem Schmerz.

Ein weiterer Gegenstand der Erfindung ist daher die Verwendung im folgenden als Verbindungsgruppe (L) bezeichneter substituierter Cyclohexan-1,4-diaminderivate gemäß der allgemeinen Formel I,

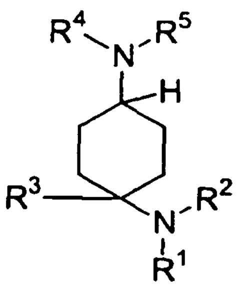

I

, worin
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen, oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR\ ^7R^8$, $C(X)OR^9$, $C(X)SR^9$, $S(O_2)_R{}^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

mit $R^8$ ausgewählt aus H, $C_{1-4}$-Alkyl, gesättigt oder ungesättigt, verzweigt oder unverzweigt, einfach oder mehrfach substituiert oder unsubstituiert oder

die Reste $R^7$ und $R^8$ zusammen einen Ring bilden und $CH_2CH_2OCH_2CH_2$, $CH_2CH_2NR^{10}$ $CH_2CH_2$ oder $(CH_2)_{3-6}$ bedeuten,

mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$, - $CHR^{11}$- $CH_2R^{12}$, $-CHR^{11}-CH_2-CH_2R^{12}$, $-CHR^{11}-CH_2-CH_2-CH_2R^{12}$ , $C(Y)R^{12}$, $-C(Y)-CH_2R^{12}$, $-C(Y)-CH_2-CH_2R^{12}$ oder $-C(Y)-CH_2-CH_2-CH_2R^{12}$

mit Y = O, S oder $H_2$,

mit $R^{11}$ ausgewählt aus

H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;

und mit $R^{12}$ ausgewählt aus

H; $C_{3-8}$-Cycloalkyl, Aryl oder Heteroaryl, jeweils unsubstituiert oder einfach oder mehrfach substituiert,

oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;

in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate; zur Herstellung eines Arzneimittels zur Behandlung von Schmerz, insbesondere von akutem, neuropathischem oder chronischem Schmerz.

Ein weiterer Gegenstand der Erfindung ist daher die Verwendung im folgenden als Verbindungsgruppe (**M**) bezeichneter substituierter Cyclohexan-1,4-diaminderivate gemäß der allgemeinen Formel I,

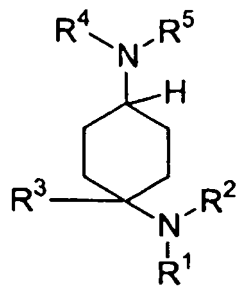

I

, worin

$R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^3$ ausgewählt ist aus $C_{1-8}$-Alkyl oder $C_{3-8}$-Cycloalkyl, jeweils gesättigt oder ungesättigt, verzweigt oder unverzweigt,

einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$, $C(X)OR^9$, $C(X)SR^9$, $S(O_2)R^9$

mit X = O oder S,

mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

mit $R^8$ ausgewählt aus H, $C_{1-4}$-Alkyl, gesättigt oder ungesättigt, verzweigt oder unverzweigt, einfach oder mehrfach substituiert oder unsubstituiert oder

die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,

mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$,$-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ oder $-C(Y)-CH_2-CH_2-CH_2R^{12}$

mit Y = O, S oder $H_2$,

mit $R^{11}$ ausgewählt aus

H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;

und mit $R^{12}$ ausgewählt aus

H; $C_{3-8}$-Cycloalkyl, Aryl oder Heteroaryl, jeweils unsubstituiert oder einfach oder mehrfach substituiert,

oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;

in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate; zur Herstellung eines Arzneimittels zur Behandlung von Schmerz, insbesondere von akutem, neuropathischem oder chronischem Schmerz.

Ein weiterer Gegenstand der Erfindung ist daher die Verwendung im folgenden als Verbindungsgruppe (**N**) bezeichneter substituierter Cyclohexan-1,4-diaminderivate gemäß der allgemeinen Formel 1,

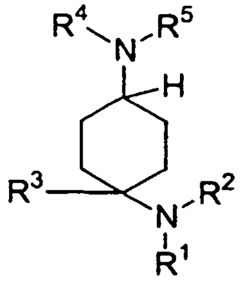

I

, worin
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C(X)R^7$, $C(X)NR^7R^8$, $C(X)OR^9$, $C(X)SR^9$ oder $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$, $-CHR^{11}-CH_2R^{12}$, $-CHR^{11}-CH_2-CH_2R^{12}$, $-CHR^{11}-CH_2CH_2-CH_2R^{12}$, $-C(Y)R^{12}$, $-C(Y)-CH_2R^{12}$, $-C(Y)-CH_2-CH_2R^{12}$ oder $-C(Y)-CH_2-CH_2-CH_2R^{12}$
mit Y = O, S oder $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert; und mit $R^{12}$ ausgewählt aus

H; $C_{3-8}$-Cycloalkyl, Aryl oder Heteroaryl, jeweils unsubstituiert oder einfach oder mehrfach substituiert, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate; zur Herstellung eines Arzneimittels zur Behandlung von Schmerz, insbesondere von akutem, neuropathischem oder chronischem Schmerz.

Wie bereits in der Einleitung ausgeführt, spielt der ORL1-Rezeptor neben der Funktion im Schmerzgeschehen noch in einer Vielzahl anderer physiologischer Prozeße insbesondere von medizinisch relevanter Bedeutung eine Rolle, so daß ein weiterer Gegenstand der Erfindung die Verwendung im folgenden als Verbindungsgruppe (**O**) bezeichneter substituierter Cyclohexan-1,4-diaminderivate gemäß der allgemeinen Formel 1,

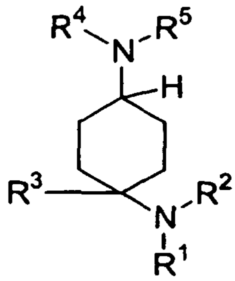

I

, worin
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen, oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$, $C(X)OR^9$, $C(X)SR^9$, $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert; mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}$ $CH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder

mehrfach substituiert oder unsubstituiert;
mit $R^9$ ausgewählt aus $C_{1-8}$-Alkyl oder $C_{3-8}$Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$, - $CHR^{11}$- $CH_2R^{12}$, $-CHR^{11}-CH_2-CH_2R^{12}$, $-CHR^{11}-CH_2-CH_2CH_2R^{12}$, - $C(Y)R^{12}$, $-C(Y)-CH_2R^{12}$, -C (Y)-$CH_2$-$CH_2R$ $^{12}$ oder $-C(Y)-CH_2-CH_2-CH_2R$ $^{12}$
mit Y = O, S oder $H_2$,
mit $R^{11}$ ausgewählt aus
H, $C_{1-7}$Alkyl, gesättigt oder ungesättigt, verzweigt oder unverzweigt, einfach oder mehrfach substituiert oder unsubstituiert; oder C(O)O-$C_{1-6}$-Alkyl, gesättigt oder ungesättigt, verzweigt oder unverzweigt, einfach oder mehrfach substituiert oder unsubstituiert;
und mit R $^{12}$ ausgewählt aus
H; $C_{3-8}$-Cycloalkyl, Aryl oder Heteroaryl, jeweils unsubstituiert oder einfach oder mehrfach substituiert,
oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate; zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Schwierigkeiten (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese und/oder Anxiolyse ist.

Dabei kann es in einer der vorstehenden Verwendungen bevorzugt sein, wenn ein verwendetes substituiertes Cyclohexan-1,4-diaminderivat als reines Diastereomer und/oder Enantiomer, als Razemat oder als nicht-äquimolare oder äquimolare Mischung der Diastereomere und/oder Enantiomere vorliegt.

Bei einer der vorstehenden Verwendungen eines substituierten Cyclohexan-1,4-diaminderivats aus einer der Verbindungsgruppen **(K), (L)** oder **(O)** kann es bevorzugt sein, wenn in Formel I
$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
oder die Reste $R^1$ und $R^2$ zusammen einen Ring bilden und $(CH_2)_{4-5}$ bedeuten,
insbesondere
$R^1$ und $R^2$ unabhängig voneinander ausgewählt sind aus Methyl oder Ethyl oder die Reste $R^1$ und $R^2$ zusammen einen Ring bilden und $(CH_2)_5$ bedeuten.

Bei einer der vorstehenden Verwendungen eines substituierten Cyclohexan-1,4-diaminderivats aus der Verbindungsgruppe **(M)** kann es bevorzugt sein, wenn in Formel I
$R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ zusammen einen Ring bilden und $(CH_2)_{4-5}$ bedeuten,
insbesondere
$R^1$ und $R^2$ zusammen einen Ring bilden und $(CH_2)_5$ bedeuten.

Bei einer der vorstehenden Verwendungen eines substituierten Cyclohexan-1,4-diaminderivats aus der Verbindungsgruppe **(N)** kann es bevorzugt sein, wenn in Formel I
$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
vorzugsweise
$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
insbesondere
$R^1$ und $R^2$ unabhängig voneinander ausgewählt sind aus Methyl oder Ethyl;
oder
$R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ zusammen einen Ring bilden und $(CH_2)_{4-5}$ bedeuten,
insbesondere
$R^1$ und $R^2$ zusammen einen Ring bilden und $(CH_2)_5$ bedeuten.

Bei einer der vorstehenden Verwendungen eines substituierten Cyclohexan-1,4-diaminderivats aus einer der Verbindungsgruppen **(K), (M), (N)** oder **(O)** kann es bevorzugt sein, wenn in Formel I
$R^3$ ausgewählt ist aus $C_{3-8}$-Cycloalkyl, Aryl oder Heteroaryl, jeweils unsubstituiert oder einfach oder mehrfach substituiert; über eine gesättigte oder ungesättigte, unverzweigte, substituierte oder unsubstituierte $C_{1-2}$-Alkyl-Gruppe gebundenem Aryl, $C_{3-8}$-Cycloalkyl oder Heteroaryl, jeweils unsubstituiert oder einfach oder mehrfach substituiert;
vorzugsweise
$R^3$ ausgewählt ist aus $C_{5-6}$-Cycloalkyl, Phenyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; über eine gesättigte, unverzweigte $C_{1-2}$-Alkyl-Gruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Phenyl, Furyl, Thiophenyl, Cyclohexanyl, Naphthyl, Benzofuranyl, Indolyl, Indanyl, Benzodioxanyl, Benzodioxolanyl, Pyrrolyl, Pyrimidyl, Pyrazinyl oder Benzothiophenyl, jeweils unsubstituiert oder einfach oder mehrfach substituiert; über eine gesättigte, unverzweigte $C_{1-2}$-Alkyl-Gruppe gebundenem Phenyl, Furyl oder Thiophenyl, jeweils unsubstituiert oder einfach oder mehrfach substituiert.

Bei einer der vorstehenden Verwendungen eines substituierten Cyclohexan-1,4-diaminderivats aus der Verbindungsgruppe (L) kann es bevorzugt sein, wenn in Formel I
$R^3$ ausgewählt ist aus $C_{3-8}$-Cycloalkyl oder Heteroaryl, jeweils unsubstituiert oder einfach oder mehrfach substituiert; über eine gesättigte oder ungesättigte, unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
vorzugsweise
$R^3$ ausgewählt ist aus $C_{5-6}$-Cycloalkyl, Thiophenyl, Benzothiophenyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; über eine gesättigte, unverzweigte $C_{1-2}$-AlkylGruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Furyl, Thiophenyl, Cyclohexanyl, Benzofuranyl, Indolyl, Indanyl, Benzodioxanyl, Benzodioxolanyl, Pyrrolyl, Pyrimidyl, Pyrazinyl oder Benzothiophenyl, jeweils unsubstituiert oder einfach oder mehrfach substituiert; über eine gesättigte, unverzweigte $C_{1-2}$-AlkylGruppe gebundenem Phenyl, Furyl oder Thiophenyl, jeweils unsubstituiert oder einfach oder mehrfach substituiert.

Bei einer der vorstehenden Verwendungen eines substituierten Cyclohexan-1,4-diaminderivats aus einer der Verbindungsgruppen (K), (L), (M), (N) oder (O) kann es bevorzugt sein, wenn in Formel I $R^4$ H ist.

Ebenso kann es bei einer der vorstehenden Verwendungen eines substituierten Cyclohexan-1,4-diaminderivats aus einer der Verbindungsgruppen (**K**), (**L**), (**M**), (**N**) oder (**O**) bevorzugt sein, wenn in Formel I
$R^4$ ausgewählt ist aus H, $C(X)R^7$, $C(X)NR^7R^8$, $C(X)OR^9$, $C(X)SR^9$ oder $S(O_2)R^9$ mit X = O oder S,
vorzugsweise
$R^4$ ausgewählt ist aus H, $C(X)R^7$, $C(X)NR^7R^8$ oder $C(X)OR^9$ mit X = O,
insbesondere
$R^4$ ausgewählt ist aus H oder $C(O)R^7$; vorzugsweise mit $R^7$ ausgewählt aus

H; oder $C_{1-8}$-Alkyl gesättigt oder ungesättigt, verzweigt oder unverzweigt, einfach oder mehrfach substituiert oder unsubstituiert;
vorzugsweise
H; oder $C_{1-3}$-Alkyl gesättigt, unsubstituiert, verzweigt oder unverzweigt;
insbesondere $CH_3$.

Bei einer der vorstehenden Verwendungen eines substituierten Cyclohexan-1,4-diaminderivats aus einer der Verbindungsgruppen **(K), (L), (M)** oder **(O)** kann es bevorzugt sein, wenn in Formel I
$R^4$ und $R^5$ zusammen einen Heterocyclus mit zwischen 3 und 8 Atomen im Ring bilden, gesättigt oder ungesättigt; einfach oder mehrfach substituiert oder unsubstituiert, vorzugsweise mit zwischen 5 und 7 Atomen im Ring, wovon neben dem obligatorischen N 0 bis 1 weitere Heteroatome, ausgewählt aus N, S oder O, im Ring sind;
wobei der von $R^4$ und $R^5$ zusammen gebildete Heterocyclus gegebenenfalls mit weiteren Ringen kondensiert sein kann, vorzugsweise mit aromatischen und/oder heteroaromatischen Ringen, wobei diese mit weiteren aromatischen und/oder heteroaromatischen Ringen kondensiert sein können,
insbesondere der von $R^4$ und $R^5$ zusammen gebildete Heterocyclus mit ein oder zwei weiteren Ringen kondensiert ist, vorzugsweise der von $R^4$ und $R^5$ zusammen gebildete Heterocyclus so mit zwei weiteren Ringen kondensiert ist, dass $R^4$ und $R^5$ zusammen

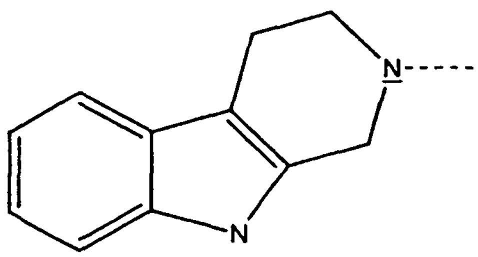

bedeuten.

Bei einer der vorstehenden Verwendungen eines substituierten Cyclohexan-1,4-diaminderivats aus einer der Verbindungsgruppen **(K), (L), (M)** oder **(O)** kann es bevorzugt sein, wenn in Formel I
$R^4$ ausgewählt ist aus H, $C_{1-8}$-Alkyl, gesättigt oder ungesättigt, verzweigt oder unverzweigt, einfach oder mehrfach substituiert oder unsubstituiert,
vorzugsweise
H, $C_{1-6}$-Alkyl, gesättigt oder ungesättigt, verzweigt oder unverzweigt, einfach oder mehrfach substituiert oder unsubstituiert,
insbesondere
H, $C_{1-3}$-Alkyl, gesättigt, unverzweigt und unsubstituiert.

Bei einer der vorstehenden Verwendungen eines substituierten Cyclohexan-1,4-diaminderivats aus einer der Verbindungsgruppen **(K), (L), (M), (N)** oder **(O)** kann es bevorzugt sein, wenn in Formel I
$R^5$ ausgewählt ist aus $C_{3-8}$-Cycloalkyl, Aryl oder Heteroaryl, jeweils unsubstituiert oder einfach oder mehrfach substituiert;
vorzugsweise
$R^5$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^5$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

Ebenso kann es bei einer der vorstehenden Verwendungen eines substituierten Cyclohexan-1,4-diaminderivats aus einer der Verbindungsgruppen **(K), (L), (M), (N)** oder **(O)** bevorzugt sein, wenn in Formel I
$R^5$ ausgewählt ist aus $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ oder $-C(Y)-CH_2-CH_2-CH_2R^{12}$
mit Y = O, S oder $H_2$,
vorzugsweise

$R^5$ ausgewählt ist aus $-CHR^{11}R^{12}$, $-CHR^{11}-CH_2R^{12}$, $-CHR^{11}-CH_2-CH_2R^{12}$, $-C(Y)R^{12}$, $-C(Y)-CH_2R^{12}$ oder $-C(Y)-CH_2CH_2R^{12}$
mit Y = O oder S,
insbesondere
$R^5$ ausgewählt ist aus $-CHR^{11}R^{12}$, $-CHR^{11}-CH_2R^{12}$, $-CHR^{11}-CH_2-CH_2R^{12}$, $-C(Y)R^{12}$ oder $-C(Y)-CH_2R^{12}$
mitY=O.

Bezüglich der vorstehenden Ausführungsform ist es bevorzugt, wenn bei den verwendeten substituierten Cyclohexan-1,4-diaminderivaten gemäß Formel I
$R^{11}$ ausgewählt ist aus
H, $C_{1-4}$-Alkyl, gesättigt oder ungesättigt, verzweigt oder unverzweigt, einfach oder mehrfach substituiert oder unsubstituiert; oder $C(O)O-C_{1-4}$-Alkyl, gesättigt oder ungesättigt, verzweigt oder unverzweigt, einfach oder mehrfach substituiert oder unsubstituiert;
vorzugsweise
H, $C_{1-4}$-Alkyl, gesättigt oder ungesättigt, verzweigt oder unverzweigt, einfach oder mehrfach substituiert oder unsubstituiert; oder $C(O)O-C_{1-2}$-Alkyl, gesättigt, unverzweigt, einfach oder mehrfach substituiert oder unsubstituiert unsubstituiert;
insbesondere
H, $CH_3$, $C_2H_5$ und $C(O)O-CH_3$;
und/oder, wenn bei den verwendeten substituierten Cyclohexan-1,4-diaminderivaten gemäß Formel 1
$R^{12}$ ausgewählt ist aus $C_{3-8}$-Cycloalkyl, Aryl oder Heteroaryl, jeweils unsubstituiert oder einfach oder mehrfach substituiert;
vorzugsweise
$R^{12}$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^{12}$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

Generell kann es unter besonderen Umständen günstig sein, wenn für alle vorstehend beschriebenen erfindungsgemäßen substituierten Cyclohexan-1,4-diaminderivate, Arzneimittel oder Verfahren gilt, daß $R^4$ und $R^5$ zusammen keinen Heterocyclus bilden.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen substituierten Cyclohexan-1,4-diaminderivats, oder eines erfindungsgemäßen Arzneimittels.

Ein weiterer Gegenstand der Erfindung ist ein Verfahren zur Herstellung der erfindungsgemäßen substituierten Cyclohexan-1,4-diaminderivate wie in der folgenden Beschreibung und Beispielen ausgeführt.

Insbesondere geeignet ist dabei ein, im folgenden Hauptverfahren genanntes Verfahren zur Herstellung eines erfindungsgemäßen substituierten Cyclohexan-1,4-diaminderivats mit folgenden Schritten:

a. ein mit den Gruppen $S^1$ und $S^2$ geschütztes Cyclohexan-1,4-dion gemäß Formel II wird in Gegenwart einer Verbindung der Formel $HNR^{01}R^{02}$ mit einem Cyanid, vorzugsweise Kaliumcyanid, zu einem geschützten N-substituierten 1-Amino-4-oxo-cyclohexancarbonitrilderivat gemäß Formel III umgesetzt;

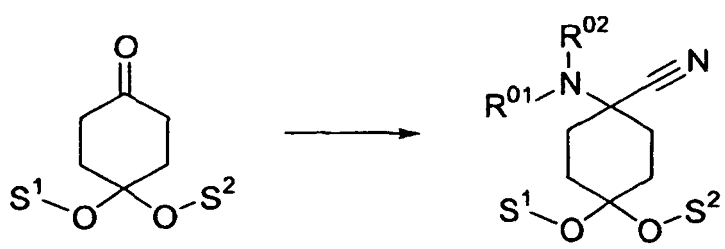

II III

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

b. das Aminonitril gemäß Formel III wird mit metallorganischen Reagenzien, bevorzugt Grignard- oder Organolithiumreagenzien, der Formel Metall-$R^3$ umgesetzt, so daß eine Verbindung gemäß Formel IVa entsteht;

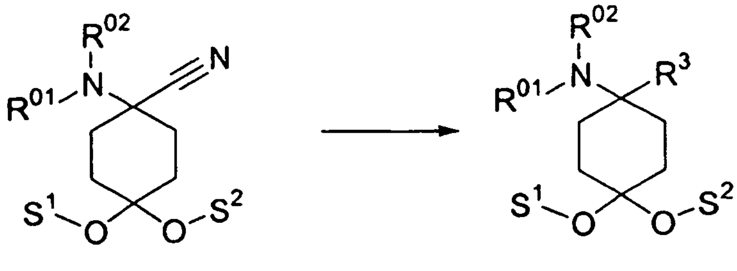

III IVa

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$= mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

c. an der Verbindung gemäß Formel IVa werden die Schutzgruppen $S^1$ und $S^2$ abgespalten, so daß ein 4-substituiertes 4-Aminocyclohexanonderivat gemäß Formel IV entsteht;

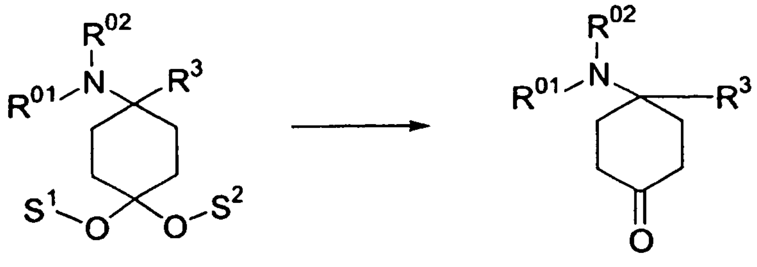

IVa IV

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

d. das 4-substituierte 4-Aminocyclohexanonderivat gemäß Formel IVa wird reduktiv mit einer Verbindung der Formel $HNR^{04}R^{05}$ aminiert, so daß ein Cyclohexan-1,4-diaminderivat gemäß Formel V entsteht;

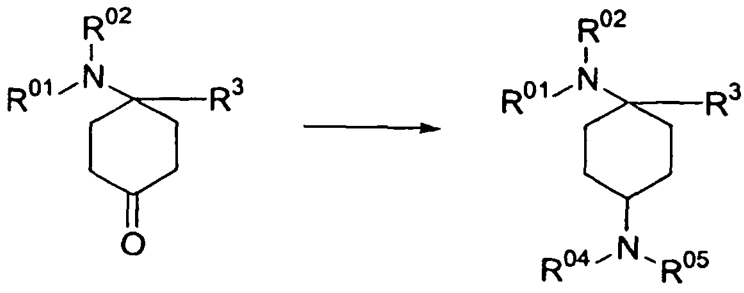

IV V

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert, bis eine Verbindung gemäß Formel I entsteht,

wobei $R^1$, $R^2$, $R^3$ $R^4$ und $R^5$ die für Verbindungsgruppe (A) gemäß Formel I angegebene Bedeutung haben
und
$R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; oder die Reste $R^{01}$ und $R^{02}$ zusammen einen Ring bilden und $CH_2CH_2OCH_2CH_2$, $CH_2CH_2NR^{06}CH_2CH_2$ oder $(CH_2)_{3-6}$ bedeuten,
mit $R^{06}$ ausgewählt aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^{04}$ ausgewählt ist aus H, mit einer Schutzgruppe versehenem H; $C_{1-8}$-Alkyl, gesättigt oder ungesättigt, verzweigt oder unverzweigt, einfach oder mehrfach substituiert oder unsubstituiert;
$R^{05}$ ausgewählt ist aus H, mit einer Schutzgruppe versehenem H; $C_{3-8}$-Cycloalkyl, Aryl oder Heteroaryl, jeweils unsubstituiert oder einfach oder mehrfach substituiert; $-CHR^{11}R^{12}$, $-CHR^{11}- CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ oder $- C(Y)-CH_2-CH_2-CH_2R^{12}$
mit $Y = H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^{04}$ und $R^{05}$ zusammen einen Heterocyclus mit zwischen 3 und 8 Atomen im Ring bilden, gesättigt oder ungesättigt; einfach oder mehrfach substituiert oder unsubstituiert,
und $S^1$ und $S^2$ unabhängig voneinander ausgewählt sind aus Schutzgruppen oder zusammen eine Schutzgruppe bedeuten, vorzugsweise Monoacetal.

Dabei versteht man unter Alkylierung auch eine reduktive Aminierung, da sie zum gleichen Ergebnis führt.

Ein weiterer bevorzugter Gegenstand der Erfindung ist ein, im folgenden Altemativ-Verfahren genanntes Verfahren zur Herstellung eines erfindungsgemäßen substituierten Cyclohexan-1,4-diaminderivats mit folgenden Schritten:

a. ein mit den Gruppen $S^1$ und $S^2$ geschütztes Cyclohexan-1,4-dion gemäß Formel II wird reduktiv mit einer Verbindung der Formel $HNR^{04}R^{05}$ aminiert, so daß ein 4-Aminocyclohexanonderivat gemäß Formel VI entsteht;

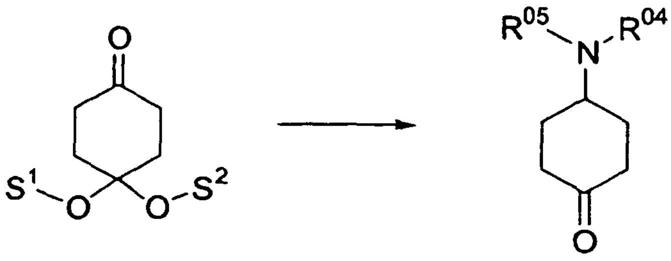

II VI

gegebenenfalls wird anschließend in beliebiger Reihenfolge und gegebenfalls wiederholt acyliert, alkyliert oder sulfoniert und/oder bei Verbindungen mit $R^{04}$ und/oder $R^{05}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{04}$ und/oder $R^{05}$ = H mindestens einmal eine Schutzgruppe eingeführt und gegebenfalls acyliert, alkyliert oder sulfoniert,

b. das 4-Aminocyclohexanonderivat gemäß Formel VI wird in Gegenwart einer Verbindung der Formel $HNR^{01}R^{02}$ mit Cyanid, vorzugsweise Kaliumcyanid, zu einem Cyclohexanonnitrilderivat der Formel VII umgesetzt,

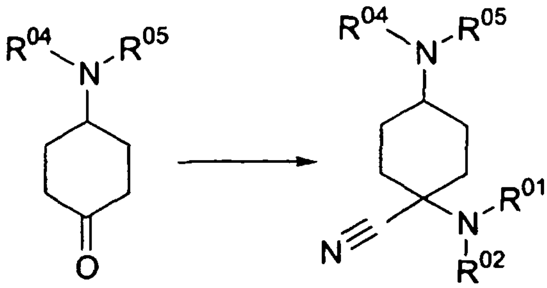

VI VII

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert,

c. das Cyclohexanonnitrilderivat der Formel VII wird mit metallorganischen Reagenzien, bevorzugt Grignard- oder Organolithiumreagenzien, der Formel Metall-$R^3$ umgesetzt und abschließend die Schutzgruppen $S^1$ und $S^2$ abgespalten, so daß ein Cyclohexan-1,4-diaminderivate gemäß Formel V entsteht,

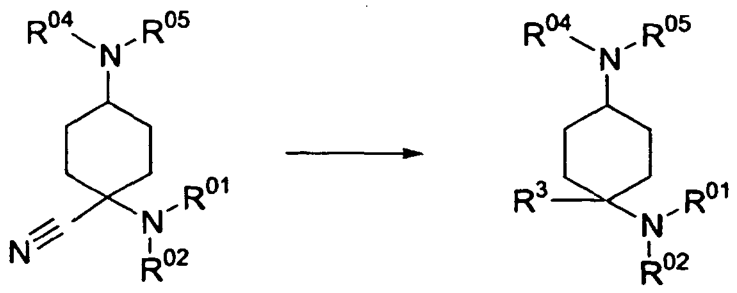

VII

V

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert, bis eine Verbindung gemäß Formel I entsteht,

wobei $R^1$, $R^2$, $R^3$ $R^4$ und $R^5$ die für Verbindungsgruppe (A) gemäß Formel I angegebene Bedeutung haben
und
$R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; oder die Reste $R^{01}$ und $R^{02}$ zusammen einen Ring bilden und $CH_2CH_2OCH_2CH_2$, $CH_2CH_2NR^{06}CH_2CH_2$ oder $(CH_2)_{3-6}$ bedeuten,
mit $R^{06}$ ausgewählt aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^{04}$ ausgewählt ist aus H, mit einer Schutzgruppe versehenem H; $C_{1-8}$-Alkyl, gesättigt oder ungesättigt, verzweigt oder unverzweigt, einfach oder mehrfach substituiert oder unsubstituiert;
$R^{05}$ ausgewählt ist aus mit einer Schutzgruppe versehenem H; $C_{3-8}$-Cycloalkyl, Aryl oder Heteroaryl, jeweils unsubstituiert oder einfach oder mehrfach substituiert; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ oder $-C(Y)-CH_2-CH_2-CH_2R^{12}$
mit Y = $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert ;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^{04}$ und $R^{05}$ zusammen einen Heterocyclus mit zwischen 3 und 8 Atomen im Ring bilden, gesättigt oder ungesättigt; einfach oder mehrfach substituiert oder unsubstituiert,
und $S^1$ und $S^2$ unabhängig voneinander ausgewählt sind aus Schutzgruppen oder zusammen eine Schutzgruppe bedeuten, vorzugsweise Monoacetal.

Für beide Verfahren ist es bevorzugt, daß die Schutzgruppen am H bei $R^{01}$, $R^{02}$, $R^{04}$, $R^{05}$ und/oder $R^{06}$ ausgewählt sind aus Alkyl, Benzyl oder Carbamaten, beispielsweise FMOC, Z oder Boc.

Weiter ist es für das Haupt-Verfahren bevorzugt, wenn die reduktive Aminierung in Schritt d in Gegenwart von Amoniumformiat, Amoniumacetat oder $NaCNBH_3$ stattfindet.

Für das Hauptverfahren ist auch eine besonders günstige Ausführungsform, wenn statt der reduktiven Aminierung mit $HNR^{04}R^{05}$ in Schritt d die Verbindung IV mit Hydroxylamin reagiert und nach der Oximbildung reduziert wird.

Ebenso günstig ist es für das Anternativ-Verfahren, wenn in Schritt b in Formel $HNR^{01}R^{02}$ der Rest $R^{01}$ = H ist, die Umsetzung mit dem Cyanid mit TMSCN geschieht und gegebenfalls anschließend eine Schutzgruppe an $R^{01}$ eingeführt wird.

Im folgenden wird die Erfindung weiter durch Beispiele erläutert, ohne sie darauf zu beschränken.

**Beispiele**

Die folgenden Beispiele dienen zur näheren Erläuterung der Erfindung, schränken aber den allgemeinen Erfindungsgedanken nicht ein.
Die Ausbeuten der hergestellten Verbindungen sind nicht optimiert.
Alle Temperaturen sind unkorrigiert.
Die Angabe "Ether" bedeutet Diethylether, "EE" Ethylacetat und "DCM" Dichlormethan. Die Angabe "Äquivalente" bedeutet Stoffmengenäquivalente, "Smp." bedeutet Schmelzpunkt bzw. Schmelzbereich, "RT" bedeutet Raumtemperatur, "Vol.%" Volumenprozent, "m%" Massenprozent und "M" ist eine Konzentrationsangabe in mol/l.
Als stationäre Phase für die Säulenchromatographie wurde Kieselgel 60 (0.040 - 0.063 mm) der Firma E. Merck, Darmstadt, eingesetzt.
Die dünnschicht-chromatographischen Untersuchungen wurden mit HPTLC-Fertigplatten, Kieselgel 60 F 254, der Firma E. Merck, Darmstadt, durchgeführt.
Die Mischungsverhältnisse von Laufmitteln für chromatographische Untersuchungen sind stets in Volumen/Volumen angegeben.

Beispiel 1: N'-Benzyl-N,N-dimethyl-1-phenyl-cyclohexan-1,4-diamin Hydrochlorid, unpolareres Diastereomer

200 g 1,4-Dioxa-spiro[4.5]decan-8-on wurden mit 200 ml Methanol, 1680 ml wäßriger Dimethylaminlösung (40 m%), 303 g Dimethylamin Hydrochlorid und 200 g Kaliumcyanid versetzt und für ca. 65 Stunden gerührt. Die erhaltene weiße Suspension wurde viermal mit je 800 ml Ether extrahiert, die vereinigten Extrakte eingeengt, der Rückstand in ca. 500 ml Dichlormethan aufgenommen und die Phasen getrennt. Die Dichlormethanphase wurde über Natriumsulfat getrocknet, filtriert und eingeengt. Es wurden 265 g 8-Dimethylamino-1,4-dioxa-spiro[4.5]decan-8-carbonitril als weißer Feststoff erhalten.
50,0 g 8-Dimethylamino-1,4-dioxa-spiro[4.5]decan-8-carbonitril wurden in 400 ml Tetrahydrofuran p.a. gelöst, bei Eisbadkühlung unter Stickstoffatmosphäre 216 ml einer kommerziell erhältlichen zweimolaren Lösung von Phenylmagnesiumchlorid in Tetrahydrofuran zugetropft und über Nacht unter Erwärmung auf Raumtemperatur gerührt. Zur Aufarbeitung wurden unter Rühren bei Eisbadkühlung 200 ml eiskalter Ammoniumchloridlösung (20 m%) zugegeben und nach 30 Minuten die Phasen getrennt. Die wäßrige Phase wurde zweimal mit je 250 ml Ether extrahiert, die Extrakte mit der organischen Phase vereinigt, mit 200 ml Wasser gefolgt von 200 ml gesättigter Natriumchloridlösung gewaschen, über Natriumsulfat getrocknet, filtriert und eingeengt. Es wurden 60,0 g Dimethyl-(8-phenyl-1,4-dioxa-spiro[4.5]dec-8-yl)-amin erhalten.
165 ml Salzsäure (32 m%) wurden mit 100 ml Wasser verdünnt, in diese ca. sechsmolare Salzsäure 60,0 g Dimethyl-(8-phenyl-1,4-dioxa-spiro[4,5]dec-8-yl)-amin gegeben und 24 Stunden gerührt. Das Reaktionsgemisch wurde dreimal mit je 50 ml Diethylether gewaschen, mit 100 ml Natronlauge (32 m%) alkalisch gestellt (pH > 10) und dreimal mit je 100 ml Dichlormethan extrahiert. Die Extrakte wurden vereinigt, über Natriumsulfat getrocknet, filtriert und eingeengt. Es wurden 36,1 g 4-Dimethylamino-4-phenyl-cyclohexanon erhalten.
2,00 g 4-Dimethylamino-4-phenyl-cyclohexanon wurden in 30 ml Tetrahydrofuran p.a. gelöst und unter Rühren im Eisbad 986 mg Benzylamin gefolgt von 794 μl Eisessig zugegeben. Anschließend wurden innerhalb von 15 Minuten 2,72 g Natriumtriacetoxyborhydrid portionsweise zugegeben und der Ansatz für ca. 65 Stunden nachgerührt. Zur Aufarbeitung wurden 15 ml zweimolare Natronlauge zugetropft (pH > 10) und dreimal mit je 25 ml Diethylether extrahiert. Die vereinigten organischen Phasen wurden anschließend zweimal mit je 20 ml Wasser gewaschen, über Natriumsulfat getrocknet, filtriert und eingeengt. Das erhaltene Rohprodukt wurde mit Diethylether unter Zusatz von einem Volumenprozent wäßriger Ammoniaklösung (25 m%) an Kieselgel chromatographiert. Es wurden 844 mg des unpolareren Diastereoisomers von N'-Benzyl-N,N-dimethyl-1-phenyl-cyclohexan-1,4-diamin erhaltenen, die durch Lösen in 6,8 ml 2-Butanon und Zugabe von 27,1 μl Wasser gefolgt von 381 μl Chlortrimethylsilan und Rühren über Nacht in 843 mg des korrespondierenden Hydrochlorids überführt wurden.

Beispiel 2: N'-Benzyl-N,N-dimethyl-1-phenyl-cyctohexan-1,4-diamin Hydrochlorid, polareres Diastereomer

Wie für Beispiel 1 beschrieben wurden auch 1,01 g des polareren Diastereomers von N'-Benryl-N,N-dimethyl-1-phenyl-cyclohexan-1,4-diamin erhaltenen, die durch Lösen in 8,1 ml 2-Butanon und Zugabe von 32,5 μl Wasser gefolgt von 457 μl Chlortrimethylsilan und Rühren über Nacht in 781 mg des korrespondierenden Hydrochlorids überführt wurden.

Beispiel 3: 1,N'-Dibenzyl-N,N-dimethyl-cyclohexan-1,4-diamin Hydrochlorid, unpolareres Diastereomer

50,0 g 8-Dimethylamino-1,4-dioxa-spiro[4,5]decan-8-carbonitril (s. Beispiel 1) wurden in 400 ml Tetrahydrofuran

p.a. gelöst, bei Eisbadkühlung unter Stickstoffatmosphäre 214 ml einer kommerziell erhältlichen zweimolaren Lösung von Benzylmagnesiumchlorid in Tetrahydrofuran zugetropft und über Nacht unter Erwärmung auf Raumtemperatur gerührt. Zur Aufarbeitung wurden unter Rühren bei Eisbadkühlung 200 ml eiskalter Ammoniumchloridlösung (20 m%) zugegeben und nach 30 Minuten die Phasen getrennt. Die wäßrige Phase wurde zweimal mit je 250 ml Ether extrahiert, die Extrakte mit der organischen Phase vereinigt, mit 200 ml Wasser gefolgt von 200 ml gesättigter Natriumchloridlösung gewaschen, über Natriumsulfat getrocknet, filtriert und eingeengt. Es wurden 78,4 g Rohprodukt erhalten, das überwiegend aus (8-Benzyl-1,4-dioxa-spiro[4.5]dec-8-yl)-dimethyl-amin bestand und ohne zusätzliche Aufreinigung weiter umgesetzt wurde.

200 ml Salzsäure (32 m%) wurden mit 120 ml Wasser verdünnt, in diese ca. sechsmolare Salzsäure 78,4 g rohes (8-Benzyl-1,4-dioxa-spiro[4.5]dec-8-yl)-dimethyl-amin gegeben und 24 Stunden gerührt. Das Reaktionsgemisch wurde dreimal mit je 100 ml Diethylether gewaschen, unter Eisbadkühlung mit 100 ml Natronlauge (32 m%) alkalisch gestellt (pH > 10) und dreimal mit je 100 ml Dichlormethan extrahiert. Die Extrakte wurden vereinigt, über Natriumsulfat getrocknet, filtriert und eingeengt. Es wurden 50,4 g 4-Benzyl-4-dimethylamino-cyclohexanon erhalten.

2,00 g 4-Benzyl-4-dimethylamino-cyclohexanon wurden in 30 ml Tetrahydrofuran p.a. gelöst und unter Rühren im Eisbad 926 mg Benzylamin gefolgt von 746 μl Eisessig zugegeben. Anschließend wurden innerhalb von 15 Minuten 2,56 g Natriumtriacetoxyborhydrid portionsweise zugegeben und der Ansatz für ca. 65 Stunden nachgerührt. Zur Aufarbeitung wurden 15 ml zweimolare Natronlauge zugetropft (pH > 10) und dreimal mit je 25 ml Diethylether extrahiert. Die vereinigten organischen Phasen wurden anschließend zweimal mit je 20 ml Wasser gewaschen, über Natriumsulfat getrocknet, filtriert und eingeengt. Das erhaltene Rohprodukt wurde mit Diethylether unter Zusatz von einem Volumenprozent wäßriger Ammoniaklösung (25 m%) an Kieselgel chromatographiert. Es wurden 774 mg des unpolareren Diastereoisomers von 1,N'-Dibenzyl-N,N-dimethyl-cyclohexan-1,4-diamin erhaltenen, die durch Lösen in 6,2 ml 2-Butanon und Zugabe von 23,8 μl Wasser gefolgt von 334 μl Chlortrimethylsilan und Rühren über Nacht in 731 mg des korrespondierenden Hydrochlorids überführt wurden.

Beispiel 4: 1,N'-Dibenzyl-N,N-dimethyl-cydohexan-1,4-diamin Hydrochlorid, polareres Diastereomer

Wie für Beispiel 3 beschrieben wurden auch 820 mg des polareren Diastereomers von 1,N'-Dibenzyl-N,N-dimethyl-cyclohexan-1,4-diamin erhaltenen, die durch Lösen in 6,6 ml 2-Butanon und Zugabe von 25,2 μl Wasser gefolgt von 354 μl Chlortrimethylsilan und Rühren über Nacht in 793 mg des korrespondierenden Hydrochlorids überführt wurden.

Beispiel 5: N-(4-Benzyl-4-dimethytamino-cyctohexyt)-N-propyt-benzamid Hydrochlorid

6,00 g 4-Benzyl-4-dimethylamino-cyclohexanon (s. Beispiel 3) wurden in 90 ml Tetrahydrofuran p.a. gelöst und unter Rühren im Eisbad 1,53 g n-Propylamin gefolgt von 3,36 ml Eisessig zugegeben. Anschließend wurden innerhalb von 15 Minuten 7,68 g Natriumtriacetoxyborhydrid portionsweise zugegeben und der Ansatz für ca. 65 Stunden nachgerührt. Zur Aufarbeitung wurden 45 ml zweimolare Natronlauge zugetropft (pH > 10) und dreimal mit je 50 ml Diethylether extrahiert. Die vereinigten organischen Phasen wurden zweimal mit je 50 ml Wasser gewaschen, über Natriumsulfat getrocknet, filtriert und eingeengt. Das erhaltene Rohprodukt (6,43 g) wurde mit Diethylether unter Zusatz von fünf Volumenprozent wäßriger Ammoniaklösung (25 m%) an Kieselgel chromatographiert. Es wurden 707 mg des unpolareren Diastereoisomers von 1-Benzyl-N,N-dimethyl-N'-propyl-cyclohexan-1,4-diamin erhaltenen.

700 mg des unpolareren Diastereomers von 1-Benzyl-N,N-dimethyl-N'-propyl-cyclohexane-1,4-diamin wurden in 10 ml Dichlormethan gelöst und 370 μl Triethylamin und ca. 10 mg DMAP (4-Dimethylaminopyridin) zugegeben. Unter Kühlung in einem Eis/Methanol-Bad wurden 311 μl Benzoylchlorid zugetropft und die Reaktionsmischung anschließend unter Erwärmung auf Raumtemperatur über Nacht gerührt. Zur Aufarbeitung wurden 10 ml fünfmolare KOH-Lösung und 10 ml Wasser zugegeben, zehn Minuten gerührt, dreimal mit je 20 ml Dichlormethan extrahiert, die vereinigten Extrakte über Magnesiumsulfat getrocknet, filtriert und eingeengt. Aus dem erhaltenen Produkt (834 mg) wurde wie für Beispiel 1 beschrieben mit Wasser und Chlortrimethylsilan in 2-Butanon 909 mg N-(4-Benzyl-4-dimethylamino-cyclohexyl)-N-propyl-benzamid Hydrochlorid hergestellt.

Beispiel 6: N,N-Dimethyl-1-phenyl-N'-propyl-cyclohexan-1,4-diamin Hydrochlorid, unpolareres Diastereomer

10,0 g 4-Dimethylamino-4-phenyl-cyclohexanon wurden in 160 ml Tetrahydrofuran p.a. gelöst und unter Rühren im Eisbad 2,72 g n-Propylamin gefolgt von 5,97 ml Eisessig zugegeben. Anschließend wurden innerhalb von 15 Minuten 13,6 g Natriumtriacetoxyborhydrid portionsweise zugegeben und der Ansatz für ca. 65 Stunden nachgerührt. Zur Aufarbeitung wurden 85 ml zweimolare Natronlauge zugetropft (pH > 10) und dreimal mit je 100 ml Diethylether extrahiert. Die vereinigten organischen Phasen wurden anschließend zweimal mit je 100 ml Wasser gewaschen, über Natriumsulfat getrocknet, filtriert und eingeengt. 5,00 g des erhaltenen Rohprodukts (9,79 g) wurden mit Diethylether, dem ein Volumenprozent wäßriger Ammoniaklösung (25 m%) zugesetzt wurde, und einem von ein auf vierzig Volumenprozent steigendem Zusatz von Methanol an Kieselgel chromatographiert . Es wurden 2,79 g des unpolareren und 1,33 g des polareren Diastereoisomers von N,N-Dimethyl-1-phenyl-N'-propyl-cyclohexan-1,4-diamin erhaltenen. Aus einer Probe von 356 mg des unpolareren Diastereoisomers wurden wie für Beispiel 1 beschrieben mit Wasser und Chlortrimethylsilan in 2-Butanon 253 mg des korrespondierenden Hydrochlorids erhalten.

Beispiel 7: N-(4-Dimethytamino-4-phenyl-cydohexyt)-N-propyt-benzamid Hydrochlorid, unpolareres Diastereomer

1,00 g des unpolareren Diastereomers von N,N-Dimethyl-1-phenyl-N'-propyl-cyclohexan-1,4-diamin (s. Beispiel 6) wurden in 15 ml Dichlormethan gelöst und 560 µl Triethylamin und ca. 10 mg DMAP zugegeben. Unter Kühlung in einem Eis/Methanol-Bad wurden 468 µl Benzoylchlorid zugetropft und die Reaktionsmischung anschließend unter Erwärmung auf Raumtemperatur über Nacht gerührt. Zur Aufarbeitung wurden 12 ml fünfmolare KOH-Lösung und 12 ml Wasser zugegeben, zehn Minuten gerührt, dreimal mit je 25 ml Dichlormethan extrahiert, die vereinigten Extrakte über Magnesiumsulfat getrocknet, filtriert und eingeengt. Aus dem erhaltenen Produkt (1,31 g) wurden wie für Beispiel 1 beschrieben mit Wasser und Chlortrimethylsilan in 2-Butanon 1,01 g des unpolareren Diastereomers von N-(4-Benzyl-4-dimethylamino-cyclohexyl)-N-propyl-benzamid Hydrochlorid hergestellt.

Beispiel 8: N-(4-Dimethy)amino-4-phenyl-cyclohexyl)-N-propyl-benzamid Hydrochlorid, polareres Diastereomer

1,00 g des polareren Diastereomers von N,N-Dimethyl-1-phenyl-N'-propyl-cyclohexan-1,4-diamin (s. Beispiel 6) wurden in 15 ml Dichlormethan gelöst und 560 µl Triethylamin und ca. 10 mg DMAP zugegeben. Unter Kühlung in einem Eis/Methanol-Bad wurden 468 µl Benzoylchlorid zugetropft und die Reaktionsmischung anschließend unter Erwärmung auf Raumtemperatur über Nacht gerührt. Zur Aufarbeitung wurden 12 ml fünfmolare KOH-Lösung und 12 ml Wasser zugegeben, zehn Minuten gerührt, dreimal mit je 25 ml Dichlormethan extrahiert, die vereinigten Extrakte über Magnesiumsulfat getrocknet, filtriert und eingeengt. Aus dem erhaltenen Produkt (1,29 g) wurden wie für Beispiel 1 beschrieben mit Wasser und Chlortrimethylsilan in 2-Butanon 752 mg des polareren Diastereomers von N-(4-Benzyl-4-dimethylamino-cyclohexyl)-N-propyl-benzamid Hydrochlorid hergestellt.

Beispiel 9: 1,N'-Dibenzyl-N.N,N'-trimethyl-cyclohexan-1,4-diamin Hydrochlorid, unpolareres Diastereomer

10,0 g 4-Benzyl-4-dimethylamino-cyclohexanon (s. Beispiel 3) wurden in 150 ml Tetrahydrofuran p.a. gelöst und unter Rühren im Eisbad 5,24 g Benzyl-methyl-amin gefolgt von 5,60 ml Eisessig zugegeben. Anschließend wurden innerhalb von 15 Minuten 12,8 g Natriumtriacetoxyborhydrid portionsweise zugegeben und der Ansatz über Nacht nachgerührt. Zur Aufarbeitung wurden 75 ml zweimolare Natronlauge zugetropft (pH > 10) und dreimal mit je 100 ml Diethylether extrahiert. Die vereinigten organischen Phasen wurden anschließend zweimal mit je 100 ml Wasser gewaschen, über Natriumsulfat getrocknet, filtriert und eingeengt. Das erhaltene Rohprodukts (13,1 g) wurde mit Ethylacetat und einem von Null auf hundert Volumenprozent steigendem Zusatz von Methanol an Kieselgel chromatographiert .

Es wurden, neben einer Mischfraktion von 5,23 g, 5,37 g des unpolareren und 1,20 g des polareren Diastereoisomers von N,N-Dimethyl-1-phenyl-N'-propyl-cyclohexan-1,4-diamin erhaltenen. Aus dem unpolareren Diastereoisomer wurden wie für Beispiel 1 beschrieben mit Wasser und Chlortrimethylsilan in 2-Butanon 5,44 g des korrespondierenden Hydrochlorids erhalten.

Beispiel 10: 1,N'-Dibenzyl-N,N,N'-trimethyl-cyclohexan-1,4-diamin Hydrochlorid, polareres Diastereomer

Wie für Beispiel 9 beschrieben wurden aus 1,20 g des polareren Diastereomers von 1,N'-Dibenzyl-N,N,N'-trimethyl-cyclohexan-1,4-diamin 1,24 g des korrespondierenden Hydrochlorids erhaltenen.

Beispiel 11: N-(4-Benzyl-4-dimethylämino-cyclohexyl)-N-methyl-benzamid Hydrochlorid, polareres Diastereomer

15,0 g 4-Benzyl-4-dimethytamino-cyclohexanon (s. Beispiel 3) wurden in 225 ml Tetrahydrofuran p.a. gelöst und unter Rühren im Eisbad 4,38 g Methylamin Hydrochlorid, 8,9 ml Triethylamin und 8,40 ml Eisessig zugegeben. Anschließend wurden innerhalb von 15 Minuten 19,2 g Natriumtriacetoxyborhydrid portionsweise zugegeben und der Ansatz über Nacht nachgerührt. Zur Aufarbeitung wurden 110 ml zweimolare Natronlauge zugetropft (pH > 10) und dreimal mit je 200 ml Diethylether extrahiert. Die vereinigten organischen Phasen wurden zweimal mit je 200 ml Wasser gewaschen, über Natriumsulfat getrocknet, filtriert und eingeengt. Das erhaltene Rohprodukt (15,0 g) wurde mit Methanol unter Zusatz von einem Volumenprozent wäßriger Ammoniaklösung (25 m%) an Kieselgel chromatographiert. Es wurden 11,6 g des noch deutlich verunreinigten Produktes erhalten, die nochmals mit Ethylacetat und einem von fünfundzwanzig auf fünfzig Volumenprozent steigendem Zusatz von Methanol an Kieselgel chromatographiert wurden. Es wurden 6,67

g 1-Benzyl-N,N,N'-trimethyl-cyclohexan-1,4-diamin als cis/trans-Gemisch erhalten.

3,00 g 1-Benzyl-N,N,N'-trimethyl-cyclohexan-1,4-diamin wurden in 50 ml Dichlormethan gelöst und 1,78 ml Triethylamin und ca. 10 mg DMAP zugegeben. Unter Kühlung in einem Eis/Methanol-Bad wurden 1,41 ml Benzoylchlorid zugetropft und die Reaktionsmischung anschließend unter Erwärmung auf Raumtemperatur über Nacht gerührt. Zur Aufarbeitung wurden 50 ml fünfmolare KOH-Lösung und 50 ml Wasser zugegeben, zehn Minuten gerührt, dreimal mit je 50 ml Dichlormethan extrahiert, die vereinigten Extrakte über Magnesiumsulfat getrocknet, filtriert und eingeengt. Das erhaltene Rohprodukt (3,61 g) wurde mit Methanol/Ether 1 : 1 an Kieselgel chromatographiert. Es wurden 231 mg des polareren Diastereomers von N-(4-Benzyl-4-dimethylamino-cyclohexyl)-N-methyl-benzamid erhalten, aus denen wie für Beispiel 1 beschrieben mit Wasser und Chlortrimethylsilan in 2-Butanon 188 mg des korrespondierenden Hydrochlorids hergestellt wurden.

Beispiel 12: N-(4-Benzyl-4-dimethylamino-cyclohexyl)-N-ethyl-benzamid Hydrochlorid, polareres Diastereomer

15,0 g 4-Benzyl-4-dimethylamino-cyclohexanon (s. Beispiel 3) wurden in 225 ml Tetrahydrofuran p.a. gelöst und unter Rühren im Eisbad 2,89 g Ethylamin gefolgt von 8,40 ml Eisessig zugegeben. Anschließend wurden innerhalb von 15 Minuten 19,2 g Natriumtriacetoxyborhydrid portionsweise zugegeben und der Ansatz über Nacht nachgerührt. Zur Aufarbeitung wurden 110 ml zweimolare Natronlauge zugetropft (pH > 10) und dreimal mit je 200 ml Diethylether extrahiert. Die vereinigten organischen Phasen wurden zweimal mit je 200 ml Wasser gewaschen, über Natriumsulfat getrocknet, filtriert und eingeengt. Das erhaltene Rohprodukt (15,7 g) wurde mit Methanol unter Zusatz von einem Volumenprozent wäßriger Ammoniaklösung (25 m%) an Kieselgel chromatographiert. Es wurden 14,1 g des noch deutlich verunreinigten Produktes erhalten, die nochmals mit Methanol unter Zusatz von einem Volumenprozent wäßriger Ammoniaklösung (25 m%) an Kieselgel chromatographiert wurden. Es wurden 12,1 g 1-Benzyl-N'-ethyl-N,N-dimethyl-cyclohexan-1,4-diamin als cis/trans-Gemisch erhalten.

3,00 g 1-Benzyl-N'-ethyl-N,N-dimethyl-cyclohexan-1,4-diamin wurden in 50 ml Dichlormethan gelöst und 1,68 ml Triethylamin und ca. 10 mg DMAP zugegeben.

Unter Kühlung in einem Eis/Methanol-Bad wurden 1,40 ml Benzoylchlorid zugetropft und die Reaktionsmischung anschließend unter Erwärmung auf Raumtemperatur über Nacht gerührt. Zur Aufarbeitung wurden 50 ml fünfmolare KOH-Lösung und 50 ml Wasser zugegeben, zehn Minuten gerührt, dreimal mit je 50 ml Dichlormethan extrahiert, die vereinigten Extrakte über Magnesiumsulfat getrocknet, filtriert und eingeengt. Das erhaltene Rohprodukt (4,05 g) wurde mit Methanol/Ether 1 : 1 an Kieselgel chromatographiert. Es wurden 1,09 g des polareren Diastereomers von N-(4-Benzyl-4-dimethylamino-cyclohexyl)-N-ethyl-benzamid erhalten, aus denen wie für Beispiel 1 beschrieben mit Wasser und Chlortrimethylsilan in 2-Butanon 1,01 mg des korrespondierenden Hydrochlorids hergestellt wurden.

Beispiel 13: 1-Benzyl-*N'*-(1*H*-indol-3-ylmethyl)-*N,N*-dimethylcyclohexan-1,4-diamin Dihydrochlorid

14,5 g 3-Formylindol und 13,9 g Hydroxylamin Hydrochlorid wurden in einem Gemisch von trockenem Pyridin (80 ml) und abs. Ethanol (80 ml) zwei Stunden zum Sieden erhitzt. Das anfangs gelbe Reaktionsgemisch färbte sich in dieser Zeit kräftig rot. Danach wurde das Lösungsmittelgemisch im Vakuum abdestilliert. Zur Entfernung des Pyridins wurde der Rückstand noch dreimal mit Ethanol (je 30 ml) zur Trockene eingedampft. Der Rückstand wurde dann mit Wasser (100 ml) versetzt und 30 Minuten mit einem Magnetrührer kräftig gerührt. Die Reaktionslösung mit dem entstandenen rosafarbenen Feststoff wurde zwei Stunden im Kühlschrank gekühlt. Das erhaltene Oxim wurde abgesaugt, mit Wasser (3 × 25 ml) gewaschen und im Exsikkator getrocknet. Es wurden 15,6 g 1 H-Indol-3-carbaldehyd-(Z)-oxim mit einen Schmelzpunkt von 190 - 193 °C erhalten.

4,8 g 1*H* Indol-3-carbaldehyd-(Z)-oxim wurden in Methanol (100 ml) suspendiert (schwerlöslich) und mit fünfmolarer Natronlauge (100 ml) verdünnt. Das Reaktionsgefäß wurde mit einem leichten Argonstrom ständig gespült. Zu dem Gemisch gab man portionsweise Devarda-Legierung (20 g). Die Zugabe richtete sich nach der Heftigkeit der Reaktion. Zeitweise wurde mit Eiswasser gekühlt. Nach zwei Stunden war der Eintrag beendet, man rührte bei RT 30 Minuten nach und verdünnte dann mit Wasser (100 ml). Das Methanol wurde im Vakuum abgezogen und die wäßrige Lösung mit Ether (4 × 50 ml) extrahiert. Nach Trocknen und Abdestillieren des Ethers wurde der Rückstand durch Umkristallisation aus Toluol (20 ml) gereinigt. Es wurden 2,2 g *C*-(1*H*-Indol-3-yl)-methylamin als beiger Feststoff mit einem Schmelzpunkt von 90 - 94 °C erhalten, der bei Licht und RT schnell seine Farbe änderte. Lagerung in dunklen Flaschen und im Kühlschrank war einige Tage möglich.

292 mg *C*-(1*H*-Indol-3-yl)-methylamin wurden unter Argon in trockenem 1,2-Dichlorethan (10 ml) teilweise gelöst. Nach Zugabe von 463 mg 4-Benzyl-4-dimethylamino-cyclohexanon (s. Beispiel 3), Eisessig (4 mmol) und Natriumtriacetoxyborhydrid (550 mg) rührte man die Suspension 72 Stunden bei Raumtemperatur. Zur Aufarbeitung wurde das Reaktionsgemisch mit Wasser (10 ml) versetzt. Die organische Phase wurde abgetrennt und die wäßrige Phase zweimal mit Ether extrahiert und dann mit Natronlauge stark alkalisch gemacht. Man extrahierte erneut mit Ethylacetat (4×10 ml). Aus den vereinigten Ethylacetat-Phasen fiel noch während der Bearbeitung ein heller Niederschlag aus.

Nach Kühlung wurde dieser abgesaugt, zweimal mit kaltem Ethylacetat gewaschen und getrocknet. Das so gewonnene Produkt (235 mg) war weiß und fest (Smp. 194 - 198 °C) . 217 mg wurden in 2-Butanon/Ethanol (30 + 10 ml) warm gelöst und bei RT unter Rühren gesättigte ethanolische Salzsäure (1,5 ml; 1,85 M) hinzugegeben. Nach zwei Stunden war noch kein Niederschlag ausgefallen. Auch nach Reduzierung der Lösungsmittelmenge und Kühlung fiel kein Hydrochlorid aus. Deshalb wurde bei 40°C im Vakuum zur Trockene eingedampft und überschüssiges HCl ausgetrieben. Als Rückstand wurden 260 mg 1-Benzyl-*N'*-(1*H*-indol-3-ylmethyl)-*N,N*-dimethylcyclohexan-1,4-diamin Dihydrochlorid als hellrosafarbener Feststoff mit Smp. 170-174°C erhalten.

Beispiel 14: 1-Benzyl-*N'*-[2-(1*H*-indol-3-yl)-1-methylethyl]-*N,N*-dimethylcyclohexan-1,4-diamin, cis/trans-Gemisch

348 mg DL-α-Methyltryptamin wurden unter Argon in trockenem 1,2-Dichtorethan (10 ml) gelöst (klare Lösung), 463 mg 4-Benzyl-4-dimethylamino-cyclohexanon (s. Beispiel 3) und Eisessig (229 μl) zugegeben und eine Stunde bei RT gerührt. Dann wurden 550 mg Natriumtriacetoxyborhydrid hinzugegeben und die Suspension weitere 72 Stunden bei RT gerührt. Zur Aufarbeitung wurde das Reaktionsgemisch mit Wasser (20 ml) versetzt, die organische Phase abgetrennt, die wäßrige Phase einmal mit Ether extrahiert und dann mit Natronlauge stark alkalisch gestellt (pH > 10). Dabei fiel ein gelartiger Niederschlag aus, der sich in Ethylacetat löste. Die wäßrige Phase wurde mit Ethylacetat (4×10 ml) extrahiert. Alle Ethylacetat-Phasen wurden vereinigt, mit Natriumsulfat getrocknet und zur Trockene eingeengt. Es wurden 766 mg eines Gemisches von cis- und trans-1-Benzyl-*N'*-[2-(1*H*-indol-3-yl)-1-methylethyl]-*N*,*N*-dimethylcyclohexan-1,4-diamin als glasartiger Feststoff erhalten (Smp. 48-53 °C).

Beispiel 15: 1-Benzyl-N'-indan-5-yl-N,N-dimethyl-cyclohexan-1,4-diamin Hydrochlorid

266 mg 5-Aminoindan und 462 mg 4-Benzyl-4-dimethylamino-cyclohexanon (s. Beispiel 3) wurden unter Argon in trockenem 1,2-Dichlorethan gelöst und mit 2 g Natriumsulfat 24 Stunden bei RT gerührt. Zu diesem Gemisch wurden 600 mg Natriumtriacetoxyborhydrid gegeben und zwei Stunden bei RT gerührt. Zur Aufarbeitung wurde das Reaktionsgemisch eingeengt und der Rückstand mit fünfmolarer Natronlauge auf pH 11 eingestellt. Die alkalische Phase wurde mit Wasser (10 ml) verdünnt und mit Ethylacetat (4 × 20 ml) extrahiert. Die vereinigten Extrakte wurden über Natriumsulfat getrocknet, filtriert und eingeengt. Das Rohprodukt wurde mit Ethylacetat an Kieselgel chromatographiert. Es wurden 440 mg 1-Benzyl-N'-indan-5-yl-N,N-dimethyl-cyclohexan-1,4-diamin als farbloses Öl erhalten. Zur Herstellung des Hydrochlorids wurde die Base in 2-Butanon (8 ml) gelöst und mit 1,85 M ethanolischer Salzsäure (1,75 ml) versetzt. Der ausgefallene Feststoff wurde abgesaugt und getrocknet. Es wurden 280 mg 1-Benzyl-N'-indan-5-yl-N,N-dimethyl-cyclohexan-1,4-diamin Hydrochlorid als weißer Feststoff (Smp. 200-203 °C) erhalten.

Beispiel 16: 1-Benzyl-N'-indan-1-yl-N,N-dimethyl-cyclohexan-1,4-diamin Dihydrochlorid, cis/trans-Gemisch

266 mg 1-Aminoindan und 462 mg 4-Benzyl-4-dimethylamino-cyclohexanon (s. Beispiel 3) wurden unter Argon in trockenem 1,2-Dichlorethan gelöst und mit 2 g Natriumsulfat 24 Stunden bei RT gerührt. Zu diesem Gemisch wurden 600 mg Natriumtriacetoxyborhydrid gegeben und zwei Stunden bei RT gerührt. Zur Aufarbeitung wurde das Reaktionsgemisch eingeengt und der Rückstand mit fünfmolarer Natronlauge auf pH 11 eingestellt. Die alkalische Phase wurde mit Wasser (10 ml) verdünnt und mit Ethylacetat (4 × 20 ml) extrahiert. Die vereinigten Extrakte wurden über Natriumsulfat getrocknet , filtriert und eingeengt. Das Rohprodukt wurde mit Ethylacetat an Kieselgel chromatographiert. Es wurden 696 mg 1-Benryl-N'-indan-1-yl-N,N-dimethyl-cyclohexan-1,4-diamin als farbloses Öl erhalten. Zur Herstellung des Hydrochlorids wurde die Base in 2-Butanon (10 ml) gelöst und mit 1,85 M ethanolischer Salzsäure (2,80 ml) versetzt. Der ausgefallene Feststoff wurde abgesaugt und getrocknet. Es wurden 540 mg eines Gemisches von cis- und trans-1-Benzyl-N'-indan-1-yl-N,N-dimethyl-cyclohexan-1,4-diamin Dihydrochlorid als weißer Feststoff (Smp. 170-172 °C) erhalten.

Beispiel 17: N'-Indan-1-yl-N,N-dimethyl-1-phenyl-cyclohexan-1,4-diamin

266 mg 1-Aminoindan und 434 mg 4-Dimethylamino-4-phenyl-cyclohexanon wurden unter Argon in trockenem 1,2-Dichlorethan (10 ml) und THF (10 ml) gelöst. Zu diesem Gemisch wurden Eisessig (2 mmol) und Natriumtriacetoxyborhydrid (600 mg) gegeben und 24 Stunden bei RT gerührt. Zur Aufarbeitung wurde eingeengt und der Rückstand mit fünfmolarer Natronlauge auf pH 11 eingestellt. Die alkalische Phase wurde mit Wasser (10 ml) verdünnt und mit Ethylacetat (5 × 20 ml) extrahiert. Die vereinigten Extrakte wurden über Natriumsulfat getrocknet, filtriert und eingeengt. Das Rohprodukt mit Ethylacetat/Ethanol (1 : 1) an Kieselgel chromatographiert. Es wurden 200 mg N'-Indan-1-yl-N,N-dimethyl-1-phenyl-cyclohexan-1,4-diamin als weißer Feststoff erhalten (Smp. 99-101 °C).

Beispiel 18: *N'*-(1*H*-Indol-5-yl)-*N*,*N*-dimethyl-1-phenylcyclohexan-1,4-diamin

264 mg 5-Aminoindan und 434 mg 4-Dimethylamino-4-phenyl-cyclohexanon wurden unter Argon in trockenem 1,2-Dichlorethan (10 ml) gelöst. Zu diesem Gemisch wurden Eisessig (2 mmol) und Natriumtriacetoxyborhydrid (600 mg) gegeben und 24 Stunden bei RT gerührt. Zur Aufarbeitung wurde eingeengt und der Rückstand mit fünfmolarer Natronlauge auf pH 11 eingestellt. Die alkalische Phase wurde mit Wasser (10 ml) verdünnt und mit Ethylacetat (4 x 20 ml) extrahiert. Die vereinigten Extrakte wurden über Natriumsulfat getrocknet, filtriert und eingeengt. Das Rohprodukt mit Ethylacetat/Ethanol (1 : 1) an Kieselgel chromatographiert. Es wurden 315 mg N'-(1H Indol-5-yl)-N,N-dimethyl-1-phenylcyclohexan-1,4-diamin als weißer Feststoff erhalten (Smp. 191-192 °C).

Beispiel 19: N'-(1 H-Indol-3-ylmethyl)-N,N-dimethyl-1-phenyl-cyclohexan-1,4-diamin, cis/trans-Gemisch

292 mg *C*-(1*H*-Indol-3-yl)methylamin wurden unter Argon in trockenem 1,2-Dichlorethan (15 ml) und THF (5 ml) fast klar gelöst. Nach Zugabe von 4-Dimethylamino-4-phenylcyclohexanon (435 mg), Eisessig (4 mmol) und Natriumtriacetoxyborhydrid (550 mg) lag eine Suspension vor, für 72 Stunden bei RT gerührt wurde. Zur Aufarbeitung wurde das Reaktionsgemisch mit Wasser (20 ml) versetzt und eine Stunde kräftig gerührt. Die organische Phase wurde abgetrennt und die wäßrige Phase zweimal mit Ether (10 ml) extrahiert und dann mit fünfmolarer Natronlauge stark alkalisch gemacht. Die wäßrige Phase wurde mit Ethylacetat extrahiert (4 × 10 ml). Dabei fiel ein Feststoff aus, der sich in Ethylacetat (50 ml) unter Erwärmen löste. Die vereinigten Extrakte wurden über Natriumsulfat getrocknet, filtriert und eingeengt. Das erhaltene Rohprodukt (382 mg) wurde aus einer Mischung von Methanol (1 ml) und Ethylacetat (5 ml) umkristallisiert. Der Niederschlag wurde abgesaugt und mit wenig kaltem Ethylacetat gewaschen. Es wurden 156 mg N'-(1H-Indol-3-ylmethyl)-N,N-dimethyl-1-phenyl-cyclohexan-1,4-diamin als cis/trans-Gemisch erhalten.

Beispiel 20: N'-(1 H-Indol-3-ylmethyl)-N,N-dimethyl-1-phenyl-cyclohexan-1,4-diamin, unpolareres Diastereomer

Die in Beispiel 19 erhaltene Mutterlauge wurde eingeengt. Es wurden 173 mg des unpolareren Diastereomers von N'-(1H-Indol-3-ylmethyl)-N,N-dimethyl-1-phenyl-cyclohexan-1,4-diamin erhalten (Smp. 170-178 °C).

Beispiel 21: N'-[2-(1H-Indol-3-yl)-ethyl]-N,N-dimethyl-1-phenyl-cyclohexan-1,4-diamin, unpolareres Diastereomer

Tryptamin (320 mg) wurde unter Argon in trockenem 1,2-Dichlorethan (10 ml) gelöst. Nach Zugabe von 4-Dimethylamino-4-phenylcyclohexanon (435 mg), Eisessig (229 µl) und Natriumtriacetoxyborhydrid (550 mg) rührte man die Suspension 3 Tage bei RT. Zur Aufarbeitung wurde das Reaktionsgemisch mit Wasser (20 ml) versetzt. Die organische Phase wurde abgetrennt, die wäßrige Phase einmal mit Ether extrahiert und dann mit Natronlauge stark alkalisch gestellt. Die wäßrige Phase war bei pH 10 milchig trüb. Es wurde mit Ethylacetat extrahiert (4 × 10 ml), die Extrakte vereinigt, über Natriumsulfat getrocknet, filtriert und eingeengt. Das erhaltene Rohprodukt (674 mg) wurde zweimal aus Ethylacetat (5 ml) umkristallisiert. Es wurden 22 mg des unpolareren Diastereoisomer von N'-[2-(1 H-Indol-3-yl)-ethyl]-N, N-dimethyl-1-phenyl-cyclohexan-1,4-diamin erhalten (Smp. 134-138 °C).

Beispiel 22: N'-[2-(1H-Indol-3-yl)-ethyl]-N,N-dimethyl-1-phenyl-cyclohexan-1,4-diamin, cis/trans-Gemisch

Wie für Beispiel 21 beschrieben wurden auch 320 mg N'-[2-(1 H-Indol-3-yl)ethyl]-N,N-dimethyl-1-phenyl-cyclohexan-1,4-diamin als Gemisch der cis/trans-Isomere erhalten (Smp.149-153 °C).

Beispiel 23: N'-Indan-5-yl-N,N-dimethyl-1-phenyl-cyclohexan-1,4-diamin, unpolareres Diastereomer

5-Aminoindan (266 mg) und 4-Dimethylamino-4-phenylcyclohexanon (434 mg) wurden unter Argon in trockenem 1 ,2-Dichlorethan (10 ml) gelöst. Es wurden Eisessig (2 mmol) und Natriumtriacetoxyborhydrid (600 mg) zugegeben und für 24 Stunden bei RT gerührt. Zur Aufarbeitung wurde das Reaktionsgemisch eingeengt und der Rückstand mit fünfmolarer Natronlauge auf pH 11 eingestellt. Die alkalische Phase wurde mit Wasser (10 ml) verdünnt und mit Ethylacetat (4 × 20 ml) extrahiert. Die vereinigten Extrakte wurden über Natriumsulfat getrocknet, filtriert und eingeengt. Das erhaltene Rohprodukt mit Ethylacetat/Ethanol (1 : 1) an Kieselgel chromatographiert. Es wurden 270 mg des unpolareren Diastereoisomers von N'-Indan-5-yl-N,N-dimethyl-1-phenyl-cyclohexan-1,4-diamin als weißer Feststoff (Smp. 162-164 °C) erhalten.

Beispiel 24: N'-[2-(1H-Indol-3-yl)-1-methyl-ethyl]-N,N-dimethyl-1-phenyl-cyclohexan-1,4-diamin, unpolareres Diastereomer

DL-α-Methyltryptamin (348 mg, 2 mmol) wurde unter Argon in trockenem 1,2-Dichlorethan (10 ml) gelöst. Nach Zugabe von 4-Dimethylamino-4-phenylcyclohexanon (435 mg) und Eisessig (229 μl) wurde eine Stunde bei RT gerührt. Dann wurde Natriumtriacetoxyborhydrid (550 mg) zugegeben und die Suspension vier Tage bei RT gerührt. Zur Aufarbeitung wurde das Reaktionsgemisch mit Wasser (15 ml) versetzt. Die klaren Phasen wurden getrennt, die wäßrige Phase wurde mit Ether (10 ml) gewaschen und dann mit Natronlauge stark alkalisch gemacht. Die wäßrige Phase wurde mit Ethylacetat extrahiert (4 × 10 ml), die vereinigten Extrakte über Natriumsulfat getrocknet, filtriert und eingeengt. Das erhaltene Rohprodukt (723 mg) wurde aus einer Mischung von Ethylacetat/Cyclohexan (2 ml/6 ml) zweimal umkristallisiert. Es wurde eine Fraktion des unpolareren Diastereoisomers von N'-[2-(1 H-Indol-3-yl)-1-methyl-ethyl]-N,N-dimethyl-1-phenyl-cyclohexan-1,4-diamin (128 mg, Smp. 155-162 °C) erhalten.

Beispiel 25: N'-[2-(1H-Indol-3-yl)-1-methyl-ethyl]-N,N-dimethyl-1-phenyl-cyclohexan-1,4-diamin, cis/trans-Gemisch

Wie für Beispiel 24 beschrieben wurden auch 375 mg N'-[2-(1H-Indol-3-yl)-1-methylethyl]-N,N-dimethyl-1-phenyl-cyclohexan-1,4-diamin als Gemisch der cis/trans-Isomere erhalten (dunkelgelbes Öl).

Beispiel 26: N'-[2-(5-Benzyloxy-1 H-indol-3-yl)-ethyl]-N,N-dimethyl-1-phenyl-cyclohexan-1,4-diamin, cis/trans-Gemisch

5-Benzyloxytryptamin (440 mg, 1.65 mmol) wurde unter Argon in trockenem 1,2-Dichlorethan (14 ml) gelöst (leicht trübe Lösung). Nach Zugabe von 4-Dimethylamino-4-phenylcyclohexanon (359 mg, 1,65 mmol) und Eisessig (189 μl, 3,3 mmol) wurde zwei Stunden bei RT gerührt. Dann wurde Natriumtriacetoxyborhydrid (462 mg) zugegeben und die Suspension vier Tage bei RT gerührt. Zur Aufarbeitung wurde das Reaktionsgemisch mit Wasser (15 ml) versetzt. Die Phasen wurden getrennt, die wäßrige Phase wurde mit Ether (20 ml) gewaschen und dann mit Natronlauge stark alkalisch gemacht. Die wäßrige Phase wurde mit Ether (2×10 ml) und Ethylacetat extrahiert (4×10 ml), die vereinigten Extrakte über Natriumsulfat getrocknet, filtriert und eingeengt. Das erhaltene Rohprodukt (686 mg) wurde aus einer Mischung von Ethylacetat/Cyclohexan (35 ml/5 ml) umkristallisiert. Es wurden 396 mg N'-[2-(5-Benzyloxy-1 H-indol-3-yl)-ethyl]-N,N-dimethyl-1-phenyl-cyclohexan-1,4-diamin als cis/trans-Gemisch erhalten (Smp. 130-134 °C).

Beispiel 27: N'-(9H-Fluoren-1-yl)-N,N-dimethyl-1-phenyl-cyclohexan-1,4-diamin Dihydrochlorid

1-Aminofluoren (181 mg, 1 mmol) und 4-Dimethylamino-4-phenylcyclohexanon (217 mg, 1 mmol) wurden unter Argon in trockenem 1,2-Dichlorethan (10 ml) gelöst. Zu diesem Gemisch wurden Eisessig (1 mmol) und Natriumtriacetoxyborhydrid (300 mg) gegeben und 24 Stunden bei RT gerührt. Zur Aufarbeitung wurde eingeengt und der Rückstand mit fünfmolarer Natronlauge auf pH 11 eingestellt. Die alkalische Phase wurde mit Wasser (10 ml) verdünnt und mit Ethylacetat (4 x 20 ml) extrahiert. Die vereinigten Extrakte wurden über Natriumsulfat getrocknet, filtriert und eingeengt. Das Rohprodukt wurde Ethylacetat/Ethanol (1 : 1) an Kieselgel chromatographiert. Es wurden 200 mg N'-(9H-Fluoren-1-yl)-N,N-dimethyl-1-phenyl-cyclohexan-1,4-diamin als farbloses Öl erhalten, das zur Herstellung des Hydrochlorids in 2-Butanon (5 ml) gelöst und mit 1,85 M ethanolischer HCl (0,7 ml) versetzt wurde. Das erhaltene N'-(9H-Fluoren-1-yl)-N, N-dimethyl-1-phenyl-cyclohexan-1,4-diamin Dihydrochlorid wurde abgesaugt und getrocknet (220 mg, Smp. 223-225 °C).

Beispiel 28: N'-Indan-2-yl-N,N-dimethyl-1-phenyl-cyclohexan-1,4-diamin Dihydrochlorid, cis/trans-Gemisch

2-Aminoindan (266 mg, 2 mmol) und 4-Dimethylamino-4-phenylcyclohexanon (434, 2 mmol) wurden unter Argon in trockenem 1,2-Dichlorethan (10 ml) gelöst. Zu diesem Gemisch wurden Eisessig (2 mmol) und Natriumtriacetoxyborhydrid (600 mg) gegeben und 24 Stunden bei RT gerührt. Zur Aufarbeitung wurde eingeengt und der Rückstand mit fünfmolarer Natronlauge auf pH 11 eingestellt. Die alkalische Phase wurde mit Wasser (10 ml) verdünnt und mit Ethylacetat (4 x 20 ml) extrahiert. Die vereinigten Extrakte wurden über Natriumsulfat getrocknet, filtriert und eingeengt. Das Rohprodukt wurde Ethylacetat/Ethanol (1 : 1) an Kieselgel chromatographiert. Es wurden 490 mg N'-Indan-2-yl-N, N-dimethyl-1-phenyl-cyclohexan-1,4-diamin als weißer Feststoff erhalten, der zur Herstellung des Hydrochlorids in 2-Butanon (10 ml) gelöst und mit 1,85 M ethanolischer HCl (2 ml) versetzt wurde. Das erhaltene Gemisch von cis- und trans-N'-Indan-2-yl-N,N-dimethyl-1-phenyl-cyclohexan-1,4-diamin Dihydrochlorid wurde abgesaugt und getrocknet (540 mg, Smp. 224-226 °C).

Beispiel 29: N'-(9H-Fluoren-9-yl)-N,N-dimethyl-1-phenyl-cyclohexan-1,4-diamin Dihydrochlorid, cis/trans-Gemisch

9-Aminofluoren (362 mg, 2 mmol) und 4-Dimethylamino-4-phenylcyclohexanon (434, 2 mmol) wurden unter Argon in trockenem 1,2-Dichlorethan (10 ml) gelöst. Zu diesem Gemisch wurden Eisessig (2 mmol) und Natriumtriacetoxyborhydrid (600 mg) gegeben und 24 Stunden bei RT gerührt. Zur Aufarbeitung wurde eingeengt und der Rückstand mit fünfmolarer Natronlauge auf pH 11 eingestellt. Die alkalische Phase wurde mit Wasser (10 ml) verdünnt und mit Ethylacetat (5 x 20 ml) extrahiert. Die vereinigten Extrakte wurden über Natriumsulfat getrocknet, filtriert und eingeengt. Das Rohprodukt wurde Ethylacetat/Ethanol (1 : 1) an Kieselgel chromatographiert. Es wurden 440 mg N'-(9H-Fluoren-9-yl)-N,N-dimethyl-1-phenyl-cyclohexan-1,4-diamin als weißer Feststoff erhalten, der zur Herstellung des Hydrochlorids in 2-Butanon (10 ml) gelöst und mit 1,85 M ethanolischer HCl (1,55 ml) versetzt wurde. Das erhaltene Gemisch von N'-(9H-Fluoren-9-yl)-N,N-dimethyl-1-phenyl-cyclohexan-1,4-diamin Dihydrochlorid wurde abgesaugt und getrocknet (460 mg, Smp. 202-205 °C).

Beispiel 30: 1-Benzyl-N'-(9H-fluoren-9-yl)-N,N-dimethyl-cyclohexan-1,4-diamin

1-Aminofluoren (181 mg, 1 mmol) und 4-Benzyl-4-dimethylamino-cyclohexanon (231 mg, 1 mmol) wurden unter Argon in trockenem 1,2-Dichlorethan (10 ml) gelöst. Zu diesem Gemisch wurden Eisessig (1 mmol) und Natriumtriacetoxyborhydrid (300 mg) gegeben und 24 Stunden bei RT gerührt. Zur Aufarbeitung wurde eingeengt und der Rückstand mit fünfmolarer Natronlauge auf pH 11 eingestellt. Die alkalische Phase wurde mit Wasser (10 ml) verdünnt und mit Ethylacetat (4 × 20 ml) extrahiert. Die vereinigten Extrakte wurden über Natriumsulfat getrocknet, filtriert und eingeengt. Das Rohprodukt wurde Ethylacetat/Ethanol (1 : 1) an Kieselgel chromatographiert. Es wurden 150 mg 1-Benzyl-N'-(9H-fluoren-9-yl)-N,N-dimethylcyclohexan-1,4-diamin als weißer Feststoff erhalten (Smp. 123-125 °C).

Beispiel 31: 1-Benzyl-N'-(1 H-indol-3-ylmethyl)-N,N-dimethyl-cyclohexan-1,4-diamin, cis/trans-Gemisch

292 mg *C*-(1*H*-Indol-3-yl)-methylamin wurden unter Argon in trockenem 1,2-Dichlorethan (10 ml) teilweise gelöst. Nach Zugabe von 463 mg 4-Benzyl-4-dimethylamino-cyclohexanon (s. Beispiel 3), Eisessig (4 mmol) und Natriumtriacetoxyborhydrid (550 mg) rührte man die Suspension 72 Stunden bei Raumtemperatur. Zur Aufarbeitung wurde das Reaktionsgemisch mit Wasser (10 ml) versetzt. Die organische Phase wurde abgetrennt und die wäßrige Phase zweimal mit Ether extrahiert und dann mit Natronlauge stark alkalisch gemacht. Man extrahierte erneut mit Ethylacetat (4 × 10 ml). Aus den vereinigten Ethylacetat-Phasen fiel noch während der Bearbeitung ein heller Niederschlag aus. Nach Kühlung wurde dieser abgesaugt, zweimal mit kaltem Ethylacetat gewaschen und getrocknet. Es wurden 235 mg 1-Benzyl-N'-(1 H-indol-3-ylmethyl)-N,N-dimethylcyclohexan-1,4-diamin als cis/trans-Gemisch erhalten (Smp. 194-198 °C)..

Beispiel32: N,N-Dimethyl-N'-(1-methyl-1H-indol-3-ylmethyl)-1-phenyl-cyclohexan-1,4-diamin, cis/trans-Gemisch

450 mg *C*-(1*H*-Indol-3-yl)methylamin wurden unter Argon in trockenem 1,2-Dichlorethan (10 ml) teilweise gelöst. Nach Zugabe von 609 mg 4-Dimethylamino-4-phenylcyclohexanon, Eisessig (5,6 mmol), Natriumsulfat (2 g) und Natriumtriacetoxyborhydrid (770 mg) wurde die Suspension fünf Tage bei Raumtemperatur gerührt. Zur Aufarbeitung wurde das Reaktionsgemisch mit Wasser (20 ml) versetzt. Die organische Phase wurde abgetrennt und die wäßrige Phase zweimal mit Ether (5 ml) gewaschen und dann mit Natronlauge stark alkalisch gemacht. Es wurde mit Ether (2 x 5 ml) und Ethylacetat (4 × 10 ml) extrahiert, die vereinigten Extrakte über Natriumsulfat getrocknet, filtriert und eingeengt. Das erhaltene Rohprodukt wurde mit Methanol/Triethylamin (100: 1) an Kieselgel chromatographiert. Es wurden 52 mg N,N-Dimethyl-N'-(1-methyl-1H-indol-3-ylmethyl)-1-phenyl-cyclohexan-1,4-diamin als cis/trans-Gemisch erhalten.

Beispiel 33: N,N-Dimethyl-N'-(1-methyl-1H-indol-3-ylmethyl)-1-phenyl-cyclohexan-1,4-diamin, polareres Diastereomer

Wie für Beispiel 32 beschrieben wurden auch 106 mg des polareren Diastereomers von N,N-Dimethyl-N'-(1-methyl-1H-indol-3-ylmethyl)-1-phenyl-cyclohexan-1,4-diamin erhalten.

Beispiel 34: N'-(2-Benzo[b]thiophen-3-yl-ethyl)-N,N-dimethyl-l-phenyl-cyclohexan-1,4-diamin Dihydrochlorid, cis/trans-Gemisch

Lithiumaluminiumhydrid (1,16 g, 30,3 mmol) wurde in trockenem Diethylether (100 ml) suspendiert. In diese Suspension trug man unter Argon wasserfreies Aluminiumchlorid (4,04 g, 30,3 mmol) ein. Nach fünf Minuten wurde eine Lösung von Benzo[*b*]thiophen-3-acetonitril (5,25 g, 30,3 mmol) in trockenem Diethylether (70 ml) zugegeben. Nach vollständiger Zugabe wurde vier Tage zum Rückfluß erhitzt. Das Reaktionsgemisch wurde erneut mit Lithiumaluminiumhydrid (930 mg) und Aluminiumchlorid (500 mg) versetzt und acht weitere Stunden zum Rückfluß erhitzt. Zur Aufarbeitung wurde mit einer wäßrigen Lösung von Kalium-natrium-tartrat (80 ml, 20 m%) neutralisiert. Nach beendeter Gasentwicklung wurden die Phasen getrennt und die trübe wäßrige Phase über eine Glasfritte abgesaugt. Der Rückstand auf der Fritte wurde mit Ethylacetat gewaschen und die klare wäßrige Phase mit Ethylacetat (3 × 50 ml) extrahiert. Die organischen Phasen wurden über Natriumsulfat getrocknet, filtriert und eingeengt. Es wurde rohes Benzo[*b*]thiophen-3-ylethylamin (3,7 g) als rotbraunes Öl erhalten. Die Behandlung mit methanolischer Salzsäure lieferte ein klebriges Hydrochlorid, das sofort wieder in die freie Base überführt wurde. Es wurden 794 mg (15 %) Benzo[*b*]thiophen-3-yl-ethylamin als gelbes Öl erhalten, das für die weitere Synthese eingesetzt wurde.

Benzo[b]thiophen-3-yl-ethylamin (289 mg, 1,6 mmol) wurde unter Argon in trockenem 1,2-Dichlorethan (10 ml) gelöst und nach Zugabe von 4-Dimethylamino-4-phenylcyclohexanon (354 mg, 1,6 mmol) und Natriumsulfat (2 g) eine Stunde bei RT gerührt. Anschließend gab man Natriumtriacetoxyborhydrid (440 mg, 2,0 mmol) in einer Portion zu dem Reaktionsgemisch. Nach 3 Tagen wurde nachträglich Eisessig (4 mmol) zugesetzt und weitere 24 Stunden bei RT gerührt. Zur Aufarbeitung wurde Wasser (20 ml) zugegeben und das Reaktionsgemisch abgesaugt. Der erhaltene Feststoff wurde mit zweimolarer Natriumcarbonatlösung und Ethylacetat in Lösung gebracht, Die organische Phase abgetrennt, über Natriumsulfat getrocknet, filtriert und eingeengt. Der erhaltene feste, aber klebrige Rückstand (213 mg) wurde in 2-Butanon (5 ml) gelöst und bei RT mit ethanolischer HCl (500 µl, 1,5 mmol) versetzt. Nach zwei Stunden wurde die Lösung wurde zur Trockene eingeengt, der Rückstand in Diethylether (5 ml) suspendiert, abgesaugt und mit Diethylether (3 × 3 ml) nachgewaschen. Es wurde ein Gemisch von cis- und trans-N'-(2-Benzo[b]thiophen-3-yl-ethyl)-N,N-dimethyl-1-phenyl-cyclohexan-1,4-diamin Dihydrochlorid (217 mg, Smp.164-167 °C) als beigebrauner Feststoff erhalten.

Beispiel 35: N'-(2-Benzo[b]thiophen-3-yl-ethyl)-1-benzyl-N,N-dimethyl-cyclohexan-1,4-diamin Dihydrochlorid, cis/trans-Gemisch

Benzo[b]thiophen-3-ylethylamin (350 mg, 1,9 mmol) wurde unter Argon in trockenem 1,2-Dichlorethan (10 ml) gelöst und nach Zugabe von 4-Benzyl-4-dimethylamino-cyclohexanon (463 mg, 2 mmol), Eisessig (2 mmol) und wasserfreiem Natriumsulfat (2 g) eine Stunde bei RT gerührt. Anschließend wurde Natriumtriacetoxyborhydrid (550 mg, 2,5 mmol) in einer Portion zugegeben und vier Tage bei RT gerührt. Zur Aufarbeitung wurde mit 1,2-Dichlorethan (10 ml) und Wasser (15 ml) verdünnt. Der verbliebene Niederschlag wurde abgesaugt (379 mg, Smp. 225-233 °C). Durch Extraktion der mit fünfmolarer Natronlauge auf pH 11 eingestellten wäßrigen Phase mit Ethylacetat wurden 353 mg eines gelben Öls gewonnen werden. Aus beiden Teilmengen konnte durch erneutes Lösen in verdünnter Salzsäure, Extraktion mit Diethylether (2 × 15 ml) und anschließende Einstellung der wäßrigen Phase mit fünfmolarer Natronlauge auf pH 11 sowie Extraktion mit Ethylacetat (3 x 20 ml) das Rohprodukt (438 mg, viskoses Öl) isoliert werden. 366 mg des erhaltenen Diastereoisomerengemisches wurden in 2-Butanon (30 ml) gelöst und bei RT mit ethanolischer Salzsäure (847 µl, 2,8 mmol) versetzt. Es bildete sich ein Niederschlag, der sich schnell wieder löste und während der Nachrührzeit (vier Tage bei RT) erneut ausfiel. Nach weiteren dreißig Minuten im Kühlschrank wurde der Niederschlag abgesaugt, mit kaltem 2-Butanon (3 × 3 ml) gewaschen und getrocknet. Der erhaltene hellgelbe Feststoff war ein Gemisch von cis- und trans- N'-(2-Benzo[b]thiophen-3-yl-ethyl)-1-benzyl-N,N-dimethyl-cyclohexan-1,4-diamin Dihydrochlorid (338 mg, Smp. 225-229 °C).

Beispiel 36: N'-Acenaphthen-1-yl-N,N-dimethyl-1-phenyl-cyclohexan-1,4-diamin Dihydrochlorid, polareres Diastereomer

339 mg Acenaphthen-1-ylamin und 435 mg 4-Dimethylamino-4-phenyl-cyclohexanon wurden unter Argon in trockenem 1,2-Dichlorethan (20 ml) gelöst. Zu diesem Gemisch wurden Eisessig (2 mmol) und 600 mg Natriumtriacetoxyborhydrid gegeben und 24 Stunden bei RT gerührt. Zur Aufarbeitung wurde das Reaktionsgemisch eingeengt und der Rückstand mit fünfmolarer Natronlauge auf pH 11 eingestellt. Die alkalische Phase wurde mit Wasser (10 ml) verdünnt und mit Ethylacetat (4 × 20 ml) extrahiert. Die vereinigten Extrakte wurden über Natriumsulfat getrocknet , filtriert und eingeengt. Das Rohprodukt wurde mit Ethylacetat/Ethanol (1 : 1) an Kieselgel chromatographiert. Es wurden 130 mg des polareren Diastereomers von N'-Acenaphthen-1-yl-N,N-dimethyl-1-phenyl-cyclohexan-1,4-diamin als weißer Feststoff erhalten, aus dem mit 1,85 M ethanolischer Salzsäure (0,5 ml) in 2-Butanon (5 ml) das korrespondierende Dihydrochlorid gefällt wurde (151 mg; Smp. 214-216 °C).

Beispiel 37: N'-Acenaphthen-1-yl-N,N-dimethyl-1-phenyl-cyclohexan-1,4-diamin Dihydrochlorid, unpolareres Diastereomer

Wie für Beispiel 36 beschrieben wurden auch 250 mg des unpolareren Diastereomers von N'-Acenaphthen-1-yl-N,N-dimethyl-1-phenyl-cyclohexan-1,4-diamin als weißer Feststoff erhalten, aus denen mit 1,85 M ethanolischer

Salzsäure (0,9 ml) in 2-Butanon (10 ml) das korrespondierende Dihydrochlorid gefällt wurde (300 mg; Smp. 190-192 °C).

Beispiel 38: N-Benzo[b]thiophen-5-yl-1-benzyl-N,N-dimethyl-cyclohexan-1,4-diamin Dihydrochlorid, unpolareres Diastereomer

300 mg 5-Aminobenzothiophen und 463 mg 4-Benzyl-4-dimethylamino-cyclohexanon wurden unter Argon in trockenem 1,2-Dichlorethan (20 ml) gelöst. Zu diesem Gemisch wurden Eisessig (2 mmol) und 600 mg Natriumtriacetoxyborhydrid gegeben und 24 Stunden bei RT gerührt. Zur Aufarbeitung wurde das Reaktionsgemisch eingeengt und der Rückstand mit fünfmolarer Natronlauge auf pH 11 eingestellt. Die alkalische Phase wurde mit Wasser (10 ml) verdünnt und mit Ethylacetat (6 × 20 ml) extrahiert. Die vereinigten Extrakte wurden über Natriumsulfat getrocknet , filtriert und eingeengt. Das Rohprodukt wurde mit Ethylacetat/Ethanol (1 : 1) an Kieselgel chromatographiert. Es wurden 520 mg des unpolareren Diastereomers von N'-Benzo[b]thiophen-5-yl-1-benzyl-N,N-dimethyl-cyclohexan-1,4-diamin als weißer Feststoff erhalten, aus dem mit 1,85 M ethanolischer Salzsäure (1,93 ml) in 2-Butanon (15 ml) das korrespondierende Dihydrochlorid gefällt wurde (621 mg; Smp. 140-142 °C).

Beispiel 39: N'-Benzo[b]thiophen-5-yl-N,N-dimethyl-l-phenyl-cyclohexan-1,4-diamin Hydrochlorid, unpolareres Diastereomer

300 mg 5-Aminobenzothiophen und 435 mg 4-Dimethylamino-4-phenyl-cyclohexanon wurden unter Argon in trockenem 1,2-Dichlorethan (20 ml) gelöst. Zu diesem Gemisch wurden Eisessig (2 mmol) und 600 mg Natriumtriacetoxyborhydrid gegeben und 24 Stunden bei RT gerührt. Zur Aufarbeitung wurde das Reaktionsgemisch eingeengt und der Rückstand mit fünfmolarer Natronlauge auf pH 11 eingestellt. Die alkalische Phase wurde mit Wasser (10 ml) verdünnt und mit Ethylacetat (3 × 20 ml) extrahiert. Die vereinigten Extrakte wurden über Natriumsulfat getrocknet, filtriert und eingeengt. Das Rohprodukt wurde mit Ethylacetat/Ethanol (1 : 1) an Kieselgel chromatographiert. Es wurden 230 mg des unpolareren Diastereomers von N'-Benzo[b]thiophen-5-yl-N,N-dimethyl-1-phenyl-cyclohexan-1,4-diamin als weißer Feststoff erhalten, aus dem mit 1,85 M ethanolischer Salzsäure (0,54 ml) in 2-Butanon (8 ml) das korrespondierende Hydrochlorid gefällt wurde (243 mg; Smp. 155-157 °C).

Beispiel 40: N'-Benzothiazol-6-yl-N ,N-dimethyl-1-phenyl-cyclohexan-1 ,4-diamin Dihydrochlorid, unpolareres Diastereomer

300 mg 6-Aminobenzothiazol und 435 mg 4-Dimethylamino-4-phenyl-cyclohexanon wurden unter Argon in trockenem 1,2-Dichlorethan (20 ml) gelöst. Zu diesem Gemisch wurden Eisessig (2 mmol) und 600 mg Natriumtriacetoxyborhydrid gegeben und 24 Stunden bei RT gerührt. Zur Aufarbeitung wurde das Reaktionsgemisch eingeengt und der Rückstand mit fünfmolarer Natronlauge auf pH 11 eingestellt. Die alkalische Phase wurde mit Wasser (10 ml) verdünnt und mit Ethylacetat (3 × 20 ml) extrahiert. Die vereinigten Extrakte wurden über Natriumsulfat getrocknet , filtriert und eingeengt. Das Rohprodukt wurde mit Ethylacetat/Ethanol (1:1) an Kieselgel chromatographiert. Es wurden 220 mg des unpolareren Diastereomers von N'-Benzothiazol-6-yl-N,N-dimethyl-1-phenyl-cyclohexan-1,4-diamin als gelber Feststoff erhalten, aus dem mit 1,85 M ethanolischer Salzsäure (0,83 ml) in 2-Butanon (10 ml) das korrespondierende Dihydrochlorid gefällt wurde (197 mg; Smp. 144-147 °C).

Beispiel 41: N'-Benzo[1,2,5]thiadiazol-4-yl-N,N-dimethyl-1-phenyl-cyclohexan-1,4-diamin Dihydrochlorid, polareres Diastereomer

302 mg Benzo[1,2,5]thiadiazol-4-ylamin und 435 mg 4-Dimethylamino-4-phenyl-cyclohexanon wurden unter Argon in trockenem 1,2-Dichlorethan (20 ml) gelöst. Zu diesem Gemisch wurden Eisessig (2 mmol) und 600 mg Natriumtriacetoxyborhydrid gegeben und 24 Stunden bei RT gerührt. Zur Aufarbeitung wurde das Reaktionsgemisch eingeengt und der Rückstand mit fünfmolarer Natronlauge auf pH 11 eingestellt. Die alkalische Phase wurde mit Wasser (10 ml) verdünnt und mit Ethylacetat (3 × 20 ml) extrahiert. Die vereinigten Extrakte wurden über Natriumsulfat getrocknet , filtriert und eingeengt. Das Rohprodukt wurde mit Ethylacetat/Ethanol (1 : 1) an Kieselgel chromatographiert. Es wurden 40 mg des polareren Diastereomers von N'-Benzo[1,2,5]thiadiazol-4-yl-N,N-dimethyl-1 -phenyl-cyclohexan-1,4-diamin als roter Feststoff erhalten, aus dem mit 1,85 M ethanolischer Salzsäure (0,15 ml) in 2-Butanon (2 ml) das korrespondierende Dihydrochlorid gefällt wurde (35 mg; Smp. 122-125 °C).

Beispiel 42: N'-[2-(1H-Indol-3-yl)-1-methyl-ethyl]-N,N-dimethyl-1-phenyl-cyclohexan-1,4-diamin Dihydrochlorid, unpolareres Diastereomer

DL-α-Methyltryptamin (3,00 g, 17,2 mmol) wurde unter Argon in trockenem 1,2-Dichlorethan (10 ml) gelöst.

Nach Zugabe von 4-Dimethylamino-4-phenyl-cyclohexanon (3,70 g) und Eisessig (1,5 ml) wurde eine Stunde bei RT gerührt. Dann wurde Natriumtriacetoxyborhydrid (4,7 g) zugegeben und die Suspension vier Tage bei RT gerührt. Zur Aufarbeitung wurde das Reaktionsgemisch mit 1,2-Dichlorethan (20 ml) und Wasser (50 ml) versetzt. Die klaren Phasen wurden getrennt, die wäßrige Phase wurde mit Ether (2 x 20 ml) gewaschen und dann mit fünfmolarer Natronlauge stark alkalisch gestellt. Die wäßrige Phase wurde mit Ethylacetat extrahiert (5 × 30 ml), die vereinigten Extrakte über Natriumsulfat getrocknet, filtriert und eingeengt. Das erhaltene Rohprodukt (5,8 g beigebrauner Feststoff) wurde mit Methanol/Triethylamin (199 : 1) an Kieselgel zunächst grob fraktioniert und anschließend nochmals feingereinigt. Es wurden 1,20 g des unpolareren Diastereomers von N'-[2-(1 H-Indol-3-yl)-1-methyl-ethyl]-N,N-dimethyl-1-phenyl-cyclohexan-1,4-diamin erhalten (Smp. 158-160 °C). Aus 1 g dieser Verbindung wurde mit Chlortrimethylsilan (840 μl) in 2-Butanon/Aceton (100 ml/30 ml) das korrespondierende Dihydrochlorid gefällt (977 mg; Smp. 170-174 °C).

Beispiel 43: N'-Adamantan-2-yl-N,N-dimethyl-1-phenyl-cyclohexan-1,4-diamin Dihydrochlorid

302 mg 2-Adamantylamin und 434 mg 4-Dimethylamino-4-phenyl-cyclohexanon wurden unter Argon in trockenem Tetrahydrofuran (15 ml) und 1,2-Dichlorethan (5 ml) gelöst. Zu diesem Gemisch wurden 600 mg Natriumtriacetoxyborhydrid gegeben und 23 Stunden bei RT gerührt. Zur Aufarbeitung wurde das Reaktionsgemisch eingeengt und der Rückstand mit einmolarer Salzsäure (20 ml) und Ether (40 ml). Die wäßrige Phase wurde mit Ether (2 x 20 ml) gewaschen, mit fünfmolarer Natronlauge alkalisch gestellt und mit Ether (3 × 30 ml) extrahiert. Die vereinigten Extrakte wurden über Natriumsulfat getrocknet , filtriert und eingeengt. Das Rohprodukt wurde mit Ethylacetat/Methanol (4 : 1) an Kieselgel chromatographiert. Es wurden 130 mg N'-Adamantan-2-yl-N,N-dimethyl-1-phenyl-cyclohexan-1,4-diamin als beigefarbener Feststoff erhalten, aus dem mit 3,3 M ethanolischer Salzsäure (0,34 ml) in 2-Butanon (6 ml) das korrespondierende Dihydrochlorid gefällt wurde (132 mg), das sich beim Erwärmen ab 237 °C zersetzt.

Beispiel 44: N'-(9-Ethyl-9H-carbazol-3-yl)-N,N-dimethyl-1-phenyl-cyclohexan-1,4-diamin Dihydrochlorid, unpolareres Diastereomer

421 mg 3-Amino-9-ethylcarbazol und 435 mg 4-Dimethylamino-4-phenyl-cyclohexanon wurden unter Argon in trockenem 1,2-Dichlorethan (20 ml) gelöst. Zu diesem Gemisch wurden Eisessig (2 mmol) und 600 mg Natriumtriacetoxyborhydrid gegeben und 24 Stunden bei RT gerührt. Zur Aufarbeitung wurde das Reaktionsgemisch eingeengt und der Rückstand mit fünfmolarer Natronlauge auf pH 11 eingestellt. Die alkalische Phase wurde mit Wasser (10 ml) verdünnt und mit Ethylacetat (3 × 20 ml) extrahiert. Die vereinigten Extrakte wurden über Natriumsulfat getrocknet, filtriert und eingeengt. Das Rohprodukt wurde mit Ethylacetat/Ethanol (1 : 1) an Kieselgel chromatographiert. Es wurden 288 mg des unpolareren Diastereomers von N'-(9-Ethyl-9H-carbazol-3-yl)-N,N-dimethyl-1-phenyl-cyclohexan-1,4-diamin als brauner Feststoff erhalten, aus dem mit 1,85 M ethanolischer Salzsäure (0,95 ml) in 2-Butanon (10 ml) das korrespondierende Dihydrochlorid gefällt wurde (339 mg; Smp. 145-150 °C).

Beispiel 45: N'-(3H-Benzotriazol-5-yl)-N,N-dimethyl-1-phenyl-cyclohexan-1,4-diamin Hydrochlorid, unpolareres Diastereomer

268 mg 5-Aminobenzotriazol und 435 mg 4-Dimethylamino-4-phenyl-cyclohexanon wurden unter Argon in trockenem 1,2-Dichlorethan (20 ml) gelöst. Zu diesem Gemisch wurden Eisessig (2 mmol) und 600 mg Natriumtriacetoxyborhydrid gegeben und 24 Stunden bei RT gerührt. Zur Aufarbeitung wurde das Reaktionsgemisch eingeengt und der Rückstand mit fünfmolarer Natronlauge auf pH 11 eingestellt. Die alkalische Phase wurde mit Wasser (10 ml) verdünnt und mit Ethylacetat (3 × 20 ml) extrahiert. Die vereinigten Extrakte wurden über Natriumsulfat getrocknet , filtriert und eingeengt. Das Rohprodukt wurde mit Ethylacetat/Ethanol (1: 1) an Kieselgel chromatographiert. Es wurden 135 mg des unpolareren Diastereomers von N'-(3H-Benzotriazol-5-yl)-N,N-dimethyl-1-phenyl-cyclohexan-1,4-diamin als weißer Feststoff erhalten, aus dem mit 1,85 M ethanolischer Salzsäure (0,54 ml) in 2-Butanon (5 ml) das korrespondierende Hydrochlorid gefällt wurde (98 mg; Smp. 168-173 °C).

Beispiel 46: N'-(3H-Benzotriazol-5-yl)-N,N-dimethyl-1-phenyl-cyclohexan-1,4-diamin Hydrochlorid, polareres Diastereomer

Wie für Beispiel 45 beschrieben wurden auch 122 mg des polareren Diastereomers von N'-(3H-Benzotriazol-5-yl)-N,N-dimethyl-1-phenyl-cyclohexan-1,4-diamin als weißer Feststoff erhalten, aus denen mit 1,85 M ethanolischer Salzsäure (0,5 ml) in 2-Butanon (5 ml) das korrespondierende Dihydrochlorid gefällt wurde (119 mg; Smp. 185-189 °C).

Beispiel 47: N'-(9H-Fluoren-9-yl)-N,N-dimethyl-1-thiophen-2-yl-cyclohexan-1,4-diamin Dihydrochlorid, cis/trans-Gemisch

2-Iodthiophen (22,9 g) wurde unter Argon in THF (80 ml) gelöst und innerhalb von 30 min bei 0 °C mit 2M Isopropylmagnesiumchlorid (35,7 ml) in THF versetzt. Nach einer Reaktionszeit von einer Stunde bei 3-5 °C wurde 8-Dimethylamino-1,4-dioxa-spiro[4.5]decan-8-carbonitril (10 g), gelöst in Tetrahydrofuran (20 ml), hinzugefügt und 20 Stunden bei Raumtemperatur gerührt. Zur Aufarbeitung wurde gesättigte $NH_4Cl$-Lösung (85 ml) zugegeben, mit Diethylether extrahiert (3×100 ml), die vereinigten Extrakte mit Wasser (50 ml) und gesättigter NaCl-Lösung (50 ml) gewaschen, getrocknet und eingeengt. Das erhaltene Rohprodukt (21,3 g dunkelbraunes Öl) wurde in 2-Butanon (140 ml) gelöst und mit Chlortrimethylsilan (9,1 ml) in das Hydrochlorid von Dimethyl-(8-thiophen-2-yl-1,4-dioxa-spiro[4.5]dec-8-yl)-amin überführt (weißer Feststoff; 8,74 g).

Dimethyl-(8-thiophen-2-yl-1,4-dioxa-spiro[4.5]dec-8-yl)-amin Hydrochlorid (8,68 g) wurde in 7,5M Salzsäure (29 ml) gelöst , 48 Stunden bei Raumtemperatur gerührt und anschließend mit Diethylether extrahiert (2 × 50 ml). Die wäßrige Phase wurde unter Eiskühlung mit 5M Natronlauge alkalisch gestellt, mit Dichlormethan (3 × 50 ml) extrahiert, getrocknet und eingeengt. 4-Dimethylamino-4-thiophen-2-yl-cyclohexanon so als gelber Feststoff erhalten (5,66 g; Smp. 108-110 °C).

362 mg 9-Aminofluoren und 434 mg 4-Dimethylamino-4-thiophen-2-yl-cyclohexanon wurden unter Argon in trockenem 1,2-Dichlorethan (10 ml) gelöst. Zu diesem Gemisch wurden Eisessig (2 mmol) und 600 mg Natriumtriacetoxyborhydrid gegeben und 24 Stunden bei RT gerührt. Zur Aufarbeitung wurde das Reaktionsgemisch eingeengt und der Rückstand mit fünfmolarer Natronlauge auf pH 11 eingestellt. Die alkalische Phase wurde mit Wasser (10 ml) verdünnt und mit Ethylacetat (5 × 20 ml) extrahiert. Die vereinigten Extrakte wurden über Natriumsulfat getrocknet , filtriert und eingeengt. Das Rohprodukt wurde mit Ethylacetat/Ethanol (1 : 1) an Kieselgel chromatographiert. Es wurden 440 mg eines cis/trans-Gemisches von N'-(9H-Fluoren-9-yl)-N,N-dimethyl-1-thiophen-2-yl-cyclohexan-1,4-diamin als weißer Feststoff erhalten, aus dem mit 1,85 M ethanolischer Salzsäure (1,55 ml) in 2-Butanon (10 ml) das korrespondierende Dihydrochlorid gefällt wurde (460 mg; Smp. 202-205 °C).

Beispiel 48: N'-Cyclooctyl-N,N-dimethyl-1-phenyl-cyclohexan-1,4-diamin Dihydrochlorid

254 mg Cyclooctylamin und 434 mg 4-Dimethylamino-4-phenyl-cyclohexanon wurden unter Argon in trockenem Tetrahydrofuran (15 ml) und 1,2-Dichlorethan (5 ml) gelöst. Zu diesem Gemisch wurden Eisessig (120 mg) und 600 mg Natriumtriacetoxyborhydrid gegeben und 18 Stunden bei RT gerührt. Zur Aufarbeitung wurde das Reaktionsgemisch eingeengt, der Rückstand mit einmolarer Salzsäure (20 ml) und mit Ether (2 x 30 ml) gewaschen. Die wäßrige Phase wurde mit fünfmolarer Natronlauge alkalisch gestellt und mit Ether (3 × 30 ml) extrahiert. Die vereinigten Extrakte wurden über Natriumsulfat getrocknet, filtriert und eingeengt. Das Rohprodukt (515 mg) wurde mit Methanol an Kieselgel chromatographiert. Es wurden 108 mg N'-Cyclooctyl-N,N-dimethyl-1-phenyl-cyclohexan-1,4-diamin als farbloses Öl erhalten, aus dem mit 3,3 M ethanolischer Salzsäure (0,25 ml) in 2-Butanon (2 ml) das korrespondierende Dihydrochlorid gefällt wurde (102 mg; Smp. 247-249 °C).

Beispiel 49: N'-(1 H-Indol-3-ylmethyl)-N,N-dimethyl-1-phenyl-cyclohexan-1,4-diamin Dihydrochlorid, unpolareres Diastereomer

970 mg *C*-(1*H*-Indol-3-yl)methylamin und 1,44 mg 4-Dimethylamino-4-phenyl-cyclohexanon wurden unter Argon in trockenem Tetrahydrofuran (15 ml) und 1,2-Dichlorethan (50 ml) gelöst. Zu diesem Gemisch wurden Eisessig (13,2 mmol) und 1,82 g Natriumtriacetoxyborhydrid gegeben und 72 Stunden bei RT gerührt. Zur Aufarbeitung wurde das Reaktionsgemisch eingeengt, der Rückstand Wasser (20 ml) und Ether (30 ml) versetzt und kräftig gerührt. Die wäßrige Phase wurde abgetrennt, mit Ether (2 x 15 ml) gewaschen, mit fünfmolarer Natronlauge auf pH 11 eingestellt und mit Ethylacetat (4 × 25 ml) extrahiert. Die vereinigten Extrakte wurden über Natriumsulfat getrocknet , filtriert und eingeengt. Das Rohprodukt (2,11 g) wurde mit Methanol/Triethylamin (199 : 1) an Kieselgel chromatographiert. Es wurden 465 mg des unpolareren Diastereomers von N'-(1 H-Indol-3-ylmethyl)-N,N-dimethyl-1-phenyl-cyclohexan-1,4-diamin erhalten (Smp. 182-184 °C), aus dem mit Chlortrimethylsilan (443 μl) in 2-Butanon/Aceton (20 ml/50 ml) das korrespondierende Dihydrochlorid gefällt wurde (498 mg; Smp. 164-168 °C).

Beispiel 50: N'-(1 H-Indol-3-ylmethyl)-N,N-dimethyl-1-phenyl-cyclohexan-1,4-diamin Dihydrochlorid, polareres Diastereomer

Wie für Beispiel 49 beschrieben wurden auch 360 mg des polareren Diastereomers von N'-(1 H-Indol-3-ylmethyl)-N,N-dimethyl-1-phenyl-cyclohexan-1,4-diamin erhalten, aus denen mit Chlortrimethylsilan (328 μl) in 2-Butanon/Aceton (10 mt/25 ml) das korrespondierende Dihydrochlorid gefällt wurde (435 mg; Smp. 185-188 °C).

Beispiel 51: N'-Benzo[b]thiophen-3-ylmethyl-N,N-dimethyl-1-phenyl-cyclohexan-1,4-diamin Dihydrochlorid, unpolareres Diastereomer

Benzothiophen-3-carbaldehyd (4,0 g, 24,6 mmol) wurde in einem Gemisch aus Pyridin (25 ml) und Ethanol (25 ml) gelöst. Unter Rühren wurde Hydroxylamin Hydrochlorid (3,4 g, 49,2 mmol) zugegeben. Die Mischung wurde 30 Minuten bei RT gerührt und dann acht Stunden zum Rückfluß erhitzt. Es entstand eine rotbraune Lösung. Zur Aufarbeitung wurde eingeengt und der Rückstand durch Destillation mit Ethanol (3 x 50 ml) von restlichem Pyridin befreit. Der ölige Rückstand wurde mit Wasser (50 ml) versetzt und über Nacht kräftig gerührt. Der vorliegende rosa Feststoff wurde abgesaugt, mit Wasser gewaschen und im Vakuum getrocknet. Es wurden 4,3 g Benzothiophen-3-carbaldehyd Oxim erhalten (Smp. 87-89 °C).

Benzothiophen-3-carbaldehyd Oxim (3,96 g, 22,3 mmol) wurde in Methanol (100 ml) und fünfmolarer Natronlauge (100 ml) gelöst und unter Argon portionsweise mit Devarda-Legierung (14,1 g) versetzt. Dabei kam es zu Erwärmung und Wasserstoffentwicklung. Es wurde 16 Stunden gerührt. Die Aufarbeitung erfolgte durch langsame Zugabe von Wasser (100 ml), wobei nochmals eine heftige Reaktion einsetzte. Die Mischung wurde über Celite filtriert, das Methanol im Vakuum entfernt und die zurückgebliebene wäßrige Phase mit Diethylether (3 × 50 ml) extrahiert. Nach dem Einengen der organischen Phase blieben 1,43 g C-Benzo[b]thiophen-3-yl-methylamin als grünes Öl zurück. Zu einer Lösung dieses Amins (1,3 g, 8 mmol) in 2-Butanon (5 ml) wurde 3,3 M ethanolische Salzsäure (3,6 ml, 12 mmol) gegeben, wobei 1,18 g C-Benzo[b]thiophen-3-yl-methylamin Hydrochlorid als weißer kristalliner Feststoff mit einem Schmelzpunkt von 254-256 °C ausfielen.

449 mg C-Benzo[b]thiophen-3-yl-methylamin und 434 mg 4-Dimethylamino-4-phenyl-cyclohexanon wurden unter Argon in trockenem Tetrahydrofuran (20 ml) und 1,2-Dichlorethan (7 ml) gelöst. Zu diesem Gemisch wurden Eisessig (165 mg) und 825 mg Natriumtriacetoxyborhydrid gegeben und 41 Stunden bei RT gerührt. Zur Aufarbeitung wurde das Reaktionsgemisch eingeengt, der Rückstand mit einmolarer Salzsäure (20 ml) und mit Ether (2 x 20 ml) gewaschen. Die wäßrige Phase wurde mit einmolarer Natronlauge auf pH 8-9 gestellt und mit Ether (3 × 20 ml) extrahiert. Die vereinigten Extrakte wurden über Natriumsulfat getrocknet , filtriert und eingeengt. Das gelbe, kristalline Rohprodukt (787 mg) wurde zur chromatographischen Trennung in Methanol (7 ml) gelöst, wobei das unpolarere Diastereomer ausfiel. Es wurden 247 mg des unpolareren Diastereomers von N'-Benzo[b]thiophen-3-ylmethyl-N,N-dimethyl-1-phenylcyclohexan-1,4-diamin als weißer Feststoff erhalten (Smp. 138-140 °C), aus dem mit 3,3 M ethanolischer Salzsäure (0,8 ml) in 2-Butanon (25 ml) das korrespondierende Dihydrochlorid gefällt wurde (187 mg; Smp. 225-230 °C).

Beispiel 52: N'-Benzo[b]thiophen-3-ylmethyl-N,N-dimethyl-1-phenyl-cyclohexan-1,4-diamin Dihydrochlorid, polareres Diastereomer

Wie für Beispiel 51 beschrieben wurde die methanolische Lösung des Rohprodukts mit Methanol an Kieselgel chromatographiert. Es wurden 113 mg des polareren Diastereomers von N'-Benzo[b]thiophen-3-ylmethyl-N,N-dimethyl-1-phenyl-cyclohexan-1,4-diamin als farbloses Öl erhalten, aus dem mit 3,3 M ethanolischer Salzsäure (0,28 ml) in 2-Butanon (10 ml) das korrespondierende Dihydrochlorid als weißer Feststoff gefällt wurde (120 mg; Smp. 252-254 °C).

Beispiel 53: N'-Anthracen-2-yl-N,N-dimethyl-1-phenyl-cyclohexan-1,4-diamin Hydrochlorid, unpolareres Diastereomer

386 mg 2-Aminoanthracen und 434 mg 4-Dimethylamino-4-phenyl-cyclohexanon wurden unter Argon in trokkenem 1,2-Dichlorethan (20 ml) gelöst. Zu diesem Gemisch wurden Eisessig (2 mmol) und 600 mg Natriumtriacetoxyborhydrid gegeben und 24 Stunden bei RT gerührt. Zur Aufarbeitung wurde das Reaktionsgemisch eingeengt, der Rückstand mit fünfmolarer Natronlauge auf pH 11 eingestellt und mit Ethylacetat (4 × 20 ml) extrahiert. Die vereinigten Extrakte wurden über Natriumsulfat getrocknet, filtriert und eingeengt. Das Rohprodukt wurde mit Ethylacetat/Ethanol (1: 1) an Kieselgel chromatographiert. Es wurden 132 mg des unpolareren Diastereomers von N'-Anthracen-2-yl-N,N-dimethyl-1-phenyl-cyclohexan-1,4-diamin als grüner Feststoff erhalten, aus dem mit 1,85 M ethanolischer Salzsäure (0,46 ml) in 2-Butanon (5 ml) das korrespondierende Hydrochlorid gefällt wurde (104 mg; Smp. 169-172 °C).

Beispiel 54: N'-Benzo[b]thiophen-3-ylmethyl-1-benzyl-N,N-dimethyl-cyclohexan-1,4-diamin Dihydrochlorid, unpolareres Diastereomer

391 mg C-Benzo[b]thiophen-3-yl-methylamin und 554 mg 4-Benzyl-4-dimethylamino-cyclohexanon wurden unter Argon in trockenem Tetrahydrofuran (18 ml) und 1,2-Dichlorethan (6 ml) gelöst. Zu diesem Gemisch wurden Eisessig (144 mg) und 720 mg Natriumtriacetoxyborhydrid gegeben und 22 Stunden bei RT gerührt. Zur Aufarbeitung wurde das Reaktionsgemisch eingeengt, der Rückstand mit einmolarer Salzsäure (20 ml) aufgenommen und mit Ether (2 x 20 ml) gewaschen. Die wäßrige Phase wurde mit einmolarer Natronlauge auf pH 8-9 eingestellt und mit Ether (3 × 20 ml) extrahiert. Die vereinigten Extrakte wurden über Natriumsulfat getrocknet, filtriert und eingeengt. Das erhaltene

hellgelbe Öl (904 mg) wurde mit Methanol an Kieselgel chromatographiert. Es wurden 368 mg des unpolareren Diastereomers von N'-Benzo[b]thiophen-3-ylmethyl-1-benzyl-N,N-dimethyl-cyclohexan-1,4-diamin erhalten, aus dem mit 3,3 M ethanolischer Salzsäure (0,88 ml) in 2-Butanon (25 ml) das korrespondierende Dihydrochlorid gefällt wurde (364 mg; Smp. 246-255 °C).

Beispiel 55: N'-Benzo[b]thiophen-3-ylmethyl-1-benzyl-N,N-dimethyl-cyclohexan-1,4-diamin Dihydrochlorid, polareres Diastereomer

Wie für Beispiel 54 beschrieben wurden auch 347 mg des polareren Diastereomers von N'-Benzo[b]thiophen-3-ylmethyl-1-benzyl-N,N-dimethyl-cyclohexan-1,4-diamin erhalten, aus denen mit 3,3 M ethanolischer Salzsäure (0,83 ml) in 2-Butanon (25 ml) das korrespondierende Dihydrochlorid gefällt wurde (418 mg; Smp. 242-248 °C).

Beispiel 56: N'-[2-(1 H-Indol-3-yl)ethyl]-N,N-dimethyl-1-naphthalin-2-yl-cyclohexan-1,4-diamin Dihydrochlorid, unpolareres Diastereomer

Aus Magnesium (2,05 g) und 2-Bromnaphthalin (17,7 g) wurde in trockenem Tetrahydrofuran (65 ml) eine Grignard-Lösung bereitet. Diese Grignard-Lösung wurde noch eine Stunde bei Siedetemperatur nachgerührt. Danach wurde in trockenem Tetrahydrofuran (70 ml) gelöstes 8-Dimethylamino-1,4-dioxa-spiro[4.5]decan-8-carbonitril (9,0 g) bei RT zugetropft und über Nacht bei RT nachgerührt. Nach beendeter Reaktion wurde der Ansatz unter Eiskühlung mit gesättigter Ammoniumchloridlösung gequencht, mit Diethylether (2 × 70 ml) extrahiert, über $Na_2SO_4$ getrocknet und eingeengt. Zur Aufreinigung wurde das Rohprodukt (24,2 g) in 2-Butanon (130 ml) gelöst und unter Eiskühlung mit $Me_3SiCl$ (14,8 ml) versetzt. Nach sechs Stunden wurde das ausgefallene Dimethyl-(8-naphthalen-2-yl-1,4-dioxa-spiro [4.5]dec-8-yl)-amin Dimethyl-(8-naphthalin-2-yl-1,4-dioxa-spiro[4.5]dec-8-yl)-amin abgesaugt (weißer Feststoff, 6,09 g).

Dimethyl-(8-naphthalen-2-yl-1,4-dioxa-spiro[4.5]dec-8-yl)-amin Hydrochlorid (6,09 g) wurde in 7,5N Salzsäure gelöst, 32 Stunden bei RT gerührt und anschließend mit Diethylether (3 × 30 ml) extrahiert. Die wäßrige Phase wurde unter Eiskühlung mit 25-proz. Ammoniaklösung alkalisch gestellt und mit 1,2-Dichlorethan (3 × 30 ml) extrahiert. Die vereinigten Extrakte wurden mit $Na_2SO_4$ getrocknet und eingeengt. Es wurden 4,48 g 4-Dimethylamino-4-naphthalin-2-yl-cyclohexanon als weißer Feststoff erhalten (Smp. 81-83 °C).

Das Dihydrochlorid des unpolareren Diastereomers von N'-[2-(1 H-Indol-3-yl)-ethyl]-N,N-dimethyl-1-naphthalin-2-yl-cyclohexan-1,4-diamin wurde analog zu den oben beschrieben Beispielen durch reduktive Aminierung von 4-Dimethylamino-4-naphthalin-2-yl-cyclohexanon mit Tryptamin erhalten.

Beispiel 57: N'-[2-(1H-Indol-3-yl)-ethyl]-N,N-dimethyl-1-phenyl-cyclohexan-1,4-diamin Dihydrochlorid, unpolareres Diastereomer

1,12 g Tryptamin und 1,52 g 4-Dimethylamino-4-phenylcyclohexanon wurden unter Argon in trockenem Tetrahydrofuran (12 ml) und 1,2-Dichlorethan (40 ml) gelöst. Zu diesem Gemisch wurden Eisessig (801 μl) und 1,92 g Natriumtriacetoxyborhydrid gegeben und vier Tage bei RT gerührt. Zur Aufarbeitung wurde das Reaktionsgemisch eingeengt, der Rückstand mit Wasser (20 ml), zweimolarer Salzsäure (5 ml) und Ether (35 ml) aufgenommen. Die wäßrige Phase wurde abgetrennt, mit Ether (2 x 15 ml) gewaschen, mit Natronlauge auf pH 11 eingestellt und mit Ethylacetat (3 × 20 ml) extrahiert. Die vereinigten Extrakte wurden über Natriumsulfat getrocknet, filtriert und eingeengt. Der erhaltene beigebraune Rückstand (2,0 g) wurde mit Methanol, das 0,75 Vol.% Triethylamin enthielt, an Kieselgel chromatographiert. Es wurden 553 mg des unpolareren Diastereomers von N'-[2-(1H-Indol-3-yl)-ethyl]-N,N-dimethyl-1-phenyl-cyclohexan-1,4-diamin erhalten (Smp. 175-178 °C), aus dem mit Chlortrimethylsilan in 2-Butanon/Aceton (20 ml/50 ml) das korrespondierende Dihydrochlorid erhalten wurde (600 mg; Smp. 216-218 °C).

Beispiel 58: N'-[2-(1 H-Indol-3-yl)-ethyl]-N,N-dimethyl-1-phenyl-cyclohexan-1,4-diamin Dihydrochlorid, polareres Diastereomer

Wie für Beispiel 57 beschrieben wurden auch 546 mg des polareren Diastereomers von N'-[2-(1 H-Indol-3-yl)-ethyl]-N,N-dimethyl-1-phenyl-cyclohexan-1,4-diamin erhalten (Smp. 175-180 °C), aus denen mit Chlortrimethylsilan (573 μl) in 2-Butanon/Aceton (3 ml/30 ml) das korrespondierende Dihydrochlorid erhalten wurde (520 mg; Smp. 223-229 °C).

Beispiel 59: N'-[2-(1 H-Indol-3-yl)-1-methyl-ethyl]-N,N-dimethyl-1-phenyl-cyclohexan-1,4-diamin Dihydrochlorid, polareres Diastereomer

Wie für Beispiel 42 beschrieben wurden auch 546 mg des polareren Diastereomers von N'-[2-(1H-Indol-3-yl)-

1-methyl-ethyl]-N,N-dimethyl-1-phenyl-cyclohexan-1,4-diamin erhalten (Smp. 50-55 °C), aus denen mit Chlortrimethylsilan (1,0 ml) in 2-Butanon (50 ml) das korrespondierende Dihydrochlorid als hellrosa Feststoff erhalten wurde (1,1 g; Smp. 194-199 °C).

Beispiel 60: Methyl 2-(4-dimethylamino-4-phenyl-cyclohexylamino)-3-(1H-indol-3-yl)-propionat Dihydrochlorid, unpolareres Diastereomer

L-Tryptophanmethylester (438 mg, 2 mmol) in 1,2-Dichlorethan (20 ml) wurde mit 4-Dimethylamino-4-phenylcyclohexanon (435 mg, 2 mmol), Eisessig (57 µl, 1 mmol) und geglühtem Natriumsulfat (2 g) versetzt. Nach zweistündigem Rühren bei RT wurde Natriumtriacetoxyborhydrid (660 mg, 3 mmol) zugesetzt und weiter gerührt. Nach 3 Tagen wurde das Reaktionsgemisch eingeengt und der Rückstand in Diethylether (20 ml) und 1 M NaOH (5 ml) suspendiert. Nach der Extraktion der wäßrigen Phase mit Diethylether und Ethylacetat (je 3 × 10 ml) wurden die vereinigten organischen Phasen im Scheidetrichter noch zweimal mit 1 M NaOH (5 ml) gewaschen, getrocknet und eingeengt. Der viskose Rückstand (718 mg) wurde durch zweimalige Flashchromatographie [50 g Kieselgel, Eluent: Ethylacetat/Methanol (3 : 1) sowie Ethylacetat/MeOH (1 : 1)] gereinigt und dabei die Diastereoisomeren getrennt. Es wurden 270 mg des unpolareren Diastereomers von Methyl 2-(4-dimethylamino-4-phenyl-cyclohexylamino)-3-(1H-indol-3-yl)-propionat erhalten, aus dem mit Chlortrimethylsilan (244 µl) in 2-Butanon/Aceton (8 ml/4 ml) das korrespondierende Dihydrochlorid als weißer Feststoff erhalten wurde (291 mg, Smp. 175-180 °C).

Beispiel 61: Methyl 2-(4-dimethylamino-4-phenyl-cyclohexylamino)-3-(1H-indol-3-yl)-propionat Dihydrochlorid, polareres Diastereomer

Wie für Beispiel 60 beschrieben wurden auch (140 mg, Smp. 60-65 °C) des polareren Diastereomers von Methyl 2-(4-dimethylamino-4-phenyl-cyclohexylamino)-3-(1 H-indol-3-yl)-propionat erhalten, aus denen mit Chlortrimethylsilan (126 µl) in 2-Butanon/Aceton (7 ml/3 ml) das korrespondierende Dihydrochlorid als weißer Feststoff erhalten wurde (129 mg; Smp. 180-185 °C).

Beispiel 62: N'-[2-(1H-Indol-3-yl)-1-methyl-ethyl]-N,N-dimethyl-1-naphthalin-2-yl-cyclohexan-1,4-diamin Dihydrochlorid, unpolareres Diastereomer

4-Dimethylamino-4-naphthalin-2-yl-cyclohexanon (534 mg) und DL-α-Methyltryptamin (348 mg) wurden unter Argon in einem Gemisch aus Tetrahydrofuran (20 ml) und 1,2-Dichlorethan (5 ml) gelöst. Es wurde Essigsäure (120 mg) hinzugefügt und nach einer Reaktionszeit von 15 Minuten mit Natriumtriacetoxyborhydrid (600 mg) versetzt. Nach 64 Stunden wurde das Reaktionsgemisch abgesaugt. Nach der Aufnahme des erhaltenen weißen Feststoffs in 1 M Natronlauge (20 ml), Extraktion mit Diethylether (3 × 20 ml) und dem Einengen der getrockneten vereinigten Extrakte wurde ein öliger Rückstand (520 mg) erhalten. Die chromatographische Trennung des Gemisches erfolgte zunächst mit Methanol, wobei 295 mg (Smp. 68-70 °C) des unpolareren Diastereoisomers als weißer Feststoff erhalten wurden. Das unpolarere Diamin wurde in 2-Butanon (5 ml) gelöst und mit 3,3N ethanolischer Salzsäure (0,52 ml) versetzt, wobei ein öliger Feststoff ausfiel. Nach dem Einengen des Reaktionsgemisches und der Zugabe von Diethylether wurde das kristalline Dihydrochlorid des unpolareren Diastereomers von N'-[2-(1 H-Indol-3-yl)-1-methyl-ethyl]-N,N-dimethyl-1-naphthalin-2-yl-cyclohexan-1,4-diamin erhalten (319 mg; Smp. 206-210 °C).

Beispiel 63: N'-Benzo[1,3]dioxol-5-ylmethyl-N,N-dimethyl-1-phenyl-cyclohexan-1,4-diamin Dihydrochlorid, cis/trans-Gemisch

3,4-(Methylendioxy)benzylamin (250 µl) und 4-Dimethylamino-4-phenylcyclohexanon (434 mg) wurden unter Ausschluß von Sauerstoff in trockenem 1,2-Dichlorethan (10 ml) gelöst. Diesem Gemisch wurden Eisessig (2 mmol) und Natriumtriacetoxyborhydrid (600 mg) zugefügt. Anschließend wurde 24 Stunden bei RT gerührt. Zur Aufarbeitung wurde eingeengt, mit 5M NaOH auf pH 11 eingestellt, mit Wasser (10 ml) verdünnt und mit Ethylacetat (4 × 20 ml) extrahiert. Die vereinigten organischen Extrakte wurden mit $Na_2SO_4$ getrocknet und eingeengt. Das erhaltene farblose Öl (795 mg) wurde in 2-Butanon (13 ml) gelöst und mit Chlortrimethylsilan (718 µl) das Dihydrochlorid von N'-Benzo [1,3]dioxol-5-ylmethyl-N,N-dimethyl-1-phenyl-cyclohexan-1,4-diamin als Gemisch der cis/trans-Isomere erhalten (weißer Feststoff; 790 mg; Smp. 128-131 °C).

Beispiel 64: N'-[2-(6-Fluor-1H-indol-3-yl)-ethyl]-N,N-dimethyl-1-phenyl-cyclohexan-1,4-diamin Dihydrochlorid, unpolareres Diastereomer

Das 6-Fluortryptamin (410 mg) und 4-Dimethylamino-4-phenylcyclohexanon (545 mg) wurden unter Argon in

THF (18 ml) und 1,2-Dichlorethan (6 ml) gelöst und mit Essigsäure (138 mg) versetzt. Nach 15 Minuten wurde Natriumtriacetoxyborhydrid (690 mg) und THF (5 ml) zugegeben. Nach 40 Stunden wurde eingeengt, der Rückstand in 1M Salzsäure (20 ml) aufgenommen und mit Ether (2 × 20 ml) extrahiert. Die wäßrige Phase wurde mit 1 M Natronlauge (30 ml) alkalisch gestellt und mit Ether (3 × 30 ml) extrahiert. Dabei schied sich zwischen den Phasen ein weißer Feststoff (785 mg) ab, der abgetrennt wurde. Es handelte sich hierbei um ein Gemisch aus den beiden Diastereomeren, das auch beim Einengen der etherischen Phase auftrat. Gemeinsam wurden die Gemische (985 mg) mit Methanol/konz. Ammoniak (500 : 1) säulenchromatographisch getrennt. Das unpolarere Diastereoisomer wurde als weißes Feststoff (321 mg, Smp. 185-187 °C) erhalten, unter Erwärmen in Ethanol (20 ml) gelöst und mit 3,3N ethanolischer HCl (0,79 ml) versetzt. Nach Stunde Rühren bei RT wurde das weiße Dihydrochlorid des unpolareren Diamins von N'-[2-(6-Fluor-1H-indol-3-yl)-ethyl]-N,N-dimethyl-1-phenyl-cyclohexan-1,4-diamin erhalten (344 mg; Smp. 190-195 °C).

Beispiel 65: N'-[2-(6-Fluor-1 H-indol-3-yl)-ethyl]-N,N-dimethyl-1-phenyl-cyclohexan-1,4-diamin Dihydrochlorid, polareres Diastereomer

Wie für Beispiel 64 beschrieben wurden auch 305 mg des polareren Diastereomers von N'-[2-(6-Fluor-1H-indol-3-yl)-ethyl]-N,N-dimethyl-1-phenyl-cyclohexan-1,4-diamin erhalten, aus denen in Ethanol (20 ml) mit 3,3N ethanolischer HCl (0,73 ml) das korrespondierende Dihydrochlorid erhalten wurde (270 mg; Smp. 208-211 °C)

Beispiel 66: N'-[2-(1H-Indol-3-yl)-ethyl]-N,N,N'-trimethyl-1-phenyl-cyclohexan-1,4-diamin Dihydrochlorid, unpolareres Diastereomer

*N*-ω-Methyltryptamin ([2-(1 H-Indol-3-yl)ethyl]methylamin, 348 mg) wurde unter Argon in trockenem 1,2-Dichlorethan (10 ml) gelöst. Nach Zugabe von 4-Dimethylamino-4-phenylcyclohexanon (435 mg) und Eisessig (114 µl) bildete sich ein voluminöser Niederschlag. Die Suspension wurde zwei Stunden bei RT gerührt, bevor Natriumtriacetoxyborhydrid (660 mg) zugesetzt wurde. Die Reaktionsmischung wurde zwei Tage bei RT gerührt, zur Aufarbeitung eingeengt, der Rückstand in Wasser (15 ml) und Diethylether (20 ml) aufgelöst und die organische Phase abgetrennt. Die wäßrige Phase wurde mit Diethylether (2 × 10 ml) extrahiert und mit 1 M NaOH auf pH 10 eingestellt. Dabei fiel ein weißer Feststoff aus, der abgesaugt, gewaschen und getrocknet wurde (174 mg, Smp. 208-210 °C, unpolareres Diastereoisomer). Die wäßrige Phase wurde mit 1 M NaOH auf pH 11 gebracht und mit Ethylacetat (4 × 25 ml) extrahiert Die Extrakte wurden vereinigt, mit $Na_2SO_4$ getrocknet und im Vakuum eingeengt. Der Rückstand (469 mg) wurde durch Flashchromatographie mit Methanol/Triethylamin (99:1) getrennt. Das so erhaltene unpolarere Diastereoisomer (172 mg) wurde in 2-Butanon/Aceton (15 ml/15 ml) warm gelöst und bei RT mit Chlortrimethylsilan (174 µl) das Hydrochlorid von N'-[2-(1H-Indol-3-yl)-ethyl]-N,N,N'-trimethyl-1-phenyl-cyclohexan-1 ,4-diamin als weißer Feststoff gefällt (173 mg; Smp. 195-198°C).

Beispiel 67: N'-[2-(1H-Indol-3-yl)-ethyl]-N,N,N'-trimethyl-1-phenyl-cyclohexan-1,4-diamin Dihydrochlorid, polareres Diastereomer

Wie für Beispiel 66 beschrieben wurden auch 129 mg des polareren Diastereomers von N'-[2-(1H-Indol-3-yl)-ethyl]-N,N,N'-trimethyl-1-phenyl-cyclohexan-1,4-diamin erhalten, die in der Wärme in 2-Butanon/Aceton (15 ml/3 ml) mit Chlortrimethylsilan (121 µl) in das korrespondierende Dihydrochlorid überführt wurden (weißer Feststoff; 141 mg; Smp. 198-206 °C).

Beispiel 68: N,N-Dimethyl-N'-[2-(7-methyl-1H-indol-3-yl)-ethyl]-1-phenyl-cyclohexan-1,4-diamin Dihydrochlorid, unpolareres Diastereomer

7-Methyltryptamin (348 mg) und 4-Dimethylamino-4-phenylcyclohexanon (435 mg) wurden unter Ausschluß von Sauerstoff in trockenem 1,2-Dichlorethan (5 ml) und Tetrahydrofuran (15 ml) gelöst. Zu diesem Gemisch wurde Eisessig (2 mmol) und Natriumtriacetoxyborhydrid (600 mg) hinzugefügt und 24 Stunden bei RT gerührt. Zur Aufarbeitung wurde eingeengt, der Ansatz mit 1 M HCl (20 ml) und Diethylether (40 ml) versetzt, saure wäßrige Phase mit Diethylether (2 × 20 ml) extrahiert und mit 5M NaOH auf pH 11 eingestellt. Die alkalische Phase wurde mit Wasser (10 ml) verdünnt und mit Ethylacetat (3 × 20 ml) extrahiert. Die vereinigten Extrakte wurden mit $Na_2SO_4$ getrocknet, eingeengt und das erhaltene Rohprodukt mit EtOH/$NH_3$ (500 : 1) an Kieselgel getrennt. Das unpolarere Diastereoisomer wurde (321 mg) als braunes Öl gewonnen, in 2-Butanon (10 ml) gelöst und mit Chlortrimethylsilan (270 µl) in das Dihydrochlorid überführt (weißer Feststoff; 420 mg; Smp. 189-191 °C).

Beispiel 69: N,N-Dimethyl-N'-[2-(7-methyl-1 H-indol-3-yl)-ethyl]-1-phenyl-cyclohexan-1,4-diamin Dihydrochlorid, polareres Diastereomer

Wie für Beispiel 68 beschrieben wurden auch 144 mg des polareren Diastereoisomer als braunes Öl erhalten, in 2-Butanon (5 ml) gelöst und mit Chlortrimethylsilan (121 μl) in das korrespondierende Hydrochlorid überführt (weißer Feststoff; 146 mg; Smp. 244-246 °C).

Beispiel 70: N'-[2-(5-Fluor-1 H-indol-3-yl)-ethyl]-N,N-dimethyl-1-phenyl-cyclohexan-1,4-diamin Dihydrochlorid, unpolareres Diastereomer

Zu einem Gemisch aus Tetrahydrofuran (12 ml) und 1,2-Dichlorethan (4 ml) wurden unter Argon zunächst das 2-(5-Fluor-1 H-indol-3-yl)ethylamin (282 mg) und 4-Dimethylamino-4-phenylcyclohexanon (343 mg) gegeben und mit Essigsäure (0,09 ml) versetzt. Nach 15 min wurde $NaBH(OAc)_3$ (474 mg) hinzugefügt und 40 Stunden bei RT gerührt. Das Reaktionsgemisch wurde eingeengt, der Rückstand mit 1 M Salzsäure (20 ml) aufgenommen und mit Ether (2 × 30 ml) extrahiert. Dabei schied sich ein weißer Niederschlag (191 mg) ab, der abgetrennt wurde. Anschließend wurde die wäßrige Lösung mit 1 M NaOH (28 ml) alkalisch gestellt und mit Ether (2 × 30 ml) und Ethylacetat (2 × 30 ml) extrahiert. Die vereinten organischen Extrakte wurden über Natriumsulfat getrocknet und eingeengt. Der Rückstand (468 mg) bestand genau wie der vorher abgetrennte Feststoff aus zwei Produkten. Sie wurden gemeinsam (459 mg) säulenchromatographisch mit Methanol/Ammoniak (500 : 1) aufgereinigt. Das unpolarere Diastereoisomer wurde als weißer Feststoff (218 mg; Smp. 191-192 °C) erhalten, unter Erwärmen in Ethanol (15 ml) gelöst und mit 3,3N ethanolischer Salzsäure (0,47 ml, 1,56 mmol) versetzt. Als nach 90 min noch kein Feststoff ausgefallen war, wurde 2-Butanon (5 ml) zugegeben. Nach kurzer Zeit begann dann die Kristallisation des Hydrochlorids (184 mg; Smp. 230-237 °C).

Beispiel 71: N'-[2-(5-Fluor-1H-indol-3-yl)-ethyl]-N,N-dimethyl-1-phenyl-cyclohexan-1,4-diamin Dihydrochlorid, polareres Diastereomer

Wie für Beispiel 70 beschrieben wurde auch das polarere Diastereoisomer (189 mg; Smp. 200-201 °C) erhalten, 159 mg davon in Ethanol (15 ml) und 2-Butanon (5 ml) gelöst und mit 3,3N ethanolischer Salzsäure (0,38 ml) in das Dihydrochlorid überführt (124 mg; Smp. 262-265 °C).

Beispiel 72: N'-Acenaphthen-5-ylmethyl-N,N-dimethyl-1-phenyl-cyclohexan-1,4-diamin Dihydrochlorid, unpolareres Diastereomer

Acenaphthen-5-ylmethylamin (366 mg) und 4-Dimethylamino-4-phenylcyclohexanon (434 mg) wurden unter Ausschluß von Sauerstoff in trockenem 1,2-Dichlorethan (10 ml) gelöst. Diesem Gemisch wurden Eisessig (2 mmol) und Natriumtriacetoxyborhydrid (600 mg) zugefügt und 24 Stunden bei RT gerührt. Zur Aufarbeitung wurde eingeengt und der Rückstand mit 5M NaOH auf pH 11 1 eingestellt. Die alkalische Phase wurde mit Wasser (10 ml) verdünnt und mit Ethylacetat (3 × 20 ml) extrahiert. Die vereinigten organischen Phasen wurden mit $Na_2S0_4$ getrocknet und eingeengt. Das erhaltene Rohprodukt wurde durch Chromatographie mit Ethylacetat/EtOH (1 : 1) aufgereinigt. Das unpolarere Diastereoisomer wurde als farbloses Öl erhalten (330 mg), in 2-Butanon (10 ml) gelöst und mit Chlortrimethylsilan (272 μl) in das korrespondierende Dihydrochlorid überführt (weißer Feststoff; 393 mg; Smp. 164-167 °C).

Beispiel 73: N'-[2-(1 H-Indol-3-yl)-1-methyl-ethyl]-N,N-dimethyl-1-thiophen-2-yl-cyclohexan-1,4-diamin Dihydrochlorid, unpolareres Diastereomer

DL-α-Methyltryptamin (N'-[2-(1H-Indol-3-yl)-1-methylethyl]-N,N-dimethyl-1-thiophen-2-yl-cyclohexan-1,4-diamin, 348 mg) wurde unter Argon in trockenem 1,2-Dichlorethan (20 ml) gelöst. Nach Zugabe von 4-Dimethylamino-4-thiophen-2-yl-cyclohexanon (447 mg) und Eisessig (114 μl) bildete sich ein voluminöser Niederschlag. Die Suspension wurde eine Stunde bei RT gerührt. Dann wurde Natriumtriacetoxyborhydrid (660 mg) zugesetzt und die Reaktionsmischung zwei Tage bei RT gerührt. Zur Aufarbeitung wurde mit 1,2-Dichlorethan (10 ml) und Wasser (15 ml) verdünnt, die organische Phase abgetrennt, die wäßrige Phase nochmals mit 1,2-Dichlorethan (2 × 5 ml) extrahiert, mit 5M NaOH alkalisch gestellt und mit Ethylacetat (4 × 15 ml) extrahiert. Die vereinigten organischen Phasen wurden getrocknet, eingeengt und durch Flashchromatographie (50 g Kieselgel 60, Eluent: Methanol/$NEt_3$ (99 : 1)) aufgereinigt. Das unpolarere Diastereoisomer (202 mg, Smp. 158-161 °C wurde in 2-Butanon (5 ml) gelöst und mit Chlortrimethylsilan (202 μl) in das korrespondierende Dihydrochlorid überführt (weißer Feststoff, 207 mg; Smp. 162-165 °C).

Beispiel 74: N'-[2-(1H-Indol-3-yl)1-methyl-ethyl]-N,N-dimethyl-1-thiophen-2-yl-cyclohexan-1,4-diamin Dihydrochlorid, cis/trans-Gemisch

Wie für Beispiel 73 beschrieben wurde auch ein Gemisch der Diastereoisomeren isoliert (195 mg), in 2-Butanon (4 ml) gelöst und mit Chlortrimethylsilan (194 μl) in das korrespondierende Dihydrochlorid überführt (weißer Feststoff; 232 mg; polar/unpolar = 70 : 30).

Beispiel 75: N'-[2-(7-Benzyloxy-1H-indol-3-yl)-ethyl]-N,N-dimethyl-1-phenyl-cyclohexan-1,4-diamin Dihydrochlorid, unpolareres Diastereomer

7-Benzyloxytryptamin (200 mg) wurde unter Argon in trockenem 1,2-Dichlorethan (10 ml) und THF (10 ml) gelöst. Nach Zugabe von 4-Dimethylamino-4-phenylcyclohexanon (180 mg) und Eisessig (43 μl) wurde eine Stunde bei RT gerührt und dann mit Natriumtriacetoxyborhydrid (248 mg) versetzt. Die Reaktionsmischung wurde drei Tage bei RT gerührt. Zur Aufarbeitung wurde eingeengt, der Rückstand wurde in Wasser (15 ml), 2M HCl (2 ml) und Diethylether (20 ml) aufgelöst, die organische Phase abgetrennt, die wäßrige Phase mit Diethylether (2 × 15 ml) gewaschen, mit 1 M NaOH auf pH 11 gebracht und mit Ethylacetat (4 × 10 ml) extrahiert. Die vereinigten Ethylacetat Extrakte wurden getrocknet, eingeengt und der erhaltene Rückstand (351 mg) durch Flashchromatographie (45 g Kieselgel 60, Eluent: MeOH/$NEt_3$ (99 : 1)) aufgereinigt. Das unpolarere Diastereoisomer (188 mg) in 2-Butanon/Aceton (6 ml/6 ml) warm gelöst und mit Chlortrimethylsilan (147 μ) in das korrespondierende Dihydrochlorid überführt (weißer Feststoff; 176 mg; Smp. 162-166 °C).

Beispiel 76: N'-Cyclooctyl-N,N-dimethyl-1-thiophen-2-yl-cyclohexan-1,4-diamin Dihydrochlorid, unpolareres Diastereomer

4-Dimethylamino-4-phenylcyclohexanon (447 mg, 2 mmol) wurde unter Argon in 1,2-Dichlorethan (25 ml) gelöst und mit Cyclooctylamin (254 mg) und Essigsäure (120 mg) versetzt. Es wurde 15 min bei RT gerührt und dann Natriumtriacetoxyborhydrid (600 mg) zugegeben. Nach 48 Stunden bei RT wurde das Reaktionsgemisch am Rotationsverdampfer eingeengt, der Rückstand mit 1 M Salzsäure (20 ml) aufgenommen und mit Diethylether (2 × 30 ml) gewaschen. Anschließend wurde die wäßrige Lösung mit 1M NaOH (28 ml) alkalisch gestellt und mit $Et_2O$ (3 × 30 ml) extrahiert. Die vereinigten organischen Extrakte wurden über Natriumsulfat getrocknet und eingeengt. Der ölige Rückstand (586 mg) wurde chromatographisch mit Methanol/Ammoniak (500 : 1) aufgereinigt. Das unpolarere Produkt war ein farbloses Öl (280 mg) und wurde in 2-Butanon (20 ml) gelöst mit 3,3N ethanolischer Salzsäure (0,76 ml) in das korrespondierende Dihydrochlorid überführt (weißer Feststoff; 273 mg; Smp. 205-207 °C).

Beispiel 77: N'-Adamantan-2-yl-N,N-dimethyl-1-thiophen-2-yl-cyclohexan-1,4-diamin Dihydrochlorid, unpolareres Diastereomer

In einem Gemisch aus THF (15 ml) und 1,2-Dichlorethan (5 ml) wurde 2-Adamantylamin (302 mg) und 4-Dimethylamino-4-phenylcyclohexanon (446 mg) unter Argon gelöst. Nach 15 min wurde die Mischung mit Natriumtriacetoxyborhydrid (600 mg) versetzt und 45 Stunden bei Raumtemperatur gerührt. Zur Aufarbeitung wurde eingeengt, der Rückstandes in 1M HCl (20 ml) und Diethylether (40 ml) aufgenommen, die Phasen getrennt und die wäßrige Phase mit Diethylether (2 × 30 ml) gewaschen. Die wäßrige Phase wurde mit 5M Natronlauge alkalisch gestellt und mit Diethylether extrahiert (3 × 30 ml). Nach dem Einengen der vereinigten organischen Extrakte wurde das erhaltene Rohprodukt mit Methanol chromatographisch getrennt. Das unpolarere Diastereoisomer (286 mg) wurde in 2-Butanon (15 ml) gelöst und mit 3,3N ethanolischer Salzsäure (0,606 ml) in das korrespondierende Dihydrochlorid überführt (weißer Feststoff; 300 mg; Smp. 266 °C).

Beispiel 78: 3-[2-(4-Dimethylamino-4-phenyl-cyclohexylamino)-ethyl]-1H-indol-5-ol Dihydrochlorid, unpolareres Diastereomer

Serotonin (405 mg) wurde in 1,2-Dichlorethan/THF (5 ml/20 ml) gelöst, mit 4-Dimethylamino-4-phenylcyclohexanon (500 mg), Eisessig (131 μl) und geglühtem Natriumsulfat (2 g) versetzt. Nach einstündigem Rühren bei RT wurde Natriumtriacetoxyborhydrid (759 mg) zugesetzt und weitere zwei Tage gerührt. Zur Aufarbeitung wurde eingeengt, der Rückstand in Diethylether (15 ml), Wasser (10 ml) und 2M HCl (1 ml) suspendiert, weiterer Diethylether (20 ml) zugegeben und die organische Phase grob abgetrennt. Die wäßrige Phase wurde mit 1 M NaOH zunächst auf pH 9 gebracht und mit Ethylacetat (3 × 5 ml) extrahiert, dann auf pH 11 eingestellt und erneut mit Ethylacetat (5×10 ml) extrahiert. Die organischen Extrakte wurden getrocknet, eingeengt und durch Flashchromatographie (Eluent: MeOH/$NEt_3$ 99,5 : 0,5) gereinigt. Es wurden 267 mg des unpolareren Diastereoisomers (Smp. 90-100 °C) isoliert, die, in Ethanol/2-Butanon (3

ml/15 ml) gelöst, mit 3,3M ethanolischer HCl (642 µl) in das korrespondierende Dihydrochlorid überführt wurden (weißer Feststoff; 304 mg; Smp. 215-217 °C).

Beispiel 79: 3-[2-(4-Dimethylamino-4-phenyl-cyclohexylamino)-ethyl]-1H-indol-5-ol Dihydrochlorid, polareres Diastereomer

Wie für Beispiel 78 beschrieben wurden auch 124 mg des polareren Diastereoisomer (Smp. 185-187 °C) erhalten und, gelöst in Ethanol/2-Butanon (6 ml/15 ml), mit 3,3N ethanolischer HCl (298 µl) in das korrespondierende Dihydrochlorid überführt (weißer Feststoff; 123 mg; Smp. 230-233 °C).

Beispiel 80: N'-[2-(5-Methoxy-1H-indol-3-yl)ethyl]-N,N-dimethyl-1-phenyl-cyclohexan-1,4-diamin Dihydrochlorid, unpolareres Diastereomer

6-Methoxytryptamin (495 mg) wurde unter Argon in trockenem 1,2-Dichlorethan und THF (5 ml/15 ml) klar gelöst. Nach Zugabe von 4-Dimethylamino-4-phenylcyclohexanon (565 mg) und Eisessig (148 µl) wurde 2 h bei RT gerührt, bevor Natriumtriacetoxyborhydrid (858 mg) zugesetzt wurde. Die Reaktionsmischung wurde zwei Tage bei RT gerührt. Zur Aufarbeitung wurden dem Reaktionsgemisch Wasser (15 ml) und 5,5M HCl (1,5 ml) zugesetzt. Die Phasen wurden getrennt, die wäßrig Phase (pH 3) wurde mit Diethylether (3 × 10 ml) gewaschen, und dann mit 1M NaOH auf pH 11 gebracht und mit Ethylacetat (5 × 15 ml) extrahiert. Die vereinigten Extrakte wurden mit $Na_2SO_4$ getrocknet und eingeengt. Das verbliebene Rückstand (1,0 g; Smp. 129-153 °C) wurde durch Flashchromatographie (Eluent: MeOH/$NEt_3$ 99,25 : 0,75 aufgereinigt. Das unpolarere Diastereoisomer (550 mg, Smp. 164-169 °C) wurde sauber abgetrennt, in 2-Butanon/Aceton (15 ml/16 ml) warm gelöst und mit Chlortrimethylsilan (533 µl) in das korrespondierende Dihydrochlorid überführt (weißer Feststoff; 633 mg ; Smp. 165-175 °C).

Beispiel 81: N'-[2-(5-Methoxy-1H-indol-3-yl)-ethyl]-N,N-dimethyl-1-phenyl-cyclohexan-1,4-diamin Dihydrochlorid, polareres Diastereomer

Wie für Beispiel 80 beschrieben wurde auch das polarere Diastereoisomer (320 mg; Smp. 136-140 °C) erhalten, in 2-Butanon/Aceton (15 ml/3 ml) gelöst und mit Chlortrimethylsilan (310 µl) in das korrespondierende Dihydrochlorid überführt (weißer Feststoff; 362 mg; Smp. 206-210 °C).

Beispiel 82: N,N-Dimethyl-N'-[2-(5-methyl-1 H-indol-3-yl)-ethyl]-1-phenyl-cyclohexan-1,4-diamin Dihydrochlorid, unpolareres Diastereomer

5-Methyltryptamin (348 mg) und 4-Dimethylamino-4-phenylcyclohexanon (435 mg) wurden unter Ausschluß von Sauerstoff in trockenem 1,2-Dichlorethan (5 ml) und Tetrahydrofuran (15 ml) gelöst. Zu diesem Gemisch wurde Eisessig (114µl) und Natriumtriacetoxyborhydrid (600 mg) hinzugefügt und 24 Stunden bei RT gerührt. Zur Aufarbeitung wurde eingeengt, der Rückstand mit 1 M HCl (20 ml) und Diethylether (40 ml) aufgenommen, die Phasen getrennt, die wäßrige Phase mit Diethylether (2 × 20 ml) extrahiert und mit 5M NaOH auf pH 11 eingestellt. Die wäßrige Phase wurde mit Wasser (10 ml) verdünnt und mit Ethylacetat (3 × 20 ml) extrahiert. Die vereinigten organischen Extrakte wurden mit $Na_2SO_4$ getrocknet und eingeengt. Der Rückstand wurde durch Chromatographie mit MeOH/$NH_3$ (500 : 1) aufgereinigt. Das unpolarere Diastereoisomer (braunes Öl, 379 mg) wurde in 2-Butanon (10 ml) gelöst und mit Chlortrimethylsilan (319 µl) versetzt in das korrespondierende Dihydrochlorid überführt (weißer Feststoff; 405 mg; Smp. 234-236 °C).

Beispiel 83: N,N-Dimethyl-N'-[2-(5-methyl-1 H-indol-3-yl)-ethyl]-1-phenyl-cyclohexan-1,4-diamin Dihydrochlorid, polareres Diastereomer

Wie für Beispiel 82 beschrieben wurde auch das polarere Diastereoisomer (266 mg) in 2-Butanon (10 ml) gelöst und mit $Me_3SiCl$ (224 µl, 1,76 mmol) in das korrespondierende Dihydrochlorid überführt (weißer Feststoff; 272 mg; Smp. 248-250 °C).

Beispiel 84: Dimethyl-[1-phenyl-4-(1,3,4,9-tetrahydro-b-carbolin-2-yl)-cyclohexyl]-amin Dihydrochlorid

2,3,4,9-Tetrahydro-1H-β-carbolin (345 mg) und 4-Dimethylamino-4-phenylcyclo-hexanon (435 mg) wurden unter Argon in einem Gemisch aus THF (10 ml) und 1,2-Dichlorethan (15 ml) gelöst und mit Essigsäure (120 mg, 2 mmol) versetzt. Nach 15 min wurde $NaBH(OAc)_3$ (600 mg) hinzugefügt, 68 Stunden gerührt, die Reaktionsmischung eingeengt, der Rückstand in 1N Salzsäure (20 ml) aufgenommen und mit Ether (2 × 20 ml) gewaschen. Die wäßrige

Lösung wurde mit 1 M NaOH (30 ml) alkalisch gestellt und mit Ether (3 × 30 ml) extrahiert. Nach Trocknen und Einengen der vereinigten Extrakte wurde ein halbfestes Rohprodukt erhalten, das nach säulenchromatographischer Trennung mit Methanol/$NH_3$ (500 : 3) das unpolarere Diastereoisomer (334 mg, Smp. 147-150 °C) lieferte, das in 2-Buatnon (20 ml) und Ethanol (10 ml) unter Erwärmen gelöst mit 3,3M ethanolischer Salzsäure (0,8 ml) in das korrespondierende Dihydrochlorid überführt wurde (335 mg; Smp. 264-269°C).

Beispiel 85: N-(4-Dimethylamino-4-phenyl-cyclohexyl)-N-[2-(4-fluor-phenyl)-ethyl]-acetamid Hydrochlorid, unpolareres Diastereomer

4-(Fluorphenyl)ethylamin (1,15 g) und 4-Dimethylamino-4-phenylcyclohexanon (1,8 g) wurden unter Ausschluß von Sauerstoff in trockenem 1,2-Dichlorethan (20 ml) und Tetrahydrofuran (60 ml) gelöst. Zu diesem Gemisch wurde Eisessig (8,28 mmol) und Natriumtriacetoxyborhydrid (2,48 g, 11,59 mmol) hinzugefügt und 24 Stunden bei RT gerührt. Zur Aufarbeitung wurde eingeengt, der Ansatz mit 1M HCl (20 ml) und Diethylether (40 ml) versetzt, die Phasen getrennt, die wäßrige Phase mit Diethylether (2 × 20 ml) extrahiert und mit 5N NaOH auf pH 11 eingestellt. Die wäßrige Phase wurde mit Wasser (10 ml) verdünnt und mit Ethylacetat (3 × 20 ml) extrahiert. Die vereinigten organischen Extrakte wurden über $Na_2SO_4$ getrocknet, eingeengt und der Rückstand durch Chromatographie an Kieselgel mit Methanol aufgereinigt. Das unpolarere Diastereoisomer (531 mg, 1,55 mmol) wurde in wasserfreiem Pyridin (10 ml) gelöst und unter Rühren mit Acetanhydrid (1,59 g, 15,59 mmol) versetzt. Nach 24 Stunden wurde das Reaktionsgemisch mit einigen Eisstücken versetzt und am Rotationsverdampfer so weit wie möglich eingeengt. Der Rückstand wurde mit 1M NaOH (20 ml) versetzt. Die wäßrige Phase wurde mit Ethylacetat (3 × 30 ml) extrahiert, die vereinigten organischen Extrakte mit $Na_2SO_4$ getrocknet und eingeengt. Das erhaltene Acetamid (545 mg) wurde in 2-Butanon (10 ml) gelöst und mit Chlortrimethylsilan (0,270 ml) in das korrespondierende Hydrochlorid überführt (weiße Feststoff; 302 mg; Smp. 196-201 °C).

Beispiel 86: 2-(4-Dimethylamino-4-phenyl-cyclohexylamino)-3-(5-fluor-1H-indol-3-yl)-propionsäure-methylester Dihydrochlorid, unpolareres Diastereomer

rac-5-Fluortryptophan-methylester (1030 mg) in 1,2-Dichlorethan (ca. 40 ml) wurde unter Argon mit 4-Dimethylamino-4-phenylcyclo-hexanon (935 mg), Natriumsulfat (4 g) und Eisessig (245 µl, 4,4 mmol) versetzt. Nach einstündigem Rühren bei RT wurde Natriumtriacetoxyborhydrid (1,4 g, 6,5 mmol) zugesetzt. Das Gemisch wurde bei RT drei Tage gerührt. Zur Aufarbeitung wurde eingeengt, der Rückstand in Ethylacetat (40 ml) und 1 N NaOH (35 ml) aufgenommen, die Phasen getrennt und die wäßrige Phase wurde dreimal mit Ethylacetat (je 10 ml) extrahiert. Die vereinigten Extrakte getrocknet, eingeengt und der erhaltene Rückstand (1,73 g) durch Flashchromatographie (Eluent: MeOH/EtOAc 1 : 3) aufgereinigt. Das erhaltene unpolarere Diastereoisomer (911 mg, Smp. 55-62 °C) wurde 2-Butanon/Aceton (7 ml/ 1 ml) gelöst und mit Chlortrimethylsilan (174 µ) in das korrespondierende Dihydrochlorid überführt (beiger Feststoff; 135 mg; Smp. 172-182 °C).

Beispiel 87: N-(4-Dimethylamino-4-phenyl-cyclohexyl)-N-(3-phenyl-propyl)-acetamid Hydrochlorid, unpolareres Diastereomer

3-Phenylpropylamin (676 mg) und 4-Dimethylamino-4-phenylcyclohexanon (1,086 g) wurden unter Ausschluß von Sauerstoff in trockenem 1,2-Dichlorethan (5 ml) und Tetrahydrofuran (15 ml) gelöst. Zu diesem Gemisch wurde Eisessig (5 mmol) und Natriumtriacetoxyborhydrid (1,5 g, 7 mmol) hinzugefügt und 24 Stunden bei RT gerührt. Zur Aufarbeitung wurde eingeengt und der Ansatz mit 1M HCl (20 ml) und Diethylether (40 ml) versetzt. Die wäßrige Phase wurde mit Diethylether (2 × 20 ml) gewaschen, abgetrennt, mit 5N NaOH auf pH 11 eingestellt, mit Wasser (10 ml) verdünnt und mit Ethylacetat (3 × 20 ml) extrahiert. Die vereinigten organischen Extrakte wurden mit $Na_2SO_4$ getrocknet und eingeengt. Das erhaltene Rohprodukt wurde durch Chromatographie an Kieselgel mit Methanol aufgereinigt. Es wurden 761 mg des unpolareren Diastereoisomer erhaltenen.
453 mg wurden in wasserfreiem Pyridin (10 ml) gelöst und unter Rühren mit Acetanhydrid (1,374 g) versetzt. Nach 24 Stunden Rühren bei RT wurde mit einigen Eisstücken versetzt und am Rotationsverdampfer so weit wie möglich eingeengt. Der Rückstand wurde mit 1 N NaOH (20 ml) versetzt und mit Ethylacetat (3 × 30 ml) extrahiert. Die vereinigten organischen Extrakte wurden über $Na_2SO_4$ getrocknet und eingeengt. Das erhaltenen Acetamid (528 mg) in 2-Butanon (10 ml) gelöst und mit Chlortrimethylsilan (0,353 ml) in das korrespondierende Hydrochlorid überführt (weißer Feststoff; 282 mg; Smp. 206-211 °C).

Beispiel 88: 2-(4-Dimethylamino-4-phenyl-cyclohexylamino)-3-(6-fluor-1H-indol-3-yl)-propionsäure-methylester Dihydrochlorid, unpolareres Diastereomer

rac-6-Fluortryptophan-methylester (952 mg) in 1,2-Dichlorethan (ca. 30 ml) wurde unter Argon mit 4-Dimethylamino-4-phenylcyclohexanon (877 mg), Natriumsulfat (2 g) und Eisessig (230 μl, 4 mmol) versetzt. Nach einstündigem Rühren bei RT wurde Natriumtriacetoxyborhydrid (1,33 g, 6 mmol) zugesetzt und bei RT zwei Tage gerührt. Zur Aufarbeitung wurde eingeengt, der Rückstand in Ethylacetat (30 ml) und 1M NaOH (25 ml) gelöst, die klaren Phasen wurden im Scheidetrichter getrennt, die wäßrige Phase wurde dreimal mit Ethylacetat (je 10 ml) extrahiert und die vereinigten Extrakte getrocknet und eingedampft. Der erhaltene Rückstand (1,72 g) wurde durch Flashchromatographie (Eluent: MeOH/EtOAc 1:2, dann MeOH/EtOAc 1:1 und MeOH/$NH_3$ 400:1). Das unpolarere Diastereoisomer (868 mg.) wurde zum Teil (261 mg) wurde in 2-Butanon (7 ml) gelöst und mit Chlortrimethylsilan (227 μl) das korrespondierende Dihydrochlorid gefällt (weißer Feststoff; 224 mg; Smp. 164-169 °C).

Beispiel 89: N-(4-Dimethylamino-4-phenyl-cyclohexyl)-2-(1 H-indol-3-yl)-acetamid Hydrochlorid, polareres Diastereomer

4-Dimethylamino-4-phenylcyclohexanon (10 g) und Hydroxylamin-Hydrochlorid (4,8 g) wurden in absolutem Ethanol (120 ml) gelöst, die Lösung mit basischem Ionenaustauscher Amberlyst A 21 (30,7 g) versetzt und über Nacht bei RT gerührt. Der Ionenaustauscher wurde abfiltriert und mit Ethanol (3 × 50 ml) auf der Fritte gewaschen. Das Ethanol wurde im Vakuum abgezogen, der Rückstand mit 5M NaOH auf pH 11 eingestellt, mit Wasser verdünnt und mit Ethylacetat (4 × 30 ml) extrahiert. Die vereinigten Extrakte wurden mit $Na_2SO_4$ getrocknet und eingeengt. Es wurden 11 g 4-Dimethylamino-4-phenyl-cyclohexanon Oxim erhalten.

4-Dimethylamino-4-phenyl-cyctohexanon Oxim (11 g) wurde in Methanol (200 ml) gelöst und mit 5M NaOH (200 ml) verdünnt. Zu diesem Gemisch wurde portionsweise Devarda-Legierung (30 g) gegeben. Die Reaktionstemperatur lag dabei zwischen 50-60 °C. 15 min nach beendeter Zugabe wurde mit Wasser (150 ml) verdünnt, Methanol i. Vak. abgezogen und die wäßrige Lösung mit Ether (5 × 50 ml) extrahiert. Die vereinigten Extrakte wurden über $Na_2SO_4$ getrocknet und eingeengt. N,N-Dimethyl-1-phenylcyclohexan-1,4-diamin wurde als gelbes Öl erhalten (10,0 g).

Zu einer Lösung von Indol-3-ylessigsäure (257 mg) in abs. THF (10 ml) wurde N-Methylmorpholin (235μl, 2,1 mmol) und 2-Chlor-4,6-dimethoxy-1,3,5-triazin (371 mg, 2,11 mmol) gegeben. Anschließend wurde eine Stunde bei RT gerührt. Danach wurde dem Ansatz das polarere Diastereoisomer von N,N-Dimethyl-1-phenylcyclohexan-1,4-diamin (320 mg) zugesetzt und 12 Stunden bei RT gerührt. Zur Aufarbeitung wurde eingeengt, der Ansatz mit 5M NaOH auf pH 11 eingestellt, die Phasen getrennt, die wäßrige Phase mit Wasser (10 ml) verdünnt und mit Ethylacetat (3 × 20 ml) extrahiert. Die vereinigten organischen Extrakte wurden mit $Na_2SO_4$ getrocknet und eingeengt. Das erhaltene Amid wurde durch Säulenchromatographie mit Ethylacetat/Ethanol (1 : 1) gereinigt und (120 mg), in 2-Butanon (3 ml) gelöst und mit Chlortrimethylsilan (61 μl) in das korrespondierende Hydrochlorid überführt (weißer Feststoff; 128 mg; Smp. 100-102 °C).

Beispiel 90: 2-(4-Dimethylamino-4-thiophen-2-yl-cyclohexylamino)-3-(1 H-indol-3-yl)-propionsäure-methylester Dihydrochlorid, unpolareres Diastereomer

Das Hydrochlorid von L-Tryptophan-methylesters (1,01 g) wurde mit 1,2-Dichlorethan (20 ml) und gesättigter $NaHCO_3$-Lösung (20 ml) 15 min kräftig gerührt und die wäßrige Phase sofort mit 1,2-Dichlorethan (2 × 20 ml) extrahiert. Nach dem Trocknen mit $Na_2SO_4$ wurde die organische Phase auf 40 ml eingeengt und unter Argon mit 4-Dimethylamino-4-phenylcyclohexanon (893 mg, 4 mmol) versetzt. Zur klaren Lösung wurde Eisessig (0,228 ml, 4 mmol) und $Na_2SO_4$ (2 g) hinzugefügt. Nach einer Reaktionszeit von 15 min wurde die Reaktionsmischung mit $NaBH(OAc)_3$ (1,2 g) versetzt und 4 Tage bei Raumtemperatur gerührt. Zur Aufarbeitung mit gesättigter $NaHCO_3$-Lösung (40 ml) versetzt und 15 min gerührt. Die wäßrige Phase wurde mit Dichlormethan (2 × 20 ml) extrahiert. Die vereinigten organischen Phasen wurden nach dem Trocknen eingeengt, wobei ein hellbraunes Öl erhalten wurde. Die säulenchromatographische Aufreinigung erfolgte mit Ethylacetat und Methanol. Das unpolarere Diastereoisomer (918 mg; Smp. 108-112 °C) wurde 2-Butanon (15 ml) gelöst und mit Chlortrimethylsilan (0,4 ml) in das korrespondierende Dihydrochlorid überführt (weißer Feststoff; 326 mg; Smp. 197-202 °C).

Beispiel 91: N-(4-Dimethylamino-4-phenyl-cyclohexyl-2-(5-methoxy-1 H-indol-3-yl)-acetamid Hydrochlorid, unpolareres Diastereomer

Zu einer Lösung von (5-Methoxy-1H-indol-3-yl)essigsäure (364 mg) in abs. Methanol (20 ml) wurden das unpolarere Diastereoisomer von N,N-Dimethyl-1-phenyl-cyclohexan-1,4-diamin (387 mg) und 4-(4,6-Dimethoxy-1,3,5-triazin-2-yl)-4-methyl-morpholiniumchlorid (267 mg, 2,0 mmol) gegeben. Anschließend wurde 24 Stunden bei RT gerührt.

Zur Aufarbeitung wurde eingeengt, der Ansatz mit Wasser (10 m) verdünnt, das Gemisch mit 5M NaOH auf pH 11 eingestellt und mit Ethylacetat (3 × 20 ml) extrahiert. Die vereinigten organischen Extrakte wurden über $Na_2SO_4$ getrocknet und eingedampft. Nach Säulenchromatographie mit MeOH wurde das unpolarere Amid (154 mg; farbloses Öl) in 2-Butanon (5 ml) gelöst und mit Chlortrimethylsilan (72 µl) in das korrespondierende Hydrochlorid überführt (weißer Feststoff; 168 mg; Smp. 143-145 °C).

**Beispiel 92:**

**Messung der ORL1-Bindung**

Die Cyclohexan-1,4-diaminderivate der allgemeinen Formel wurden in einem Rezeptorbindungsassay mit $^3$H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^3$H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM $MgCl_2$ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei Raumtemperatur und anschließende Messung im Szintillationscounter Trilux (Wallac, Finnland), bestimmt. Die Affinität wird als $K_i$-Wert in µM angegeben.

| Beispiel | ORL1 Ki/µM |
|---|---|
| 1 | 0,010 |
| 2 | 0,050 |
| 3 | 0,50 |
| 4 | 0,30 |
| 5 | 0,14 |
| 6 | 0,040 |
| 7 | 0,0030 |
| 8 | 0,28 |
| 9 | 0,34 |
| 10 | 0,12 |
| 11 | 0,38 |
| 12 | 0,25 |
| 13 | 0,22 |
| 14 | 0,10 |
| 15 | 0,093 |
| 16 | 0,066 |
| 17 | 0,010 |
| 18 | 0,027 |
| 19 | 0,0051 |
| 20 | 0,0054 |
| 21 | 0,0099 |
| 22 | 0,0060 |
| 23 | 0,250 |
| 24 | 0,0011 |
| 25 | 0,0020 |
| 26 | 0,210 |

(fortgesetzt)

| Beispiel | ORL1 Ki/μM |
|---|---|
| 27 | 0,017 |
| 28 | 0,039 |
| 29 | 0,19 |
| 30 | 0,49 |
| 31 | 0,051 |
| 32 | 0,0069 |
| 33 | 0,057 |
| 34 | 0,0084 |
| 35 | 0,45 |
| 36 | 0,54 |
| 37 | 0,0090 |
| 38 | 0,60 |
| 39 | 0,10 |
| 40 | 0,26 |
| 41 | 0,29 |
| 42 | 0,0013 |
| 43 | 0,042 |
| 44 | 0,066 |
| 45 | 0,63 |
| 46 | 0,75 |
| 47 | 0,045 |
| 48 | 0,030 |
| 49 | 0,0026 |
| 50 | 0,039 |
| 51 | 0,0033 |
| 52 | 0,15 |
| 53 | 0,33 |
| 54 | 0,42 |
| 55 | 0,45 |
| 56 | 0,75 |
| 57 | 0,0015 |
| 58 | 0,25 |
| 59 | 0,18 |
| 60 | 0,0090 |
| 61 | 0,090 |
| 62 | 0,39 |
| 63 | 0,051 |
| 64 | 0,0036 |

(fortgesetzt)

| Beispiel | ORL1 Ki/µM |
|---|---|
| 65 | 0,17 |
| 66 | 0,0033 |
| 67 | 0,051 |
| 68 | 0,017 |
| 69 | 0,18 |
| 70 | 0,0004 |
| 71 | 0,18 |
| 72 | 0,019 |
| 73 | 0,0037 |
| 74 | 0,013 |
| 75 | 0,10 |
| 76 | 0,020 |
| 77 | 0,0063 |
| 78 | 0,0092 |
| 79 | 0,095 |
| 80 | 0,023 |
| 81 | 0,55 |
| 82 | 0,0015 |
| 83 | 0,095 |
| 84 | 0,043 |
| 85 | 0,002 |
| 86 | 0,004 |
| 87 | 0,023 |
| 89 | 0,014 |
| 90 | 0,025 |
| 90 | 0,004 |
| 91 | 0,006 |

**Messung der ORL1-Bindung**

Die Cyclohexan-1,4-diaminderivate der allgemeinen Formel I wurden in einem Rezeptorbindungsassay mit $^{3}$H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^{3}$H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM $MgCl_2$ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei Raumtemperatur und anschliessende Messung im Szintillationscounter Trilux (Wallac, Finnland), bestimmt. Die Affinität wird als $K_i$-Wert angegeben.

**Beispiel 93:**

**Analgesieprüfung im Tail-Flick-Test an der Maus**

Die Mäuse wurden jeweils einzeln in einen Testkäfig gesetzt und die Schwanzbasis dem fokussierten Wärmestrahl einer elektrischen Lampe (Tail-flick-Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Mäusen 3 bis 5 Sekunden betrug. Vor der Applikation der Lösungen enthaltend die erfindungsgemäße Verbindung bzw. der jeweiligen Vergleichslösungen wurden die Mäuse innerhalb von fünf Minuten zweimal vorgetestet und der Mittelwert dieser Messungen als Vortestmittelwert berechnet.

Die Lösungen der erfindungsgemäßen Verbindung der allgemeinen Formel I sowie die Vergleichslösungen wurden dann intravenös appliziert. Die Schmerzmessung wurde jeweils 10, 20, 40 und 60 Minuten nach der intravenösen Applikation durchgeführt. Die analgetische Wirkung wurde als Zunahme der Schmerzlatenz (% des maximal möglichen antinociceptiven Effektes) nach der folgenden Formel bestimmt:

$$[(T_1 - T_0)/(T_2 - T_0)] \times 100$$

Hierbei ist die Zeit $T_0$ die Latenzzeit vor der Applikation, die Zeit $T_1$ die Latenzzeit nach der Applikation der Wirkstoffkombination und die Zeit $T_2$ die maximale Expositionsdauer (12 Sekunden).

Die vertiefte Untersuchung auf analgetische Wirksamkeit wurde im Tail-Flick-Test an der Maus durchgeführt, wie obenstehend beschrieben.

Die untersuchten erfindungsgemäßen Verbindungen zeigten eine analgetische Wirkung. Die Ergebnisse ausgewählter Untersuchungen sind ebenfalls in der nachfolgenden Tabelle zusammengefaßt.

**Tabelle:**

| Beispiel Nr. | Antinociceptive Wirkung prozentual zur Kontrollgruppe* |
|---|---|
| | |
| 1 | 98 (10) |
| 3 | 40 (10) |
| 4 | 44 (10) |
| 7 | 100 (1) |
| 12 | 47 (10) |
| 27 | 49 (2,15) |
| 60 | 100(1) |
| 85 | 91 (1) |
| 86 | 100(1) |
| 88 | 100 (10) |
| 89 | 37(1) |
| 90 | 94 (1) |
| 91 | 100 (1) |
| *: In Klammern ist jeweils die Dosierung in mg/kg bei intravenöser Applikation angegeben. | |

Die untersuchten beispielgemäßen Verbindungen zeigen eine gute analgetische Wirksamkeit.

**Beispiel 94:**

**Parenterale Lösung eines erfindungsgemäßen substituierten Cyclohexan-1,4-diaminderivats**

38 g eines der erfindungsgemäßen substituierten Cyclohexan-1,4-diaminderivate, hier gemäß Beispiel 91, wird in 1 l Wasser für Injektionszwecke bei Raumtemperatur gelöst und anschließend durch Zugabe von wasserfreier Glukose für Injektionszwecke auf isotone Bedingungen eingestellt.

## Patentansprüche

**1.** Substituierte Cyclohexan-1,4-diaminderivate der allgemeinen Formel I,

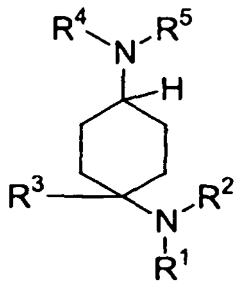

I

, worin
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C_{1-8}$-Alkyl, gesättigt oder ungesättigt, verzweigt oder unverzweigt, einfach oder mehrfach substituiert oder unsubstituiert; oder $C(X)R^7$, $C(X)NR^7R^8$, $C(X)OR^9$, $C(X)SR^9$, $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach

substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt ist aus $C_{3-8}$-Cycloalkyl, Aryl oder Heteroaryl, jeweils unsubstituiert oder einfach oder mehrfach substituiert; -CHR11$R^{12}$, - $CHR^{11}$- $CH_2R^{12}$, -$CHR^{11}$-$CH_2$-$CH_2R^{12}$, -$CHR^{11}$-$CH_2$-$CH_2$-$CH_2R^{12}$, - C(Y)$R^{12}$, -C (Y)-$CH_2R^{12}$, -C(Y)-$CH_2$-$CH_2R^{12}$ oder -C(Y)-$CH_2$-$CH_2$-$CH_2R^{12}$
mit Y = O, S oder $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder C(O)O-$C_{1-6}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann,
mit der Maßgabe,

- daß, wenn $R^3$ substituiertes oder unsubstituiertes Phenyl ist und mindestens einer von $R^1$ oder $R^2$ H oder $C_{1-8}$-Alkyl ist, $R^4$ nicht Alkyl sein darf und $R^4$ und $R^5$ nicht zusammen einen Heterocyclus bilden dürfen oder
- daß, wenn $R^3$ unsubstituiertes Phenyl ist und $R^1$ und $R^2$ zusammen $(CH_2)_5$ bedeuten, $R^4$ ausgewählt ist aus H oder $C_{1-8}$-Alkyl, Y nicht O oder S bedeutet und $R^5$ nicht $C_{1-6}$-Alkyl ist,

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

**2.** Substituierte Cyclohexan-1,4-diaminderivate gemäß Anspruch 1,
, worin
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, C(X)$R^7$, C(X)$NR^7R^8$, C(X)$OR^9$ oder C(X)$SR^9$, S($O_2$)$R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder

unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$,$-CHR^{11}-$ $CH_2R^{12}$, $-CHR^{11}-CH_2-CH_2R^{12}$, $-CHR^{11}-CH_2-CH_2-CH_2R^{12}$, - $C(Y)R^{12}$, -C (Y)-$CH_2R^{12}$, -C(Y)-$CH_2$-$CH_2R^{12}$ oder -C(Y)-$CH_2$-$CH_2$-$CH_2R^{12}$
mit Y = O, S oder $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder C(O)O-$C_{1-6}$-Alkyl, gesättigt oder ungesättigt, verzweigt oder unverzweigt, einfach oder mehrfach substituiert oder unsubstituiert;
und mit $R^{12}$ ausgewählt aus
$C_{3-8}$-Cycloalkyl, Aryl oder Heteroaryl, jeweils unsubstituiert oder einfach oder mehrfach substituiert,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

**3.** Substituierte Cyclohexan-1,4-diaminderivate gemäß Anspruch 1,
, worin
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C(X)R^7$, $C(X)NR^7R^8$, $C(X)OR^9$ oder $C(X)SR^9$, $S(O_2)R^9$
mit X=O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
oder die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils

einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$,$-CHR^{11}$- $CH_2R^{12}$, $-CHR^{11}-CH_2-CH_2R^{12}$, $-CHR^{11}-CH_2-CH_2-CH_2R^{12}$, - $C(Y)R^{12}$, -C (Y)-$CH_2R^{12}$, -C(Y)-$CH_2$-$CH_2R^{12}$ oder-C(Y)-$CH_2CH_2$-$CH_2R^{12}$
mit Y = O, S oder $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder C(O)O-$C_{1-6}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhättnis;
in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

**4.** Substituierte Cyclohexan-1,4-diaminderivate gemäß Anspruch 1,
, worin
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Heteroaryl, unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$, $C(X)OR^9$, $C(X)SR^9$, $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
oder die Reste $R^7$ und $R^8$ zusammen einen Ring bilden und $CH_2CH_2OCH_2CH_2$, $CH_2CH_2NR^{10}OCH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt,

einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$,-CHR"- $CH_2R^{12}$, $-CHR^{11}-CH_2-CH_2R^{12}$, $-CHR^{11}-CH_2-CH_2-CH_2R^{12}$,$-C(Y)R^{12}$, -C (Y)-$CH_2R^{12}$, -C(Y)$CH_2$-$CH_2R^{12}$ oder-C(Y)-$CH_2$-$CH_2$-$CH_2R^{12}$

mit Y = O, S oder $H_2$,

mit $R^{11}$ ausgewählt aus

H, $C_{1-7}$-Alkyl, gesättigt oder ungesättigt, verzweigt oder unverzweigt, einfach oder mehrfach substituiert oder unsubstituiert; oder C(O)O-$C_{1-6}$-Alkyl, gesättigt oder ungesättigt, verzweigt oder unverzweigt, einfach oder mehrfach substituiert oder unsubstituiert;

und mit $R^{12}$ ausgewählt aus

H; $C_{3-8}$-Cycloalkyl, Aryl oder Heteroaryl, jeweils unsubstituiert oder einfach oder mehrfach substituiert,

oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann;

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;

in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

**5.** Substituierte Cyclohexan-1,4-diaminderivate gemäß Anspruch 1,

, worin

die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$, $C(X)OR^9$, $C(X)SR^9$, $S(O_2)R^9$

mit X = O oder S,

mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

mit $R^8$ ausgewählt aus H, $C_{1-4}$-Alkyl, gesättigt oder ungesättigt, verzweigt oder unverzweigt, einfach oder mehrfach substituiert oder unsubstituiert oder

die Reste $R^7$ und $R^8$ zusammen einen Ring bilden und $CH_2CH_2OCH_2CH_2$, $CH_2CH_2NR^{10}$ $CH_2CH_2$ oder $(CH_2)_{3-6}$ bedeuten,

mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$,$-CHR^{11}-CH_2R^{12}$, $-CHR^{11}-CH_2-CH_2R^{12}$, $-CHR^{11}-CH_2-CH_2-CH_2R^{12}$,$-C(Y)R^{12}$, $-C(Y)-CH_2R^{12}$, $-C(Y)-CH_2-CH_2R^{12}$ oder $-C(Y)-CH_2-CH_2-CH_2R^{12}$
mit Y = O, S oder $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder C(O)O-$C_{1-6}$-Alkyl, gesättigt oder ungesättigt, verzweigt oder unverzweigt, einfach oder mehrfach substituiert oder unsubstituiert;
und mit $R^{12}$ ausgewählt aus
$C_{3-8}$-Cycloalkyl, Aryl oder Heteroaryl, jeweils unsubstituiert oder einfach oder mehrfach substituiert,
oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann;
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

**6.** Substituierte Cyclohexan-1,4-diaminderivate gemäß einem der Ansprüche 1, 2 oder 4 **dadurch gekennzeichnet, daß**
$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
oder die Reste $R^1$ und $R^2$ zusammen einen Ring bilden und $(CH_2)_{4-5}$ bedeuten,
insbesondere
$R^1$ und $R^2$ unabhängig voneinander ausgewählt sind aus Methyl oder Ethyl oder die Reste $R^1$ und $R^2$ zusammen einen Ring bilden und $(CH_2)_5$ bedeuten.

**7.** Substituierte Cyclohexan-1,4-diaminderivate gemäß Anspruch 5, **dadurch gekennzeichnet, daß**
$R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ zusammen einen Ring bilden und $(CH_2)_{4-5}$ bedeuten,
insbesondere
$R^1$ und $R^2$ zusammen einen Ring bilden und $(CH_2)_5$ bedeuten.

**8.** Substituierte Cyclohexan-1,4-diaminderivate gemäß Anspruch 3, **dadurch gekennzeichnet, daß**
$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
vorzugsweise
$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
insbesondere
$R^1$ und $R^2$ unabhängig voneinander ausgewählt sind aus Methyl oder Ethyl.

**9.** Substituierte Cyclohexan-1,4-diaminderivate gemäß einem der Ansprüche 1 bis 3 oder 5, **dadurch gekennzeichnet, daß**
$R^3$ ausgewählt ist aus $C_{3-8}$-Cycloalkyl, Aryl oder Heteroaryl, jeweils unsubstituiert oder einfach oder mehrfach substituiert; über eine gesättigte oder ungesättigte, unverzweigte, substituierte oder unsubstituierte $C_{1-2}$-Alkyl-Grup-

pe gebundenem Aryl, $C_{3-8}$-Cycloalkyl oder Heteroaryl, jeweils unsubstituiert oder einfach oder mehrfach substituiert; vorzugsweise
$R^3$ ausgewählt ist aus $C_{5-6}$-Cycloalkyl, Phenyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; über eine gesättigte, unverzweigte $C_{1-2}$-Alkyl-Gruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Phenyl, Furyl, Thiophenyl, Cyclohexanyl, Naphthyl, Benzofuranyl, Indolyl, Indanyl, Benzodioxanyl, Benzodioxolanyl, Pyrrolyl, Pyrimidyl, Pyrazinyl oder Benzothiophenyl, jeweils unsubstituiert oder einfach oder mehrfach substituiert; über eine gesättigte, unverzweigte $C_1$-$_2$-Alkyl-Gruppe gebundenem Phenyl, Furyl oder Thiophenyl, jeweils unsubstituiert oder einfach oder mehrfach substituiert.

**10.** Substituierte Cyclohexan-1,4-diaminderivate gemäß Anspruch 4, **dadurch gekennzeichnet, daß**
$R^3$ ausgewählt ist aus $C_{3-8}$-Cycloalkyl oder Heteroaryl, jeweils unsubstituiert oder einfach oder mehrfach substituiert; über eine gesättigte oder ungesättigte, unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
vorzugsweise
$R^3$ ausgewählt ist aus $C_{5-6}$-Cycloalkyl, Thiophenyl, Benzothiophenyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; über eine gesättigte, unverzweigte $C_{1-2}$-AlkylGruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Furyl, Thiophenyl, Cyclohexanyl, Benzofuranyl, Indolyl, Indanyl, Benzodioxanyl, Benzodioxolanyl, Pyrrolyl, Pyrimidyl, Pyrazinyl oder Benzothiophenyl, jeweils unsubstituiert oder einfach oder mehrfach substituiert; über eine gesättigte, unverzweigte $C_{1-2}$-AlkylGruppe gebundenem Phenyl, Furyl oder Thiophenyl, jeweils unsubstituiert oder einfach oder mehrfach substituiert.

**11.** Substituierte Cyclohexan-1,4-diaminderivate gemäß einem der Ansprüche 1 bis 10, **dadurch gekennzeichnet, daß** $R^4$ H ist.

**12.** Substituierte Cyclohexan-1,4-diaminderivate gemäß einem der Ansprüche 1 bis 10, **dadurch gekennzeichnet, daß**
$R^4$ ausgewählt ist aus H, $C(X)R^7$, $C(X)NR^7R^8$, $C(X)OR^9$, $C(X)SR^9$ oder $S(O_2)R^9$ mit X = O oder S,
vorzugsweise
$R^4$ ausgewählt ist aus H, $C(X)R^7$, $C(X)NR^7R^8$ oder $C(X)OR^9$ mit X = O,
insbesondere
$R^4$ ausgewählt ist aus H oder $C(O)R^7$; vorzugsweise mit $R_7$ ausgewählt aus
H; oder $C_{1-8}$-Alkyl gesättigt oder ungesättigt, verzweigt oder unverzweigt, einfach oder mehrfach substituiert oder unsubstituiert;
vorzugsweise
H; oder $C_{1-3}$-Alkyl gesättigt, unsubstituiert, verzweigt oder unverzweigt;
insbesondere $CH_3$.

**13.** Substituierte Cyclohexan-1,4-diaminderivate gemäß einem der Ansprüche 1, 4 oder 5, **dadurch gekennzeichnet, daß**
$R^4$ und $R^5$ zusammen einen Heterocyclus mit zwischen 3 und 8 Atomen im Ring bilden, gesättigt oder ungesättigt; einfach oder mehrfach substituiert oder unsubstituiert, vorzugsweise mit zwischen 5 und 7 Atomen im Ring, wovon neben dem obligatorischen N 0 bis 1 weitere Heteroatome, ausgewählt aus N, S oder O, im Ring sind;
wobei der von $R^4$ und $R^5$ zusammen gebildete Heterocyclus gegebenenfalls mit weiteren Ringen kondensiert sein kann,
vorzugsweise mit aromatischen und/oder heteroaromatischen Ringen, wobei diese mit weiteren aromatischen und/oder heteroaromatischen Ringen kondensiert sein können,
insbesondere der von $R^4$ und $R^5$ zusammen gebildete Heterocyclus mit ein oder zwei weiteren Ringen kondensiert ist, vorzugsweise der von $R^4$ und $R^5$ zusammen gebildete Heterocyclus so mit zwei weiteren Ringen kondensiert ist, dass $R^4$ und $R^5$ zusammen

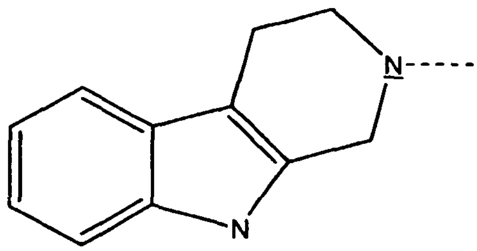

bedeuten.

**14.** Substituierte Cyclohexan-1,4-diaminderivate gemäß einem der Ansprüche 1, 4 oder 5, **dadurch gekennzeichnet, daß**
$R^4$ ausgewählt ist aus H oder $C_{1-8}$-Alkyl, gesättigt oder ungesättigt, verzweigt oder unverzweigt, einfach oder mehrfach substituiert oder unsubstituiert,
vorzugsweise
H oder $C_{1-6}$-Alkyl, gesättigt oder ungesättigt, verzweigt oder unverzweigt, einfach oder mehrfach substituiert oder unsubstituiert,
insbesondere
H oder $C_{1-3}$-Alkyl, gesättigt, unverzweigt und unsubstituiert.

**15.** Substituierte Cyclohexan-1,4-diaminderivate gemäß einem der Ansprüche 1 bis 14, **dadurch gekennzeichnet, daß**
$R^5$ ausgewählt ist aus $C_{3-8}$-Cycloalkyl, Aryl oder Heteroaryl, jeweils unsubstituiert oder einfach oder mehrfach substituiert;
vorzugsweise
$R^5$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^5$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

**16.** Substituierte Cyclohexan-1,4-diaminderivate gemäß einem der Ansprüche 1 bis 14, **dadurch gekennzeichnet, daß**
$R^5$ ausgewählt ist aus $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ oder $-C(Y)-CH_2-CH_2-CH_2R^{12}$
mit Y = O, S oder $H_2$,
vorzugsweise
$R^5$ ausgewählt ist aus $-CHR^{11}R^{12}$, $-CHR^{11}-CH_2R^{12}$, $-CHR^{11}-CH_2-CH_2R^{12}$, $-C(Y)R^{12}$, $-C(Y)-CH_2R^{12}$ oder $-C(Y)-CH_2CH_2R^{12}$
mit Y = O oder S,
insbesondere
$R^5$ ausgewählt ist aus $-CHR^{11}R^{12}$, $-CHR^{11}CH_2R^{12}$, $-CHR^{11}CH_2-CH_2R^{12}$, $-C(Y)R^{12}$ oder $-C(Y)-CH_2R^{12}$
mit Y = O.

**17.** Substituierte Cyclohexan-1,4-diaminderivate gemäß Anspruch 16, **dadurch gekennzeichnet, daß**
$R^{11}$ ausgewählt ist aus
H, $C_{1-4}$-Alkyl, gesättigt oder ungesättigt, verzweigt oder unverzweigt, einfach oder mehrfach substituiert oder unsubstituiert; oder $C(O)O-C_{1-4}Alkyl$, gesättigt oder ungesättigt, verzweigt oder unverzweigt, einfach oder mehrfach substituiert oder unsubstituiert;
vorzugsweise
H, $C_{1-4}$-Alkyl, gesättigt oder ungesättigt, verzweigt oder unverzweigt, einfach oder mehrfach substituiert oder unsubstituiert; oder C(O)O-$C_{1-2}$-Alkyl, gesättigt, unverzweigt, einfach oder mehrfach substituiert oder unsubstituiert unsubstituiert;
insbesondere
H, $CH_3$, $C_2H_5$ und C(O)O-$CH_3$.

**18.** Substituierte Cyclohexan-1,4-diaminderivate gemäß Anspruch 16, **dadurch gekennzeichnet, daß**
$R^{12}$ ausgewählt ist aus $C_{3-8}$-Cycloalkyl, Aryl oder Heteroaryl, jeweils unsubstituiert oder einfach oder mehrfach substituiert;
vorzugsweise
$R^{12}$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^{12}$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

**19.** Substituierte Cyclohexan-1,4-diaminderivate gemäß einem der Ansprüche 1 bis 18, **dadurch gekennzeichnet, daß** sie ausgewählt sind aus der folgenden Gruppe:

■ N'-Benzyl-N,N-dimethyl-1-phenyl-cyclohexan-1,4-diamin Hydrochlorid, unpolareres Diastereomer
■ N'-Benzyl-N,N-dimethyl-1-phenyl-cyclohexan-1,4-diamin Hydrochlorid, polareres Diastereomer
■ 1,N'-Dibenzyl-N,N-dimethyl-cyclohexan-1,4-diamin Hydrochlorid, unpolareres Diastereomer
■ 1,N'-Dibenzyl-N,N-dimethyl-cyclohexan-1,4-diamin Hydrochlorid, polareres Diastereomer
■ N-(4-Benzyl-4-dimethylamino-cyclohexyl)-N-propyl-benzamid Hydrochlorid
■ N,N-Dimethyl-1-phenyl-N'-propyl-cyclohexan-1,4-diamin Hydrochlorid, unpolareres Diastereomer
■ N-(4-Dimethylamino-4-phenyl-cyclohexyl)-N-propyl-benzamid Hydrochlorid unpolareres Diastereomer
■ N-(4-Dimethylamino-4-phenyl-cyclohexyl)-N-propyl-benzamid Hydrochlorid, polareres Diastereomer
■ 1,N'-Dibenzyl-N,N,N'-trimethyl-cyclohexan-1,4-diamin Hydrochlorid, unpolareres Diastereomer
■ 1,N'-Dibenzyl-N,N,N'-trimethyl-cyclohexan-1,4-diamin Hydrochlorid, polareres Diastereomer
■ N-(4-Benzyl-4-dimethylamino-cyclohexyl)-N-methyl-benzamid Hydrochlorid, polareres Diastereomer
■ N-(4-Benzyl-4-dimethylamino-cyclohexyl)-N-ethyl-benzamid Hydrochlorid, polareres Diastereomer
■ 1-Benzyl-N'-(1H-indol-3-ylmethyl)-N,N-dimethyl-cyclohexan-1,4-diamin Dihydrochlorid
■ 1-Benzyl-N'-[2-(1H-indol-3-yl)-1-methyl-ethyl]-N,N-dimethyl-cyclohexan-1,4-diamin, cis/trans-Gemisch
■ 1-Benzyl-N'-indan-5-yl-N,N-dimethyl-cyclohexan-1,4-diamin Hydrochlorid
■ 1-Benzyl-N'-indan-1-yl-N,N-dimethyl-cyclohexan-1,4-diamin Dihydrochlorid, cis/trans-Gemisch
■ N'-Indan-1-yl-N,N-dimethyl-1-phenyl-cyclohexan-1,4-diamin
■ N'-(1H-Indol-5-yl)-N,N-dimethyl-1-phenyl-cyclohexan-1,4-diamin
■ N'-(1H-Indol-3-ylmethyl)-N,N-dimethyl-1-phenyl-cyclohexan-1,4-diamin, cis/trans-Gemisch
■ N'-(1H-Indol-3-ylmethyl)-N,N-dimethyl-1-phenyl-cyclohexan-1,4-diamin, unpolareres Diastereomer
■ N'-[2-(1H-Indol-3-yl)-ethyl]-N,N-dimethyl-1-phenyl-cyclohexan-1,4-diamin, unpolareres Diastereomer
■ N'-[2-(1H-Indol-3-yl)-ethyl]-N,N-dimethyl-1-phenyl-cyclohexan-1 ,4-diamin, cis/trans-Gemisch
■ N'-Indan-5-yl-N,N-dimethyl-1-phenyl-cyclohexan-1,4-diamin, unpolareres Diastereomer
■ N'-[2-(1 H-Indol-3-yl)-1-methyl-ethyl]-N,N-dimethyl-1-phenyl-cyclohexan-1,4-diamin, unpolareres Diastereomer
■ N'-[2-(1 H-Indol-3-yl)-1-methyl-ethyl]-N,N-dimethyl-1-phenyl-cyclohexan-1,4-diamin, cis/trans-Gemisch
■ N'-[2-(5-Benzyloxy-1 H-indol-3-yl)-ethyl]-N,N-dimethyl-1-phenyl-cyclohexan-1,4-diamin, cis/trans-Gemisch
■ N'-(9H-Fluoren-1-yl)-N,N-dimethyl-1-phenyl-cyclohexan-1,4-diamin Dihydrochlorid
■ N'-Indan-2-yl-N,N-dimethyl-1-phenyl-cyclohexan-1,4-diamin Dihydrochlorid, cis/trans-Gemisch
■ N'-(9H-Fluoren-9-yl)-N,N-dimethyl-1-phenyl-cyclohexan-1,4-diamin Dihydrochlorid, cis/trans-Gemisch
■ 1-Benzyl-N'-(9H-fluoren-9-yl)-N,N-dimethyl-cyclohexan-1,4-diamin
■ 1-Benzyl-N'-(1 H-indol-3-ylmethyl)-N,N-dimethyl-cyclohexan-1,4-diamin, cis/trans-Gemisch
■ N,N-Dimethyl-N'-(1-methyl-1H-indol-3-ylmethyl)-1-phenyl-cyclohexan-1,4-diamin, cis/trans-Gemisch
■ N,N-Dimethyl-N'-(1-methyl-1H-indol-3-ylmethyl)-1-phenyl-cyclohexan-1,4-diamin, polareres Diastereomer
■ N'-(2-Benzo[b]thiophen-3-yl-ethyl)-N,N-dimethyl-1-phenyl-cyclohexan-1,4-diamin Dihydrochlorid, cis/trans-Gemisch

■ N'-(2-Benzo[b]thiophen-3-yl-ethyl)-1-benzyl-N,N-dimethyl-cyclohexan-1,4-diamin Dihydrochlorid, cis/trans-Gemisch

■ N'-Acenaphthen-1-yl-N,N-dimethyl-1-phenyl-cyclohexan-1,4-diamin Dihydrochlorid, polareres Diastereomer

■ N'-Acenaphthen-1-yl-N,N-dimethyl-1-phenyl-cyclohexan-1,4-diamin Dihydrochlorid, unpolareres Diastereomer

■ N'-Benzo[b]thiophen-5-yl-1-benzyl-N,N-dimethyl-cyclohexan-1,4-diamin Dihydrochlorid, unpolareres Diastereomer

■ N'-Benzo[b]thiophen-5-yl-N,N-dimethyl-1-phenyl-cyclohexan-1,4-diamin Hydrochlorid, unpolareres Diastereomer

■ N'-Benzothiazol-6-yl-N,N-dimethyl-1-phenyl-cyclohexan-1,4-diamin Dihydrochlorid, unpolareres Diastereomer

■ N'-Benzo[1,2,5]thiadiazol-4-yl-N,N-dimethyl-1-phenyl-cyclohexan-1,4-diamin Dihydrochlorid, polareres Diastereomer

■ N'-[2-(1H-Indol-3-yl)-1-methyl-ethyl]-N,N-dimethyl-1-phenyl-cyclohexan-1,4-diamin Dihydrochlorid, unpolareres Diastereomer

■ N'-Adamantan-2-yl-N,N-dimethyl-1-phenyl-cyclohexan-1,4-diamin Dihydrochlorid

■ N'-(9-Ethyl-9H-carbazol-3-yl)-N,N-dimethyl-1-phenyl-cyclohexan-1,4-diamin Dihydrochlorid, unpolareres Diastereomer

■ N'-(3H-Benzotriazol-5-yl)-N,N-dimethyl-1-phenyl-cyclohexan-1,4-diamin Hydrochlorid, unpolareres Diastereomer

■ N'-(3H-Benzotriazol-5-yl)-N,N-dimethyl-1-phenyl-cyclohexan-1,4-diamin Hydrochlorid, polareres Diastereomer

■ N'-(9H-Fluoren-9-yl)-N,N-dimethyl-1-thiophen-2-yl-cyclohexan-1,4-diamin Dihydrochlorid, cis/trans-Gemisch

■ N'-Cyclooctyl-N,N-dimethyl-1-phenyl-cyclohexan-1,4-diamin Dihydrochlorid

■ N'-(1 H-Indol-3-ylmethyl)-N,N-dimethyl-1-phenyl-cyclohexan-1,4-diamin Dihydrochlorid, unpolareres Diastereomer

■ N'-(1 H-Indol-3-ylmethyl)-N,N-dimethyl-1-phenyl-cyclohexan-1,4-diamin Dihydrochlorid, polareres Diastereomer

■ N'-Benzo[b]thiophen-3-ylmethyl-N,N-dimethyl-1-phenyl-cyclohexan-1,4-diamin Dihydrochlorid, unpolareres Diastereomer

■ N'-Benzo[b]thiophen-3-ylmethyl-N,N-dimethyl-1-phenyt-cyclohexan-1,4-diamin Dihydrochlorid, polareres Diastereomer

■ N'-Anthracen-2-yl-N,N-dimethyl-1-phenyl-cyclohexan-1,4-diamin Hydrochlorid, unpolareres Diastereomer

■ N'-Benzo[b]thiophen-3-ylmethyl-1-benzyl-N,N-dimethyl-cyclohexan-1,4-diamin Dihydrochlorid, unpolareres Diastereomer

■ N'-Benzo[b]thiophen-3-ylmethyl-1-benzyl-N,N-dimethyl-cyclohexan-1,4-diamin Dihydrochlorid, polareres Diastereomer

■ N'-[2-(1 H-Indol-3-yl)-ethyl]-N,N-dimethyl-1-naphthalin-2-yl-cyclohexan-1,4-diamin Dihydrochlorid, unpolareres Diastereomer

■ N'-[2-(1H-Indol-3-yl)-ethyl]-N,N-dimethyl-1-phenyl-cyclohexan-1,4-diamin Dihydrochlorid, unpolareres Diastereomer

■ N'-[2-(1 H-Indol-3-yl)-ethyl]-N ,N-dimethyl.1-phenyl-cyclohexan-1 ,4-diamin Dihydrochlorid, polareres Diastereomer

■ N'-[2-(1H-Indol-3-yl)-1-methyl-ethyl]-N,N-dimethyl-1-phenyl-cyclohexan-1,4-diamin Dihydrochlorid, polareres Diastereomer

■ Methyl 2-(4-dimethylamino-4-phenyl-cyclohexylamino)-3-(1 H-indol-3-yl)-propionat Dihydrochlorid, unpolareres Diastereomer

■ Methyl 2-(4-dimethylamino-4-phenyl-cyclohexylamino)-3-(1H-indol-3-yl)-propionat Dihydrochlorid, polareres Diastereomer

■ N'-[2-(1 H-Indol-3-yl)-1-methyl-ethyl]-N,N-dimethyl-1-naphthalin-2-yl-cyclohexan-1,4-diamin Dihydrochlorid, unpolareres Diastereomer

■ N'-Benzo[1,3]dioxol-5-ylmethyl-N,N-dimethyl-1-phenyl-cyclohexan-1,4-diamin Dihydrochlorid, cis/trans-Gemisch

■ N'-[2-(6-Fluor-1H-indol-3-yl)-ethyl]-N,N-dimethyl-1-phenyl-cyclohexan-1,4-diamin Dihydrochlorid, unpolareres Diastereomer

■ N'-[2-(6-Fluor-1H-indol-3-yl)-ethyl]-N,N-dimethyl-1-phenyl-cyclohexan-1,4-diamin Dihydrochlorid, polareres Diastereomer

- ■ N'-[2-(1H-Indol-3-yl)-ethyl]-N,N,N'-tnmethyl-1-phenyl-cyclohexan-1,4-diamin Dihydrochlorid, unpolareres Diastereomer
- ■ N'-[2-(1H-Indol-3-y)-ethyl]-N,N,N'-trimethyl-1-phenyl-cyclohexan-1,4-diamin Dihydrochlorid, polareres Diastereomer
- ■ N,N-Dimethyl-N'-[2-(7-methyl-1H-indol-3-yl)-ethyl]-1-phenyl-cyclohexan-1,4-diamin Dihydrochlorid, unpolareres Diastereomer
- ■ N,N-Dimethyl-N'-[2-(7-methyl-1H-indol-3-yl)-ethyl]-1-phenyl-cyclohexan-1,4-diamin Dihydrochlorid, polareres Diastereomer
- ■ N'-[2-(5-Fluor-1 H-indol-3-yl)-ethyl]-N,N-dimethyl-1-phenyl-cyclohexan-1,4-diamin Dihydrochlorid, unpolareres Diastereomer
- ■ N'-[2-(5-Fluor-1H-indol-3-yl)-ethyl]-N,N-dimethyl-1-phenyl-cyclohexan-1,4-diamin Dihydrochlorid, polareres Diastereomer
- ■ N'-Acenaphthen-5-ylmethyl-N,N-dimethyl-1-phenyl-cyclohexan-1,4-diamin Dihydrochlorid, unpolareres Diastereomer
- ■ N'-[2-(1H-Indol-3-yl)-1-methyl-ethyl]-N,N-dimethyl-1-thiophen-2-yl-cyclohexan-1,4-diamin Dihydrochlorid, unpolareres Diastereomer
- ■ N'-[2-(1H-Indol-3-yl)-1-methyl-ethyl]-N,N-dimethyl-1-thiophen-2-yl-cyclohexan-1,4-diamin Dihydrochlorid, cis/trans-Gemisch
- ■ N'-[2-(7-Benzyloxy-1H-indol-3-yl)-ethyl]-N,N-dimethyl-1-phenyl-cyclohexan-1,4-diamin Dihydrochlorid, unpolareres Diastereomer
- ■ N'-Cyclooctyl-N,N-dimethyl-1-thiophen-2-yl-cyclohexan-1,4-diamin Dihydrochlorid, unpolareres Diastereomer
- ■ N'-Adamantan-2-yl-N,N-dimethyl-1-thiophen-2-yl-cyclohexan-1,4-diamin Dihydrochlorid, unpolareres Diastereomer
- ■ 3-[2-(4-Dimethylamino-4-phenyl-cyclohexylamino)-ethyl]-1 H-indol-5-ol Dihydrochlorid, unpolareres Diastereomer
- ■ 3-[2-(4-Dimethylamino-4-phenyl-cyclohexylamino)-ethyl]-1H-indol-5-ol Dihydrochlorid, polareres Diastereomer
- ■ N'-[2-(5-Methoxy-1H-indol-3-yl)-ethyl]-N,N-dimethyl-1-phenyl-cyclohexan-1,4-diamin Dihydrochlorid, unpolareres Diastereomer
- ■ N'-[2-(5-Methoxy-1H-indol-3-yl)-ethyl]-N,N-dimethyl-1-phenyl-cyclohexan-1,4-diamin Dihydrochlorid, polareres Diastereomer
- ■ N,N-Dimethyl-N'-[2-(5-methyl-1H-indol-3-yl)-ethyl)-1-phenyl-cyclohexan-1,4-diamin Dihydrochlorid, unpolareres Diastereomer
- ■ N,N-Dimethyl-N'-[2-(5-methyl-1H-indol-3-yl)-ethyl]-1-phenyl-cyclohexan-1,4-diamin Dihydrochlorid, polareres Diastereomer
- ■ Dimethyl-[1-phenyl-4-(1,3,4,9-tetrahydro-b-carbolin-2-yl}-cyclohexyl)-amin Dihydrochlorid
- ■ N-(4-Dimethylamino-4-phenyl-cyclohexyl)-N-[2-(4-fluor-phenyl)-ethyl]-acetamid Hydrochlorid, unpolareres Diastereomer
- ■ 2-(4-Dimethylamino-4-phenyl-cyclohexylamino)-3-(5-fluor-1 -indol-3-yl)-propionsäure-methylester Dihydrochlorid, unpolareres Diastereomer
- ■ N-(4-Dimethylamino-4-phenyl-cyclohexyl)-N-(3-phenyl-propyl)-acetamid Hydrochlorid, unpolareres Diastereomer
- ■ 2-(4-Dimethylamino-4-phenyl-cyclohexylamino)-3-(6-fluor-1H-indol-3-yl)-propionsäure-methylester Dihydrochlorid, unpolareres Diastereomer
- ■ N-(4-Dimethylamino-4-phenyl-cyclohexyl)-2-(1H-indol-3-yl)-acetamid Hydrochlorid, polareres Diastereomer
- ■ 2-(4-Dimethylamino-4-thiophen-2-yl-cyclohexylamino)-3-(1H-indol-3-yl)-propionsäure-methylester Dihydrochlorid, unpolareres Diastereomer
- ■ N-(4-Dimethylamino-4-phenyl-cyclohexyl)-2-(5-methoxy-1 H-indol-3-yl)-acetamid Hydrochlorid, unpolareres Diastereomer

gegebenenfalls auch in Form ihrer Racemate, der genannten oder anderen reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; gegebenenfalls auch in Form der Säuren oder Basen oder in Form anderer Salze, insbesondere physiologisch verträglicher Salze oder Salze physiologisch verträglicher Säuren oder Kationen; oder in Form ihrer Solvate, insbesondere der Hydrate.

**20.** Arzneimittel enthaltend wenigstens ein substituiertes Cyclohexan-1,4-diaminderivat gemäß Anspruch 1,

, worin
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$, $C(X)OR^9$, $C(X)SR^9$, $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$, $- CHR^{11}- CH_2R^{12}$, $-CHR^{11}-CH_2-CH_2R^{12}$, $-CHR^{11}-CH_2-CH_2-CH_2R^{12}$, $- C(Y)R^{12}$, $-C(Y)-CH_2R^{12}$, $-C(Y)-CH_2-CH_2R^{12}$ oder $-C(Y)-CH_2-CH_2-CH_2R^{12}$
mit Y = O, S oder $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann,
mit der Maßgabe,

- daß, wenn $R^3$ substituiertes oder unsubstituiertes Phenyl ist und mindestens einer von $R^1$ oder $R^2$ H oder $C_{1-8}$-Alkyl ist, $R^4$ nicht Alkyl sein darf und $R^4$ und $R^5$ nicht zusammen einen Heterocyclus bilden dürfen

gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/ oder gegebenenfalls weitere Wirkstoffe.

**21.** Arzneimittel enthaltend wenigstens ein substituiertes Cyclohexan-1,4-diaminderivat gemäß Anspruch 1, , worin
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$, $C(X)OR^9$, $C(X)SR^9$, $S(O_2)_R{}^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$, $- CHR^{11}- CH_2R^{12}$, $-CHR^{11}-CH_2-CH_2R^{12}$, $-CHR^{11}-CH_2-CH_2-CH_2R^{12}$, $- C(Y)R^{12}$, $-C(Y)-CH_2R^{12}$, $-C(Y)-CH_2CH_2R^{12}$ oder $-C(Y)-CH_2-CH_2-CH_2R^{12}$
mit Y = O, S oder $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann,

gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weitere Wirkstoffe.

**22.** Arzneimittel enthaltend wenigstens ein substituiertes Cyclohexan-1,4-diaminderivat gemäß Anspruch 1,
, worin
$R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkyfen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$, $C(X)OR^9$, $C(X)SR^9$, $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}CH^2CH^2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2CH_2R^{12}$ oder $-C(Y)-CH_2-CH_2-CH_2R^{12}$
mit Y = O, S oder $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann,
gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form seiner Säuren oder seiner Basen oder in Form seiner Salze, insbesondere der

physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weitere Wirkstoffe.

**23.** Arzneimittel enthaltend wenigstens ein substituiertes Cyclohexan-1,4-diaminderivat gemäß Anspruch 1, , worin
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C(X)R^7$, $C(X)NR^7R^8$, $C(X)OR^9$, $C(X)SR^9$ oder $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$, $- CHR^{11}- CH_2R^{12}$, $-CHR^{11}-CH_2-CH_2R^{12}$, $-CHR^{11}CH_2-CH_2-CH_2R^{12}$, $- C(Y)R^{12}$, $-C(Y)-CH_2R^{12}$, $-C(Y)-CH_2CH_2R^{12}$ oder $-C(Y)-CH_2-CH_2-CH_2R^{12}$
mit Y = O, S oder $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weitere Wirkstoffe.

**24.** Arzneimittel gemäß einem der Ansprüchen 20 oder 21, **dadurch gekennzeichnet, daß** bei den enthaltenen substituierten Cyclohexan-1,4-diaminderivaten
$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen, oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen, oder die Reste $R^1$ und $R^2$ zusammen einen Ring bilden und $(CH_2)_{4-5}$ bedeuten,
insbesondere
$R^1$ und $R^2$ unabhängig voneinander ausgewählt sind aus Methyl oder Ethyl oder die Reste $R^1$ und $R^2$ zusammen einen Ring bilden und $(CH_2)_5$ bedeuten.

**25.** Arzneimittel gemäß Anspruch 22, **dadurch gekennzeichnet, daß** bei den enthaltenen substituierten Cyclohexan-1,4-diaminderivaten
$R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ zusammen einen Ring bilden und $(CH_2)_{4-5}$ bedeuten,
insbesondere
$R^1$ und $R^2$ zusammen einen Ring bilden und $(CH_2)_5$ bedeuten.

**26.** Arzneimittel gemäß Anspruch 23, **dadurch gekennzeichnet, daß** bei den enthaltenen substituierten Cyclohexan-1,4-diaminderivaten
$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
vorzugsweise
$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
insbesondere
$R^1$ und $R^2$ unabhängig voneinander ausgewählt sind aus Methyl oder Ethyl;
oder
$R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ zusammen einen Ring bilden und $(CH_2)_{4-5}$ bedeuten,
insbesondere
$R^1$ und $R^2$ zusammen einen Ring bilden und $(CH_2)_5$ bedeuten.

**27.** Arzneimittel gemäß einem der Ansprüchen 20, 22 oder 23, **dadurch gekennzeichnet, daß** bei den enthaltenen substituierten Cyclohexan-1,4-diaminderivaten
$R^3$ ausgewählt ist aus $C_{3-8}$-Cycloalkyl, Aryl oder Heteroaryl, jeweils unsubstituiert oder einfach oder mehrfach substituiert; über eine gesättigte oder ungesättigte, unverzweigte, substituierte oder unsubstituierte $C_{1-2}$-Alkyl-Gruppe gebundenem Aryl, $C_{3-8}$-Cycloalkyl oder Heteroaryl, jeweils unsubstituiert oder einfach oder mehrfach substituiert;
vorzugsweise
$R^3$ ausgewählt ist aus $C_{5-6}$-Cycloalkyl, Phenyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; über eine gesättigte, unverzweigte $C_{1-2}$-Alkyl-Gruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;

insbesondere
$R^3$ ausgewählt ist aus Phenyl, Furyl, Thiophenyl, Cyclohexanyl, Naphthyl, Benzofuranyl, Indolyl, Indanyl, Benzodioxanyl, Benzodioxolanyl, Pyrrolyl, Pyrimidyl, Pyrazinyl oder Benzothiophenyl, jeweils unsubstituiert oder einfach oder mehrfach substituiert; über eine gesättigte, unverzweigte $C_{1-2}$-Alkyl-Gruppe gebundenem Phenyl, Furyl oder Thiophenyl, jeweils unsubstituiert oder einfach oder mehrfach substituiert.

**28.** Arzneimittel gemäß Anspruch 21, **dadurch gekennzeichnet, daß** bei den enthaltenen substituierten Cyclohexan-1,4-diaminderivaten
$R^3$ ausgewählt ist aus $C_{3-8}$-Cycloalkyl oder Heteroaryl, jeweils unsubstituiert oder einfach oder mehrfach substituiert; über eine gesättigte oder ungesättigte, unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
vorzugsweise
$R^3$ ausgewählt ist aus $C_{5-6}$-Cycloalkyl, Thiophenyl, Benzothiophenyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; über eine gesättigte, unverzweigte $C_{1-2}$-AlkylGruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Furyl, Thiophenyl, Cyclohexanyl, Benzofuranyl, Indolyl, Indanyl, Benzodioxanyl, Benzodioxolanyl, Pyrrolyl, Pyrimidyl, Pyrazinyl oder Benzothiophenyl, jeweils unsubstituiert oder einfach oder mehrfach substituiert; über eine gesättigte, unverzweigte $C_{1-2}$-AlkylGruppe gebundenem Phenyl, Furyl oder Thiophenyl, jeweils unsubstituiert oder einfach oder mehrfach substituiert.

**29.** Arzneimittel gemäß einem der Ansprüchen 20 bis 28, **dadurch gekennzeichnet, daß** bei den enthaltenen substituierten Cyclohexan-1,4-diaminderivaten $R^4$ H ist.

**30.** Arzneimittel gemäß einem der Ansprüchen 20 bis 28, **dadurch gekennzeichnet, daß** bei den enthaltenen substituierten Cyclohexan-1,4-diaminderivaten
$R^4$ ausgewählt ist aus H, $C(X)R^7$, $C(X)NR^7R^8$, $C(X)OR^9$, $C(X)SR^9$ oder $S(O_2)R^9$ mit X = O oder S,
vorzugsweise
$R^4$ ausgewählt ist aus H, $C(X)R^7$, $C(X)NR^7R^8$ oder $C(X)OR^9$ mit $\times$ = O,
insbesondere
$R^4$ ausgewählt ist aus H oder $C(O)R^7$; vorzugsweise mit $R^7$ ausgewählt aus
H; oder $C_{1-8}$-Alkyl gesättigt oder ungesättigt, verzweigt oder unverzweigt, einfach oder mehrfach substituiert oder unsubstituiert;
vorzugsweise
H; oder $C_{1-3}$-Alkyl gesättigt, unsubstituiert, verzweigt oder unverzweigt;
insbesondere $CH_3$.

**31.** Arzneimittel gemäß einem der Ansprüchen 20 bis 22, **dadurch gekennzeichnet, daß** bei den enthaltenen substituierten Cyclohexan-1,4-diaminderivaten
$R^4$ und $R^5$ zusammen einen Heterocyclus mit zwischen 3 und 8 Atomen im Ring bilden, gesättigt oder ungesättigt; einfach oder mehrfach substituiert oder unsubstituiert, vorzugsweise mit zwischen 5 und 7 Atomen im Ring, wovon neben dem obligatorischen N 0 bis 1 weitere Heteroatome, ausgewählt aus N, S oder O, im Ring sind;
wobei der von $R^4$ und $R^5$ zusammen gebildete Heterocyclus gegebenenfalls mit weiteren Ringen kondensiert sein kann,
vorzugsweise mit aromatischen und/oder heteroaromatischen Ringen, wobei diese mit weiteren aromatischen und/oder heteroaromatischen Ringen kondensiert sein können,
insbesondere der von $R^4$ und $R^5$ zusammen gebildete Heterocyclus mit ein oder zwei weiteren Ringen kondensiert ist, vorzugsweise der von $R^4$ und $R^5$ zusammen gebildete Heterocyclus so mit zwei weiteren Ringen kondensiert ist, dass $R^4$ und $R^5$ zusammen

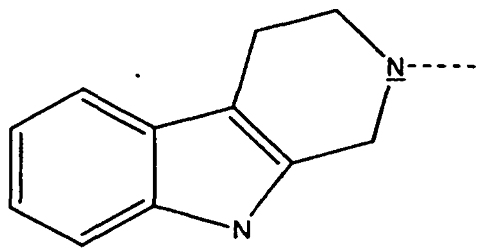

bedeuten.

**32.** Arzneimittel gemäß einem der Ansprüchen 20 bis 22, **dadurch gekennzeichnet, daß** bei den enthaltenen substituierten Cyclohexan-1,4-diaminderivaten
$R^4$ ausgewählt ist aus H, $C_{1-8}$-Alkyl, gesättigt oder ungesättigt, verzweigt oder unverzweigt, einfach oder mehrfach substituiert oder unsubstituiert,
vorzugsweise
H, $C_{1-6}$-Alkyl, gesättigt oder ungesättigt, verzweigt oder unverzweigt, einfach oder mehrfach substituiert oder unsubstituiert,
insbesondere
H, $C_{1-3}$-Alkyl, gesättigt, unverzweigt und unsubstituiert.

**33.** Arzneimittel gemäß einem der Ansprüchen 20 bis 32, **dadurch gekennzeichnet, daß** bei den enthaltenen substituierten Cyclohexan-1,4-diaminderivaten
$R^5$ ausgewählt ist aus $C_{3-8}$-Cycloalkyl, Aryl oder Heteroaryl, jeweils unsubstituiert oder einfach oder mehrfach substituiert;
vorzugsweise
$R^5$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^5$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

**34.** Arzneimittel gemäß einem der Ansprüchen 20 bis 32, **dadurch gekennzeichnet, daß** bei den enthaltenen substituierten Cyclohexan-1,4-diaminderivaten
$R^5$ ausgewählt ist aus $-CHR^{11}R^{12}$, $-CHR^{11}-CH_2R^{12}$, $-CHR^{11}-CH_2-CH_2R^{12}$, $-CHR^{11}-CH_2-CH_2CH_2R^{12}$, $-C(Y)R^{12}$, $-C(Y)-CH_2R^{12}$, $-C(Y)-CH_2-CH_2R^{12}$ oder $-C(Y)-CH_2-CH_2-CH_2R^{12}$
mit Y = O, S oder $H_2$,
vorzugsweise
$R^5$ ausgewählt ist aus $-CHR^{11}R^{12}$, $-CHR^{11}-CH_2R^{12}$, $-CHR^{11}-CH_2-CH_2R^{12}$, $-C(Y)R^{12}$, $-C(Y)-CH_2R^{12}$ oder $-C(Y)-CH_2-CH_2R^{12}$
mit Y = O oder S,
insbesondere
$R^5$ ausgewählt ist aus $-CHR^{11}R^{12}$, $-CHR^{11}-CH_2R^{12}$, $-CHR^{11}-CH_2-CH_2R^{12}$, $-C(Y)R^{12}$ oder $-C(Y)-CH_2R^{12}$
mit Y = O.

**35.** Arzneimittel gemäß Anspruch 34, **dadurch gekennzeichnet, daß** bei den enthaltenen substituierten Cyclohexan-1,4-diaminderivaten
$R^{11}$ ausgewählt ist aus
H, $C_{1-4}$-Alkyl, gesättigt oder ungesättigt, verzweigt oder unverzweigt, einfach oder mehrfach substituiert oder unsubstituiert; oder $C(O)O-C_{1-4}$-Alkyl, gesättigt oder ungesättigt, verzweigt oder unverzweigt, einfach oder mehrfach substituiert oder unsubstituiert;

vorzugsweise
H, $C_{1-4}$-Alkyl, gesättigt oder ungesättigt, verzweigt oder unverzweigt, einfach oder mehrfach substituiert oder unsubstituiert; oder C(O)O-$C_{1-2}$-Alkyl, gesättigt, unverzweigt, einfach oder mehrfach substituiert oder unsubstituiert unsubstituiert;
insbesondere
H, $CH_3$, $C_2H_5$ und C(O)O-$CH_3$.

**36.** Arzneimittel gemäß Anspruch 34, **dadurch gekennzeichnet, daß** bei den enthaltenen substituierten Cyclohexan-1,4-diaminderivaten
$R^{12}$ ausgewählt ist aus $C_{3-8}$-Cycloalkyl, Aryl oder Heteroaryl, jeweils unsubstituiert oder einfach oder mehrfach substituiert;
vorzugsweise
$R^{12}$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^{12}$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

**37.** Arzneimittel gemäß einem der Ansprüchen 20 bis 36, **dadurch gekennzeichnet, daß** das Arzneimittel neben wenigstens einem substituierten Cyclohexan-1,4-diaminderivat noch ein Opioid, vorzugsweise ein starkes Opioid, insbesondere Morphin, oder ein Anesthetikum, vorzugsweise Hexobarbital oder Halothan, enthält.

**38.** Verwendung eines substituierten Cyclohexan-1,4-diaminderivats gemäß Anspruch 1,
, worin
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$, $C(X)OR^9$, $C(X)SR^9$, $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert, über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^1$ $OCH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder.mehrfach substituiert oder unsubstituiert oder über $C_{1}$-$_{3}$-Alkylen gebundenem Aryl, $C_{3-8}$-Cycloalkyl oder Heteroaryl, jeweils einfach oder mehrfach substituiert oder unsubstituiert;

mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$,$-CHR^{11}$- $CH_2R^{12}$, $-CHR^{11}-CH_2-CH_2R^{12}$, $-CHR^{11}-CH_2-CH_2-CH_2R^{12}$,$-C(Y)R^{12}$, -C (Y)-$CH_2R^{12}$, $-C(Y)CH_2-CH_2R^{12}$ oder-$C(Y)-CH_2-CH_2CH_2R^{12}$

mit Y = O, S oder $H_2$,

mit $R^{11}$ ausgewählt aus

H, $C_{1-7}$-Alkyl, gesättigt oder ungesättigt, verzweigt oder unverzweigt, einfach oder mehrfach substituiert oder unsubstituiert; oder C(O)O-$C_{1-6}$-Alkyl, gesättigt oder ungesättigt, verzweigt oder unverzweigt, einfach oder mehrfach substituiert oder unsubstituiert;

und mit $R^{12}$ ausgewählt aus

H; $C_{3-8}$-Cycloalkyl, Aryl oder Heteroaryl, jeweils unsubstituiert oder einfach oder mehrfach substituiert,

oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenecifialls mit weiteren Ringen kondensiert sein kann,

mit der Maßgabe,

- daß, wenn $R^3$ substituiertes oder unsubstituiertes Phenyl ist und mindestens einer von $R^1$ oder $R^2$ H oder $C_{1-8}$-Alkyl ist, $R^4$ nicht Alkyl sein darf und $R^4$ und $R^5$ nicht zusammen einen Heterocyclus bilden dürfen

gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;

in dargestellter Form oder in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate; zur Herstellung eines Arzneimittels zur Behandlung von Schmerz, insbesondere von akutem, neuropathischem oder chronischem Schmerz, bzw. zur Coadministration bei Behandlung mit einem opioiden Analgetikum.

**39.** Verwendung eines substituierten Cyclohexan-1,4-diaminderivats gemäß Anspruch 1,

, worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert: wobei $R^1$ und $R^2$ nicht beide H sein dürfen,

oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder. Heteroaryl, jeweils einfach oder mehrfach substituiert oder unsubstituiert;

$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^1$, $C(X)NR^7R^8$, $C(X)OR^9$, $C(X)SR^9$, $S(O_2)R^9$

mit X =O oder S,

mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach

oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,
mit $R^{10}$ ausgewählt aus H; $C_{1-8}$-Alkyl oder $C_{3-8}$Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$, - $CHR^{11}-CH_2R^{12}$, $-CHR^{11}-CH_2CH_2R^{12}$, $-CHR^{11}-CH_2-CH_2-CH_2R^{12}$,$-C(Y)R^{12'}$, $-C(Y)-CH_2R^{12}$, $-C(Y)-CH_2CH_2R^{12}$ oder $-C(Y)-CH_2-CH_2CH_2R^{12}$
mit Y = O, S oder $H_2$,
mit $R^{11}$ ausgewählt aus
H, $C_{1-7}$-Alkyl, gesättigt oder ungesättigt, verzweigt oder unverzweigt einfach oder mehrfach substituiert oder unsubstituiert; oder $C(O)O-C_{1-6}$Alkyl, gesättigt oder ungesättigt, verzweigt oder unverzweigt, einfach oder mehrfach substituiert oder unsubstituiert:
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann,
gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate; zur Herstellung eines Arzneimittels zur Behandlung von Schmerz, insbesondere von akutem, neuropathischem oder chronischem Schmerz, bzw. zur Coadministration bei Behandlung mit einem opioiden Analgetikum.

**40.** Verwendung eines substituierten Cyclohexan-1,4-diaminderivats gemäß Anspruch 1,
, worin
$R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert, über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$ ,$C(X)OR^9$, $C(X)SR^9$, $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

mit $R^8$ ausgewählt aus H, $C_{1-4}$-Alkyl, gesättigt oder ungesättigt, verzweigt oder unverzweigt, einfach oder mehrfach substituiert oder unsubstituiert oder

die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,

mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert, oder über $C_{1-3}$-Alkylen gebundenem Aryl, $C_{3-8}$-Cycloalkyl oder Heteroaryl, jeweils einfach oder mehrfach substituiert oder unsubstituiert,

mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$,-CHR11- $CH^2R^{12}$, $-CHR^{11}-CH_2-CH_2R^{12}$, $-CH_2-CHR^{11}-CH_2-CH_2R^{12}$,$-C(Y)R^{12}$, -C (Y)-$CH_2R^{12}$, -C(Y)-$CH_2CH_2R^{12}$ oder -C(Y)-$CH_2$-$CH_2$-$CH_2R^{12}$

mit Y = O, S oder $H_2$,

mit $R^{11}$ ausgewählt aus

H, $C_{1-7}$-Alkyl, gesättigt oder ungesättigt, verzweigt oder unverzweigt, einfach oder mehrfach substituiert oder unsubstituiert; oder C(0)0-$C_{1-6}$-Alkyl, gesättigt oder ungesättigt, verzweigt oder unverzweigt einfach oder mehrfach substituiert oder unsubstituiert,

und mit $R^{12}$ ausgewählt aus

H; $C_{3-8}$-Cycloalkyl, Aryl oder Heteroaryl, jeweils unsubstituiert oder einfach oder mehrfach substituiert,

oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert; der gegebenenfalls mit weiteren Ringen kondensiert sein kann,

gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;

in dargestellter Form oder in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate; zur Herstellung eines Arzneimittels zur Behandlung von Schmerz, insbesondere von akutem, neuropathischem oder chronischem Schmerz, bzw. zur Coadministration bei Behandlung mit einem opioiden Analgetikum.

**41.** Verwendung eines substituierten Cyclohexan-1 ,4-diaminderivats gemäß Anspruch 1, worin

$R^1$ und $R^2$ unabhängig voneinander ausgewählt sind aus H; C-$_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,

oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert:

$R^4$ ausgewählt ist aus H, $C(X)R^7$, $C(X)NR^7R^8$, $C(X)OR^9$, $C(X)SR^9$ oder $S(O_2)R^9$

mit X = O oder S.

mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; Ober eine gesättigte oder ungesättigte, verzweigte oder unverzweigte, substituierte oder

unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesattigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt aus $C_{3-8}$-Cycloalkyl, Aryl oder Heteroaryl, jeweils unsubstituiert oder einfach oder mehrfach substituiert; $-CHR^{11}R^{12}$, $CHR^{11}$- $CH_2R^{12}$, $-CHR^{11}-CH_2-CH_2R^{12}$, $-CHR^{11}-CH_2-CH_2-CH_2R^{12}$, - $C(Y)R^{12}$, $-C(Y)-CH_2R^{12}$, -C (Y)-$CH_2$-$CH_2R^{12}$ oder-$C(Y)-CH_2CH_2CH_2R^{12}$
mit Y=O, S oder $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder C(O)O-$C_{1-6}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate; zur Herstellung eines Arzneimittels zur Behandlung von Schmerz, insbesondere von akutem, neuropathischem oder chronischem Schmerz, bzw. zur Coadministration bei Behandlung mit einem opioiden Analgetikum.

**42.** Verwendung eines substituierten Cyclohexan-1,4-diaminderivats gemäß Anspruch 1,
, worin
$R^1$ und $R^2$ unabhängig voneinander ausgewählt sind aus H; $C_{1-8}$-Alkyl oder $C_{3-8}$-Cycloalkyl, jeweils gesättigt oder ungesättigt verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert,
$R^3$ ausgewählt ist aus $C_{1-8}$-Alkyl oder $C_{3-8}$-Cycloalkyl, jeweils gesättigt oder ungesättigt, verzweigt oder unverzweigt einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C_{1-8}$-Alkyl, gesättigt oder ungesättigt, verzweigt oder unverzweigt, einfach oder mehrfach substituiert oder unsubstituiert oder $C(X)R^7$, $C(X)NR^7R^8$, $C(X)OR^9$, $C(X)SR^9$, $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder

unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

mit $R^8$ ausgewählt aus H, $C_{1-4}$-Alkyl, gesättigt oder ungesättigt, verzweigt oder unverzweigt, einfach oder mehrfach substituiert oder unsubstituiert oder

die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,

mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$Alkylen gebundenem Aryl, $C_{3-8}$-Cycloalkyl oder Heteroaryl, jeweils einfach oder mehrfach substituiert oder unsubstituiert;

mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$,$-CHR^{11}$- $CH_2R^{12}$, $-CHR^{11}-CH_2-CH_2R^{12}$, $-CHR^{11}-CH_2-CH_2CH_2R^{12}$,$-C(Y)R^{12}$, $-C(Y)-CH_2R^{12}$, $-C(Y)-CH_2-CH_2R^{12}$ oder $-C(Y)-CH_2-CH_2-CH_2R^{12}$

mit Y = O, S oder $H_2$,

mit $R^{11}$ ausgewählt aus

H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;

und mit $R^{12}$ ausgewählt aus

H; $C_{3-8}$-Cycloalkyl, Aryl oder Heteroaryl, jeweils unsubstituiert oder einfach oder mehrfach substituiert,

oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann,

gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, In einem beliebigen Mischungsverhältnis;

in dargestellter Form oder in Form seiner Säuren oder seiner Basen oder in Form seiner Salze, insbesondere der physiologisch verträglichen Salze oder Salze physiologisch verträglicher Säuren oder Kationen; oder in Form seiner-Solvate, insbesondere der Hydrate; zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Schwierigkeiten (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypötension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Haminkontinenz bzw. als Muskelrelaxanz, Antikonvulsivum oder Anesthetikum, bzw. zur Coadministration bei Behandlung mit einem Anesihetikum, zur Diurese oder Antinatriurese und/oder Anxiolyse.

**43.** Verwendung gemäß einem der Ansprüchen 38, 39 oder 42, **dadurch gekennzeichnet, daß** bei den verwendeten substituierten Cyclohexan-1,4-diaminderivaten

$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,

oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

mit $R^6$ ausgewählt aus H; $C_{1-8}$-Alkyl, gesättigt oder ungesättigt, verzweigt oder unverzweigt, einfach oder mehrfach substituiert oder unsubstituiert,

vorzugsweise

$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,

oder die Reste $R^1$ und $R^2$ zusammen einen Ring bilden und $(CH_2)_{4-5}$ bedeuten,

insbesondere

$R^1$ und $R^2$ unabhängig voneinander ausgewählt sind aus Methyl oder Ethyl oder die Reste $R^1$ und $R^2$ zusammen einen Ring bilden und $(CH_2)_5$ bedeuten.

**44.** Verwendung gemäß Anspruch 40, **dadurch gekennzeichnet, daß** bei den verwendeten substituierten Cyclohexan-1,4-diaminderivaten
$R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten, mit $R^6$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ zusammen einen Ring bilden und $(CH_2)_{4-5}$ bedeuten,
insbesondere
$R^1$ und $R^2$ zusammen einen Ring bilden und $(CH_2)_5$ bedeuten.

**45.** Verwendung gemäß Anspruch 41, **dadurch gekennzeichnet, daß** bei den verwendeten substituierten Cyclohexan-1,4-diaminderivaten
$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
vorzugsweise
$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
insbesondere
$R^1$ und $R^2$ unabhängig voneinander ausgewählt sind aus Methyl oder Ethyl;
oder
$R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten, mit $R^6$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ zusammen einen Ring bilden und $(CH_2)_{4-5}$ bedeuten,
insbesondere
$R^1$ und $R^2$ zusammen einen Ring bilden und $(CH_2)_5$ bedeuten.

**46.** Verwendung gemäß einem der Ansprüchen 38 oder 40 bis 42, **dadurch gekennzeichnet, daß** bei den verwendeten substituierten Cyclohexan-1,4-diaminderivaten
$R^3$ ausgewählt ist aus $C_{3-8}$-Cycloalkyl, Aryl oder Heteroaryl, jeweils unsubstituiert oder einfach oder mehrfach substituiert; über eine gesättigte oder ungesättigte, unverzweigte, substituierte oder unsubstituierte $C_{1-2}$-Alkyl-Gruppe gebundenem Aryl, $C_{3-8}$-Cycloalkyl oder Heteroaryl, jeweils unsubstituiert oder einfach oder mehrfach substituiert;
vorzugsweise
$R^3$ ausgewählt ist aus $C_{5-6}$-Cycloalkyl, Phenyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; über eine gesättigte, unverzweigte $C_{1-2}$-Alkyl-Gruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Phenyl, Furyl, Thiophenyl, Cyclohexanyl, Naphthyl, Benzofuranyl, Indolyl, Indanyl, Benzodioxanyl, Benzodioxolanyl, Pyrrolyl, Pyrimidyl, Pyrazinyl oder Benzothiophenyl, jeweils unsubstituiert oder einfach oder mehrfach substituiert; über eine gesättigte, unverzweigte $C_{1-2}$-Alkyl-Gruppe gebundenem Phenyl, Furyl oder Thiophenyl, jeweils unsubstituiert oder einfach oder mehrfach substituiert.

**47.** Verwendung gemäß Anspruch 39, **dadurch gekennzeichnet, daß** bei den verwendeten substituierten Cyclohexan-1,4-diaminderivaten
$R^3$ ausgewählt ist aus $C_{3-8}$-Cycloalkyl oder Heteroaryl, jeweils unsubstituiert oder einfach oder mehrfach substituiert; über eine gesättigte oder ungesättigte, unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
vorzugsweise
$R^3$ ausgewählt ist aus $C_{5-6}$-Cycloalkyl, Thiophenyl, Benzothiophenyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; über eine gesättigte, unverzweigte $C_{1-2}$-AlkylGruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere

$R^3$ ausgewählt ist aus Furyl, Thiophenyl, Cyclohexanyl, Benzofuranyl, Indolyl, Indanyl, Benzodioxanyl, Benzodioxolanyl, Pyrrolyl, Pyrimidyl, Pyrazinyl oder Benzothiophenyl, jeweils unsubstituiert oder einfach oder mehrfach substituiert; über eine gesättigte, unverzweigte $C_{1-2}$-AlkylGruppe gebundenem Phenyl, Furyl oder Thiophenyl, jeweils unsubstituiert oder einfach oder mehrfach substituiert.

**48.** Verwendung gemäß einem der Ansprüchen 38 bis 47, **dadurch gekennzeichnet, daß** bei den verwendeten substituierten Cyclohexan-1,4-diaminderivaten $R^4$ H ist.

**49.** Verwendung gemäß einem der Ansprüchen 38 bis 47, **dadurch gekennzeichnet, daß** bei den verwendeten substituierten Cyclohexan-1,4-diaminderivaten
$R^4$ ausgewählt ist aus H, $C(X)R^7$, $C(X)NR^7R^8$, $C(X)OR^9$, $C(X)SR^9$ oder $S(O_2)R^9$ mit X = O oder S,
vorzugsweise
$R^4$ ausgewählt ist aus H, $C(X)R^7$, $C(X)NR^7R^8$ oder $C(X)OR^9$ mit X = O,
insbesondere
$R^4$ ausgewählt ist aus H oder $C(O)R^7$; vorzugsweise mit $R^7$ ausgewählt aus
H; oder $C_{1-8}$-Alkyl gesättigt oder ungesättigt, verzweigt oder unverzweigt, einfach oder mehrfach substituiert oder unsubstituiert;
vorzugsweise
H; oder $C_{1-3}$-Alkyl gesättigt, unsubstituiert, verzweigt oder unverzweigt;
insbesondere $CH_3$.

**50.** Verwendung gemäß einem der Ansprüchen 38 bis 40 oder 42, **dadurch gekennzeichnet, daß** bei den verwendeten substituierten Cyclohexan-1,4-diaminderivaten
$R^4$ und $R^5$ zusammen einen Heterocyclus mit zwischen 3 und 8 Atomen im Ring bilden, gesättigt oder ungesättigt; einfach oder mehrfach substituiert oder unsubstituiert, vorzugsweise mit zwischen 5 und 7 Atomen im Ring, wovon neben dem obligatorischen N 0 bis 1 weitere Heteroatome, ausgewählt aus N, S oder O, im Ring sind;
wobei der von $R^4$ und $R^5$ zusammen gebildete Heterocyclus gegebenenfalls mit weiteren Ringen kondensiert sein kann,
vorzugsweise mit aromatischen und/oder heteroaromatischen Ringen,
wobei diese mit weiteren aromatischen und/oder heteroaromatischen Ringen kondensiert sein können,
insbesondere der von $R^4$ und $R^5$ zusammen gebildete Heterocyclus mit ein oder zwei weiteren Ringen kondensiert ist,
vorzugsweise der von $R^4$ und $R^5$ zusammen gebildete Heterocyclus so mit zwei weiteren Ringen kondensiert ist, dass $R^4$ und $R^5$ zusammen

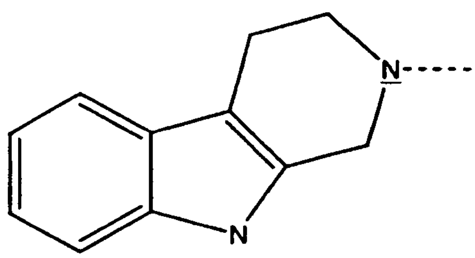

bedeuten.

**51.** Verwendung gemäß einem der Ansprüchen 38 bis 40 oder 42, **dadurch gekennzeichnet, daß** bei den verwendeten substituierten Cyclohexan-1,4-diaminderivaten
$R^4$ ausgewählt ist aus H, $C_{1-8}$-Alkyl, gesättigt oder ungesättigt, verzweigt oder unverzweigt, einfach oder mehrfach substituiert oder unsubstituiert,
vorzugsweise
H, $C_{1-6}$-Alkyl, gesättigt oder ungesättigt, verzweigt oder unverzweigt, einfach oder mehrfach substituiert oder unsubstituiert,
insbesondere
H, $C_{1-3}$-Alkyl, gesättigt, unverzweigt und unsubstituiert.

**52.** Verwendung gemäß einem der Ansprüchen 38 bis 51, **dadurch gekennzeichnet, daß** bei den verwendeten substituierten Cyclohexan-1,4-diaminderivaten
$R^5$ ausgewählt ist aus $C_{3-8}$-Cycloalkyl, Aryl oder Heteroaryl, jeweils unsubstituiert oder einfach oder mehrfach substituiert;
vorzugsweise
$R^5$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^5$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

**53.** Verwendung gemäß einem der Ansprüchen 38 bis 51, **dadurch gekennzeichnet, daß** bei den verwendeten substituierten Cyclohexan-1,4-diaminderivaten
$R^5$ ausgewählt ist aus $-CHR^{11}R^{12}$, $-CHR^{11}$- $CH_2R^{12}$, $-CHR^{11}-CH_2-CH_2R^{12}$, $-CHR^{11}-CH_2-CH_2CH_2R^{12}$, $-C(Y)R^{12}$, $-C(Y)-CH_2R^{12}$, $-C(Y)-CH_2CH_2R^{12}$ oder $-C(Y)-CH_2-CH_2CH_2R^{12}$
mit Y = O, S oder $H_2$,
vorzugsweise
$R^5$ ausgewählt ist aus $-CHR^{11}R^{12}$, $-CHR^{11}$- $CH_2R^{12}$, $-CHR^{11}-CH_2-CH_2R^{12}$, $-C(Y)R^{12}$, $-C(Y)-CH_2R^{12}$ oder $-C(Y)-CH_2-CH_2R^{12}$
mit Y = O oder S,
insbesondere
$R^5$ ausgewählt ist aus $-CHR^{11}R^{12}$, $-CHR^{11}$- $CH_2R^{12}$, $-CHR^{11}-CH_2-CH_2R^{12}$, $-C(Y)R^{12}$ oder $-C(Y)-CH_2R^{12}$
mit Y = O.

**54.** Verwendung gemäß Anspruch 53, **dadurch gekennzeichnet, daß** bei den verwendeten substituierten Cyclohexan-1,4-diaminderivaten
$R^{11}$ ausgewählt ist aus
H, $C_{1-4}$-Alkyl, gesättigt oder ungesättigt, verzweigt oder unverzweigt, einfach oder mehrfach substituiert oder unsubstituiert; oder $C(O)O-C_{1-4}$-Alkyl, gesättigt oder ungesättigt, verzweigt oder unverzweigt, einfach oder mehrfach substituiert oder unsubstituiert;
vorzugsweise
H, $C_{1-4}$-Alkyl, gesättigt oder ungesättigt, verzweigt oder unverzweigt, einfach oder mehrfach substituiert oder unsubstituiert; oder $C(O)O-C_{1-2}$-Alkyl, gesättigt, unverzweigt, einfach oder mehrfach substituiert oder unsubstituiert unsubstituiert;
insbesondere
H, $CH_3$, $C_2H_5$ und $C(O)O-CH_3$.

**55.** Verwendung gemäß Anspruch 53, **dadurch gekennzeichnet, daß** bei den verwendeten substituierten Cyclohexan-1,4-diaminderivaten
$R^{12}$ ausgewählt ist aus $C_{3-8}$-Cycloalkyl, Aryl oder Heteroaryl, jeweils unsubstituiert oder einfach oder mehrfach substituiert;
vorzugsweise
$R^{12}$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^{12}$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

**56.** Substituierte Cyclohexan-1,4-diaminderivate, Arzneimittel oder Verfahren gemäß einem der vorstehenden Ansprü-

che, **dadurch gekennzeichnet, daß** $R^4$ und $R^5$ zusammen keinen Heterocyclus bilden.

**57.** Verfahren zur Herstellung eines substituierten Cyclohexan-1,4-diaminderivats gemäß einem der Ansprüche 1 bis 19 mit folgenden Schritten:

a. ein mit den Gruppen $S^1$ und $S^2$ geschütztes Cyclohexan-1,4-dion gemäß Formel II wird in Gegenwart einer Verbindung der Formel $HNR^{01}R^{02}$ mit einem Cyanid, vorzugsweise Kaliumcyanid, zu einem geschützten N-substituierten 1-Amino-4-oxo-cyclohexancarbonitrilderivat gemäß Formel III umgesetzt;

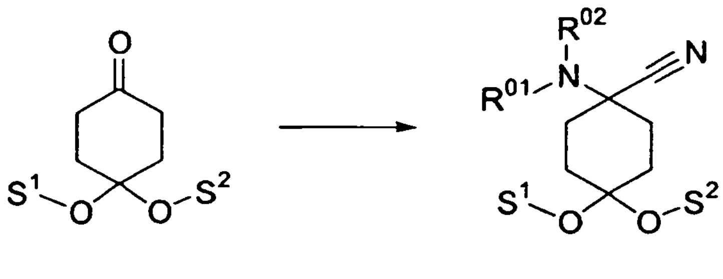

II III

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/ oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

b. das Aminonitril gemäß Formel III wird mit metallorganischen Reagenzien, bevorzugt Grignard- oder Organolithiumreagenzien, der Formel Metall-$R^3$ umgesetzt, so daß eine Verbindung gemäß Formel IVa entsteht;

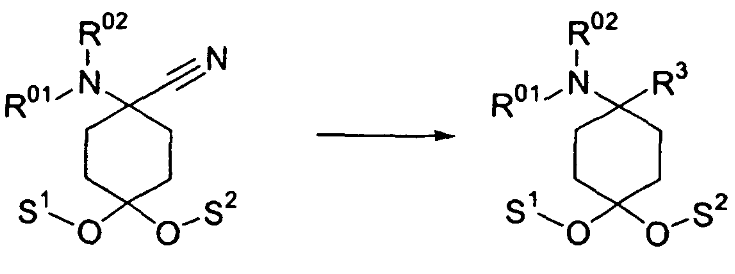

III IVa

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/ oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

c. an der Verbindung gemäß Formel IVa werden die Schutzgruppen $S^1$ und $S^2$ abgespalten, so daß ein 4-substituiertes 4-Aminocyclohexanonderivat gemäß Formel IV entsteht;

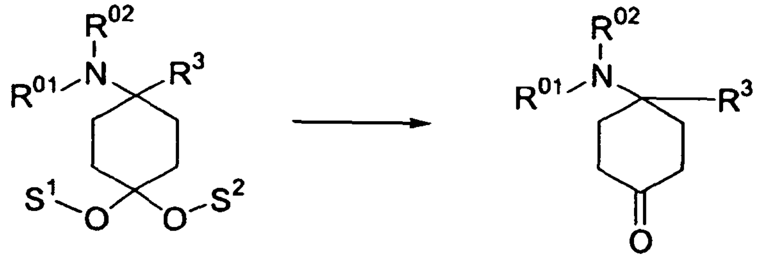

IVa IV

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/ oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

d. das 4-substituierte 4-Aminocyclohexanonderivat gemäß Formel IVa wird reduktiv mit einer Verbindung der Formel $HNR^{04}R^{05}$ aminiert, so daß ein Cyclohexan-1,4-diaminderivat gemäß Formel V entsteht;

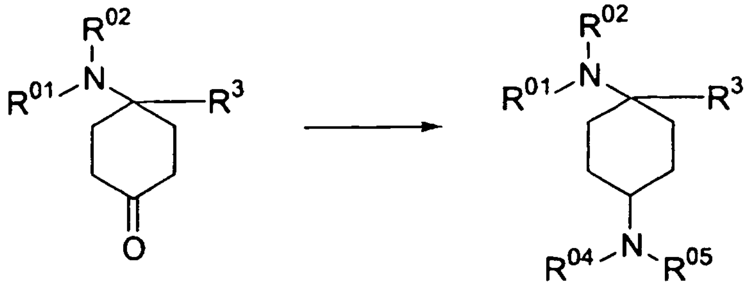

IV V

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/ oder $R^{06}$ = H mindestens einmal eine Schutzgruppe eingeführt und gegebenfalls acyliert, alkyliert oder sulfoniert, bis eine Verbindung gemäß Formel I entsteht,

wobei $R^1$, $R^2$, $R^3$ $R^4$ und $R^5$ die in Anspruch 1 angegebene Bedeutung haben

und

$R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

oder die Reste $R^{01}$ und $R^{02}$ zusammen einen Ring bilden und $CH_2CH_2OCH_2CH_2$, $CH_2CH_2NR^{06}CH_2CH_2$ oder $(CH_2)_{3-6}$ bedeuten,

mit $R^{06}$ ausgewählt aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^{04}$ ausgewählt ist aus H, mit einer Schutzgruppe versehenem H; $C_{1-8}$-Alkyl, gesättigt oder ungesättigt, verzweigt

oder unverzweigt, einfach oder mehrfach substituiert oder unsubstituiert;
$R^{05}$ ausgewählt ist aus H, mit einer Schutzgruppe versehenem H; $C_{3-8}$-Cycloalkyl, Aryl oder Heteroaryl, jeweils unsubstituiert oder einfach oder mehrfach substituiert; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ oder $-C(Y)-CH_2-CH_2-CH_2R^{12}$
mit Y = $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^{04}$ und $R^{05}$ zusammen einen Heterocyclus mit zwischen 3 und 8 Atomen im Ring bilden, gesättigt oder ungesättigt; einfach oder mehrfach substituiert oder unsubstituiert,
und $S^1$ und $S^2$ unabhängig voneinander ausgewählt sind aus Schutzgruppen oder zusammen eine Schutzgruppe bedeuten, vorzugsweise Monoacetal.

**58.** Verfahren zur Herstellung eines substituierten Cyclohexan-1,4-diaminderivats gemäß einem der Ansprüche 1 bis 19 mit folgenden Schritten:

a. ein mit den Gruppen $S^1$ und $S^2$ geschütztes Cyclohexan-1,4-dion gemäß Formel II wird reduktiv mit einer Verbindung der Formel $HNR^{04}R^{05}$ aminiert, so daß ein 4-Aminocyclohexanonderivat gemäß Formel VI entsteht;

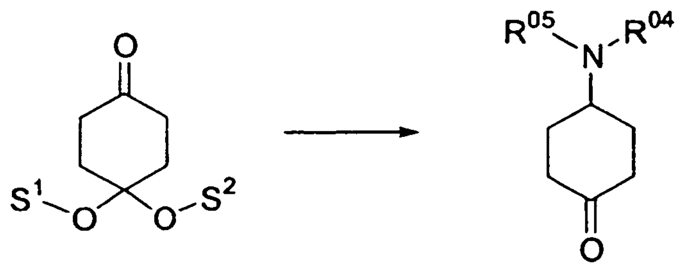

II VI

gegebenenfalls wird anschließend in beliebiger Reihenfolge und gegebenfalls wiederholt acyliert, alkyliert oder sulfoniert und/oder bei Verbindungen mit $R^{04}$ und/oder $R^{05}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{04}$ und/oder $R^{05}$ = H mindestens einmal eine Schutzgruppe eingeführt und gegebenfalls acyliert, alkyliert oder sulfoniert,
b. das 4-Aminocyclohexanonderivat gemäß Formel VI wird in Gegenwart einer Verbindung der Formel $HNR^{01}R^{02}$ mit Cyanid, vorzugsweise Kaliumcyanid, zu einem Cyclohexanonnitrilderivat der Formel VII umgesetzt,

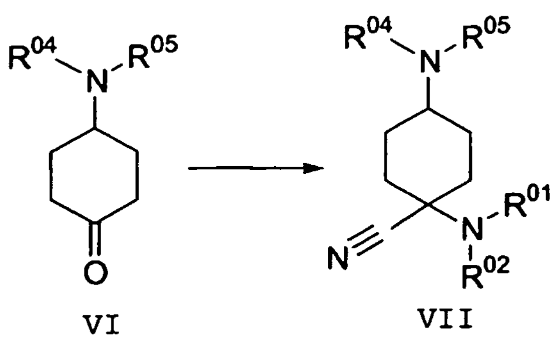

VI VII

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$= mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert,

c. das Cyclohexanonnitrilderivat der Formel VII wird mit metallorganischen Reagenzien, bevorzugt Grignard- oder Organolithiumreagenzien, der Formel Metall-$R^3$ umgesetzt und abschließend die Schutzgruppen $S^1$ und $S^2$ abgespalten, so daß ein Cyclohexan-1,4-diaminderivate gemäß Formel V entsteht,

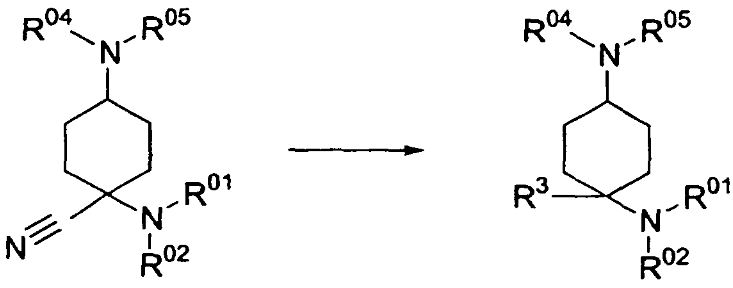

VII V

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert, bis eine Verbindung gemäß Formel I entsteht,

wobei $R^1$, $R^2$, $R^3$ $R^4$ und $R^5$ die in Anspruch 1 angegebene Bedeutung haben
und
$R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
oder die Reste $R^{01}$ und $R^{02}$ zusammen einen Ring bilden und $CH_2CH_2OCH_2CH_2$, $CH_2CH_2NR^{06}CH_2CH_2$ oder $(CH_2)_{3-6}$ bedeuten,
mit $R^{06}$ ausgewählt aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^{04}$ ausgewählt ist aus H, mit einer Schutzgruppe versehenem H; $C_{1-8}$-Alkyl, gesättigt oder ungesättigt, verzweigt oder unverzweigt, einfach oder mehrfach substituiert oder unsubstituiert;
$R^{05}$ ausgewählt ist aus mit einer Schutzgruppe versehenem H; $C_{3-8}$-Cycloalkyl, Aryl oder Heteroaryl, jeweils unsubstituiert oder einfach oder mehrfach substituiert; $-CHR^{11}R^{12}$, $-CHR^{11}$- $CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ oder $-C(Y)-CH_2-CH_2-CH_2R^{12}$
mit Y = $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^{04}$ und $R^{05}$ zusammen einen Heterocyclus mit zwischen 3 und 8 Atomen im Ring bilden, gesättigt oder ungesättigt; einfach oder mehrfach substituiert oder unsubstituiert,
und $S^1$ und $S^2$ unabhängig voneinander ausgewählt sind aus Schutzgruppen oder zusammen eine Schutzgruppe bedeuten, vorzugsweise Monoacetal.

**59.** Verfahren zur Herstellung eines substituierten Cyclohexan-1,4-diaminderivats gemäß einem der Ansprüche 57 oder 58, **dadurch gekennzeichnet, daß** die Schutzgruppen am H bei $R^{01}$, $R^{02}$, $R^{04}$, $R^{05}$ und/oder $R^{06}$ ausgewählt sind aus Alkyl, Benzyl oder Carbamaten, beispielsweise FMOC, Z oder Boc.

**60.** Verfahren zur Herstellung eines substituierten Cyclohexan-1,4-diaminderivats gemäß Anspruch 57, **dadurch gekennzeichnet, daß** die reduktive Aminierung in Schritt d in Gegenwart von Amoniumformiat, Amoniumacetat oder $NaCNBH_3$ stattfindet.

**61.** Verfahren zur Herstellung eines substituierten Cyclohexan-1,4-diaminderivats gemäß Anspruch 57, **dadurch gekennzeichnet, daß** statt der reduktiven Aminierung mit $HNR^{04}R^{05}$ in Schritt d die Verbindung IV mit Hydroxylamin reagiert und nach der Oximbildung reduziert wird.

**62.** Verfahren zur Herstellung eines substituierten Cyclohexan-1,4-diaminderivats gemäß Anspruch 58, **dadurch gekennzeichnet, daß** in Schritt b in Formel $HNR^{01}R^{02}$ der Rest $R^{01}$ = H ist, die Umsetzung mit dem Cyanid mit TMSCN geschieht und gegebenfalls anschließend eine Schutzgruppe an $R^{01}$ eingeführt wird.

## Claims

**1.** Substituted cyclohexane-1,4-diamine derivatives of the general formula I

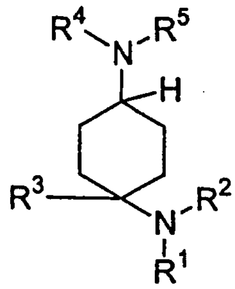

I

wherein
$R^1$ and $R^2$ independently of one another are chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted; wherein $R^1$ and $R^2$ may not both be H,
or the radicals $R^1$ and $R^2$ together form a ring and denote $CH_2CH_2OCH_2CH_2$, $CHO_2CH_2NR^6CH_2CH_2$ or $(CH_2)_{3-6}$,
where $R^6$ is chosen from H; $C_{1-8}$-alkyl or $C_{3-8}$-cycloalkyl, in each case saturated or unsaturated, branched or unbranched, mono-or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
$R^3$ is chosen from $C_1$-$_8$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^4$ is chosen from H, $C_{1-8}$-alkyl, saturated or unsaturated, branched or unbranched, mono- or polysubstituted or unsubstituted; or $C(X)R^7$, $C(X)NR^7R^8$, $C(X)OR^9$, $C(X)SR^9$, $S(O_2)R^9$
where X = O or S,
where $R^7$ is chosen from H, $C_{1-8}$-alkyl or $C_{3-8}$-cycloalkyl, in each case saturated or unsaturated, branched or unbranched, mono-or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
where $R^8$ is chosen from H, $C_{1-4}$-alkyl, saturated or unsaturated, branched or unbranched, mono- or polysubstituted or unsubstituted or

the radicals $R^7$ and $R^8$ together form a ring and denote $CH_2CH_2OCH_2CH_2$, $CH_2CH_2NR^{10}CH_2CH_2$ or $(CH_2)_{3-6}$, where $R^{10}$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
where $R^9$ is chosen from $C_{1-8}$-alkyl or $C_{3-8}$-cycloalkyl, in each case saturated or unsaturated, branched or unbranched, mono-or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^5$ is chosen from $C_{3-8}$-cycloalkyl, aryl or heteroaryl, in each case unsubstituted or mono- or polysubstituted; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, -C (Y) $R^{12}$, -C (Y) $-CH_2R^{12}$, -C (Y)$-CH_2-CH_2R^{12}$ or -C (Y) $-CH_2-CH_2-CH_2R^{12}$
where Y = O, S or $H_2$,
where $R^{11}$ is chosen from
H, $C_{1-7}$-alkyl, saturated or unsaturated, branched or unbranched, mono- or polysubstituted or unsubstituted; or C (O)O-$C_{1-6}$-alkyl, saturated or unsaturated, branched or unbranched, mono- or polysubstituted or unsubstituted;
and where $R^{12}$ is chosen from
H; $C_{3-8}$-cycloalkyl, aryl or heteroaryl, in each case unsubstituted or mono- or polysubstituted,
or $R^4$ and $R^5$ together form a heterocyclic radical having between 3 and 8 atoms in the ring, saturated or unsaturated; mono- or polysubstituted or unsubstituted, which heterocyclic radical may optionally be fused to further rings,
with the proviso,

- ■ that if $R^3$ is substituted or unsubstituted phenyl and at least one of $R^1$ or $R^2$ is H or $C_{1-8}$-alkyl, $R^4$ may not be alkyl and $R^4$ and $R^5$ may not together form a heterocyclic radical
or
- ■ that if $R^3$ is unsubstituted phenyl and $R^1$ and $R^2$ together denote $(CH_2)_5$, $R^4$ is chosen from H or $C_{1-8}$-alkyl, Y is not O or S and $R^5$ is not $C_{1-6}$-alkyl,

optionally in the form of their racemates, of their pure stereoisomers, in particular enantiomers or diastereomers, or in the form of mixtures of the stereoisomers, in particular of the enantiomers or diastereomers, in any mixing ratio; in the prepared form or in the form of their acids or their bases or in the form of their salts, in particular the physiologically acceptable salts or salts of physiologically acceptable acids or cations; or in the form of their solvates, in particular the hydrates.

**2.** Substituted cyclohexane-1,4-diamine derivatives according to claim 1,
wherein
$R^1$ and $R^2$ independently of one another are chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted; wherein $R^1$ and $R^2$ may not both be H,
or the radicals $R^1$ and $R^2$ together form a ring and denote $CH_2CH_2OCH_2CH_2$, $CH_2CH_2NR^6CH_2CH_2$ or $(CH_2)_{3-6}$, where $R^6$ is chosen from H; $C_{1-8}$-alkyl or $C_{3-8}$-cycloalkyl, in each case saturated or unsaturated, branched or unbranched, mono-or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
$R^3$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^4$ is chosen from H, $C(X)R^7$, $C(X)NR^7R^8$, $C(X)OR^9$ or $C(X)SR^9$, $S(O_2)R^9$
where X = O or S,
where $R^7$ is chosen from H, $C_{1-8}$-alkyl or $C_{3-8}$-cycloalkyl, in each case saturated or unsaturated, branched or unbranched, mono-or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
where $R^8$ is chosen from H, $C_{1-4}$-alkyl, saturated or unsaturated, branched or unbranched, mono- or polysubstituted or unsubstituted or
the radicals $R^7$ and $R^8$ together form a ring and denote $CH_2CH_2OCH_2CH_2$, $CH_2CH_2NR^{10}CH_2CH_2$ or $(CH_2)_{3-6}$,

where $R^{10}$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
where $R^9$ is chosen from $C_{1-8}$-alkyl or $C_{3-8}$-cycloalkyl, in each case saturated or unsaturated, branched or unbranched, mono-or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^5$ is chosen from $C_{3-8}$-cycloalkyl, aryl or heteroaryl, in each case unsubstituted or mono- or polysubstituted; $-CHR^{11}R^{12}$, $-CHR^{11}-CHR^{12}$, $-CHR^{11}-CH_2-CH_2R^{12}$, $-CHR^{11}-CH_2-CH_2-CH_2R^{12}$, -C (Y) $R^{12}$, -C (Y)-$CH_2R^{12}$, -C (Y) -$CH_2$-$CH_2R^{12}$ or -C (Y) -$CH_2$-$CH_2$-$CH_2R^{12}$
where Y = O, S or $H_2$,
where $R^{11}$ is chosen from
H, $C_{1-7}$-alkyl, saturated or unsaturated, branched or unbranched, mono- or polysubstituted or unsubstituted; or C (O)O-$C_{1-6}$-alkyl, saturated or unsaturated, branched or unbranched, mono- or polysubstituted or unsubstituted;
and where $R^{12}$ is chosen from
$C_{3-8}$-cycloalkyl, aryl or heteroaryl, in each case unsubstituted or mono- or polysubstituted,
optionally in the form of their racemates, of their pure stereoisomers, in particular enantiomers or diastereomers, or in the form of mixtures of the stereoisomers, in particular of the enantiomers or diastereomers, in any mixing ratio; in the prepared form or in the form of their acids or their bases or in the form of their salts, in particular the physiologically acceptable salts or salts of physiologically acceptable acids or cations; or in the form of their solvates, in particular the hydrates.

**3.** Substituted cyclohexane-1,4-diamine derivatives according to claim 1,
wherein
$R^1$ and $R^2$ independently of one another are chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted; wherein $R^1$ and $R^2$ may not both be H,
$R^3$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^4$ is chosen from H, $C(X)R^7$, $C(X)NR^7R^8$, C (X) $OR^9$ or C (X) $SR^9$, S $(O_2)$ $R^9$
where X = O or S,
where $R^7$ is chosen from H, $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
where $R^8$ is chosen from H, $C_{1-4}$-alkyl, saturated or unsaturated, branched or unbranched, mono- or polysubstituted or unsubstituted or
the radicals $R^7$ and $R^8$ together form a ring and denote $CH_2CH_2OCH_2CH_2$, $CH_2CH_2NR^{10}CH_2CH_2$ or $(CH_2)_{3-6}$,
where $R^{10}$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
where $R^9$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^5$ is chosen from $C_{3-8}$-cycloalkyl, aryl or heteroaryl, in each case unsubstituted or mono- or polysubstituted; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, -C (Y)-$CH_2$-$CH^2R^{12}$ or -C(Y)-$CH_2$-$CH_2$-$CH_2R^{12}$
where Y = O, S or $H_2$,
where $R^{11}$ is chosen from
H, $C_{1-7}$-alkyl, saturated or unsaturated, branched or unbranched, mono- or polysubstituted or unsubstituted; or C (O)O-$C_{1-6}$-alkyl, saturated or unsaturated, branched or unbranched, mono- or polysubstituted or unsubstituted;
and where $R^{12}$ is chosen from H; $C_{3-8}$-cycloalkyl, aryl or heteroaryl, in each case unsubstituted or mono- or polysubstituted,
optionally in the form of their racemates, of their pure stereoisomers, in particular enantiomers or diastereomers, or in the form of mixtures of the stereoisomers, in particular of the enantiomers or diastereomers, in any mixing ratio; in the prepared form or in the form of their acids or their bases or in the form of their salts, in particular the physiologically acceptable salts or salts of physiologically acceptable acids or cations; or in the form of their solvates, in particular the hydrates.

**4.** Substituted cyclohexane-1,4-diamine derivatives according to claim 1,
wherein
$R^1$ and $R^2$ independently of one another are chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted; wherein $R^1$ and $R^2$ may not both be H,
or the radicals $R^1$ and $R^2$ together form a ring and denote $CH_2CH_2OCH_2CH_2$, $CH_2CH_2NR^6CH_2CH_2$ or $(CH_2)_{3-6}$, where $R^6$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
$R^3$ is chosen from $C_{1-8}$-alkyl or $C_{3-8}$-cycloalkyl, in each case saturated or unsaturated, branched or unbranched, mono- or polysubstituted or unsubstituted; heteroaryl, unsubstituted or mono-or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^4$ is chosen from H, $C_{1-8}$-alkyl, saturated or unsaturated, branched or unbranched, mono- or polysubstituted or unsubstituted; or $C(X)R^7$, $C(X)NR^7R^8$, $C(X)OR^9$, $C(X)SR^9$, $S(O_2)R^9$
where X = O or S,
where $R^7$ is chosen from H, $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
where $R^8$ is chosen from H, $C_{1-4}$-alkyl, saturated or unsaturated, branched or unbranched, mono- or polysubstituted or unsubstituted or
the radicals $R^7$ and $R^8$ together form a ring and denote $CH_2CH_2OCH_2CH_2$, $CH_2CH_2NR^{10}CH_2CH_2$ or $(CH_2)_{3-6}$,
where $R^{10}$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
where $R^9$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^5$ is chosen from $C_{3-8}$-cycloalkyl, aryl or heteroaryl, in each case unsubstituted or mono- or polysubstituted; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, -C (Y) $R^{12}$, -C (Y) - $CH_2R^{12}$, -C (Y) $-CH_2-CH_2R^{12}$ or -C (Y) $-CH_2-CH_2-CH_2R^{12}$
where Y = O, S or $H_2$,
where $R^{11}$ is chosen from
H, $C_{1-7}$-alkyl, saturated or unsaturated, branched or unbranched, mono- or polysubstituted or unsubstituted; or C (O)O-$C_{1-6}$-alkyl, saturated or unsaturated, branched or unbranched, mono- or polysubstituted or unsubstituted;
and where $R^{12}$ is chosen from
H; $C_{3-8}$-cycloalkyl, aryl or heteroaryl, in each case unsubstituted or mono- or polysubstituted,
or $R^4$ and $R^5$ together form a heterocyclic radical having between 3 and 8 atoms in the ring, saturated or unsaturated; mono- or polysubstituted or unsubstituted, which heterocyclic radical may optionally be fused to further rings;
optionally in the form of their racemates, of their pure stereoisomers, in particular enantiomers or diastereomers, or in the form of mixtures of the stereoisomers, in particular of the enantiomers or diastereomers, in any mixing ratio; in the prepared form or in the form of their acids or their bases or in the form of their salts, in particular the physiologically acceptable salts or salts of physiologically acceptable acids or cations; or in the form of their solvates, in particular the hydrates.

**5.** Substituted cyclohexane-1,4-diamine derivatives according to claim 1,

wherein
the radicals $R^1$ and $R^2$ together form a ring and denote $CH_2CH_2OCH_2CH_2$, $CH_2CH_2NR^6CH_2CH_2$ or $(CH_2)_{3-6}$, where $R^6$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
$R^3$ is chosen from $C_{1-8}$-alkyl or $C_{3-13}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or
polysubstituted;
$R^4$ is chosen from H, $C_{1-8}$-alkyl, saturated or unsaturated, branched or unbranched, mono- or polysubstituted or unsubstituted; or $C(X)R^7$, $C(X)NR^7R^8$, $C(X)OR^9$, $C(X)SR^9$, $S(O_2)R^9$
where X = O or S,
where $R^7$ is chosen from H, $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
where $R^8$ is chosen from H, $C_{1-4}$-alkyl, saturated or unsaturated, branched or unbranched, mono- or polysubstituted or unsubstituted or
the radicals $R^7$ and $R^8$ together form a ring and denote $CH_2CH_2OCH_2CH_2$, $CH_2CH_2NR^{10}CH_2CH_2$ or $(CH_2)_{3-6}$, where $R^{10}$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
where $R^9$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^5$ is chosen from $C_{3-8}$-cycloalkyl, aryl or heteroaryl, in each case unsubstituted or mono- or polysubstituted; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C (Y) - CH_2R^{12}$, $-C (Y) -CH_2-CH_2R^{12}$ or $-C (Y) -CH_2-CH_2-CH_2R^{12}$
where Y = O, S or $H_2$,
where $R^{11}$ is chosen from
H, $C_{1-7}$-alkyl, saturated or unsaturated, branched or unbranched, mono- or polysubstituted or unsubstituted; or $C(O)O-C_{1-6}$-alkyl, saturated or unsaturated, branched or unbranched, mono- or polysubstituted or unsubstituted;
and where $R^{12}$ is chosen from
$C_{3-8}$-cycloalkyl, aryl or heteroaryl, in each case unsubstituted or mono- or polysubstituted,
or $R^4$ and $R^5$ together form a heterocyclic radical having between 3 and 8 atoms in the ring, saturated or unsaturated; mono- or polysubstituted or unsubstituted, which heterocyclic radical may optionally be fused to further rings;
optionally in the form of their racemates, of their pure stereoisomers, in particular enantiomers or diastereomers, or in the form of mixtures of the stereoisomers, in particular of the enantiomers or diastereomers, in any mixing ratio; in the prepared form or in the form of their acids or their bases or in the form of their salts, in particular the physiologically acceptable salts or salts of physiologically acceptable acids or cations; or in the form of their solvates, in particular the hydrates.

**6.** Substituted cyclohexane-1,4-diamine derivatives according to one of claims 1, 2 or 4, **characterized in that**
$R^1$ and $R^2$ independently of one another are chosen from H; $C_{1-8}$-alkyl, saturated or unsaturated, branched or unbranched, mono- or polysubstituted or unsubstituted; wherein $R^1$ and $R^2$ may not both be H,
or the radicals $R^1$ and $R^2$ together form a ring and denote $CH_2CH_2OCH_2CH_2$, $CH_2CH_2NR^6CH_2CH_2$ or $(CH_2)_{3-6}$, where $R^6$ is chosen from H; $C_{1-8}$-alkyl, saturated or unsaturated, branched or unbranched, mono- or polysubstituted or unsubstituted,
preferably
$R^1$ and $R^2$ independently of one another are chosen from H; $C_{1-4}$-alkyl, saturated or unsaturated, branched or unbranched, mono- or polysubstituted or unsubstituted; wherein $R^1$ and $R^2$ may not both be H,
or the radicals $R^1$ and $R^2$ together form a ring and denote $(CH_2)_{4-5}$,
in particular
$R^1$ and $R^2$ independently of one another are chosen from methyl or ethyl or the radicals $R^1$ and $R^2$ together form a

ring and denote $(CH_2)_5$.

7. Substituted cyclohexane-1,4-diamine derivatives according to claim 5, **characterized in that** $R^1$ and $R^2$ together form a ring and denote $CH_2CH_2OCH_2CH_2$, $CH_2CH_2NR^6CH_2CH_2$ or $(CH_2)_{3-6}$, where $R^6$ is chosen from H; $C_{1-8}$-alkyl, saturated or unsaturated, branched or unbranched, mono- or polysubstituted or unsubstituted,
preferably
$R^1$ and $R^2$ together form a ring and denote $(CH_2)_{4-5}$,
in particular
$R^1$ and $R^2$ together form a ring and denote $(CH_2)_5$.

8. Substituted cyclohexane-1,4-diamine derivatives according to claim 3, **characterized in that** $R^1$ and $R^2$ independently of one another are chosen from H; $C_{1-8}$-alkyl, saturated or unsaturated, branched or unbranched, mono- or polysubstituted or unsubstituted; wherein $R^1$ and $R^2$ may not both be H,
preferably
$R^1$ and $R^2$ independently of one another are chosen from H; $C_{1-4}$-alkyl, saturated or unsaturated, branched or unbranched, mono- or polysubstituted or unsubstituted; wherein $R^1$ and $R^2$ may not both be H,
in particular
$R^1$ and $R^2$ independently of one another are chosen from methyl or ethyl.

9. Substituted cyclohexane-1,4-diamine derivatives according to one of claims 1 to 3 or 5, **characterized in that** $R^3$ is chosen from $C_{3-8}$-cycloalkyl, aryl or heteroaryl, in each case unsubstituted or mono- or polysubstituted; aryl, $C_{3-8}$-cycloalkyl or heteroaryl, bonded via a saturated or unsaturated, unbranched, substituted or unsubstituted $C_{1-2}$-alkyl group and in each case unsubstituted or mono- or polysubstituted;
preferably
$R^3$ is chosen from $C_{5-6}$-cycloalkyl, phenyl, naphthyl, anthracenyl, thiophenyl, benzothiophenyl, furyl, benzofuranyl, benzodioxolanyl, indolyl, indanyl, benzodioxanyl, pyrrolyl, pyrimidyl or pyrazinyl, in each case unsubstituted or mono- or polysubstituted; $C_{5-6}$-cycloalkyl, phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl, bonded via a saturated, unbranched $C_{1-2}$-alkyl group and in each case unsubstituted or mono- or polysubstituted;
in particular
$R^3$ is chosen from phenyl, furyl, thiophenyl, cyclohexanyl, naphthyl, benzofuranyl, indolyl, indanyl, benzodioxanyl, benzodioxolanyl, pyrrolyl, pyrimidyl, pyrazinyl or benzothiophenyl, in each case unsubstituted or mono- or polysubstituted; phenyl, furyl or thiophenyl, bonded via a saturated, unbranched $C_{1-2}$-alkyl group and in each case unsubstituted or mono- or polysubstituted.

10. Substituted cyclohexane-1,4-diamine derivatives according to claim 4, **characterized in that** $R^3$ is chosen from $C_{3-8}$-cycloalkyl or heteroaryl, in each case unsubstituted or mono- or polysubstituted; aryl, $C_{3-8}$-cycloalkyl or heteroaryl, bonded via a saturated or unsaturated, unbranched, substituted or unsubstituted $C_{1-4}$-alkyl group and in each case unsubstituted or mono- or polysubstituted;
preferably
$R^3$ is chosen from $C_{5-6}$-cycloalkyl, thiophenyl, benzothiophenyl, furyl, benzofuranyl, benzodioxolanyl, indolyl, indanyl, benzodioxanyl, pyrrolyl, pyrimidyl or pyrazinyl, in each case unsubstituted or mono- or polysubstituted; $C_{5-6}$-cycloalkyl, phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl, bonded via a saturated, unbranched $C_{1-2}$-alkyl group and in each case unsubstituted or mono- or polysubstituted;
in particular
$R^3$ is chosen from furyl, thiophenyl, cyclohexanyl, benzofuranyl, indolyl, indanyl, benzodioxanyl, benzodioxolanyl, pyrrolyl, pyrimidyl, pyrazinyl or benzothiophenyl, in each case unsubstituted or mono- or polysubstituted; phenyl, furyl or thiophenyl, bonded via a saturated, unbranched $C_{1-2}$-alkyl group and in each case unsubstituted or mono- or polysubstituted.

11. Substituted cyclohexane-1,4-diamine derivatives according to one of claims 1 to 10, **characterized in that** $R^4$ is H.

12. Substituted cyclohexane-1,4-diamine derivatives according to one of claims 1 to 10, **characterized in that** $R^4$ is chosen from H, $C(X)R^7$, $C(X)NR^7R^8$, $C(X)OR^9$, $C(X)SR^9$ or $S(O_2)R^9$, where X = O or S,
preferably
$R^4$ is chosen from H, $C(X)R^7$, $C(X)NR^7R^8$ or $C(X)OR^9$, where X = O,

in particular
$R^4$ is chosen from H or $C(O)R^7$; preferably where $R^7$ is chosen from
H; or $C_{1-8}$-alkyl, saturated or unsaturated, branched or unbranched, mono- or polysubstituted or unsubstituted;
preferably
H; or $C_{1-3}$-alkyl, saturated, unsubstituted, branched or unbranched;
in particular $CH_3$.

**13.** Substituted cyclohexane-1,4-diamine derivatives according to one of claims 1, 4 or 5, **characterized in that**
$R^4$ and $R^5$ together form a heterocyclic radical having between 3 and 8 atoms in the ring, saturated or unsaturated; mono- or polysubstituted or unsubstituted, preferably having between 5 and 7 atoms in the ring, of which, in addition to the obligatory N, 0 to 1 further heteroatoms chosen from N, S or O are in the ring;
wherein the heterocyclic radical formed by $R^4$ and $R^5$ together may optionally be fused to further rings,
preferably to aromatic and/or heteroaromatic rings, wherein those rings can be fused to further aromatic and/or heteroaromatic rings,
in particular the heterocyclic radical formed by $R^4$ and $R^5$ together is fused to one or two further rings,
preferably the heterocyclic radical formed by $R^4$ and $R^5$ together is so fused to two further rings that $R^4$ and $R^5$ together denote

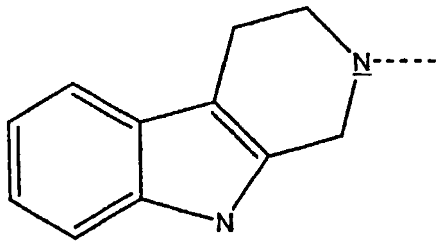

.

**14.** Substituted cyclohexane-1,4-diamine derivatives according to one of claims 1, 4 or 5, **characterized in that**
$R^4$ is chosen from H or $C_{1-8}$-alkyl, saturated or unsaturated, branched or unbranched, mono- or polysubstituted or unsubstituted,
preferably
H or $C_{1-6}$-alkyl, saturated or unsaturated, branched or unbranched, mono- or polysubstituted or unsubstituted,
in particular
H or $C_{1-3}$-alkyl, saturated, unbranched and unsubstituted.

**15.** Substituted cyclohexane-1,4-diamine derivatives according to one of claims 1 to 14, **characterized in that**
$R^5$ is chosen from $C_{3-8}$-cycloalkyl, aryl or heteroaryl, in each case unsubstituted or mono- or polysubstituted;
preferably
$R^5$ is chosen from cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo [1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl, in each case unsubstituted or mono- or polysubstituted;
in particular
$R^5$ is chosen from cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, in each case unsubstituted or mono- or polysubstituted.

**16.** Substituted cyclohexane-1,4-diamine derivatives according to one of claims 1 to 14, **characterized in that**
$R^5$ is chosen from $-CHR^{11}R^{12}$, $-CHR^{11}-CH_2R^{12}$, $-CHR^{11}-CH_2-CH_2R^{12}$; $-CHR^{11}-CH_2-CH_2-CH_2R^{12}$,- $C(Y)R^{12}$, $-C(Y)-CH_2R^{12}$, $-C(Y)-CH_2-CH_2R^{12}$ or $-C(Y)-CH_2-CH_2-CH_2R^{12}$
where Y = O, S or $H_2$,
preferably

$R^5$ is chosen from $-CHR^{11}R^{12}$, $-CHR^{11}-CH_2R^{12}$, $-CHR^{11}-CH_2-CH_2R^{12}$ -C (Y) $R^{12}$, C (Y) - $CH_2R^{12}$ or -C (Y) $-CH_2-CH_2R^{12}$
where Y = O or S,
in particular
$R^5$ is chosen from $-CHR^{11}R^{12}$, $-CHR^{11}-CH_2R^{12}$, $-CHR^{11}-CH_2-CH_2R^{12}$, $-C(Y)R^{12}$ or $-C(Y)-CH_2R^{12}$
where Y = O.

**17.** Substituted cyclohexane-1,4-diamine derivatives according to claim 16, **characterized in that**
$R^{11}$ is chosen from
H, $C_{1-4}$-alkyl, saturated or unsaturated, branched or unbranched, mono- or polysubstituted or unsubstituted; or C (O)O-$C_{1-4}$-alkyl, saturated or unsaturated, branched or unbranched, mono- or polysubstituted or unsubstituted;
preferably
H, $C_{1-4}$-alkyl, saturated or unsaturated, branched or unbranched, mono- or polysubstituted or unsubstituted; or C (O)O-$C_{1-2}$-alkyl, saturated, unbranched, mono- or polysubstituted or unsubstituted;
in particular
H, $CH_3$, $C_2H_5$ and C(O)O-$CH_3$.

**18.** Substituted cyclohexane-1,4-diamine derivatives according to claim 16, **characterized in that**
$R^{12}$ is chosen from $C_{3-8}$-cycloalkyl, aryl or heteroaryl, in each case unsubstituted or mono- or polysubstituted;
preferably
$R^{12}$ is chosen from cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl, in each case unsubstituted or mono- or polysubstituted;
in particular
$R^{12}$ is chosen from cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, in each case unsubstituted or mono- or polysubstituted.

**19.** Substituted cyclohexane-1,4-diamine derivatives according to one of claims 1 to 18, **characterized in that** they are chosen from the following group:

- N'-benzyl-N,N-dimethyl-1-phenyl-cyclohexane-1,4-diamine hydrochloride, nonpolar diastereomer
- N'-benzyl-N,N-dimethyl-1-phenyl-cyclohexane-1,4-diamine hydrochloride, polar diastereomer
- 1,N'-dibenzyl-N,N-dimethyl-cyclohexane-1,4-diamine hydrochloride, nonpolar diastereomer
- 1,N'-dibenzyl-N,N-dimethyl-cyclohexane-1,4-diamine hydrochloride, polar diastereomer
- N-(4-benzyl-4-dimethylamino-cyclohexyl)-N-propyl-benzamide hydrochloride
- N,N-dimethyl-1-phenyl-N'-propyl-cyclohexane-1,4-diamine hydrochloride, nonpolar diastereomer
- N-(4-dimethylamino-4-phenyl-cyclohexyl)-N-propyl-benzamide hydrochloride, nonpolar diastereomer
- N-(4-dimethylamino-4-phenyl-cyclohexyl)-N-propyl-benzamide hydrochloride, polar diastereomer
- 1,N'-dibenzyl-N,N,N'-trimethyl-cyclohexane-1,4-diamine hydrochloride, nonpolar diastereomer
- 1,N'-dibenzyl-N,N,N'-trimethyl-cyclohexane-1,4-diamine hydrochloride, polar diastereomer
- N-(4-benzyl-4-dimethylamino-cyclohexyl)-N-methyl-benzamide hydrochloride, polar diastereomer
- N-(4-benzyl-4-dimethylamino-cyclohexyl)-N-ethyl-benzamide hydrochloride, polar diastereomer
- 1-benzyl-N'-(1H-indol-3-ylmethyl)-N,N-dimethylcyclohexane-1,4-diamine dihydrochloride
- 1-benzyl-N'-[2-(1H-indol-3-yl)-1-methyl-ethyl]-N,N-dimethyl-cyclohexane-1,4-diamine, cis/trans mixture
- 1-benzyl-N'-indan-5-yl-N,N-dimethyl-cyclohexane-1,4-diamine hydrochloride
- 1-benzyl-N'-indan-1-yl-N,N-dimethyl-cyclohexane-1,4-diamine dihydrochloride, cis/trans mixture
- N'-indan-1-yl-N,N-dimethyl-1-phenyl-cyclohexane-1,4-diamine
- N'-(1H-indol-5-yl)-N,N-dimethyl-1-phenylcyclohexane-1,4-diamine
- N'-(1H-indol-3-ylmethyl)-N,N-dimethyl-1-phenylcyclohexane-1,4-diamine, cis/trans mixture
- N'-(1H-indol-3-ylmethyl)-N,N-dimethyl-1-phenylcyclohexane-1,4-diamine, nonpolar diastereomer
- N'-[2-(1H-indol-3-yl)-ethyl]-N,N-dimethyl-1-phenyl-cyclohexane-1,4-diamine, nonpolar diastereomer
- N'-[2-(1H-indol-3-yl)-ethyl]-N,N-dimethyl-1-phenyl-cyclohexane-1,4-diamine, cis/trans mixture
- N'-indan-5-yl-N,N-dimethyl-1-phenyl-cyclohexane-1,4-diamine, nonpolar diastereomer
- N'-[2-(1H-indol-3-yl)-1-methyl-ethyl]-N,N-dimethyl-1-phenyl-cyclohexane-1,4-diamine, nonpolar diastereomer

- N'-[2-(1H-indol-3-yl)-1-methyl-ethyl]-N,N-dimethyl-1-phenyl-cyclohexane-1,4-diamine, cis/trans mixture
- N'-[2-(5-benzyloxy-1H-indol-3-yl)-ethyl]-N,N-dimethyl-1-phenyl-cyclohexane-1,4-diamine, cis/trans mixture
- N'-(9H-fluoren-1-yl)-N,N-dimethyl-1-phenylcyclohexane-1,4-diamine dihydrochloride
- N'-indan-2-yl-N,N-dimethyl-1-phenyl-cyclohexane-1,4-diamine dihydrochloride, cis/trans mixture
- N'-(9H-fluoren-9-yl)-N,N-dimethyl-1-phenylcyclohexane-1,4-diamine dihydrochloride, cis/trans mixture
- 1-benzyl-N'-(9H-fluoren-9-yl)-N,N-dimethylcyclohexane-1,4-diamine
- 1-benzyl-N'-(1H-indol-3-ylmethyl)-N,N-dimethylcyclohexane-1,4-diamine, cis/trans mixture
- N,N-dimethyl-N'-(1-methyl-1H-indol-3-ylmethyl)-1-phenyl-cyclohexane-1,4-diamine, cis/trans mixture
- N,N-dimethyl-N'-(1-methyl-1H-indol-3-ylmethyl)-1-phenyl-cyclohexane-1,4-diamine, polar diastereomer
- N'-(2-benzo[b]thiophen-3-yl-ethyl)-N,N-dimethyl-1-phenyl-cyclohexane-1,4-diamine dihydrochloride, cis/trans mixture
- N'-(2-benzo[b]thiophen-3-yl-ethyl)-1-benzyl-N,N-dimethyl-cyclohexane-1,4-diamine dihydrochloride, cis/trans mixture
- N'-acenaphthen-1-yl-N,N-dimethyl-1-phenylcyclohexane-1,4-diamine dihydrochloride, polar diastereomer
- N'-acenaphthen-1-yl-N,N-dimethyl-1-phenylcyclohexane-1,4-diamine dihydrochloride, nonpolar diastereomer
- N'-benzo[b]thiophen-5-yl-1-benzyl-N,N-dimethylcyclohexane-1,4-diamine dihydrochloride, nonpolar diastereomer
- N'-benzo[b]thiophen-5-yl-N,N-dimethyl-1-phenylcyclohexane-1,4-diamine hydrochloride, nonpolar diastereomer
- N'-benzothiazol-6-yl-N,N-dimethyl-1-phenylcyclohexane-1,4-diamine dihydrochloride, nonpolar diastereomer
- N'-benzo[1,2,5]thiadiazol-4-yl-N,N-dimethyl-1-phenyl-cyclohexane-1,4-diamine dihydrochloride, polar diastereomer
- N'-[2-(1H-indol-3-yl)-1-methyl-ethyl]-N,N-dimethyl-1-phenyl-cyclohexane-1,4-diamine dihydrochloride, nonpolar diastereomer
- N'-adamantan-2-yl-N,N-dimethyl-1-phenylcyclohexane-1,4-diamine dihydrochloride
- N'-(9-ethyl-9H-carbazol-3-yl)-N,N-dimethyl-1-phenyl-cyclohexane-1,4-diamine dihydrochloride, nonpolar diastereomer
- N'-(3H-benzotriazol-5-yl)-N,N-dimethyl-1-phenylcyclohexane-1,4-diamine hydrochloride, nonpolar diastereomer
- N'-(3H-benzotriazol-5-yl)-N,N-dimethyl-1-phenylcyclohexane-1,4-diamine hydrochloride, polar diastereomer
- N'-(9H-fluoren-9-yl)-N,N-dimethyl-1-thiophen-2-yl-cyclohexane-1,4-diamine dihydrochloride, cis/trans mixture
- N'-cyclooctyl-N,N-dimethyl-l-phenyl-cyclohexane-1,4-diamine dihydrochloride
- N'-(1H-indol-3-ylmethyl)-N,N-dimethyl-1-phenylcyclohexane-1,4-diamine dihydrochloride, nonpolar diastereomer
- N'-(1H-indol-3-ylmethyl)-N,N-dimethyl-1-phenylcyclohexane-1,4-diamine dihydrochloride, polar diastereomer
- N'-benzo[b]thiophen-3-ylmethyl-N,N-dimethyl-1-phenyl-cyclohexane-1,4-diamine dihydrochloride, nonpolar diastereomer
- N'-benzo [b] thiophen-3-ylmethyl-N,N-dimethyl-1-phenyl-cyclohexane-1,4-diamine dihydrochloride, polar diastereomer
- N'-anthracen-2-yl-N,N-dimethyl-1-phenylcyclohexane-1,4-diamine hydrochloride, nonpolar diastereomer
- N'-benzo[b]thiophen-3-ylmethyl-1-benzyl-N,N-dimethyl-cyclohexane-1,4-diamine dihydrochloride, nonpolar diastereomer
- N'-benzo[b] thiophen-3-ylmethyl-1-benzyl-N,N-dimethyl-cyclohexane-1,4-diamine dihydrochloride, polar diastereomer
- N'-[2-(1H-indol-3-yl)-ethyl]-N,N-dimethyl-1-naphthalen-2-yl-cyclohexane-1,4-diamine dihydrochloride, nonpolar diastereomer
- N'-[2-(1H-indol-3-yl)-ethyl]-N,N-dimethyl-1-phenyl-cyclohexane-1,4-diamine dihydrochloride, nonpolar diastereomer
- N'-[2-(1H-indol-3-yl)-ethyl]-N,N-dimethyl-1-phenyl-cyclohexane-1,4-diamine dihydrochloride, polar diastereomer
- N'-[2-(1H-indol-3-yl)-1-methyl-ethyl]-N,N-dimethyl-1-phenyl-cyclohexane-1,4-diamine dihydrochloride, polar diastereomer
- methyl 2-(4-dimethylamino-4-phenyl-cyclohexylamino)-3-(1H-indol-3-yl)-propionate dihydrochloride, nonpolar diastereomer

● methyl 2-(4-dimethylamino-4-phenyl-cyclohexylamino)-3-(1H-indol-3-yl)-propionate dihydrochloride, polar diastereomer

● N'-[2-(1H-indol-3-yl)-1-methyl-ethyl]-N,N-dimethyl-1-naphthalen-2-yl-cyclohexane-1,4-diamine dihydrochloride, nonpolar diastereomer

● N'-benzo[1,3]dioxol-5-ylmethyl-N,N-dimethyl-1-phenyl-cyclohexane-1,4-diamine dihydrochloride, cis/trans mixture

● N'-[2-(6-fluoro-1H-indol-3-yl)-ethyl]-N,N-dimethyl-1-phenyl-cyclohexane-1,4-diamine dihydrochloride, nonpolar diastereomer

● N'-[2-(6-fluoro-1H-indol-3-yl)-ethyl]-N,N-dimethyl-1-phenyl-cyclohexane-1,4-diamine dihydrochloride, polar diastereomer

● N'-[2-(1H-indol-3-yl)-ethyl]-N,N,N'-trimethyl-1-phenyl-cyclohexane-1,4-diamine dihydrochloride, nonpolar diastereomer

● N'-[2-(1H-indol-3-yl)-ethyl]-N,N,N'-trimethyl-1-phenyl-cyclohexane-1,4-diamine dihydrochloride, polar diastereomer

● N,N-dimethyl-N'-[2-(7-methyl-1H-indol-3-yl)-ethyl]-1-phenyl-cyclohexane-1,4-diamine dihydrochloride, nonpolar diastereomer

● N,N-dimethyl-N'-[2-(7-methyl-1H-indol-3-yl)-ethyl]-1-phenyl-cyclohexane-1,4-diamine dihydrochloride, polar diastereomer

● N'-[2-(5-fluoro-1H-indol-3-yl)-ethyl]-N,N-dimethyl-1-phenyl-cyclohexane-1,4-diamine dihydrochloride, nonpolar diastereomer

● N'-[2-(5-fluoro-1H-indol-3-yl)-ethyl]-N,N-dimethyl-l-phenyl-cyclohexane-1,4-diamine dihydrochloride, polar diastereomer

● N'-acenaphthen-5-ylmethyl-N,N-dimethyl-1-phenylcyclohexane-1,4-diamine dihydrochloride, nonpolar diastereomer

● N'-[2-(1H-indol-3-yl)-1-methyl-ethyl]-N,N-dimethyl-1-thiophen-2-yl-cyclohexane-1,4-diamine dihydrochloride, nonpolar diastereomer

● N'-[2-(1H-indol-3-yl)-1-methyl-ethyl]-N,N-dimethyl-1-thiophen-2-yl-cyclohexane-1,4-diamine dihydrochloride, cis/trans mixture

● N'-[2-(7-benzyloxy-1H-indol-3-yl)-ethyl]-N,N-dimethyl-1-phenyl-cyclohexane-1,4-diamine dihydrochloride, nonpolar diastereomer

● N'-cyclooctyl-N,N-dimethyl-1-thiophen-2-yl-cyclohexane-1,4-diamine dihydrochloride, nonpolar diastereomer

● N'-adamantan-2-yl-N,N-dimethyl-1-thiophen-2-yl-cyclohexane-1,4-diamine dihydrochloride, nonpolar diastereomer

● 3-[2-(4-dimethylamino-4-phenyl-cyclohexylamino)-ethyl]-1H-indol-5-ol dihydrochloride, nonpolar diastereomer

● 3-[2-(4-dimethylamino-4-phenyl-cyclohexylamino)-ethyl]-1H-indol-5-0l dihydrochloride, polar diastereomer

● N'-[2-(5-methoxy-1H-indol-3-yl)-ethyl]-N,N-dimethyl-1-phenyl-cyclohexane-1,4-diamine dihydrochloride, nonpolar diastereomer

● N'-[2-(5-methoxy-1H-indol-3-yl)-ethyl]-N,N-dimethyl-1-phenyl-cyclohexane-1,4-diamine dihydrochloride, polar diastereomer

● N,N-dimethyl-N'-[2-(5-methyl-1H-indol-3-yl)-ethyl]-1-phenyl-cyclohexane-1,4-diamine dihydrochloride, nonpolar diastereomer

● N,N-dimethyl-N'-[2-(5-methyl-1H-indol-3-yl)-ethyl]-1-phenyl-cyclohexane-1,4-diamine dihydrochloride, polar diastereomer

● dimethyl-[1-phenyl-4-(1,3,4,9-tetrahydro-b-carbolin-2-yl)-cyclohexyl]-amine dihydrochloride

● N-(4-dimethylamino-4-phenyl-cyclohexyl)-N-[2-(4-fluoro-phenyl)-ethyl]-acetamide hydrochloride, nonpolar diastereomer

● 2-(4-dimethylamino-4-phenyl-cyclohexylamino)-3-(5-fluoro-1H-indol-3-yl)-propionic acid methyl ester dihydrochloride, nonpolar diastereomer

● N-(4-dimethylamino-4-phenyl-cyclohexyl)-N-(3-phenyl-propyl)-acetamide hydrochloride, nonpolar diastereomer

● 2-(4-dimethylamino-4-phenyl-cyclohexylamino)-3-(6-fluoro-1H-indol-3-yl)-propionic acid methyl ester dihydrochloride, nonpolar diastereomer

● N-(4-dimethylamino-4-phenyl-cyclohexyl)-2-(1H-indol-3-yl)-acetamide hydrochloride, polar diastereomer

● 2-(4-dimethylamino-4-thiophen-2-yl-cyclohexylamino)-3-(1H-indol-3-yl)-propionic acid methyl ester dihydrochloride, nonpolar diastereomer

● N-(4-dimethylamino-4-phenyl-cyclohexyl)-2-(5-methoxy-1H-indol-3-yl)-acetamide hydrochloride, nonpolar diastereomer

optionally also in the form of their racemates, of the mentioned or other pure stereoisomers, in particular enantiomers or diastereomers, or in the form of mixtures of the stereoisomers, in particular of the enantiomers or diastereomers, in any mixing ratio; optionally also in the form of the acids or bases or in the form of other salts, in particular physiologically acceptable salts or salts of physiologically acceptable acids or cations; or in the form of their solvates, in particular the hydrates.

**20.** Medicaments comprising at least one substituted cyclohexane-1,4-diamine derivative according claim 1, wherein
$R^1$ and $R^2$ independently of one another are chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted; wherein $R^1$ and $R^2$ may not both be H,
or the radicals $R^1$ and $R^2$ together form a ring and denote $CH_2CH_2OCH_2CH_2$, $CH_2CH_2NR^6H_2CH_2$ or $(CH_2)_{3-6}$,
where $R^6$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
$R^3$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^4$ is chosen from H, $C_{1-8}$-alkyl, saturated or unsaturated, branched or unbranched, mono- or polysubstituted or unsubstituted; or $C(X)R^7$, $C(X)NR^7R^8$, $C(X)OR^9$, $C(X)SR^9$, $S(O_2)R^9$
where X = O or S,
where $R^7$ is chosen from H, $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
where $R^8$ is chosen from H, $C_{1-4}$-alkyl, saturated or unsaturated, branched or unbranched, mono- or polysubstituted or unsubstituted or
the radicals $R^7$ and $R^8$ together form a ring and denote $CH_2CH_2OCH_2CH_2$, $CH_2CH_2NR^{10}CH_2CH_2$ or $(CH_2)_{3-6}$,
where $R^{10}$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
where $R^9$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^5$ is chosen from $C_{3-8}$-cycloalkyl, aryl or heteroaryl, in each case unsubstituted or mono- or polysubstituted; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C (Y) -CH_2-CH_2R^{12}$ or $-C (Y) -CH_2-CH_2-CH_2R^{12}$
where Y = O, S or $H_2$,
where $R^{11}$ is chosen from
H, $C_{1-7}$-alkyl, saturated or unsaturated, branched or unbranched, mono- or polysubstituted or unsubstituted; or C (O) O-$C_{1-6}$-alkyl, saturated or unsaturated, branched or unbranched, mono- or polysubstituted or unsubstituted;
and where $R^{12}$ is chosen from
H; $C_{3-8}$-cycloalkyl, aryl or heteroaryl, in each case unsubstituted or mono- or polysubstituted,
or $R^4$ and $R^5$ together form a heterocyclic radical having between 3 and 8 atoms in the ring, saturated or unsaturated; mono- or polysubstituted or unsubstituted, which heterocyclic radical may optionally be fused to further rings,
with the proviso,

■ that if $R^3$ is substituted or unsubstituted phenyl and at least one of $R^1$ or $R^2$ is H or $C_{1-8}$-alkyl, $R^4$ may not be alkyl and $R^4$ and $R^5$ may not together form a heterocyclic radical

optionally in the form of its racemate, of the pure stereoisomers, in particular enantiomers or diastereomers, or in the form of mixtures of the stereoisomers, in particular of the enantiomers or diastereomers, in any mixing ratio; in the prepared form or in the form of its acids or its bases or in the form of its salts, in particular the physiologically acceptable salts or salts of physiologically acceptable acids or cations; or in the form of its solvates, in particular the hydrates, and optionally comprising suitable additives and/or auxiliary substances and/or optionally further active compounds.

**21.** Medicaments comprising at least one substituted cyclohexane-1,4-diamine derivative according to claim 1, wherein
$R^1$ and $R^2$ independently of one another are chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted; wherein $R^1$ and $R^2$ may not both be H,
or the radicals $R^1$ and $R^2$ together form a ring and denote $CH_2CH_2OCH_2CH_2$, $CH_2CH_2NR^6CH_2CH_2$ or $(CH_2)_{3-6}$,
where $R^6$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
$R^3$ is chosen from $C_{1-8}$-alkyl or $C_{3-8}$-cycloalkyl, in each case saturated or unsaturated, branched or unbranched, mono- or polysubstituted or unsubstituted; heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^4$ is chosen from H, $C_{1-8}$-alkyl, saturated or unsaturated, branched or unbranched, mono- or polysubstituted or unsubstituted; or $C(X)R^7$, $C(X)NR^7R^8$, $C(X)OR^9$, $C(X)SR^9$, $S(O_2)R^9$
where X = O or S,
where $R^7$ is chosen from H, $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
where $R^8$ is chosen from H, $C_{1-4}$-alkyl, saturated or unsaturated, branched or unbranched, mono- or polysubstituted or unsubstituted or
the radicals $R^7$ and $R^8$ together form a ring and denote $CH_2CH_2OCH_2CH_2$, $CH_2CH_2NR^{10}CH_2CH_2$ or $(CH_2)_{3-6}$,
where $R^{10}$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
where $R^9$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^5$ is chosen from $C_{3-8}$-cycloalkyl, aryl or heteroaryl, in each case unsubstituted or mono- or polysubstituted; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ or $-C(Y)-CH_2-CH_2-CH_2R^{12}$
where Y = O, S or $H_2$,
where $R^{11}$ is chosen from
H, $C_{3-7}$-alkyl, saturated or unsaturated, branched or unbranched, mono- or polysubstituted or unsubstituted; or C (O) O-$C_{1-6}$-alkyl, saturated or unsaturated, branched or unbranched, mono- or polysubstituted or unsubstituted;
and where $R^{12}$ is chosen from
H; $C_{3-8}$-cycloalkyl, aryl or heteroaryl, in each case unsubstituted or mono- or polysubstituted,
or $R^4$ and $R^5$ together form a heterocyclic radical having between 3 and 8 atoms in the ring, saturated or unsaturated; mono- or polysubstituted or unsubstituted, which heterocyclic radical may optionally be fused to further rings,
optionally in the form of its racemate, of the pure stereoisomers, in particular enantiomers or diastereomers, or in the form of mixtures of the stereoisomers, in particular of the enantiomers or diastereomers, in any mixing ratio;
in the prepared form or in the form of its acids or its bases or in the form of its salts, in particular the physiologically acceptable salts or salts of physiologically acceptable acids or cations; or in the form of its solvates, in particular the hydrates, and optionally comprising suitable additives and/or auxiliary substances and/or optionally further active compounds.

**22.** Medicaments comprising at least one substituted cyclohexane-1,4-diamine derivative according to claim 1, wherein
$R^1$ and $R^2$ together form a ring and denote $CH_2CH_2OCH_2CH_2$, $CH_2CH_2NR^6CH_2CH_2$ or $(CH_2)_{3-6}$,
where $R^6$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
$R^3$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_3$-$_8$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^4$ is chosen from H, $C_{1-8}$-alkyl, saturated or unsaturated, branched or unbranched, mono- or polysubstituted or unsubstituted; or $C(X)R^7$, $C(X)NR^7R^8$, $C(X)OR^9$, $C(X)SR^9$, $S(O_2)R^9$
where X = O or S,
where $R^7$ is chosen from H, $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
where $R^8$ is chosen from H, $C_{1-4}$-alkyl, saturated or unsaturated, branched or unbranched, mono- or polysubstituted or unsubstituted or
the radicals $R^7$ and $R^8$ together form a ring and denote $CH_2CH_2OCH_2CH_2$, $CH_2CH_2NR^{10}CH_2CH_2$ or $(CH_2)_{3-6}$,
where $R^{10}$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
where $R^9$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^5$ is chosen from $C_{3-8}$-cycloalkyl, aryl or heteroaryl, in each case unsubstituted or mono- or polysubstituted; $-CHR^{11}R^{12}$, $-CHR^{11}-CH_2R^{12}$, $-CHR^{11}-CH_2-CH_2R^{12}$, $CHR^{11}-CH_2-CH_2-CH_2R^{12}$, $-C(Y)R^{12}$, $-C(Y)-CH_2R^{12}$, $-C(Y)-CH_2-CH_2R^{12}$ or $-C(Y)-CH_2-CH_2-CH_2R^{12}$
where Y = O, S or $H_2$,
where $R^{11}$ is chosen from
H, $C_{1-7}$-alkyl, saturated or unsaturated, branched or unbranched, mono- or polysubstituted or unsubstituted; or C(O)O-$C_{1-6}$-alkyl, saturated or unsaturated, branched or unbranched, mono- or polysubstituted or unsubstituted;
and where $R^{12}$ is chosen from
H; $C_{3-8}$-cycloalkyl, aryl or heteroaryl, in each case unsubstituted or mono- or polysubstituted,
or $R^4$ and $R^5$ together form a heterocyclic radical having between 3 and 8 atoms in the ring, saturated or unsaturated; mono- or polysubstituted or unsubstituted, which heterocyclic radical may optionally be fused to further rings,
optionally in the form of its racemate, of the pure stereoisomers, in particular enantiomers or diastereomers, or in the form of mixtures of the stereoisomers, in particular of the enantiomers or diastereomers, in any mixing ratio; in the prepared form or in the form of its acids or its bases or in the form of its salts, in particular the physiologically acceptable salts or salts of physiologically acceptable acids or cations; or in the form of its solvates, in particular the hydrates, and optionally comprising suitable additives and/or auxiliary substances and/or optionally further active compounds.

**23.** Medicaments comprising at least one substituted cyclohexane-1,4-diamine derivative according to claim 1, wherein
$R^1$ and $R^2$ independently of one another are chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted; wherein $R^1$ and $R^2$ may not both be H,
or the radicals $R^1$ and $R^2$ together form a ring and denote $CH_2CH_2OCH_2CH_2$, $CH_2CH_2NR^6CH_2CH_2$ or $(CH_2)_{3-6}$,
where $R^6$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;

$R^3$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^4$ is chosen from H, $C(X)R^7$, $C(X)NR^7R^8$, C(X) $OR^9$, C (X) $SR^9$ or $S(O_2)R^9$
where X = O or S,
where $R^7$ is chosen from H, $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
where $R^8$ is chosen from H, $C_{1-4}$-alkyl, saturated or unsaturated, branched or unbranched, mono- or polysubstituted or unsubstituted or
the radicals $R^7$ and $R^8$ together form a ring and denote $CH_2CH_2OCH_2CH_2$, $CH_2CH_2NR^{10}CH_2CH_2$ or $(CH_2)_{3-6}$,
where $R^{10}$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
where $R^9$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^5$ is chosen from $C_{3-8}$-cycloalkyl, aryl or heteroaryl, in each case unsubstituted or mono- or polysubstituted; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, -C $(Y)-CH_2R^{12}$, -C $(Y)-CH_2-CH_2R^{12}$ or $-C(Y)-CH_2-CH_2-CH_2R^{12}$
where Y = O, S or $H_2$,
where $R^{11}$ is chosen from
H, $C_{1-7}$-alkyl, saturated or unsaturated, branched or unbranched, mono- or polysubstituted or unsubstituted; or C (O)O-$C_{1-6}$-alkyl, saturated or unsaturated, branched or unbranched, mono- or polysubstituted or unsubstituted;
and where $R^{12}$ is chosen from
H; $C_{3-8}$-cycloalkyl, aryl or heteroaryl, in each case unsubstituted or mono- or polysubstituted,
optionally in the form of its racemate, of the pure stereoisomers, in particular enantiomers or diastereomers, or in the form of mixtures of the stereoisomers, in particular of the enantiomers or diastereomers, in any mixing ratio;
in the prepared form or in the form of its acids or its bases or in the form of its salts, in particular the physiologically acceptable salts or salts of physiologically acceptable acids or cations; or in the form of its solvates, in particular the hydrates, and optionally comprising suitable additives and/or auxiliary substances and/or optionally further active compounds.

**24.** Medicaments according to one of claims 20 or 21, **characterized in that** in the substituted cyclohexane-1,4-diamine derivatives contained therein
$R^1$ and $R^2$ independently of one another are chosen from H; $C_{1-8}$-alkyl, saturated or unsaturated, branched or unbranched, mono- or polysubstituted or unsubstituted; wherein $R^1$ and $R^2$ may not both be H,
or the radicals $R^1$ and $R^2$ together form a ring and denote $CH_2CH_2OCH_2CH_2$, $CH_2CH_2NR^6CH_2CH_2$ or $(CH_2)_{3-6}$,
where $R^6$ is chosen from H; $C_{1-8}$-alkyl, saturated or unsaturated, branched or unbranched, mono- or polysubstituted or unsubstituted,
preferably
$R^1$ and $R^2$ independently of one another are chosen from H; $C_{1-4}$-alkyl, saturated or unsaturated, branched or unbranched, mono- or polysubstituted or unsubstituted; wherein $R^1$ and $R^2$ may not both be H,
or the radicals $R^1$ and $R^2$ together form a ring and denote $(CH_2)_{4-5}$,
in particular
$R^1$ and $R^2$ independently of one another are chosen from methyl or ethyl or the radicals $R^1$ and $R^2$ together form a ring and denote $(CH_2)_5$.

**25.** Medicaments according to claim 22, **characterized in that** in the substituted cyclohexane-1,4-diamine derivatives contained therein
$R^1$ and $R^2$ together form a ring and denote $CH_2CH_2OCH_2CH_2$, $CH_2CH_2NR^6CH_2CH_2$ or $(CH_2)_{3-6}$,
where $R^6$ is chosen from H; $C_{1-8}$-alkyl, saturated or unsaturated, branched or unbranched, mono- or polysubstituted or unsubstituted,
preferably

$R^1$ and $R^2$ together form a ring and denote $(CH_2)_{4-5}$,
in particular
$R^1$ and $R^2$ together form a ring and denote $(CH_2)_5$.

**26.** Medicaments according to claim 23, **characterized in that** in the substituted cyclohexane-1,4-diamine derivatives contained therein
$R^1$ and $R^2$ independently of one another are chosen from H; $C_{1-8}$-alkyl, saturated or unsaturated, branched or unbranched, mono- or polysubstituted or unsubstituted; wherein $R^1$ and $R^2$ may not both be H,
preferably
$R^1$ and $R^2$ independently of one another are chosen from H; $C_{1-4}$-alkyl, saturated or unsaturated, branched or unbranched, mono- or polysubstituted or unsubstituted; wherein $R^1$ and $R^2$ may not both be H,
in particular
$R^1$ and $R^2$ independently of one another are chosen from methyl or ethyl;
or
$R^1$ and $R^2$ together form a ring and denote $CH_2CH_2OCH_2CH_2$, $CH_2CH_2NR^6CH_2CH_2$ or $(CH_2)_{3-6,}$
where $R^6$ is chosen from H; $C_{1-8}$-alkyl, saturated or unsaturated, branched or unbranched, mono- or polysubstituted or unsubstituted,
preferably
$R^1$ and $R^2$ together form a ring and denote $(CH_2)_{4-5}$,
in particular
$R^1$ and $R^2$ together form a ring and denote $(CH_2)_5$.

**27.** Medicaments according to one of claims 20, 22 or 23, **characterized in that** in the substituted cyclohexane-1,4-diamine derivatives contained therein
$R^3$ is chosen from $C_{3-8}$-cycloalkyl, aryl or heteroaryl, in each case unsubstituted or mono- or polysubstituted; aryl, $C_{3-8}$-cycloalkyl or heteroaryl, bonded via a saturated or unsaturated, unbranched, substituted or unsubstituted $C_{1-2}$-alkyl group and in each case unsubstituted or mono- or polysubstituted;
preferably
$R^3$ is chosen from $C_{5-6}$-cycloalkyl, phenyl, naphthyl, anthracenyl, thiophenyl, benzothiophenyl, furyl, benzofuranyl, benzodioxolanyl, indolyl, indanyl, benzodioxanyl, pyrrolyl, pyrimidyl or pyrazinyl, in each case unsubstituted or mono- or polysubstituted; $C_{5-6}$-cycloalkyl, phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl, bonded via a saturated, unbranched $C_{1-2}$-alkyl group and in each case unsubstituted or mono- or polysubstituted;
in particular
$R^3$ is chosen from phenyl, furyl, thiophenyl, cyclohexanyl, naphthyl, benzofuranyl, indolyl, indanyl, benzodioxanyl, benzodioxolanyl, pyrrolyl, pyrimidyl, pyrazinyl or benzothiophenyl, in each case unsubstituted or mono- or polysubstituted; phenyl, furyl or thiophenyl, bonded via a saturated, unbranched $C_{1-2}$-alkyl group and in each case unsubstituted or mono- or polysubstituted.

**28.** Medicaments according to claim 21, **characterized in that** in the substituted cyclohexane-1,4-diamine derivatives contained therein
$R^3$ is chosen from $C_{3-8}$-cycloalkyl or heteroaryl, in each case unsubstituted or mono- or polysubstituted; aryl, $C_{3-8}$-cycloalkyl or heteroaryl, bonded via a saturated or unsaturated, unbranched, substituted or unsubstituted $C_{1-4}$-alkyl group and in each case unsubstituted or mono- or polysubstituted;
preferably
$R^3$ is chosen from $C_{5-6}$-cycloalkyl, thiophenyl, benzothiophenyl, furyl, benzofuranyl, benzodioxolanyl, indolyl, indanyl, benzodioxanyl, pyrrolyl, pyrimidyl or pyrazinyl, in each case unsubstituted or mono- or polysubstituted; $C_{5-6}$-cycloalkyl, phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl, bonded via a saturated, unbranched $C_{1-2}$-alkyl group and in each case unsubstituted or mono- or polysubstituted;
in particular
$R^3$ is chosen from furyl, thiophenyl, cyclohexanyl, benzofuranyl, indolyl, indanyl, benzodioxanyl, benzodioxolanyl, pyrrolyl, pyrimidyl, pyrazinyl or benzothiophenyl, in each case unsubstituted or mono- or polysubstituted; phenyl, furyl or thiophenyl, bonded via a saturated, unbranched $C_{1-2}$-alkyl group and in each case unsubstituted or mono- or polysubstituted.

**29.** Medicaments according to one of claims 20 to 28, **characterized in that** in the substituted cyclohexane-1,4-diamine derivatives contained therein, $R^4$ is H.

**30.** Medicaments according to one of claims 20 to 28, **characterized in that** in the substituted cyclohexane-1,4-diamine derivatives contained therein
$R^4$ is chosen from H, $C(X)R^7$, $C(X)NR^7R^8$, $C(X)OR^9$, $C(X)SR^9$ or $S(O_2)R^9$, where X = O or S,
preferably
$R^4$ is chosen from H, $C(X)R^7$, C (X) $NR^7R^8$ or C (X) $OR^9$, where X = O,
in particular
$R^4$ is chosen from H or $C(O)R^7$; preferably where $R^7$ is chosen from
H; or $C_{1-8}$-alkyl, saturated or unsaturated, branched or unbranched, mono- or polysubstituted or unsubstituted;
preferably
H; or $C_{1-3}$-alkyl, saturated, unsubstituted, branched or unbranched;
in particular $CH_3$.

**31.** Medicaments according to one of claims 20 to 22, **characterized in that** in the substituted cyclohexane-1,4-diamine derivatives contained therein
$R^4$ and $R^5$ together form a heterocyclic radical having between 3 and 8 atoms in the ring, saturated or unsaturated; mono- or polysubstituted or unsubstituted, preferably having between 5 and 7 atoms in the ring, of which, in addition to the obligatory N, 0 to 1 further heteroatoms chosen from N, S or O are in the ring;
wherein the heterocyclic radical formed by $R^4$ and $R^5$ together may optionally be fused to further rings,
preferably to aromatic and/or heteroaromatic rings, wherein those rings can be fused to further aromatic and/or heteroaromatic rings,
in particular the heterocyclic radical formed by $R^4$ and $R^5$ together is fused to one or two further rings,
preferably the heterocyclic radical formed by $R^4$ and $R^5$ together is so fused to two further rings that $R^4$ and $R^5$ together denote

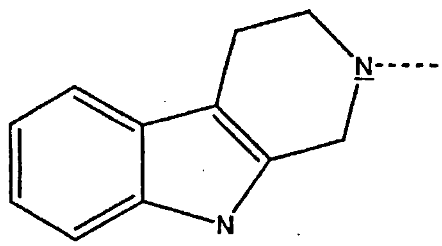

**32.** Medicaments according to one of claims 20 to 22, **characterized in that** in the substituted cyclohexane-1,4-diamine derivatives contained therein
$R^4$ is chosen from H, $C_{1-8}$-alkyl, saturated or unsaturated, branched or unbranched, mono- or polysubstituted or unsubstituted,
preferably
H, $C_{1-6}$-alkyl, saturated or unsaturated, branched or unbranched, mono- or polysubstituted or unsubstituted,
in particular
H, $C_{1-3}$-alkyl, saturated, unbranched and unsubstituted.

**33.** Medicaments according to one of claims 20 to 32, **characterized in that** in the substituted cyclohexane-1,4-diamine derivatives contained therein
$R^5$ is chosen from $C_{3-8}$-cycloalkyl, aryl or heteroaryl, in each case unsubstituted or mono- or polysubstituted;
preferably
$R^5$ is chosen from cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo [1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl, in each case unsubstituted or mono- or polysubstituted;
in particular
$R^5$ is chosen from cyclopentyl, cyclohexyl, cycloheptyl, cyclooctyl, anthracenyl, indolyl, naphthyl, benzofuranyl,

benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, in each case unsubstituted or mono- or polysubstituted.

**34.** Medicaments according to one of claims 20 to 32, **characterized in that** in the substituted cyclohexane-1,4-diamine derivatives contained therein
$R^5$ is chosen from $-CHR^{11}R^{12}$, $-CHR^{11}-CH_2R^{12}$, $-CHR^{11}-$ $-CH_2-CH_2R^{12}$, $CHR^{11}-$ $CH_2-CH_2-CH_2R^{12}$, $-C(Y)R^{12}$, $-C(Y)-CH_2R^{12}$, $-C(Y)-$ $CH_2-CH_2R^{12}$ or $-C(Y)-CH_2-CH_2-CH_2R^{12}$
where Y = O, S or $H_2$,
preferably
$R^5$ is chosen from $-CHR^{11}R^{12}$, $-CHR^{11}-CH_2R^{12}$, $-CHR^{11}-CH_2-CH_2R^{12}$, $-C(Y)-CH_2R^{12}$ or $-C(Y)-CH_2-CH_2R^{12}$
where Y = O or S,
in particular
$R^5$ is chosen from $-CHR^{11}R^{12}$, $-CHR^{11}-CH_2R^{12}$, $-CHR^{11}-CH_2-CH_2R^{12}$, $-C(Y)R^{12}$ or $-C(Y)-CH_2R^{12}$
where Y = O.

**35.** Medicaments according to claim 34, **characterized in that** in the substituted cyclohexane-1,4-diamine derivatives contained therein
$R^{11}$ is chosen from
H, $C_{1-4}$-alkyl, saturated or unsaturated, branched or unbranched, mono- or polysubstituted or unsubstituted; or C(O)O-$C_{1-4}$-alkyl, saturated or unsaturated, branched or unbranched, mono- or polysubstituted or unsubstituted;
preferably
H, $C_{1-4}$-alkyl, saturated or unsaturated, branched or unbranched, mono- or polysubstituted or unsubstituted; or C(O)O-$C_{1-2}$-alkyl, saturated, unbranched, mono- or polysubstituted or unsubstituted;
in particular
H, $CH_3$, $C_2H_5$ and C(O)O-$CH_3$.

**36.** Medicaments according to claim 34, **characterized in that** in the substituted cyclohexane-1,4-diamine derivatives contained therein
$R^{12}$ is chosen from $C_{3-8}$-cycloalkyl, aryl or heteroaryl, in each case unsubstituted or mono- or polysubstituted;
preferably
$R^{12}$ is chosen from cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl, in each case unsubstituted or mono- or polysubstituted;
in particular
$R^{12}$ is chosen from cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, in each case unsubstituted or mono- or polysubstituted.

**37.** Medicaments according to one of claims 20 to 36, **characterized in that** in addition to at least one substituted cyclohexane-1,4-diamine derivative, the medicament also comprises an opioid, preferably a potent opioid, in particular morphine, or an anaesthetic, preferably hexobarbital or halothane.

**38.** Use of a substituted cyclohexane-1,4-diamine derivative according to claim 1,
wherein
$R^1$ and $R^2$ independently of one another are chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted; wherein $R^1$ and $R^2$ may not both be H,
or the radicals $R^1$ and $R^2$ together form a ring and denote $CH_2CH_2OCH_2CH_2$, $CH_2CH_2NR^6CH_2CH^2$ or $(CH_2)_{3-6}$,
where $R^6$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
$R^3$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted;

aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^4$ is chosen from H, $C_{1-8}$-alkyl, saturated or unsaturated, branched or unbranched, mono- or polysubstituted or unsubstituted or $C(X)R^7$, $C(X)\ NR^7R^8$, $C\ (X)\ OR^9$, $C\ (X)\ SR^9$, $S\ (O_2)\ R^9$
where X = O or S,
where $R^7$ is chosen from H, $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
where $R^8$ is chosen from H, $C_{1-4}$-alkyl, saturated or unsaturated, branched or unbranched, mono- or polysubstituted or unsubstituted or
the radicals $R^7$ and $R^8$ together form a ring and denote $CH_2CH_2OCH_2CH_2$, $CH_2CH_2NR^{10}CH_2CH_2$ or $(CH_2)_{3-6}$,
where $R^{10}$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
where $R^9$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^5$ is chosen from $C_{3-8}$-cycloalkyl, aryl or heteroaryl, in each case unsubstituted or mono- or polysubstituted; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)\ R^{12}$, $C\ (Y)-CH_2R^{12}$, $-C\ (Y)\ -CH_2-CH_2R^{12}$ or $-C\ (Y)\ -CH_2CH_2-CH_2R^{12}$
where Y = O, S or $H_2$,
where $R^{11}$ is chosen from
H, $C_{1-7}$-alkyl, saturated or unsaturated, branched or unbranched, mono- or polysubstituted or unsubstituted; or C (O)O-$C_{1-6}$-alkyl, saturated or unsaturated, branched or unbranched, mono- or polysubstituted or unsubstituted;
and where $R^{12}$ is chosen from
H; $C_{3-8}$-cycloalkyl, aryl or heteroaryl, in each case unsubstituted or mono- or polysubstituted,
or $R^4$ and $R^5$ together form a heterocyclic radical having between 3 and 8 atoms in the ring, saturated or unsaturated; mono- or polysubstituted or unsubstituted, which can optionally be fused with further rings,
with the proviso,

- that if $R^3$ is substituted or unsubstituted phenyl and at least one of $R^1$ or $R^2$ is H or $C_{1-8}$-alkyl, $R^4$ may not be alkyl and $R^4$ and $R^5$ may not together form a heterocyclic radical

optionally in the form of its racemate, the pure stereoisomers, in particular enantiomers or diastereomers, or in the form of mixtures of the stereoisomers, in particular the enantiomers or diastereomers, in any desired mixing ratio; in the form shown or in the form of its acids or its bases or in the form of its salts, in particular the physiologically acceptable salts or salts of physiologically acceptable acids or cations; or in the form of its solvates, in particular the hydrates; for the preparation of a medicament for treatment of pain, in particular acute, neuropathic or chronic pain, or for coadministration in treatment with an opioid analgesic.

**39.** Use of a substituted cyclohexane-1,4-diamine derivative according to claim 1
wherein
$R^1$ and $R^2$ independently of one another are chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted; wherein $R^1$ and $R^2$ may not both be H,
or the radicals $R^1$ and $R^2$ together form a ring and denote $CH_2CH_2OCH_2CH_2$, $CH_2CH_2NR^6CH_2CH_2$ or $(CH_2)_{3-6}$,
where $R^6$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
$R^3$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;

$R^4$ is chosen from H, $C_{1-8}$-alkyl, saturated or unsaturated, branched or unbranched, mono- or polysubstituted or unsubstituted or $C(X)R^7$, $C(X)NR^7R^8$, $C(X)OR^9$, C (X) $SR^9$, $S(O_2)R^9$
where X =O or S,
where $R^7$ is chosen from H, $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
where $R^8$ is chosen from H, $C_{1-4}$-alkyl, saturated or unsaturated, branched or unbranched, mono- or polysubstituted or unsubstituted or
the radicals $R^7$ and $R^8$ together form a ring and denote $CH_2CH_2OCH_2CH_2$, $CH_2CH_2NR^{10}CH_2CH_2$ or $(CH_2)_{3-6}$,
where $R^{10}$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
where $R^9$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^5$ is chosen from $C_{3-8}$-cycloalkyl, aryl or heteroaryl, in each case unsubstituted or mono- or polysubstituted; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, -C (Y) $R^{12}$, -C (Y) - $CH_2R^{12}$, -C (Y)-$CH_2$-$CH_2R^{12}$ or -C(Y)-$CH_2$-$CH_2$-$CH_2R^{12}$
where Y = O , S or $H_2$,
where $R^{11}$ is chosen from
H, $C_{1-7}$-alkyl, saturated or unsaturated, branched or unbranched, mono- or polysubstituted or unsubstituted; or C (O)O-$C_{1-6}$-alkyl, saturated or unsaturated, branched or unbranched, mono- or polysubstituted or unsubstituted;
and where $R^{12}$ is chosen from
H; $C_{3-8}$-cycloalkyl, aryl or heteroaryl, in each case unsubstituted or mono- or polysubstituted,
or $R^4$ and $R^5$ together form a heterocyclic radical having between 3 and 8 atoms in the ring, saturated or unsaturated; mono- or polysubstituted or unsubstituted, which can optionally be fused with further rings,
optionally in the form of its racemate, the pure stereoisomers, in particular enantiomers or diastereomers, or in the form of mixtures of the stereoisomers, in particular the enantiomers or diastereomers, in any desired mixing ratio; in the form shown or in the form of its acids or its bases or in the form of its salts, in particular the physiologically acceptable salts or salts of physiologically acceptable acids or cations; or in the form of its solvates, in particular the hydrates; for the preparation of a medicament for treatment of pain, in particular acute, neuropathic or chronic pain, or for coadministration in treatment with an opioid analgesic.

**40.** Use of a substituted cyclohexane-1,4-diamine derivative according to claim 1,
wherein
$R^1$ and $R^2$ together form a ring and denote $CH_2CH_2OCH_2CH_2$, $CH_2CH_2NR^6CH_2CH_2$ or $(CH_2)_{3-6}$,
where $R^6$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
$R^3$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^4$ is chosen from H, $C_{1-8}$-alkyl, saturated or unsaturated, branched or unbranched, mono- or polysubstituted or unsubstituted; or $C(X)R^7$, $C(X)NR^7R^8$, C (X) $OR^9$, C (X) $SR^9$, S $(O_2)$ $R^9$
where X = O or S,
where $R^7$ is chosen from H, $C_{1-8}$-alkyl or $C_3$-$_8$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
where $R^8$ is chosen from H, $C_{1-4}$-alkyl, saturated or unsaturated, branched or unbranched, mono- or polysubstituted or unsubstituted or
the radicals $R^7$ and $R^8$ together form a ring and denote $CH_2CH_2OCH_2CH_2$, $CH_2CH_2NR^{10}CH_2CH_2$ or $(CH_2)_{3-6}$,
where $R^{10}$ is chosen from H; $C_{1-8}$-alkyl or $C_{3-8}$-cycloalkyl, in each case saturated or unsaturated, branched or

unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
where $R^9$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^5$ is chosen from $C_{3-8}$-cycloalkyl, aryl or heteroaryl, in each case unsubstituted or mono- or polysubstituted; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, -C (Y) $R^{12}$, -C (Y) - $CH_2R^{12}$, -C (Y) $-CH^2-CH_2R^{12}$ or -C (Y) $-CH_2-CH_2-CH_2R^{12}$
where Y = O, S or $H_2$,
where $R^{11}$ is chosen from
H, $C_{1-7}$-alkyl, saturated or unsaturated, branched or unbranched, mono- or polysubstituted or unsubstituted; or C (O)O-$C_{1-6}$-alkyl, saturated or unsaturated, branched or unbranched, mono- or polysubstituted or unsubstituted;
and where $R^{12}$ is chosen from
H; $C_{3-8}$-cycloalkyl, aryl or heteroaryl, in each case unsubstituted or mono- or polysubstituted,
or $R^4$ and $R^5$ together form a heterocyclic radical having between 3 and 8 atoms in the ring, saturated or unsaturated; mono- or polysubstituted or unsubstituted, which heterocyclic radical may optionally be fused to further rings,
optionally in the form of its racemate, of the pure stereoisomers, in particular enantiomers or diastereomers, or in the form of mixtures of the stereoisomers, in particular of the enantiomers or diastereomers, in any mixing ratio;
in the prepared form or in the form of its acids or its bases or in the form of its salts, in particular the physiologically acceptable salts or salts of physiologically acceptable acids or cations; or in the form of its solvates, in particular the hydrates; for the preparation of a medicament for treatment of pain, in particular acute, neuropathic or chronic pain, or for co-administration in treatment with an opioid analgesic.

**41.** Use of a substituted cyclohexane-1,4-diamine derivative according to claim 1,
wherein
$R^1$ and $R^2$ independently of one another are chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted; wherein $R^1$ and $R^2$ may not both be H,
or the radicals $R^1$ and $R^2$ together form a ring and denote $CH_2CH_2OCH_2CH_2$, $CH_2CH_2NR^6CH_2CH_2$ or $(CH_2)_{3-6}$,
where $R^6$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
$R^3$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^4$ is chosen from H, $C(X)R^7$, C (X) $NR^7R^8$, C (X) $OR^9$, C (X) $SR^9$ or S $(O_2)$ $R^9$
where X = O or S,
where $R^7$ is chosen from H, $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
where $R^8$ is chosen from H, $C_{1-4}$-alkyl, saturated or unsaturated, branched or unbranched, mono- or polysubstituted or unsubstituted or
the radicals $R^7$ and $R^8$ together form a ring and denote $CH_2CH_2OCH_2CH_2$, $CH_2CH_2NR^{10}CH_2CH_2$ or $(CH_2)_{3-6}$,
where $R^{10}$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
where $R^9$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^5$ is chosen from $C_{3-8}$-cycloalkyl, aryl or heteroaryl, in each case unsubstituted or mono- or polysubstituted;

$-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ or $-C(Y)-CH_2-CH_2-CH_2R^{12}$
where Y = O, S or $H_2$,
where $R^{11}$ is chosen from
H, $C_{1-7}$-alkyl, saturated or unsaturated, branched or unbranched, mono- or polysubstituted or unsubstituted; or C (O)O-$C_{1-6}$-alkyl, saturated or unsaturated, branched or unbranched, mono- or polysubstituted or unsubstituted;
and where $R^{12}$ is chosen from
H; $C_{3-8}$-cycloalkyl, aryl or heteroaryl, in each case unsubstituted or mono- or polysubstituted,
optionally in the form of its racemate, of the pure stereoisomers, in particular enantiomers or diastereomers, or in the form of mixtures of the stereoisomers, in particular of the enantiomers or diastereomers, in any mixing ratio; in the prepared form or in the form of its acids or its bases or in the form of its salts, in particular the physiologically acceptable salts or salts of physiologically acceptable acids or cations; or in the form of its solvates, in particular the hydrates; for the preparation of a medicament for treatment of pain, in particular acute, neuropathic or chronic pain, or for co-administration in treatment with an opioid analgesic.

**42.** Use of a substituted cyclohexane-1,4-diamine derivative according to claim 1,
wherein
$R^1$ and $R^2$ independently of one another are chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted; wherein $R^1$ and $R^2$ may not both be H,
or the radicals $R^1$ and $R^2$ together form a ring and denote $CH_2CH_2OCH_2CH_2$, $CH_2CH_2NR^6CH_2CH_2$ or $(CH_2)_{3-6}$,
where $R^6$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
$R^3$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^4$ is chosen from H, $C_{1-8}$-alkyl, saturated or unsaturated, branched or unbranched, mono- or polysubstituted or unsubstituted; or $C(X)R^7$, $C(X)NR^7R^8$, $C(X)OR^9$, $C(X)SR^9$, $S(O_2)R^9$
where X = O or S,
where $R^7$ is chosen from H, $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
where $R^8$ is chosen from H, $C_{1-4}$-alkyl, saturated or unsaturated, branched or unbranched, mono- or polysubstituted or unsubstituted or
the radicals $R^7$ and $R^8$ together form a ring and denote $CH_2CH_2OCH_2CH_2$, $CH_2CH_2NR^{10}CH_2CH_2$ or $(CH_2)_{3-6}$,
where $R^{10}$ is chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
where $R^9$ is chosen from $C_{1-8}$-alkyl or $C_{3-8}$-cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$-cycloalkyl or heteroaryl, bonded via a saturated or unsaturated, branched or unbranched, substituted or unsubstituted $C_{1-4}$-alkyl group and in each case unsubstituted or mono- or polysubstituted;
$R^5$ is chosen from $C_{3-8}$-cycloalkyl, aryl or heteroaryl, in each case unsubstituted or mono- or polysubstituted; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ or $-C(Y)-CH_2-CH_2-CH_2R^{12}$
where Y = O, S or $H_2$,
where $R^{11}$ is chosen from
H, $C_{1-7}$-alkyl, saturated or unsaturated, branched or unbranched, mono- or polysubstituted or unsubstituted; or C (O) O-$C_{1-6}$-alkyl, saturated or unsaturated, branched or unbranched, mono- or polysubstituted or unsubstituted;
and where $R^{12}$ is chosen from
H; $C_{3-8}$-cycloalkyl, aryl or heteroaryl, in each case unsubstituted or mono- or polysubstituted,
or $R^4$ and $R^5$ together form a heterocyclic radical having between 3 and 8 atoms in the ring, saturated or unsaturated;

mono- or polysubstituted or unsubstituted, which heterocyclic radical may optionally be fused to further rings optionally in the form of its racemate, of the pure stereoisomers, in particular enantiomers or diastereomers, or in the form of mixtures of the stereoisomers, in particular of the enantiomers or diastereomers, in any mixing ratio; in the prepared form or in the form of its acids or its bases or in the form of its salts, in particular the physiologically acceptable salts or salts of physiologically acceptable acids or cations; or in the form of its solvates, in particular the hydrates; for the preparation of a medicament for treatment of anxiety states, of stress and stress-associated syndromes, depression, epilepsy, Alzheimer's disease, senile dementia, general cognitive dysfunctions, learning and memory difficulties (as a nootropic), withdrawal symptoms, alcohol and/or drug and/or medicament abuse and/or dependency, sexual dysfunctions, cardiovascular diseases, hypotension, hypertension, tinnitus, pruritus, migraine, impaired hearing, deficient intestinal motility, impaired food intake, anorexia, obesity, locomotor disorders, diarrhoea, cachexia, urinary incontinence or as a muscle relaxant, anticonvulsive or anaesthetic or for co-administration in treatment with an anaesthetic, for diuresis or antinatriuresis and/or anxiolysis.

**43.** Use according to one of claims 38, 39 or 42, **characterized in that** in the substituted cyclohexane-1,4-diamine derivatives used
$R^1$ and $R^2$ independently of one another are chosen from H; $C_{1-8}$-alkyl, saturated or unsaturated, branched or unbranched, mono- or polysubstituted or unsubstituted; wherein $R^1$ and $R^2$ may not both be H,
or the radicals $R^1$ and $R^2$ together form a ring and denote $CH_2CH_2OCH_2CH_2$, $CH_2CH_2NR^6$ $CH_2CH_2$ or $(CH_2)_{3-6}$,
where $R^6$ is chosen from H; $C_{1-8}$-alkyl, saturated or unsaturated, branched or unbranched, mono- or polysubstituted or unsubstituted,
preferably
$R^1$ and $R^2$ independently of one another are chosen from H; $C_{1-4}$-alkyl, saturated or unsaturated, branched or unbranched, mono- or polysubstituted or unsubstituted; wherein $R^1$ and $R^2$ may not both be H,
or the radicals $R^1$ and $R^2$ together form a ring and denote $(CH_2)_{4-5}$,
in particular
$R^1$ and $R^2$ independently of one another are chosen from methyl or ethyl or the radicals $R^1$ and $R^2$ together form a ring and denote $(CH_2)_5$.

**44.** Use according to claim 40, **characterized in that** in the substituted cyclohexane-1,4-diamine derivatives used
$R^1$ and $R^2$ together form a ring and denote $CH_2CH_2OCH_2CH_2$, $CH_2CH_2NR^6CH_2CH_2$ or $(CH_2)_{3-6}$,
where $R^6$ is chosen from H; $C_{1-8}$-alkyl, saturated or unsaturated, branched or unbranched, mono- or polysubstituted or unsubstituted,
preferably
$R^1$ and $R^2$ together form a ring and denote $(CH_2)_{4-5}$,
in particular
$R^1$ and $R^2$ together form a ring and denote $(CH_2)_5$.

**45.** Use according to claim 41, **characterized in that** in the substituted cyclohexane-1,4-diamine derivatives used
$R^1$ and $R^2$ independently of one another are chosen from H; $C_{1-8}$-alkyl, saturated or unsaturated, branched or unbranched, mono- or polysubstituted or unsubstituted; wherein $R^1$and $R^2$ may not both be H,
preferably
$R^1$ and $R^2$ independently of one another are chosen from H; $C_{1-4}$-alkyl, saturated or unsaturated, branched or unbranched, mono- or polysubstituted or unsubstituted; wherein $R^1$ and $R^2$ may not both be H,
in particular
$R^1$ and $R^2$ independently of one another are chosen from methyl or ethyl;
or
$R^1$ and $R^2$ together form a ring and denote $CH_2CH_2OCH_2CH_2$, $CH_2CH_2NR^6CH_2CH_2$ or $(CH_2)_{3-6}$,
where $R^6$ is chosen from H; $C_{1-8}$-alkyl, saturated or unsaturated, branched or unbranched, mono- or polysubstituted or unsubstituted,
preferably
$R^1$ and $R^2$ together form a ring and denote $(CH_2)_{4-5}$,
in particular
$R^1$ and $R^2$ together form a ring and denote $(CH_2)_5$.

**46.** Use according to one of claims 38 or 40 to 42, **characterized in that** in the substituted cyclohexane-1,4-diamine derivatives used
$R^3$ is chosen from $C_{3-8}$-cycloalkyl, aryl or heteroaryl, in each case unsubstituted or mono- or polysubstituted; aryl, $C_{3-8}$-cycloalkyl or heteroaryl, bonded via a saturated or unsaturated, unbranched, substituted or unsubstituted

$C_{1-2}$-alkyl group and in each case unsubstituted or mono- or polysubstituted;
preferably
$R^3$ is chosen from $C_{5-6}$-cycloalkyl, phenyl, naphthyl, anthracenyl, thiophenyl, benzothiophenyl, furyl, benzofuranyl, benzodioxolanyl, indolyl, indanyl, benzodioxanyl, pyrrolyl, pyrimidyl or pyrazinyl, in each case unsubstituted or mono- or polysubstituted; $C_{5-6}$-cycloalkyl, phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl, bonded via a saturated, unbranched $C_{1-2}$-alkyl group and in each case unsubstituted or mono- or polysubstituted;
in particular
$R^3$ is chosen from phenyl, furyl, thiophenyl, cyclohexanyl, naphthyl, benzofuranyl, indolyl, indanyl, benzodioxanyl, benzodioxolanyl, pyrrolyl, pyrimidyl, pyrazinyl or benzothiophenyl, in each case unsubstituted or mono- or polysubstituted; phenyl, furyl or thiophenyl, bonded via a saturated, unbranched $C_{1-2}$-alkyl group and in each case unsubstituted or mono- or polysubstituted.

**47.** Use according to claim 39, **characterized in that** in the substituted cyclohexane-1,4-diamine derivatives used
$R^3$ is chosen from $C_{3-8}$-cycloalkyl or heteroaryl, in each case unsubstituted or mono- or polysubstituted; aryl, $C_{3-8}$-cycloalkyl or heteroaryl, bonded via a saturated or unsaturated, unbranched, substituted or unsubstituted $C_{1-4}$-alkyl group and in each case unsubstituted or mono- or polysubstituted;
preferably
$R^3$ is chosen from $C_{5-6}$-cycloalkyl, thiophenyl, benzothiophenyl, furyl, benzofuranyl, benzodioxolanyl, indolyl, indanyl, benzodioxanyl, pyrrolyl, pyrimidyl or pyrazinyl, in each case unsubstituted or mono- or polysubstituted; $C_{5-6}$-cycloalkyl, phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl, bonded via a saturated, unbranched $C_{1-2}$-alkyl group and in each case unsubstituted or mono- or polysubstituted;
in particular
$R^3$ is chosen from furyl, thiophenyl, cyclohexanyl, benzofuranyl, indolyl, indanyl, benzodioxanyl, benzodioxolanyl, pyrrolyl, pyrimidyl, pyrazinyl or benzothiophenyl, in each case unsubstituted or mono- or polysubstituted; phenyl, furyl or thiophenyl, bonded via a saturated, unbranched $C_{1-2}$-alkyl group and in each case unsubstituted or mono- or polysubstituted.

**48.** Use according to one of claims 38 to 47, **characterized in that** in the substituted cyclohexane-1,4-diamine derivatives used, $R^4$ is H.

**49.** Use according to one of claims 38 to 47, **characterized in that** in the substituted cyclohexane-1,4-diamine derivatives used
$R^4$ is chosen from H, $C(X)R^7$, $C(X)NR^7R^8$, $C(X)OR^9$, $C(X)SR^9$ or $S(O_2)R^9$, where X = O or S,
preferably
$R^4$ is chosen from H, $C(X)R^7$, $C(X)NR^7R^8$ or C (X) $OR^9$, where X = O,
in particular
$R^4$ is chosen from H or $C(O)R^7$; preferably where $R^7$ is chosen from
H; or $C_{1-8}$-alkyl, saturated or unsaturated, branched or unbranched, mono- or polysubstituted or unsubstituted;
preferably
H; or $C_{1-3}$-alkyl, saturated, unsubstituted, branched or unbranched;
in particular $CH_3$.

**50.** Use according to one of claims 38 to 40 or 42, **characterized in that** in the substituted cyclohexane-1,4-diamine derivatives used
$R^4$ and $R^5$ together form a heterocyclic radical having between 3 and 8 atoms in the ring, saturated or unsaturated; mono- or polysubstituted or unsubstituted, preferably having between 5 and 7 atoms in the ring, of which, in addition to the obligatory N, 0 to 1 further heteroatoms chosen from N, S or O are in the ring;
wherein the heterocyclic radical formed by $R^4$ and $R^5$ together may optionally be fused to further rings,
preferably to aromatic and/or heteroaromatic rings, wherein those rings can be fused to further aromatic and/or heteroaromatic rings,
in particular the heterocyclic radical formed by $R^4$ and $R^5$ together is fused to one or two further rings,
preferably the heterocyclic radical formed by $R^4$ and $R^5$ together is so fused to two further rings that $R^4$ and $R^5$ together denote

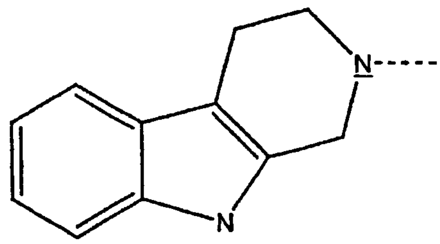

.

**51.** Use according to one of claims 38 to 40 or 42, **characterized in that** in the substituted cyclohexane-1,4-diamine derivatives used
$R^4$ is chosen from H, $C_{1-8}$-alkyl, saturated or unsaturated, branched or unbranched, mono- or polysubstituted or unsubstituted,
preferably
H, $C_{1-6}$-alkyl, saturated or unsaturated, branched or unbranched, mono- or polysubstituted or unsubstituted,
in particular
H, $C_{1-3}$-alkyl, saturated, unbranched and unsubstituted.

**52.** Use according to one of claims 38 to 51, **characterized in that** in the substituted cyclohexane-1,4-diamine derivatives used
$R^5$ is chosen from $C_{3-8}$-cycloalkyl, aryl or heteroaryl, in each case unsubstituted or mono- or polysubstituted;
preferably
$R^5$ is chosen from cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo [1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl, in each case unsubstituted or mono- or polysubstituted;
in particular
$R^5$ is chosen from cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, in each case unsubstituted or mono- or polysubstituted.

**53.** Use according to one of claims 38 to 51, **characterized in that** in the substituted cyclohexane-1,4-diamine derivatives used
$R^5$ is chosen from $-CHR^{11}R^{12}$, $-CHR^{11}-CH_2R^{12}$, $-CHR^{11}-CH_2-CH_2R^{12}$, $CHR_2-CH_2-CH_2R^{12}$, $-C(Y)R^{12}$, $-C(Y)-CH_2R^{12}$, $-C(Y)-CH_2-CH_2R^{12}$ or $-C(Y)-CH_2-CH_2-CH_2R^{12}$
where Y = 0, S or $H_2$,
preferably
$R^5$ is chosen from $-CHR^{11}R^{12}$, $-CHR^{11}-CH_2R^{12}$, $-CHR^{11}-CH_2-CH_2R^{12}$, $-C(Y)R^{12}$, $-C(Y)-CH_2R^{12}$ or $-C(Y)-CH_2-CH_2R^{12}$
where Y = O or S,
in particular
$R^5$ is chosen from $-CHR^{11}R^{12}$, $-CHR^{11}-CH_2R^{12}$, $-CHR^{11}-CH_2-CH_2R^{12}$, $-C(Y)R^{12}$ or $-C(Y)-CH_2R^{12}$
where Y = O.

**54.** Use according to claim 53, **characterized in that** in the substituted cyclohexane-1,4-diamine derivatives used
$R^{11}$ is chosen from
H, $C_{1-4}$-alkyl, saturated or unsaturated, branched or unbranched, mono- or polysubstituted or unsubstituted; or C (O)O-$C_{1-4}$-alkyl, saturated or unsaturated, branched or unbranched, mono- or polysubstituted or unsubstituted;
preferably
H, $C_{1-4}$-alkyl, saturated or unsaturated, branched or unbranched, mono- or polysubstituted or unsubstituted; or C (O)O-$C_{1-2}$-alkyl, saturated, unbranched, mono- or polysubstituted or unsubstituted;
in particular
H, $CH_3$, $C_2H_5$ and C (O) O-$CH_3$.

**55.** Use according to claim 53, **characterized in that** in the substituted cyclohexane-1,4-diamine derivatives used $R^{12}$ is chosen from $C_{3-8}$-cycloalkyl, aryl or heteroaryl, in each case unsubstituted or mono- or polysubstituted; preferably
$R^{12}$ is chosen from cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl, in each case unsubstituted or mono- or polysubstituted;
in particular
$R^{12}$ is chosen from cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, in each case unsubstituted or mono- or polysubstituted.

**56.** Substituted cyclohexane-1,4-diamine derivatives, medicaments or processes according to one of the preceding claims, **characterized in that** $R^4$ and $R^5$ together do not form a heterocyclic radical.

**57.** Process for the preparation of a substituted cyclohexane-1,4-diamine derivative according to one of claims 1 to 19 with the following steps:

a. a cyclohexane-1,4-dione, protected with groups $S^1$ and $S^2$, according to formula II is reacted with a cyanide, preferably potassium cyanide, in the presence of a compound of the formula $HNR^{01}R^{02}$ to give a protected N-substituted 1-amino-4-oxo-cyclohexane-carbonitrile derivative according to formula III;

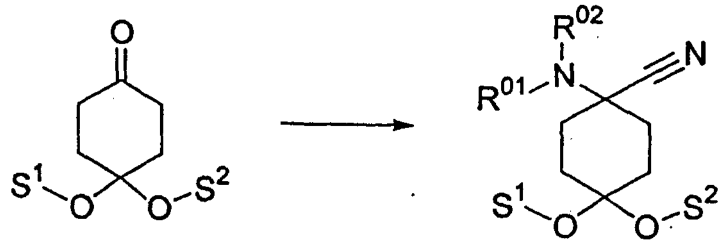

II III

and optionally subsequently, in any desired sequence and optionally repeatedly, acylation, alkylation or sulfonation is carried out and/or in the case of compounds where $R^{01}$ and/or $R^{02}$ and/or $R^{06}$ = H protected with a protective group, at least once a protective group is split off and acylation, alkylation or sulfonation is optionally carried out and/or in the case of compounds where $R^{01}$ and/or $R^{02}$ and/or $R^{06}$ = H, at least once a protective group is introduced and acylation, alkylation or sulfonation is optionally carried out,
b. the aminonitrile according to formula III is reacted with organometallic reagents, preferably Grignard or organolithium reagents, of the formula metal-$R^3$, so that a compound according to formula IVa is formed;

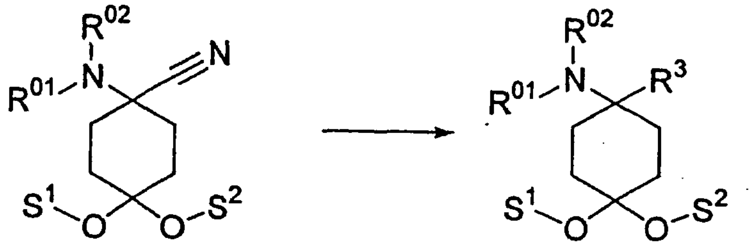

III IVa

and optionally subsequently, in any desired sequence and optionally repeatedly, acylation, alkylation or sulfonation is carried out and/or in the case of compounds where $R^{01}$ and/or $R^{02}$ and/or $R^{06}$ = H protected with a protective group, at least once a protective group is split off and acylation, alkylation or sulfonation is optionally carried out and/or in the case of compounds where $R^{01}$ and/or $R^{02}$ and/or $R^{06}$= H, at least once a protective group is introduced and acylation, alkylation or sulfonation is optionally carried out,

c. on the compound according to formula IVa, the protective groups $S^1$ and $S^2$ are split off, so that a 4-substituted 4-aminocyclohexanone derivative according to formula IV is formed;

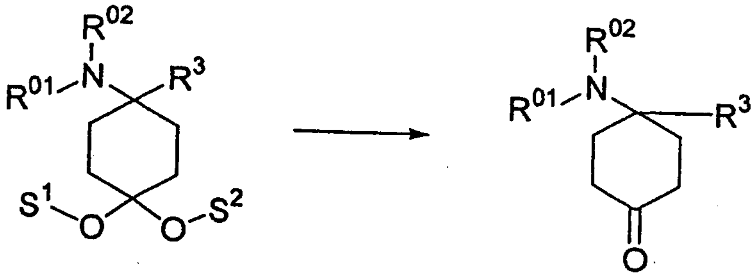

IVa IV

and optionally subsequently, in any desired sequence and optionally repeatedly, acylation, alkylation or sulfonation is carried out and/or in the case of compounds where $R^{01}$ and/or $R^{02}$ and/or $R^{06}$ = H protected with a protective group, at least once a protective group is split off and acylation, alkylation or sulfonation is optionally carried out and/or in the case of compounds where $R^{01}$ and/or $R^{02}$ and/or $R^{06}$ = H, at least once a protective group is introduced and acylation, alkylation or sulfonation is optionally carried out,

d. the 4-substituted 4-aminocyclohexanone derivative according to formula IVa is aminated reductively with a compound of the formula $HNR^{04}R^{05}$, so that a cyclohexane-1,4-diamine derivative according to formula V is formed;

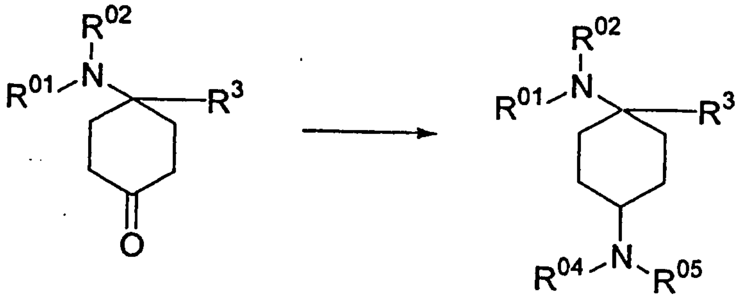

IV V

and optionally subsequently, in any desired sequence and optionally repeatedly, acylation, alkylation or sulfonation is carried out and/or in the case of compounds where $R^{01}$ and/or $R^{02}$ and/or $R^{04}$ and/or $R^{05}$ and/or $R^{06}$= H protected with a protective group, at least once a protective group is split off and acylation, alkylation or sulfonation is optionally carried out and/or in the case of compounds where $R^{01}$ and/or $R^{02}$ and/or $R^{04}$ and/or $R^{05}$ and/or $R^{06}$ = H, at least once a protective group is introduced and acylation, alkylation or sulfonation is optionally carried out, until a compound according to formula I is formed,

wherein $R^1$, $R^2$, $R^3$, $R^4$ and $R^5$ have the meaning given in claim 1

and

$R^{01}$ and $R^{02}$ independently of one another are chosen from H; H provided with a protective group; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;

or the radicals $R^{01}$ and $R^{02}$ together form a ring and denote $CH_2CH_2OCH_2CH_2$, $CH_2CH_2NR^{06}CH_2CH_2$ or $(CH_2)_{3-6}$,

where $R^{06}$ is chosen from H; H provided with a protective group; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;

$R^{04}$ is chosen from H, H provided with a protective group; $C_{1-8}$-alkyl, saturated or unsaturated, branched or unbranched, mono- or polysubstituted or unsubstituted;

$R^{05}$ is chosen from H, H provided with a protective group; $C_{3-8}$-cycloalkyl, aryl or heteroaryl, in each case unsubstituted or mono- or polysubstituted; $-CHR^{11}R^{12}$, $CRR^{11}-CH_2R^{12}$, $-CHR^{11}-CH_2-CH_2R^{12}$, $-CHR^{11}-CH_2-CH_2-CH_2R^{12}$,- C(Y) $R^{12}$, -C (Y) $-CH_2R^{12}$, -C (Y) $-CH_2CH_2R^{12}$ or -C (Y) - $CH_2-CH_2-CH_2R^{12}$

where Y = $H_2$,

where $R^{11}$ is chosen from

H, $C_{1-7}$-alkyl, saturated or unsaturated, branched or unbranched, mono- or polysubstituted or unsubstituted;

and where $R^{12}$ is chosen from

H; $C_{3-8}$-cycloalkyl, aryl or heteroaryl, in each case unsubstituted or mono- or polysubstituted,

or $R^{04}$ and $R^{05}$ together form a heterocyclic radical having between 3 and 8 atoms in the ring, saturated or unsaturated; mono- or polysubstituted or unsubstituted,

and $S^1$ and $S^2$ independently of one another are chosen from protective groups or together denote a protective group, preferably monoacetal.

**58.** Process for the preparation of a substituted cyclohexane-1,4-diamine derivative according to one of claims 1 to 19 with the following steps:

a. a cyclohexane-1,4-dione, protected with the groups $S^1$ and $S^2$, according to formula II is aminated reductively with a compound of the formula $HNR^{04}R^{05}$, so that a 4-aminocyclohexanone derivative according to formula VI is formed;

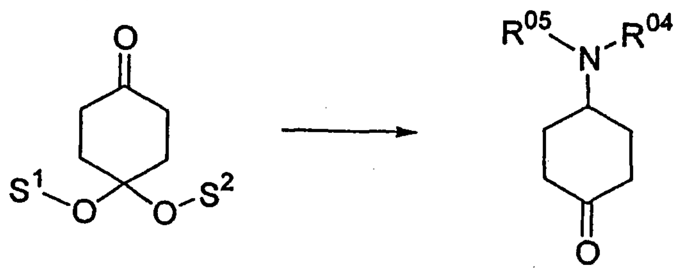

II VI

and optionally subsequently, in any desired sequence and optionally repeatedly, acylation, alkylation or sulfonation is carried out and/or in the case of compounds where $R^{04}$ and/or $R^{05}$ = H protected with a protective group, at least once a protective group is split off and acylation, alkylation or sulfonation is optionally carried out and/or in the case of compounds where $R^{04}$ and/or $R^{05}$ = H, at least once a protective group is introduced and acylation, alkylation or sulfonation is optionally carried out,

b. the 4-aminocyclohexanone derivative according to formula VI is reacted with cyanide, preferably potassium cyanide, in the presence of a compound of the formula $HNR^{01}R^{02}$ to give a cyclohexanone-nitrile derivative of the formula VII,

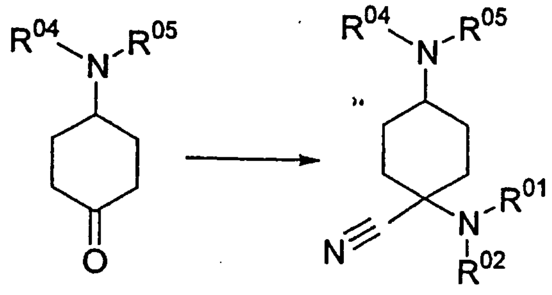

VI VII

and optionally subsequently, in any desired sequence and optionally repeatedly, acylation, alkylation or sulfonation is carried out and/or in the case of compounds where $R^{01}$ and/or $R^{02}$ and/or $R^{04}$ and/or $R^{05}$ and/or $R^{06}$ = H protected with a protective group, at least once a protective group is split off and acylation, alkylation or sulfonation is optionally carried out and/or in the case of compounds where $R^{01}$ and/or $R^{02}$ and/or $R^{04}$ and/or $R^{05}$ and/or $R^{06}$= H, at least once a protective group is introduced and acylation, alkylation or sulfonation is optionally carried out,

c. the cyclohexanone-nitrile derivative of the formula VII is reacted with organometallic reagents, preferably Grignard or organolithium reagents, of the formula metal-$R^3$ and, finally, the protective groups $S^1$ and $S^2$ are split off, so that a cyclohexane-1,4-diamine derivative according to formula V is formed,

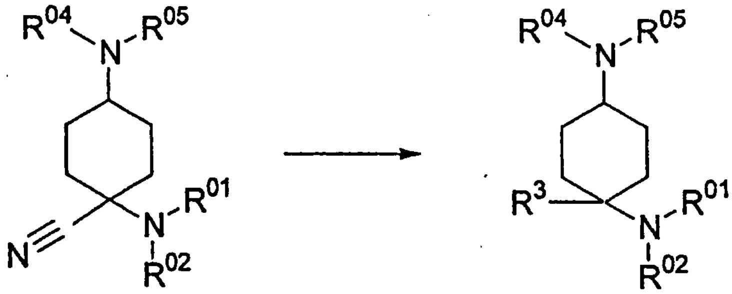

VII V

and optionally subsequently, in any desired sequence and optionally repeatedly, acylation, alkylation or sulfonation is carried out and/or in the case of compounds where $R^{01}$ and/or $R^{02}$ and/or $R^{04}$ and/or $R^{05}$ and/or $R^{06}$ = H protected with a protective group, at least once a protective group is split off and acylation, alkylation or sulfonation is optionally carried out and/or in the case of compounds where $R^{01}$ and/or $R^{02}$ and/or $R^{04}$ and/or $R^{05}$ and/or $R^{06}$ = H, at least once a protective group is introduced and acylation, alkylation or sulfonation is optionally carried out, until a compound according to formula I is formed,

wherein $R^1$, $R^2$, $R^3$, $R^4$ and $R^5$ have the meaning given in claim 1
and
$R^{01}$ and $R^{02}$ independently of one another are chosen from H; H provided with a protective group; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
or the radicals $R^{01}$ and $R^{02}$ together form a ring and denote $CH_2CH_2OCH_2CH_2$, $CH_2CH_2NR^{06}CH_2CH_2$ or $(CH_2)_{3-6}$, where $R^{06}$ is chosen from H; H provided with a protective group; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkylene and in each case mono- or polysubstituted or unsubstituted;
$R^{04}$ is chosen from H, H provided with a protective group; $C_{1-8}$-alkyl, saturated or unsaturated, branched or unbranched, mono- or polysubstituted or unsubstituted;

$R^{05}$ is chosen from H, H provided with a protective group; $C_{3-8}$-cycloalkyl, aryl or heteroaryl, in each case unsubstituted or mono- or polysubstituted; $-CHR^{11}R^{12}$, $-CHR^{11}-CH_2R^{12}$, $CHR^{11}-CH_2-CH_2R^{12}$, $-CHR^{11}-CH_2-CH_2-CH_2R^{12}$, -C (Y) $R^{12}$, $-C(Y)-CH_2R^{12}$, $-C(Y)-CH_2-CH_2R^{12}$ or $-C(Y)-CH_2-CH_2-CH_2R^{12}$
where $Y = H_2$,
where $R^{11}$ is chosen from
H, $C_{1-7}$-alkyl, saturated or unsaturated, branched or unbranched, mono- or polysubstituted or unsubstituted;
and where $R^{12}$ is chosen from
H; $C_{3-8}$-cycloalkyl, aryl or heteroaryl, in each case unsubstituted or mono- or polysubstituted,
or $R^{04}$ and $R^{05}$ together form a heterocyclic radical having between 3 and 8 atoms in the ring, saturated or unsaturated; mono- or polysubstituted or unsubstituted,
and $S^1$ and $S^2$ independently of one another are chosen from protective groups or together denote a protective group, preferably monoacetal.

**59.** Process for the preparation of a substituted cyclohexane-1,4-diamine derivative according to one of claims 57 or 58, **characterized in that** the protective groups on the H in $R^{01}$, $R^{02}$, $R^{04}$, $R^{05}$ and/or $R^{06}$ are chosen from alkyl, benzyl or carbamates, for example FMOC, Z or Boc.

**60.** Process for the preparation of a substituted cyclohexane-1,4-diamine derivative according to claim 57, **characterized in that** the reductive amination in step d takes place in the presence of ammonium formate, ammonium acetate or $NaCNBH_3$.

**61.** Process for the preparation of a substituted cyclohexane-1,4-diamine derivative according to claim 57, **characterized in that** instead of the reductive amination with $HNR^{04}R^{05}$ in step d, the compound IV is reacted with hydroxylamine and reduction is carried out after the oxime formation.

**62.** Process for the preparation of a substituted cyclohexane-1,4-diamine derivative according to claim 58, **characterized in that** in step b the radical $R^{01}$ in formula $HNR^{01}R^{02}$ is H, the reaction with the cyanide is carried out with TMSCN and optionally subsequently a protective group is introduced on $R^{01}$.

## Revendications

**1.** Dérivés substitués de cyclohexane-1,4-diamine, de formule générale I,

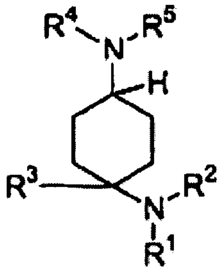

I

dans laquelle
$R^1$ et $R^2$ sont choisis, indépendamment l'un de l'autre, entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ; $R^1$ et $R^2$ ne pouvant pas représenter tous deux H,
ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,
$R^6$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou bien un reste, lié par l'intermédiaire d'un groupe alkylène en $C_1$ à $C_3$,

aryle, cycloalkyle en $C_3$ à $C_8$ ou hétéroaryle, chacun substitué une ou plusieurs fois ou non substitué ;
$R^3$ est choisi entre un reste alkyle en $C_1$ à $C_8$ ou un reste cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;
$R^4$ est choisi entre H, un reste alkyle en $C_1$ à $C_8$, saturé ou non saturé, ramifié ou non ramifié, substitué une ou plusieurs fois ou non substitué; ou bien un groupe $C(X)R^7$, $C(X)NR^7R^8$, $C(X)OR^9$, $C(X)SR^9$, $S(O_2)R^9$,
avec X = O ou S,
$R^7$ étant choisi entre H, un reste alkyle en $C_1$ à $C_8$ ou cycloalkyle en $C_3$ à $C_8$, chacun saturé ou non saturé, ramifié ou non ramifié, substitué une ou plusieurs fois ou non substitué; un reste aryle, hétéroaryle, chacun non substitué ou substitué une ou plusieurs fois; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;
$R^8$ étant choisi entre H, un reste alkyle en $C_1$ à $C_4$, saturé ou non saturé, ramifié ou non ramifié, substitué une ou plusieurs fois ou non substitué, ou bien
les restes $R^7$ et $R^8$ forment ensemble un noyau et représentent $CH_2CH_2OCH_2CH_2$, $CH_2CH_2NR^{10}CH_2CH_2$ ou $(CH_2)_{3-6}$,
$R^{10}$ étant choisi entre H; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou bien un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;
$R^9$ étant choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;
$R^5$ est choisi entre un reste cycloalkyle en $C_3$ à $C_8$, aryle ou hétéroaryle, chacun non substitué ou substitué une ou plusieurs fois ; un groupe $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, -C (Y) $R^{12}$, -C (Y) $-CH_{2R}{}^{12}$, -C (Y) $-CH_2-CH_2R^{12}$ ou -C (Y) $-CH_2-CH_2-CH_2R^{12}$ ,
avec Y = 0, S ou $H_2$,
$R^{11}$ étant choisi entre
H, un reste alkyle en $C_1$ à $C_7$, saturé ou non saturé, ramifié ou non ramifié, substitué une ou plusieurs fois ou non substitué; ou bien un groupe C(O)O-(alkyle en $C_1$ à $C_6$), saturé ou non saturé, ramifié ou non ramifié, substitué une ou plusieurs fois ou non substitué;
et $R^{12}$ étant choisi entre
H ; un reste cycloalkyle en $C_3$ à $C_8$, aryle ou hétéroaryle, chacun non substitué ou substitué une ou plusieurs fois, ou bien $R^4$ et $R^5$ forment ensemble un hétérocycle ayant entre 3 et 8 atomes dans le noyau, saturé ou non saturé, substitué une ou plusieurs fois ou non substitué et pouvant éventuellement être condensé avec d'autres noyaux, sous réserve que

• lorsque $R^3$ est un reste phényle substitué ou non substitué et l'un au moins de $R^1$ ou $R^2$ représente H ou un reste alkyle en $C_1$ à $C_8$, $R^4$ ne puisse pas être un reste alkyle et $R^4$ et $R^5$ ne puissent pas former ensemble un hétérocycle,
ou bien
• lorsque $R^3$ est un reste phényle non substitué et $R^1$ et $R^2$ forment ensemble un groupe $(CH_2)_5$, $R^4$ soit choisi entre H et un reste alkyle en $C_1$ à $C_8$, Y ne représente pas O ou S et $R^5$ ne soit pas un reste alkyle en $C_1$ à $C_6$,

le cas échéant sous forme de leurs racémates, de leurs stéréo-isomères purs, en particulier de leurs énantiomères ou de leurs diastéréo-isomères, ou sous forme de mélanges des stéréo-isomères, en particulier des énantiomères ou des diastéréo-isomères, dans un rapport de mélange quelconque ; sous la forme représentée ou sous forme de leurs acides ou de leurs bases ou sous forme de leurs sels, en particulier des sels physiologiquement acceptables ou de sels d'acides ou de cations physiologiquement acceptables ; ou sous forme de leurs produits de solvatation, en particulier des hydrates.

**2.** Dérivés substitués de cyclohexane-1,4-diamine, suivant la revendication 1,
où
$R^1$ et $R^2$ sont choisis, indépendamment l'un de l'autre, entre H ; un reste alkyle en $C_1$ à $C_8$ ou cycloalkyle en $C_3$ à

$C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ; $R^1$ et $R^2$ ne pouvant pas représenter tous deux H,
ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,
$R^6$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou bien un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;
$R^3$ est choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;
$R^4$ est choisi entre H, $C(X)R^7$, $C (X) NR^7R^8$, $C (X) OR^9$ ou $C (X) SR^9$, $S(O_2)R^9$,
avec X = O ou S,
$R^7$ étant choisi entre H, un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;
$R^8$ étant choisi entre H, un reste alkyle en $C_1$ à $C_4$, saturé ou non saturé, ramifié ou non ramifié, substitué une ou plusieurs fois ou non substitué, ou bien
les restes $R^7$ et $R^8$ forment ensemble un noyau et représentent $CH_2CH_2OCH_2CH_2$, $CH_2CH_2NR^{10}CH_2CH_2$ ou $(CH_2)_{3-6}$,
$R^{10}$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;
$R^9$ étant choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;
$R^5$ est choisi entre un reste cycloalkyle en $C_3$ à $C_8$, aryle ou hétéroaryle, chacun non substitué ou substitué une ou plusieurs fois ; un groupe $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C (Y) R^{12}$, $-C (Y) -CH_2R^{12}$, $-C (Y) -CH_2-CH_2R^{12}$ ou $-C (Y) -CH_2-CH_2-CH_2R^{12}$,
avec Y = 0, S ou $H_2$,
$R^{11}$ étant choisi entre
H, un reste alkyle en $C_1$ à $C_7$, saturé ou non saturé, ramifié ou non ramifié, substitué une ou plusieurs fois ou non substitué ; ou un groupe C(O)O-(alkyle en $C_1$ à $C_6$), saturé ou non saturé, ramifié ou non ramifié, substitué une ou plusieurs fois ou non substitué ;
et $R^{12}$ étant choisi entre
un reste cycloalkyle en $C_3$ à $C_8$, aryle ou hétéroaryle, chacun non substitué ou substitué une ou plusieurs fois,
le cas échéant sous forme de leurs racémates, de leurs stéréo-isomères purs, en particulier leurs énantiomères ou leurs diastéréo-isomères, ou sous forme de mélanges des stéréo-isomères, en particulier des énantiomères ou des diastéréo-isomères, dans un rapport de mélange quelconque ; sous la forme représentée ou sous forme de leurs acides ou de leurs bases ou sous forme de leurs sels, en particulier des sels physiologiquement acceptables ou de sels d'acides ou de cations physiologiquement acceptables ; ou sous forme de leurs produits de solvatation, en particulier des hydrates.

**3.** Dérivés substitués de cyclohexane-1,4-diamine suivant la revendication 1,
où
$R^1$ et $R^2$ sont choisis, indépendamment l'un de l'autre, entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs

fois ou non substitué; $R^1$ et $R^2$ ne pouvant pas représenter tous deux H;

$R^3$ est choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^4$ est choisi entre H, C (X) $R^7$, C (X) $NR^7R^8$, C (X) $OR^9$ ou C (X) $SR^9$, $S(O_2)R^9$,

avec X = O ou S,

$R^7$ étant choisi entre H, un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois;

$R^8$ étant choisi entre H, un reste alkyle en $C_1$ à $C_4$, saturé ou non saturé, ramifié ou non ramifié, substitué une ou plusieurs fois ou non substitué, ou bien

les restes $R^7$ et $R^8$ forment ensemble un noyau et représentent $CH_2CH_2OCH_2CH_2$, $CH_2CH_2NR^{10}CH_2CH_2$ ou $(CH_2)_{3-6}$,

$R^{10}$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué;

$R^9$ étant choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^5$ est choisi entre un reste cycloalkyle en $C_3$ à $C_8$, aryle ou hétéroaryle, chacun non substitué ou substitué une ou plusieurs fois ; un groupe $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ ou $-C(Y)-CH_2-CH_2-CH_2R^{12}$,

avec Y = O, S ou $H_2$,

$R^{11}$ étant choisi entre

H, un reste alkyle en $C_1$ à $C_7$, saturé ou non saturé, ramifié ou non ramifié, substitué une ou plusieurs fois ou non substitué ; ou bien un groupe C(O)O-(alkyle en $C_1$ à $C_6$), saturé ou non saturé, ramifié ou non ramifié, substitué une ou plusieurs fois ou non substitué ;

et $R^{12}$ étant choisi entre

H ; un reste cycloalkyle en $C_3$ à $C_8$, aryle ou hétéroaryle, chacun non substitué ou substitué une ou plusieurs fois, le cas échéant sous forme de leurs racémates, de leurs stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ; sous la forme représentée ou sous forme de leurs acides ou de leurs bases ou sous forme de leurs sels, en particulier des sels physiologiquement acceptables ou de sels d'acides ou de cations physiologiquement acceptables ; ou sous forme de leurs produits de solvatation, en particulier des hydrates.

**4.** Dérivés substitués de cyclohexane-1,4-diamine, suivant la revendication 1,

où

$R^1$ et $R^2$ sont choisis, indépendamment l'un de l'autre, entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ; $R^1$ et $R^2$ ne pouvant pas représenter tous deux H,

ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,

$R^6$ étant choisi entre H; un reste alkyle en $C_1$ à $C_8$ ou cycloalkyle en $C_3$ à $C_8$, chacun saturé ou non saturé, ramifié ou non ramifié, substitué une ou plusieurs fois ou non substitué; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

$R^3$ est choisi entre un reste alkyle en $C_1$ à $C_8$ ou un reste cycloalkyle en $C_3$ à $C_8$, chacun saturé ou non saturé, ramifié ou non ramifié, substitué une ou plusieurs fois ou non substitué; un reste hétéroaryle, non substitué ou

substitué une ou plusieurs fois, ou un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^4$ est choisi entre H ; un reste alkyle en $C_1$ à $C_8$, saturé ou non saturé, ramifié ou non ramifié, substitué une ou plusieurs fois ou non substitué, ou bien un groupe $C(X)R^7$, $C(X)NR^7R^8$, $C(X)OR^9$, $C(X)SR^9$, $S(O_2)R^9$,

avec X = 0 ou S,

$R^7$ étant choisi entre H, un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^8$ étant choisi entre H, un reste alkyle en $C_1$ à $C_4$, saturé ou non saturé, ramifié ou non ramifié, substitué une ou plusieurs fois ou non substitué, ou bien

les restes $R^7$ et $R^8$ forment ensemble un noyau et représentent $CH_2CH_2OCH_2CH_2$, $CH_2CH_2NR^{10}CH_2CH_2$ ou $(CH_2)_{3-6}$,

$R^{10}$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

$R^9$ étant choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^5$ est choisi entre un reste cycloalkyle en $C_3$ à $C_8$, aryle ou hétéroaryle, chacun non substitué ou substitué une ou plusieurs fois ; un groupe $-CHR^{11}R^{12}$,- $CHR^{11}-CH_2R^{12}$, $-CHR^{11}-CH_2-CH_2R^{12}$, $-CHR^{11}-CH_2-CH_2-CH_2R^{12}$, -C(Y) $R^{12}$, -C (Y) $-CH_2R^{12}$, -C (Y) $-CH_2-CH_2R^{12}$ ou -C(Y)$-CH_2-CH_2-CH_2R^{12}$,

avec Y = O, S ou $H_2$,

$R^{11}$ étant choisi entre

H, un reste alkyle en $C_1$ à $C_7$, saturé ou non saturé, ramifié ou non ramifié, substitué une ou plusieurs fois ou non substitué ; ou un groupe C (O) O- (alkyle en $C_1$ à $C_6$), saturé ou non saturé, ramifié ou non ramifié, substitué une ou plusieurs fois ou non substitué ;

et $R^{12}$ étant choisi entre

H ; un reste cycloalkyle en $C_3$ à $C_8$, aryle ou hétéroaryle, chacun non substitué ou substitué une ou plusieurs fois, ou bien $R^4$ et $R^5$ forment ensemble un hétérocycle ayant entre 3 et 8 atomes dans le noyau, saturé ou non saturé ; substitué une ou plusieurs fois ou non substitué, qui peut éventuellement être condensé avec d'autres noyaux ;

le cas échéant sous forme de leurs racémates, de leurs stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ; sous la forme représentée ou sous forme de leurs acides ou de leurs bases, ou sous forme de leurs sels, en particulier des sels physiologiquement acceptables ou de sels d'acides ou de cations physiologiquement acceptables; ou sous forme de leurs produits de solvatation, en particulier des hydrates.

**5.** Dérivés substitués de cyclohexane-1,4-diamine, suivant la revendication 1,

où

les restes $R^1$ et $R^2$ forment ensemble un noyau et représentent $CH_2CH_2OCH_2CH_2$, $CH_2CH_2NR^6CH_2CH_2$ ou $(CH_2)_{3-6}$, $R^6$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

$R^3$ est choisi entre un reste alkyle en $C_1$ à $C_8$ ou un reste cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^4$ est choisi entre H, un reste alkyle en $C_1$ à $C_8$, saturé ou non saturé, ramifié ou non ramifié, substitué une ou plusieurs fois ou non substitué, ou un groupe C (X) $R^7$, $C(X)NR^7R^8$, C (X) $OR^9$, $C(X)SR^9$, $S(O_2)R^9$,

avec X = O ou S,

$R^7$ étant choisi entre H, un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^8$ étant choisi entre H, un reste alkyle en $C_1$ à $C_4$, saturé ou non saturé, ramifié ou non ramifié, substitué une ou plusieurs fois ou non substitué, ou bien

les restes $R^7$ et $R^8$ forment ensemble un noyau et représentent $CH_2CH_2OCH_2CH_2$, $CH_2CH_2NR^{10}CH_2CH_2$ ou $(CH_2)_{3-6}$,

$R^{10}$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

$R^9$ étant choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^5$ est choisi entre un reste cycloalkyle en $C_3$ à $C_8$, aryle ou hétéroaryle, chacun non substitué ou substitué une ou plusieurs fois ; un groupe $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C (Y)-CH_2R^{12}$, $-C(Y)-CH_2-CH_2R^{12}$ ou $-C(Y)-CH_2-CH_2-CH_2R^{12}$,

avec Y = O , S ou $H_2$,

$R^{11}$ étant choisi entre

H, un reste alkyle en $C_1$ à $C_7$, saturé ou non saturé, ramifié ou non ramifié, substitué une ou plusieurs fois ou non substitué ; ou un groupe C (O) O- (alkyle en $C_1$ à $C_6$), saturé ou non saturé, ramifié ou non ramifié, substitué une ou plusieurs fois ou non substitué ;

et $R^{12}$ étant choisi entre

un reste cycloalkyle en $C_3$ à $C_8$, aryle ou hétéroaryle, chacun non substitué ou substitué une ou plusieurs fois,

ou bien $R^4$ et $R^5$ forment ensemble un hétérocycle ayant entre 3 et 8 atomes dans le noyau, saturé ou non saturé, substitué une ou plusieurs fois ou non substitué, qui peut éventuellement être condensé avec d'autres noyaux ;

le cas échéant sous forme de leurs racémates, de leurs stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque; sous la forme représentée ou sous forme de leurs acides ou de leurs bases ou sous forme de leurs sels, en particulier des sels physiologiquement acceptables ou de sels d'acides ou de cations physiologiquement acceptables ; ou sous forme de leurs produits de solvatation, en particulier des hydrates.

6. Dérivés substitués de cyclohexane-1,4-diamine suivant l'une des revendications 1, 2 ou 4, **caractérisés en ce que**
$R^1$ et $R^2$ sont choisis, indépendamment l'un de l'autre, entre H ; un reste alkyle en $C_1$ à $C_8$, saturé ou non saturé, ramifié ou non ramifié, substitué une ou plusieurs fois ou non substitué ; $R^1$ et $R^2$ ne pouvant pas représenter tous deux H,

ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,

$R^6$ étant choisi entre H ; un reste alkyle en $C_1$ à $C_8$, saturé ou non saturé, ramifié ou non ramifié, substitué une ou plusieurs fois ou non substitué,

avantageusement

$R^1$ et $R^2$ sont choisis indépendamment l'un de l'autre entre H ; un reste alkyle en $C_1$ à $C_4$, saturé ou non saturé, ramifié ou non ramifié, substitué une ou plusieurs fois ou non substitué ; $R^1$ et $R^2$ ne pouvant pas représenter tous deux H,

ou bien les restes $R^1$ et $R^2$ forment ensemble un noyau et représentent $(CH_2)_{4-5}$,

en particulier

$R^1$ et $R^2$ sont choisis indépendamment l'un de l'autre entre un reste méthyle ou éthyle ou bien les restes $R^1$ et $R^2$ forment ensemble un noyau et représentent $(CH_2)_5$.

7. Dérivés substitués de cyclohexane-1,4-diamine suivant la revendication 5, **caractérisés en ce que**
$R^1$ et $R^2$ forment ensemble un noyau et représentent $CH_2CH_2OCH_2CH_2$, $CH_2CH_2NR^6CH_2CH_2$ ou $(CH_2)_{3-6}$,

$R^6$ étant choisi entre H; un reste alkyle en $C_1$ à $C_8$, saturé ou non saturé, ramifié ou non ramifié, substitué une ou plusieurs fois ou non substitué,

avantageusement
$R^1$ et $R^2$ forment ensemble un noyau et représentent $(CH_2)_{4-5}$,
en particulier
$R^1$ et $R^2$ forment ensemble un noyau et représentent $(CH_2)_5$.

8. Dérivés substitués de cyclohexane-1,4-diamine suivant la revendication 3, **caractérisés en ce que**
$R^1$ et $R^2$ sont choisis, indépendamment l'un de l'autre, entre H ; un reste alkyle en $C_1$ à $C_8$ saturé ou non saturé, ramifié ou non ramifié, substitué une ou plusieurs fois ou non substitué ; $R^1$ et $R^2$ ne pouvant pas représenter tous deux H,
avantageusement,
$R^1$ et $R^2$ sont choisis indépendamment l'un de l'autre entre H; un reste alkyle en $C_1$ à $C_4$, saturé ou non saturé, ramifié ou non ramifié, substitué une ou plusieurs fois ou non substitué ; $R^1$ et $R^2$ ne pouvant pas représenter tous deux H,
en particulier
$R^1$ et $R^2$ sont choisis indépendamment l'un de l'autre entre un reste méthyle ou éthyle.

9. Dérivés substitués de cyclohexane-1,4-diamine suivant l'une des revendications 1 à 3 ou 5, **caractérisés en ce que**
$R^3$ est choisi entre un reste cycloalkyle en $C_3$ à $C_8$, aryle ou hétéroaryle, chacun non substitué ou substitué une ou plusieurs fois ; un reste, lié par l'intermédiaire d'un groupe alkyle en $C_1$ ou $C_2$ saturé ou non saturé, non ramifié, substitué ou non substitué, aryle, cycloalkyle en $C_3$ à $C_8$ ou hétéroaryle, chacun non substitué ou substitué une ou plusieurs fois ;
avantageusement,
$R^3$ est choisi entre un reste cycloalkyle en $C_5$ ou $C_6$, phényle, naphtyle, anthracényle, thiophényle, benzothiophényle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois ; un reste, lié par l'intermédiaire d'un groupe alkyle en $C_1$ ou $C_2$ saturé non ramifié, cycloalkyle en $C_5$ ou $C_6$, phényle, naphtyle, anthracényle, thiophényle, benzothiophényle, pyridyle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois;
en particulier,
$R^3$ est choisi entre un reste phényle, furyle, thiophényle, cyclohexanyle, naphtyle, benzofurannyle, indolyle, indanyle, benzodioxannyle, benzodioxolannyle, pyrrolyle, pyrimidyle, pyrazinyle ou benzothiophényle, chacun non substitué ou substitué une ou plusieurs fois ; ou un reste, lié par l'intermédiaire d'un groupe alkyle en $C_1$ ou $C_2$ saturé non ramifié, phényle, furyle ou thiophényle, chacun non substitué ou substitué une ou plusieurs fois.

10. Dérivés substitués de cyclohexane-1,4-diamine suivant la revendication 4, **caractérisés en ce que**
$R^3$ est choisi entre un reste cycloalkyle en $C_3$ à $C_8$ ou hétéroaryle, chacun non substitué ou substitué une ou plusieurs fois; un reste, lié par l'intermédiaire d'un groupe alkyle en $C_1$ à $C_4$ saturé ou non saturé, non ramifié, substitué ou non substitué, aryle, cycloalkyle en $C_3$ à $C_8$ ou hétéroaryle, chacun non substitué ou substitué une ou plusieurs fois;
avantageusement
$R^3$ est choisi entre un reste cycloalkyle en $C_5$ ou $C_6$, thiophényle, benzothiophényle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois ; un reste, lié par l'intermédiaire d'un groupe alkyle en $C_1$ ou $C_2$ saturé non ramifié, cycloalkyle en $C_5$ ou $C_6$, phényle, naphtyle, anthracényle, thiophényle, benzothiophényle, pyridyle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois;
en particulier,
$R^3$ est choisi entre un reste furyle, thiophényle, cyclohexanyle, benzofurannyle, indolyle, indanyle, benzodioxannyle, benzodioxolannyle, pyrrolyle, pyrimidyle, pyrazinyle ou benzothiophényle, chacun non substitué ou substitué une ou plusieurs fois ; un reste, lié par l'intermédiaire d'un groupe alkyle en $C_1$ ou $C_2$ saturé non ramifié, phényle, furyle ou thiophényle, chacun non substitué ou substitué une ou plusieurs fois.

11. Dérivés substitués de cyclohexane-1,4-diamine suivant l'une des revendications 1 à 10, **caractérisés en ce que**
$R^4$ représente H.

12. Dérivés substitués de cyclohexane-1,4-diamine suivant l'une des revendications 1 à 10, **caractérisés en ce que**
$R^4$ représente H, $C(X)R^7$, $C(X)NR^7R^8$, C (X) $OR^9$, $C(X)SR^9$ ou $S(O_2)R^9$ avec X = O ou S,
avantageusement,
$R^4$ est choisi entre H, $C(X)R^7$, $C(X)NR^7R^8$ ou $C(X)OR^9$ avec X = O,

en particulier
$R^4$ est choisi entre H ou un groupe $C(O)R^7$; $R^7$ étant avantageusement choisi entre
H ; ou un reste alkyle en $C_1$ à $C_8$ saturé ou non saturé, ramifié ou non ramifié, substitué une ou plusieurs fois ou non substitué ;
avantageusement
H ; ou un reste alkyle en $C_1$ à $C_3$, saturé, non substitué, ramifié ou non ramifié ;
en particulier $CH_3$.

**13.** Dérivés substitués de cyclohexane-1,4-diamine selon l'une des revendications 1, 4 ou 5, **caractérisés en ce que** $R^4$ et $R^5$ forment ensemble un hétérocycle ayant entre 3 et 8 atomes dans le noyau, saturé ou non saturé substitué une ou plusieurs fois ou non substitué, ayant avantageusement entre 5 et 7 atomes dans le noyau dont, à côté de l'atome N obligatoire, 0 à 1 autre hétéroatome choisi entre N, S ou O est présent dans le noyau;
l'hétérocycle formé conjointement par $R^4$ et $R^5$ pouvant éventuellement être condensé avec d'autres noyaux, avantageusement avec des noyaux aromatiques et/ou hétéroaromatiques, lesquels peuvent être condensés avec d'autres noyaux aromatiques et/ou hétéroaromatiques,
en particulier l'hétérocycle formé conjointement par $R^4$ et $R^5$ est condensé avec un ou deux autres noyaux, l'hétérocycle formé conjointement par $R^4$ et $R^5$ étant avantageusement condensé avec deux autres noyaux de manière que $R^4$ et $R^5$ représentent ensemble

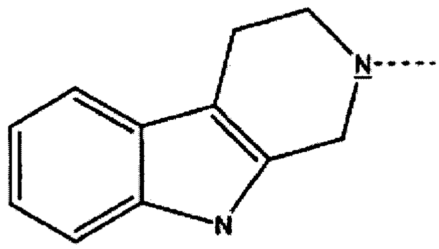

**14.** Dérivés substitués de cyclohexane-1,4-diamine suivant l'une des revendications 1, 4 ou 5, **caractérisés en ce que** $R^4$ est choisi entre H ou un reste alkyle en $C_1$ à $C_8$, saturé ou non saturé, ramifié ou non ramifié, substitué une ou plusieurs fois ou non substitué,
avantageusement
H ou un reste alkyle en $C_1$ à $C_6$, saturé ou non saturé, ramifié ou non ramifié, substitué une ou plusieurs fois ou non substitué,
en particulier
H ou un reste alkyle en $C_1$ à $C_3$, saturé, non ramifié et non substitué.

**15.** Dérivés substitués de cyclohexane-1,4-diamine suivant l'une des revendications 1 à 14, **caractérisés en ce que** $R^5$ est choisi entre un reste cycloalkyle en $C_3$ à $C_8$, aryle ou hétéroaryle, chacun non substitué ou substitué une ou plusieurs fois;
avantageusement
$R^5$ est choisi entre un reste cyclobutyle, cyclopropyle, cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]-thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou substitué une ou plusieurs fois ;
en particulier
$R^5$ est choisi entre un reste cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

**16.** Dérivés substitués de cyclohexane-1,4-diamine suivant l'une des revendications 1 à 14, **caractérisés en ce que** $R^5$ est choisi entre un groupe $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ ou $-C(Y)-CH_2-CH_2-CH_2R^{12}$, avec Y = O, S ou $H_2$,

avantageusement
$R^5$ est choisi entre $-CHR^{11}R^{12}$, $-CHR^{11}CH_2R^{12}$, $-CHR^{11}-CH_2-CH_2R^{12}$, -C (Y) $R^{12}$, -C (Y) $-CH_2R^{12}$ ou -C (Y) $-CH_2CH_2R^{12}$, avec Y = O ou S,
en particulier
$R^5$ est choisi entre $-CHR^{11}R^{12}$, $-CHR^{11}-CH_2R^{12}$, $-CHR^{11}-CH_2-CH_2R^{12}$, $C(Y)R^{12}$ ou -C (Y) $-CH_2R^{12}$, avec Y = O.

**17.** Dérivés substitués de cyclohexane-1,4-diamine suivant la revendication 16, **caractérisés en ce que**
$R^{11}$ est choisi entre
H, un reste alkyle en $C_1$ à $C_4$, saturé ou non saturé, ramifié ou non ramifié, substitué une ou plusieurs fois ou non substitué ; ou un groupe C(O)O-(alkyle en $C_1$ à $C_4$), saturé ou non saturé, ramifié ou non ramifié, substitué une ou plusieurs fois ou non substitué;
avantageusement
H, un reste alkyle en $C_1$ à $C_4$, saturé ou non saturé, ramifié ou non ramifié, substitué une ou plusieurs fois ou non substitué ; ou un reste C(O)O-(alkyle en $C_1$ ou $C_2$), saturé, non ramifié, substitué une ou plusieurs fois ou non substitué ;
en particulier
H, $CH_3$, $C_2H_5$ et C (O) $O-CH_3$.

**18.** Dérivés substitués de cyclohexane-1,4-diamine suivant la revendication 16, **caractérisés en ce que**
$R^{12}$ est choisi entre un reste cycloalkyle en $C_3$ à $C_8$, aryle ou hétéroaryle, chacun non substitué ou substitué une ou plusieurs fois ;
avantageusement
$R^{12}$ est choisi entre un reste cyclobutyle, cyclopropyle, cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]-thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou substitué une ou plusieurs fois ;
en particulier
$R^{12}$ est choisi entre un reste cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

**19.** Dérivés substitués de cyclohexane-1,4-diamine suivant l'une des revendications 1 à 18, **caractérisés en ce qu'**ils sont choisis dans le groupe suivant :

- chlorhydrate de N'-benzyl-N,N-diméthyl-1-phényl-cyclohexane-1,4-diamine, diastéréo-isomère le moins polaire
- chlorhydrate de N'-benzyl-N,N-diméthyl-1-phényl-cyclohexane-1,4-diamine, diastéréo-isomère le plus polaire
- chlorhydrate de 1,N'-dibenzyl-N,N-diméthylcyclohexane-1,4-diamine, diastéréo-isomère le moins polaire
- chlorhydrate de 1,N'-dibenzyl-N,N-diméthylcyclohexane-1,4-diamine, diastéréo-isomère le plus polaire
- chlorhydrate de N-(4-benzyl-4-diméthylaminocyclohexyl)-N-propylbenzamide
- chlorhydrate de N,N-diméthyl-1-phényl-N'-propyl-cyclohexane-1,4-diamine, diastéréo-isomère le moins polaire
- chlorhydrate de N-(4-diméthylamino-4-phénylcyclohexyl)-N-propylbenzamide, diastéréo-isomère le moins polaire
- chlorhydrate de N-(4-diméthylamino-4-phénylcyclohexyl)-N-propylbenzamide, diastéréo-isomère le plus polaire
- chlorhydrate de 1,N'-dibenzyl-N,N,N'-triméthyl-cyclohexane-1,4-diamine, diastéréo-isomère le moins polaire
- chlorhydrate de 1,N'-dibenzyl-N,N,N'-triméthyl-cyclohexane-1,4-diamine, diastéréo-isomère le plus polaire
- chlorhydrate de N-(4-benzyl-4-diméthylaminocyclohexyl)-N-méthyl-benzamide, diastéréo-isomère le plus polaire
- chlorhydrate de N-(4-benzyl-4-diméthylaminocyclohexyl)-N-éthyl-benzamide, diastéréo-isomère le plus polaire
- dichlorhydrate de 1-benzyl-N'-(1H-indole-3-ylméthyl)-N,N-diméthylcyclohexane-1,4-diamine
- 1-benzyl-N'-[2-(1H-indole-3-yl)-1-méthyléthyl]-N,N-diméthylcyclohexane-1,4-diamine, mélange cis/trans

- chlorhydrate de 1-benzyl-N'-indane-5-yl-N,N-diméthyl-cyclohexane-1,4-diamine
- dichlorhydrate de 1-benzyl-N'-indane-1-yl-N,N-diméthyl-cyclohexane-1,4-diamine, mélange cis/trans
- N'-indane-1-yl-N,N-diméthyl-1-phénylcyclohexane-1,4-diamine
- N'-(1H-indole-5-yl)-N,N-diméthyl-1-phénylcyclohexane-1,4-diamine
- N'-(1H-indole-3-ylméthyl)-N,N-diméthyl-1-phénylcyclo-hexane-1,4-diamine, mélange cis/trans
- N'-(1H-indole-3-ylméthyl)-N,N-diméthyl-1-phénylcyclo-hexane-1,4-diamine, diastéréo-isomère le moins polaire
- N'-[2-(1H-indole-3-yl)-éthyl]-N,N-diméthyl-1-phényl-cyclohexane-1,4-diamine, diastéréo-isomère le moins polaire
- N'-[2-(1H-indole-3-yl)-éthyl]-N,N-diméthyl-1-phényl-cyclohexane-1,4-diamine, mélange cis/trans
- N'-indane-5-yl-N,N-diméthyl-1-phénylcyclohexane-1,4-diamine, diastéréo-isomère le moins polaire
- N'-[2-(1H-indole-3-yl)-1-méthyléthyl]-N,N-diméthyl-1-phénylcyclohexane-1,4-diamine, diastéréo-isomère le moins polaire
- N'-[2-(1H-indole-3-yl)-1-méthyléthyl]-N,N-diméthyl-1-phénylcyclohexane-1,4-diamine, mélange cis/trans
- N'-[2-(5-benzyloxy-1H-indole-3-yl)-éthyl]-N,N-diméthyl-1-phénylcyclohexane-1,4-diamine, mélange cis/trans
- dichlorhydrate de N'-(9H-fluorène-1-yl)-N,N-diméthyl-1-phénylcyclohexane-1,4-diamine
- dichlorhydrate de N'-indane-2-yl-N,N-diméthyl-1-phényl-cyclohexane-1,4-diamine, mélange cis/trans
- dichlorhydrate de N'-(9H-fluorène-9-yl)-N,N-diméthyl-1-phénylcyclohexane-1,4-diamine, mélange cis/trans
- 1-benzyl-N'-(9H-fluorène-9-yl)-N,N-diméthylcyclohexane-1,4-diamine
- 1-benzyl-N'-(1H-indole-3-ylméthyl)-N,N-diméthylcyclo-hexane-1,4-diamine, mélange cis/trans
- N,N-diméthyl-N'-(1-méthyl-1H-indole-3-ylméthyl)-1-phénylcyclohexane-1,4-diamine, mélange cis/trans
- N,N-diméthyl-N'-(1-méthyl-1H-indole-3-ylméthyl)-1-phénylcyclohexane-1,4-diamine, diastéréo-isomère le plus polaire
- dichlorhydrate de N'-(2-benzo[b]thiophène-3-yléthyl)-N,N-diméthyl-1-phénylcyclohexane-1,4-diamine, mélange cis/trans
- dichlorhydrate de N'-(2-benzo[b]thiophène-3-yléthyl)-1-benzyl-N,N-diméthylcyclohexane-1,4-diamine, mélange cis/trans
- dichlorhydrate de N'-acénaphtène-1-yl-N,N-diméthyl-1-phénylcyclohexane-1,4-diamine, diastéréo-isomère le plus polaire
- dichlorhydrate de N'-acénaphtène-1-yl-N,N-diméthyl-1-phénylcyclohexane-1,4-diamine, diastéréo-isomère le moins polaire
- dichlorhydrate de N'-benzo[b]thiophène-5-yl-1-benzyl-N,N-diméthylcyclohexane-1,4-diamine, diastéréo-isomère le moins polaire
- chlorhydrate de N'-benzo[b]thiophène-5-yl-N,N-diméthyl-1-phénylcyclohexane-1,4-diamine, diastéréo-isomère le moins polaire
- dichlorhydrate de N'-benzothiazole-6-yl-N,N-diméthyl-1-phénylcyclohexane-1,4-diamine, diastéréo-isomère le moins polaire
- dichlorhydrate de N'-benzo[1,2,5]thiadiazole-4-yl-N,N-diméthyl-1-phénylcyclohexane-1,4-diamine, diastéréo-isomère le plus polaire
- dichlorhydrate de N'-[2-(1H-indole-3-yl)-1-méthyléthyl]-N,N-diméthyl-1-phénylcyclohexane-1,4-diamine, diastéréo-isomère le moins polaire
- dichlorhydrate de N'-adamantane-2-yl-N,N-diméthyl-1-phénylcyclohexane-1,4-diamine
- dichlorhydrate de N'-(9-éthyl-9H-carbazole-3-yl)-N,N-diméthyl-1-phénylcyclohexane-1,4-diamine, diastéréo-isomère le moins polaire
- chlorhydrate de N'-(3H-benzotriazole-5-yl)-N,N-diméthyl-1-phénylcyclohexane-1,4-diamine, diastéréo-isomère le moins polaire
- chlorhydrate de N'-(3H-benzotriazole-5-yl)-N,N-diméthyl-1-phénylcyclohexane-1,4-diamine, diastéréo-isomère le plus polaire
- dichlorhydrate de N'-(9H-fluorène-9-yl)-N,N-diméthyl-1-thiophène-2-ylcyclohexane-1,4-diamine, mélange cis/trans
- dichlorhydrate de N'-cyclooctyl-N,N-diméthyl-1-phényl-cyclohexane-1,4-diamine
- dichlorhydrate de N'-(1H-indole-3-ylméthyl)-N,N-diméthyl-1-phénylcyclohexane-1,4-diamine, diastéréo-isomère le moins polaire
- dichlorhydrate de N'-(1H-indole-3-ylméthyl)-N,N-diméthyl-1-phénylcyclohexane-1,4-diamine, diastéréo-isomère le plus polaire
- dichlorhydrate de N'-benzo[b]thiophène-3-ylméthyl-N,N-diméthyl-1-phénylcyclohexane-1,4-diamine, diastéréo-isomère le moins polaire
- dichlorhydrate de N'-benzo[b]thiophène-3-ylméthyl-N,N-diméthyl-1-phénylcyclohexane-1,4-diamine, diastéréo-isomère le plus polaire

- chlorhydrate de N'-anthracène-2-yl-N,N-diméthyl-1-phénylcyclohexane-1,4-diamine, diastéréo-isomère le moins polaire
- dichlorhydrate de N'-benzo[b]thiophène-3-ylméthyl-1-benzyl-N,N-diméthylcyclohexane-1,4-diamine, diastéréo-isomère le moins polaire
- dichlorhydrate de N'-benzo[b]thiophène-3-ylméthyl-1-benzyl-N,N-diméthylcyclohexane-1,4-diamine, diastéréo-isomère le plus polaire
- dichlorhydrate de N'-[2-(1H-indole-3-yl)-éthyl]-N,N-diméthyl-1-naphtalène-2-ylcyclohexane-1,4-diamine, diastéréo-isomère le moins polaire
- dichlorhydrate de N'-[2-(1H-indole-3-yl)-éthyl]-N,N-diméthyl-1-phényl-cyclohexane-1,4-diamine, diastéréo-isomère le moins polaire
- dichlorhydrate de N'-[2-(1H-indole-3-yl)-éthyl]-N,N-diméthyl-1-phényl-cyclohexane-1,4-diamine, diastéréo-isomère le plus polaire
- dichlorhydrate de N'-[2-(1H-indole-3-yl)-1-méthyléthyl]-N,N-diméthyl-1-phénylcyclohexane-1,4-diamine, diastéréo-isomère le plus polaire
- dichlorhydrate de 2-(4-diméthylamino-4-phénylcyclohexyl-amino)-3-(1H-indole-3-yl)-propionate de méthyle, diastéréo-isomère le moins polaire
- dichlorhydrate de 2-(4-diméthylamino-4-phénylcyclohexyl-amino)-3-(1H-indole-3-yl)-propionate de méthyle, diastéréo-isomère le plus polaire
- dichlorhydrate de N'-[2-(1H-indole-3-yl)-1-méthyléthyl]-N,N-diméthyl-1-naphtalène-2-ylcyclohexane-1,4-diamine, diastéréo-isomère le moins polaire
- dichlorhydrate de N'-benzo[1,3]dioxole-5-ylméthyl-N,N-diméthyl-1-phénylcyclohexane-1,4-diamine, mélange cis/trans
- dichlorhydrate de N'-[2-(6-fluoro-1H-indole-3-yl)-éthyl]-N,N-diméthyl-1-phénylcyclohexane-1,4-diamine, diastéréo-isomère le moins polaire
- dichlorhydrate de N'-[2-(6-fluoro-1H-indole-3-yl)-éthyl]-N,N-diméthyl-1-phénylcyclohexane-1,4-diamine, diastéréo-isomère le plus polaire
- dichlorhydrate de N'-[2-(1H-indole-3-yl)-éthyl]-N,N,N'-triméthyl-1-phénylcyclohexane-1,4-diamine, diastéréo-isomère le moins polaire
- dichlorhydrate de N'-[2-(1H-indole-3-yl)-éthyl]-N,N,N'-triméthyl-1-phénylcyclohexane-1,4-diamine, diastéréo-isomère le plus polaire
- dichlorhydrate de N,N-diméthyl-N'-[2-(7-méthyl-1H-indole-3-yl)-éthyl]-1-phénylcyclohexane-1,4-diamine, diastéréo-isomère le moins polaire
- dichlorhydrate de N,N-diméthyl-N'-[2-(7-méthyl-1H-indole-3-yl)-éthyl]-1-phénylcyclohexane-1,4-diamine, diastéréo-isomère le plus polaire
- dichlorhydrate de N'-[2-(5-fluoro-1H-indole-3-yl)-éthyl]-N,N-diméthyl-1-phénylcyclohexane-1,4-diamine, diastéréo-isomère le moins polaire
- dichlorhydrate de N'-[2-(5-fluoro-1H-indole-3-yl)-éthyl]-N,N-diméthyl-1-phénylcyclohexane-1,4-diamine, diastéréo-isomère le plus polaire
- dichlorhydrate de N'-acénaphtène-5-ylméthyl-N,N-diméthyl-1-phénylcyclohexane-1,4-diamine, diastéréo-isomère le moins polaire
- dichlorhydrate de N'-[2-(1H-indole-3-yl)-1-méthyléthyl]-N,N-diméthyl-1-thiophène-2-ylcyclohexane-1,4-diamine, diastéréo-isomère le moins polaire
- dichlorhydrate de N'-[2-(1H-indole-3-yl)-1-méthyléthyl]-N,N-diméthyl-1-thiophène-2-ylcyclohexane-1,4-diamine, mélange cis/trans
- dichlorhydrate de N'-[2-(7-benzyloxy-1H-indole-3-yl)-éthyl]-N,N-diméthyl-1-phénylcyclohexane-1,4-diamine, diastéréo-isomère le moins polaire
- dichlorhydrate de N'-cyclooctyl-N,N-diméthyl-1-thiophène-2-ylcyclohexane-1,4-diamine, diastéréo-isomère le moins polaire
- dichlorhydrate de N'-adamantane-2-yl-N,N-diméthyl-1-thiophène-2-ylcyclohexane-1,4-diamine, diastéréo-isomère le moins polaire
- dichlorhydrate de 3-[2-(4-diméthylamino-4-phénylcyclo-hexylamino)-éthyl]-1H-indole-5-ol, diastéréo-isomère le moins polaire
- dichlorhydrate de 3-[2-(4-diméthylamino-4-phénylcyclo-hexylamino)-éthyl]-1H-indole-5-ol, diastéréo-isomère le plus polaire
- dichlorhydrate de N'-[2-(5-méthoxy-1H-indole-3-yl)-éthyl]-N,N-diméthyl-1-phénylcyclohexane-1,4-diamine, diastéréo-isomère le moins polaire
- dichlorhydrate de N'-[2-(5-méthoxy-1H-indole-3-yl)-éthyl]-N,N-diméthyl-1-phénylcyclohexane-1,4-diamine,

diastéréo-isomère le plus polaire

● dichlorhydrate de N,N-diméthyl-N'-[2-(5-méthyl-1H-indole-3-yl)-éthyl]-1-phénylcyclohexane-1,4-diamine, diastéréo-isomère le moins polaire

● dichlorhydrate de N,N-diméthyl-N'-[2-(5-méthyl-1H-indole-3-yl)-éthyl]-1-phénylcyclohexane-1,4-diamine, diastéréo-isomère le plus polaire

● dichlorhydrate de diméthyl-[1-phényl-4-(1,3,4,9-tétrahydro-b-carboline-2-yl)-cyclohexyl]-amine

● chlorhydrate de N-(4-diméthylamino-4-phénylcyclohexyl)-N-[2-(4-fluorophényl)-éthyl]-acétamide, diastéréo-isomère le moins polaire

● dichlorhydrate d'ester méthylique d'acide 2-(4-diméthylamino-4-phénylcyclohexylamino)-3-(5-fluoro-1H-indole-3-yl)-propionique, diastéréo-isomère le moins polaire

● chlorhydrate de N-(4-diméthylamino-4-phénylcyclohexyl)-N-(3-phénylpropyl)-acétamide, diastéréo-isomère le moins polaire

● dichlorhydrate d'ester méthylique d'acide 2-(4-diméthylamino-4-phénylcyclohexylamino)-3-(6-fluoro-1H-indole-3-yl)-propionique, diastéréo-isomère le moins polaire

● chlorhydrate de N-(4-diméthylamino-4-phénylcyclohexyl)-2-(1H-indole-3-yl)-acétamide, diastéréo-isomère le plus polaire

● dichlorhydrate d'ester méthylique d'acide 2-(4-diméthylamino-4-thiophène-2-ylcyclohexylamino)-3-(1H-indole-3-yl)-propionique, diastéréo-isomère le moins polaire

● chlorhydrate de N-(4-diméthylamino-4-phénylcyclohexyl)-2-(5-méthoxy-1H-indole-3-yl)-acétamide, diastéréo-isomère le moins polaire,

le cas échéant également sous forme de leurs racémates, des stéréo-isomères purs mentionnés ou d'autres stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ; le cas échéant également sous forme des acides ou des bases ou sous forme d'autres sels, en particulier de sels physiologiquement acceptables ou de sels d'acides ou de cations physiologiquement acceptables ; ou sous forme de leurs produits de solvatation, en particulier des hydrates.

**20.** Médicament contenant au moins un dérivé substitué de cyclohexane-1,4-diamine suivant la revendication 1, dans lequel

$R^1$ et $R^2$ sont choisis, indépendamment l'un de l'autre, entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ; $R^1$ et $R^2$ ne pouvant pas représenter tous deux de l'hydrogène,

ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,

$R^6$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

$R^3$ est choisi entre un reste alkyle en $C_1$ à $C_8$ ou un reste cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ; $R^4$ est choisi entre H ; un reste alkyle en $C_1$ à $C_8$, saturé ou non saturé, ramifié ou non ramifié, substitué une ou plusieurs fois ou non substitué ; ou un groupe C (X) $R^7$, C (X)$NR^7R^8$, C(X)$OR^9$, C (X) $SR^9$, S $(O_2)R^9$,

avec X = O ou S,

$R^7$ étant choisi entre H, un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^8$ étant choisi entre H, un reste alkyle en $C_1$ à $C_4$, saturé ou non saturé, ramifié ou non ramifié, substitué une ou plusieurs fois ou non substitué, ou bien

les restes $R^7$ et $R^8$ forment ensemble un noyau et représentent $CH_2CH_2OCH_2CH_2$, $CH_2CH_2NR^{10}CH_2CH_2$ ou $(CH_2)_{3-6}$,

$R^{10}$ étant choisi entre H ; un reste alkyle en $C_1$ à $C_8$ ou cycloalkyle en $C_3$ à $C_8$, chacun saturé ou non saturé, ramifié

ou non ramifié, substitué une ou plusieurs fois ou non substitué ; un reste aryle ou hétéroaryle, chacun substitué une ou plusieurs fois ou non substitué ; ou bien un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

$R^9$ étant choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^5$ est choisi entre un reste cycloalkyle en $C_3$ à $C_8$, aryle ou hétéroaryle, chacun non substitué ou substitué une ou plusieurs fois; un groupe $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ ou $-C(Y)-CH_2-CH_2-CH_2R^{12}$,

avec Y = O, S ou $H_2$,

$R^{11}$ étant choisi entre

H, un reste alkyle en $C_1$ à $C_7$, saturé ou non saturé, ramifié ou non ramifié, substitué une ou plusieurs fois ou non substitué ; ou un groupe C(O)O-(alkyle en $C_1$ à $C_6$), saturé ou non saturé, ramifié ou non ramifié, substitué une ou plusieurs fois ou non substitué ;

et $R^{12}$ étant choisi entre

H ; un reste cycloalkyle en $C_3$ à $C_8$, aryle ou hétéroaryle, chacun non substitué ou substitué une ou plusieurs fois,

ou bien $R^4$ et $R^5$ forment ensemble un hétérocycle ayant entre 3 et 8 atomes dans le noyau, saturé ou non saturé ; substitué une ou plusieurs fois ou non substitué, et pouvant éventuellement être condensé avec d'autres noyaux, sous réserve que

- lorsque $R^3$ est un reste phényle substitué ou non substitué et l'un au moins de $R^1$ ou $R^2$ représente H ou un reste alkyle en $C_1$ à $C_8$, $R^4$ ne puisse pas représenter un reste alkyle et $R^4$ et $R^5$ ne puissent pas former ensemble un hétérocycle,

éventuellement sous forme de son racémate, des stéréo-isomeres purs, en particulier des énantiomères ou des diastéréo-isomères, ou sous forme de mélanges des stéréo-isomères, en particulier des énantiomères ou des diastéréo-isomères, dans un rapport de mélange quelconque ;

sous la forme représentée ou sous forme de ses acides ou de ses bases ou sous forme de ses sels, en particulier des sels physiologiquement acceptables ou de sels d'acides ou de cations physiologiquement acceptables ; ou sous forme de ses produits de solvatation, en particulier des hydrates, et contenant le cas échéant des additifs et/ou des substances auxiliaires convenables et/ou éventuellement d'autres substances actives.

**21.** Médicament contenant au moins un dérivé substitué de cyclohexane-1,4-diamine suivant la revendication 1, dans lequel

$R^1$ et $R^2$ sont choisis, indépendamment l'un de l'autre, entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou bien un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ; $R^1$ et $R^2$ ne pouvant pas représenter tous deux H,

ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,

$R^6$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

$R^3$ est choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste hétéroaryle, chaque fois non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^4$ est choisi entre H ; un reste alkyle en $C_1$ à $C_8$, saturé ou non saturé, ramifié ou non ramifié, substitué une ou plusieurs fois ou non substitué, ou un groupe $C(X)R^7$ $C(X)NR^7R^8$ C (X) $OR^9$, C (X) $SR^9$, S $(O_2)$ $R^9$,

avec X = O ou S,

$R^7$ étant choisi entre H, un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué

ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^8$ étant choisi entre H, un reste alkyle en $C_1$ à $C_4$, saturé ou non saturé, ramifié ou non ramifié, substitué une ou plusieurs fois ou non substitué, ou bien

les restes $R^7$ et $R^8$ forment ensemble un noyau et représentent $CH_2CH_2OCH_2CH_2$, $CH_2CH_2NR^{10}CH_2CH_2$ ou $(CH_2)_{3-6}$,

$R^{10}$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

$R^9$ étant choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^5$ est choisi entre un reste cycloalkyle en $C_3$ à $C_8$, aryle ou hétéroaryle, chacun non substitué ou substitué une ou plusieurs fois ; un groupe $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ ou $-C(Y)-CH_2-CH_2-CH_2R^{12}$,

avec Y = O, S ou $H_2$,

$R^{11}$ étant choisi entre

H, un reste alkyle en $C_1$ à $C_7$, saturé ou non saturé, ramifié ou non ramifié, substitué une ou plusieurs fois ou non substitué ; ou un groupe C(O)O-(alkyle en $C_1$ à $C_6$), saturé ou non saturé, ramifié ou non ramifié, substitué une ou plusieurs fois ou non substitué ;

et $R^{12}$ étant choisi entre

H ; un reste cycloalkyle en $C_3$ à $C_8$, aryle ou hétéroaryle, chacun non substitué ou substitué une ou plusieurs fois,

ou bien $R^4$ et $R^5$ forment ensemble un hétérocycle ayant entre 3 et 8 atomes dans le noyau, saturé ou non saturé ; substitué une ou plusieurs fois ou non substitué, qui peut éventuellement être condensé avec d'autres noyaux,

éventuellement sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ;

sous la forme représentée ou sous forme de ses acides ou de ses bases ou sous forme de ses sels, en particulier des sels physiologiquement acceptables ou de sels d'acides ou de cations physiologiquement acceptables ; ou sous forme de ses produits de solvatation, en particulier des hydrates, et contenant aussi éventuellement des additifs et/ou d'autres substances auxiliaires convenables et/ou, éventuellement, d'autres substances actives.

**22.** Médicament contenant au moins un dérivé substitué de cyclohexane-1,4-diamine suivant la revendication 1, dans lequel

$R^1$ et $R^2$ forment ensemble un noyau et représentent $CH_2CH_2OCH_2CH_2$, $CH_2CH_2NR^6CH_2CH_2$ ou $(CH_2)_{3-6}$,

$R^6$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

$R^3$ est choisi entre un reste alkyle en $C_1$ à $C_8$ ou un reste cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ; $R^4$ est choisi entre H, un reste alkyle en $C_1$ à $C_8$, saturé ou non saturé, ramifié ou non ramifié, substitué une ou plusieurs fois ou non substitué, ou un groupe C (X) $R^7$, C (X) $NR^7R^8$, C (X) $OR^9$, C (X) $SR^9$, $S(O_2)R^9$, avec X = O ou S,

$R^7$ étant choisi entre H, un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^8$ étant choisi entre H, un reste alkyle en $C_1$ à $C_4$, saturé ou non saturé, ramifié ou non ramifié, substitué une ou plusieurs fois ou non substitué,

ou bien

les restes $R^7$ et $R^8$ forment ensemble un noyau et représentent $CH_2CH_2OCH_2CH_2$, $CH_2CH_2NR^{10}CH_2CH_2$ ou $(CH_2)_{3-6}$,

$R^{10}$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

$R^9$ étant choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^5$ est choisi entre un reste cycloalkyle en $C_3$ à $C_8$, aryle ou hétéroaryle, chacun non substitué ou substitué une ou plusieurs fois ; un groupe $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ ou $-C(Y)-CH_2-CH_2-CH_2R^{12}$,

avec Y = O, S ou $H_2$,

$R^{11}$ étant choisi entre

H, un reste alkyle en $C_1$ à $C_7$, saturé ou non saturé, ramifié ou non ramifié, substitué une ou plusieurs fois ou non substitué; ou un groupe C(O)O-(alkyle en $C_1$ à $C_6$), saturé ou non saturé, ramifié ou non ramifié, substitué une ou plusieurs fois ou non substitué ;

et $R^{12}$ étant choisi entre

H ; un reste cycloalkyle en $C_3$ à $C_8$, aryle ou hétéroaryle, chacun non substitué ou substitué une ou plusieurs fois,

ou bien $R^4$ et $R^5$ forment ensemble un hétérocycle ayant entre 3 et 8 atomes dans le noyau, saturé ou non saturé ; substitué une ou plusieurs fois ou non substitué, qui peut éventuellement être condensé avec d'autres noyaux,

le cas échéant sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ;

sous la forme représentée ou sous forme de ses acides ou de ses bases ou sous forme de ses sels, en particulier des sels physiologiquement acceptables ou de sels d'acides ou de cations physiologiquement acceptables ; ou sous forme de ses produits de solvatation, en particulier des hydrates, et contenant éventuellement des additifs et/ou des substances auxiliaires convenables et/ou, le cas échéant, d'autres substances actives.

**23.** Médicament contenant au moins un dérivé substitué de cyclohexane-1,4-diamine suivant la revendication 1, dans lequel

$R^1$ et $R^2$ sont choisis, indépendamment l'un de l'autre, entre H ; un reste alkyle en $C_1$ à $C_8$ ou un reste cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué; $R^1$ et $R^2$ ne pouvant pas représenter tous deux H,

ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,

$R^6$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

$R^3$ est choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^4$ est choisi entre H, un groupe $C(X)R^7$, $C(X)NR^7R^8$, $C(X)OR^9$, $C(X)SR^9$ ou $S(O_2)R^9$,

avec X = O ou S,

$R^7$ étant choisi entre H, un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^8$ étant choisi entre H, un reste alkyle en $C_1$ à $C_4$, saturé ou non saturé, ramifié ou non ramifié, substitué une ou plusieurs fois ou non substitué, ou bien

les restes $R^7$ et $R^8$ forment ensemble un noyau et représentent $CH_2CH_2OCH_2CH_2$, $CH_2CH_2NR^{10}CH_2CH_2$ ou $(CH_2)_{3-6}$,

$R^{10}$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

$R^9$ étant choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^5$ est choisi entre un reste cycloalkyle en $C_3$ à $C_8$, aryle ou hétéroaryle, chacun non substitué ou substitué une ou plusieurs fois ; un groupe $-CHR^{11}R^{12}$, $-CHR^{11}-CH_2R^{12}$ $-CHR^{11}-CH_2-CH_2R^{12}$, $-CHR^{11}-CH_2-CH_2-CH_2R^{12}$- C (Y) $R^{12}$, -C (Y) $-CH_2R^{12}$, -C (Y) $-CH_2-CH_2R^{12}$ ou -C (Y) $-CH_2-CH_2-CH_2R^{12}$,

avec Y = O, S ou $H_2$,

$R^{11}$ étant choisi entre

H, un reste alkyle en $C_1$ à $C_7$, saturé ou non saturé, ramifié ou non ramifié, substitué une ou plusieurs fois ou non substitué ; ou bien un groupe C (O) O- (alkyle en $C_1$ à $C_6$) , saturé ou non saturé, ramifié ou non ramifié, substitué une ou plusieurs fois ou non substitué ;

et $R^{12}$ étant choisi entre

H ; un reste cycloalkyle en $C_3$ à $C_8$, aryle ou hétéroaryle, chacun non substitué ou substitué une ou plusieurs fois, éventuellement sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ;

sous la forme représentée ou sous forme de ses acides ou de ses bases ou sous forme de ses sels, en particulier des sels physiologiquement acceptables ou de sels d'acides ou de cations physiologiquement acceptables ; ou sous forme de ses produits de solvatation, en particulier des hydrates, contenant aussi, le cas échéant, des additifs et/ou des substances auxiliaires convenables et/ou, éventuellement, d'autres substances actives.

**24.** Médicament suivant l'une des revendications 20 ou 21, **caractérisé en ce que** dans les dérivés substitués de cyclohexane-1,4-diamine qu'il contient

$R^1$ et $R^2$ sont choisis indépendamment l'un de l'autre entre H ; un reste alkyle en $C_1$ à $C_8$, saturé ou non saturé, ramifié ou non ramifié, substitué une ou plusieurs fois ou non substitué ; $R^1$ et $R^2$ ne pouvant pas représenter tous deux H,

ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,

$R^6$ étant choisi entre H ; un reste alkyle en $C_1$ à $C_8$, saturé ou non saturé, ramifié ou non ramifié, substitué une ou plusieurs fois ou non substitué,

avantageusement

$R^1$ et $R^2$ sont choisis indépendamment l'un de l'autre entre H; un reste alkyle en $C_1$ à $C_4$, saturé ou non saturé, ramifié ou non ramifié, substitué une ou plusieurs fois ou non substitué ; $R^1$ et $R^2$ ne pouvant pas représenter tous deux H,

ou bien les restes $R^1$ et $R^2$ forment ensemble un noyau et représentent $(CH_2)_{4-5}$,

en particulier

$R^1$ et $R^2$ sont choisis indépendamment l'un de l'autre entre un reste méthyle ou éthyle ou bien les restes $R^1$ et $R^2$ forment ensemble un noyau et représentent $(CH_2)_5$.

**25.** Médicament suivant la revendication 22, **caractérisé en ce que** dans les dérivés substitués de cyclohexane-1,4-diamine qu'il contient

$R^1$ et $R^2$ forment ensemble un noyau et représentent $CH_2CH_2OCH_2CH_2$, $CH_2CH_2NR^6CH_2CH_2$ ou $(CH_2)_3$-6,

$R^6$ étant choisi entre H ; un reste alkyle en $C_1$ à $C_8$, saturé ou non saturé, ramifié ou non ramifié, substitué une ou plusieurs fois ou non substitué,

avantageusement

$R^1$ et $R^2$ forment ensemble un noyau et représentent $(CH_2)_{4-5}$,

en particulier

$R^1$ et $R^2$ forment ensemble un noyau et représentent $(CH_2)_5$.

**26.** Médicament suivant la revendication 23, **caractérisé en ce que** dans les dérivés substitués de cyclohexane-1,4-diamine qu'il contient
$R^1$ et $R^2$ sont choisis indépendamment l'un de l'autre entre H; un reste alkyle en $C_1$ à $C_8$, saturé ou non saturé, ramifié ou non ramifié, substitué une ou plusieurs fois ou non substitué ; $R^1$ et $R^2$ ne pouvant pas représenter tous deux H,
avantageusement
$R^1$ et $R^2$ sont choisis indépendamment l'un de l'autre entre H ; un reste alkyle en $C_1$ à $C_4$, saturé ou non saturé, ramifié ou non ramifié, substitué une ou plusieurs fois ou non substitué ; $R^1$ et $R^2$ ne pouvant pas représenter tous deux H,
en particulier
$R^1$ et $R^2$ sont choisis indépendamment l'un de l'autre entre les restes méthyle ou éthyle ;
ou bien
$R^1$ et $R^2$ forment ensemble un noyau et représentent $CH_2CH_2OCH_2CH_2$, $CH_2CH_2NR^6CH_2CH_2$ ou $(CH_2)_{3-6}$,
$R^6$ étant choisi entre H ; un reste alkyle en $C_1$ à $C_8$, saturé ou non saturé, ramifié ou non ramifié, substitué une ou plusieurs fois ou non substitué,
avantageusement
$R^1$ et $R^2$ forment ensemble un noyau et. représentent $(CH_2)_{4-5}$,
en particulier
$R^1$ et $R^2$ forment ensemble un noyau et représentent $(CH_2)_5$.

**27.** Médicament suivant l'une des revendications 20, 22 ou 23, **caractérisé en ce que** dans les dérivés substitués de cyclohexane-1,4-diamine qu'il contient
$R^3$ est choisi entre un reste cycloalkyle en $C_3$ à $C_8$, aryle ou hétéroaryle, chacun non substitué ou substitué une ou plusieurs fois ; un reste, lié par l'intermédiaire d'un groupe alkyle en $C_1$ ou $C_2$ saturé ou non saturé, non ramifié, substitué ou non substitué, aryle, cycloalkyle en $C_3$ à $C_8$ ou hétéroaryle, chacun non substitué ou substitué une ou plusieurs fois ;
avantageusement
$R^3$ est choisi entre un reste cycloalkyle en $C_5$ ou $C_6$, phényle, naphtyle, anthracényle, thiophényle, benzothiophényle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois ; un reste, lié par l'intermédiaire d'un groupe alkyle en $C_1$ ou $C_2$ saturé non ramifié, cycloalkyle en $C_5$ ou $C_6$, phényle, naphtyle, anthracényle, thiophényle, benzothiophényle, pyridyle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois ;
en particulier
$R^3$ est choisi entre un reste phényle, furyle, thiophényle, cyclohexanyle, naphtyle, benzofurannyle, indolyle, indanyle, benzodioxannyle, benzodioxolannyle, pyrrolyle, pyrimidyle, pyrazinyle ou benzothiophényle, chacun non substitué ou substitué une ou plusieurs fois ; un reste, lié par l'intermédiaire d'un groupe alkyle en $C_1$ ou $C_2$ saturé non ramifié, phényle, furyle ou thiophényle, chacun non substitué ou substitué une ou plusieurs fois.

**28.** Médicament suivant la revendication 21, **caractérisé en ce que** dans les dérivés substitués de cyclohexane-1,4-diamine qu'il contient
$R^3$ est choisi entre un reste cycloalkyle en $C_3$ à $C_8$ ou hétéroaryle, chacun non substitué ou substitué une ou plusieurs fois ; un reste, lié par l'intermédiaire d'un groupe alkyle en $C_1$ à $C_4$ saturé ou non saturé, non ramifié, substitué ou non substitué, aryle, cycloalkyle en $C_3$ à $C_8$ ou hétéroaryle, chacun non substitué ou substitué une ou plusieurs fois ;
avantageusement
$R^3$ est choisi entre un reste cycloalkyle en $C_5$ ou $C_6$, thiophényle, benzothiophényle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois ; un reste, lié par l'intermédiaire d'un groupe alkyle en $C_1$ ou $C_2$ saturé non ramifié, cycloalkyle en $C_5$ ou $C_6$, phényle, naphtyle, anthracényle, thiophényle, benzothiophényle, pyridyle, furyle, benzofurannyle, benzodioxolannyle, indolyle, indanyle, benzodioxannyle, pyrrolyle, pyrimidyle ou pyrazinyle, chacun non substitué ou substitué une
ou plusieurs fois;
en particulier
$R^3$ est choisi entre un reste furyle, thiophényle, cyclohexanyle, benzofurannyle, indolyle, indanyle, benzodioxannyle, benzodioxolannyle, pyrrolyle, pyrimidyle, pyrazinyle ou benzothiophényle, chacun non substitué ou substitué une ou plusieurs fois ; un reste, lié par l'intermédiaire d'un groupe alkyle en $C_1$ ou $C_2$ saturé non ramifié, phényle, furyle ou thiophényle, chacun non substitué ou substitué une ou plusieurs fois.

**29.** Médicament suivant l'une des revendications 20 à 28, **caractérisé en ce que**, dans les dérivés substitués de cyclohexane-1,4-diamine qu'il contient, $R^4$ représente H.

**30.** Médicament suivant l'une des revendications 20 à 28, **caractérisé en ce que** dans les dérivés substitués de cyclohexane-1,4-diamine qu'il contient
$R^4$ est choisi entre H, un reste $C(X)R^7$, $C(X)NR^7R^8$, C (X) $OR^9$, C (X) $SR^9$ ou $S(O_2)R^9$, avec X = O ou S, avantageusement
$R^4$ est choisi entre H, un groupe $C(X)R^7$, $C(X)NR^7R^8$ ou $C(X)OR^9$ avec X = O,
en particulier
$R^4$ est choisi entre H ou un groupe $C(O)R^7$; $R^7$ étant avantageusement choisi entre
H; ou un reste alkyle en $C_1$ à $C_8$ saturé ou non saturé, ramifié ou non ramifié, substitué une ou plusieurs fois ou non substitué ;
avantageusement
H ; ou un reste alkyle en $C_1$ à $C_3$ saturé, non substitué, ramifié ou non ramifié ;
en particulier $CH_3$.

**31.** Médicament suivant l'une des revendications 20 à 22, **caractérisé en ce que**, dans les dérivés substitués de cyclohexane-1,4-diamine qu'il contient,
$R^4$ et $R^5$ forment ensemble un hétérocycle ayant entre 3 et 8 atomes dans le noyau, saturé ou non saturé ; substitué une ou plusieurs fois ou non substitué, ayant avantageusement dans le noyau entre 5 et 7 atomes dont, à côté de l'atome N obligatoire, 0 à 1 autre hétéroatome est choisi entre N, S ou 0 dans le noyau ;
l'hétérocycle formé conjointement par $R^4$ et $R^5$ pouvant éventuellement être condensé avec d'autres noyaux, avantageusement avec des noyaux aromatiques et/ou hétéroaromatiques, lesquels peuvent être condensés avec d'autres noyaux aromatiques et/ou hétéroaromatiques,
en particulier, l'hétérocycle formé conjointement par $R^4$ et $R^5$ est condensé avec un ou deux autres noyaux, avantageusement l'hétérocycle formé conjointement par $R^4$ et $R^5$ est condensé avec deux autres noyaux de façon telle que $R^4$ et $R^5$ forment

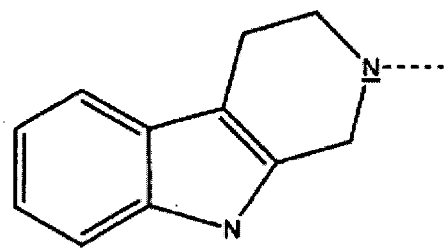

**32.** Médicament suivant l'une des revendications 20 à 22, **caractérisé en ce que** dans les dérivés substitués de cyclohexane-1,4-diamine qu'il contient
$R^4$ est choisi entre H, un reste alkyle en $C_1$ à $C_8$, saturé ou non saturé, ramifié ou non ramifié, substitué une ou plusieurs fois ou non substitué, avantageusement
H, un reste alkyle en $C_1$ à $C_6$, saturé ou non saturé, ramifié ou non ramifié, substitué une ou plusieurs fois ou non substitué,
en particulier
H, un reste alkyle en $C_1$ à $C_3$, saturé, non ramifié et non substitué.

**33.** Médicament suivant l'une des revendications 20 à 32, **caractérisé en ce que** dans les dérivés substitués de cyclohexane-1,4-diamine qu'il contient
$R^5$ est choisi entre un reste cycloalkyle en $C_3$ à $C_8$, aryle ou hétéroaryle, chacun non substitué ou substitué une ou plusieurs fois ;
avantageusement
$R^5$ est choisi entre un reste cyclobutyle, cyclopropyle, cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]-thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou substitué une ou plusieurs fois;
en particulier
$R^5$ est choisi entre un reste cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

**34.** Médicament suivant l'une des revendications 20 à 32, **caractérisé en ce que** dans les dérivés substitués de cyclohexane-1,4-diamine qu'il contient
$R^5$ est choisi entre $-CHR^{11}R^{12}$, $-CHR^{11}-CH_2R^{12}$, $-CHR^{11}-CH_2CH_2R^{12}$, $-CHR^{11}-CH_2-CH_2-CH_2R^{12}$, $-C(Y)R^{12}$, $-C (Y) -CH_2R^{12}$, $-C (Y) -CH_2-CH_2R^{12}$ ou $-C (Y) -CH_2-CH_2-CH_2R^{12}$,
avec Y = O, S ou $H_2$,
avantageusement
$R^5$ est choisi entre $-CHR^{11}R^{12}$, $-CHR^{11}-CH_2R^{12}$, $-CHR^{11}-CH_2-CH_2R^{12}$, $-C(Y)R^{12}$, $-C(Y)-CH_2R^{12}$ ou $-C (Y)-CH_2-CH_2-CH_2R^{12}$,
avec Y = O ou S,
en particulier
$R^5$ est choisi entre $-CHR^{11}R^{12}$, $-CHR^{11}-CH_2R^{12}$, $-CHR^{11}-CH_2-CH_2R^{12}$, $-C(Y)R^{12}$ ou $-C (Y) -CH_2R^{12}$,
avec Y = O.

**35.** Médicament suivant la revendication 34, **caractérisé en ce que** dans les dérivés substitués de cyclohexane-1,4-diamine qu'il contient
$R^{11}$ est choisi entre
H, un reste alkyle en $C_1$ à $C_4$, saturé ou non saturé, ramifié ou non ramifié, substitué une ou plusieurs fois ou non substitué ; ou un groupe C (O) O- (alkyle en $C_1$ à $C_4$), saturé ou non saturé, ramifié ou non ramifié, substitué une ou plusieurs fois ou non substitué ;
avantageusement
H, un reste alkyle en $C_1$ à $C_4$, saturé ou non saturé, ramifié ou non ramifié, substitué une ou plusieurs fois ou non substitué ; ou un groupe C(O)O-(alkyle en $C_1$ ou $C_2$), saturé, non ramifié, substitué une ou plusieurs fois ou non substitué ;
en particulier
H, $CH_3$, $C_2H_5$ et $C (O) O-CH_3$.

**36.** Médicament suivant la revendication 34, **caractérisé en ce que** dans les dérivés substitués de cyclohexane-1,4-diamine qu'il contient
$R^{12}$ est choisi entre un reste cycloalkyle en $C_3$ à $C_8$, aryle ou hétéroaryle, chacun non substitué ou substitué une ou plusieurs fois;
avantageusement
$R^{12}$ est choisi entre un reste cyclobutyle, cyclopropyle, cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]-thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou substitué une ou plusieurs fois ;
en particulier
$R^{12}$ est choisi entre un reste cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

**37.** Médicament suivant l'une des revendications 20 à 36, **caractérisé en ce qu'**il contient encore, à côté d'au moins un dérivé substitué de cyclohexane-1,4-diamine, un opioïde, avantageusement un opioïde puissant, en particulier la morphine, ou un anesthésique, avantageusement l'hexobarbital ou l'halothane.

**38.** Utilisation d'un dérivé substitué de cyclohexane-1,4-diamine suivant la revendication 1,
dans lequel,
$R^1$ et $R^2$ sont choisis, indépendamment l'un de l'autre, entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste

aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ; $R^1$ et $R^2$ ne pouvant pas représenter tous deux H,

ou bien les restes $R^1$ et $R^2$ forment conjointement un noyau et représentent $CH_2CH_2OCH_2CH_2$, $CH_2CH_2NR^6CH_2CH_2$ ou $(CH_2)_{3-6}$,

$R^6$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

$R^3$ est choisi entre un reste allkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^4$ est choisi entre H, un reste alkyle en $C_1$ à $C_8$, saturé ou non saturé, ramifié ou non ramifié, substitué une ou plusieurs fois ou non substitué, ou un groupe $C(X)R^7$, $C(X)NR^7R^8$, $C(X)OR^9$, $C(X)SR^9$, $S(O_2)R^9$,

avec X = O ou S,

$R^7$ étant choisi entre H, un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^8$ étant choisi entre H, un reste alkyle en $C_1$ à $C_4$, saturé ou non saturé, ramifié ou non ramifié, substitué une ou plusieurs fois ou non substitué, ou bien

les restes $R^7$ et $R^8$ forment ensemble un noyau et représentent $CH_2CH_2OCH_2CH_2$, $CH_2CH_2NR^{10}CH_2CH_2$ ou $(CH_2)_{3-6}$,

$R^{10}$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

$R^9$ étant choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;

$R^5$ est choisi entre un reste cycloalkyle en $C_3$ à $C_8$, aryle ou hétéroaryle, chacun non substitué ou substitué une ou plusieurs fois ; un groupe $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ ou $-C(Y)-CH_2-CH_2-CH_2R^{12}$,

avec Y = O, S ou $H_2$,

$R^{11}$ étant choisi entre

H, un reste alkyle en $C_1$ à $C_7$, saturé ou non saturé, ramifié ou non ramifié, substitué une ou plusieurs fois ou non substitué ; ou bien un groupe C(O) O- (alkyle en $C_1$ à $C_6$), saturé ou non saturé, ramifié ou non ramifié, substitué une ou plusieurs fois ou non substitué ;

et $R^{12}$ étant choisi entre

H ; un reste cycloalkyle en $C_3$ à $C_8$, aryle ou hétéroaryle, chacun non substitué ou substitué une ou plusieurs fois,

ou bien $R^4$ et $R^5$ forment ensemble un hétérocycle ayant entre 3 et 8 atomes de carbone dans le noyau, saturé ou non saturé, substitué une ou plusieurs fois ou non substitué, qui peut éventuellement être condensé avec d'autres noyaux,

sous réserve

- ■ que lorsque $R^3$ est un reste phényle substitué ou non substitué et que l'un au moins de $R^1$ ou $R^2$ représente H ou un reste alkyle en $C_1$ à $C_8$, $R^4$ ne puisse pas être un reste alkyle et $R^4$ et $R^5$ ne puissent pas former ensemble un hétérocycle,

le cas échéant sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ;

sous la forme représentée ou sous forme de ses acides ou de ses bases ou sous forme de ses sels, en particulier

des sels physiologiquement acceptables ou de sels d'acides ou de cations physiologiquement acceptables; ou sous forme de ses produits de solvatation, en particulier des hydrates ; pour la préparation d'un médicament destiné au traitement de la douleur, en particulier d'une douleur aiguë, neuropathique ou chronique, ou destiné à la coadministration en cas de traitement avec un analgésique opioïde.

**39.** Utilisation d'un dérivé substitué de cyclohexane-1,4-diamine suivant la revendication 1,
dans lequel
les restes $R^1$ et $R^2$ sont choisis, indépendamment l'un de l'autre, entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou bien un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ; $R^1$ et $R^2$ ne pouvant pas être tous deux H,
ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,
$R^6$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou bien un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;
$R^3$ est choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois;
$R^4$ est choisi entre H, un reste alkyle en $C_1$ à $C_8$, saturé ou non saturé, ramifié ou non ramifié, substitué une ou plusieurs fois ou non substitué ou un groupe $C(X)R^7$, $C(X)NR^7R^8$, $C(X)OR^9$, $C(X)SR^9$, $S(O_2)R^9$,
avec X = O ou S,
$R^7$ étant choisi entre H, un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;
$R^8$ étant choisi entre H, un reste alkyle en $C_1$ à $C_4$, saturé ou non saturé, ramifié ou non ramifié, substitué une ou plusieurs fois ou non substitué, ou bien
les restes $R^7$ et $R^8$ forment ensemble un noyau et représentent $CH_2CH_2OCH_2CH_2$, $CH_2CH_2NR^{10}CH_2CH_2$ ou $(CH_2)_{3-6}$,
$R^{10}$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;
$R^9$ étant choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;
$R^5$ est choisi entre un reste cycloalkyle en $C_3$ à $C_8$, aryle ou hétéroaryle, chacun non substitué ou substitué une ou plusieurs fois ; un groupe $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C (Y) R^{12}$, $-C (Y) -CH_2R^{12}$, $-C (Y) CH_2 - CH_2R^{12}$ ou $-C(Y)-CH_2-CH_2-CH_2R^{12}$,
avec Y = O, S ou $H_2$,
$R^{11}$ étant choisi entre
H, un reste alkyle en $C_1$ à $C_7$, saturé ou non saturé, ramifié ou non ramifié, substitué une ou plusieurs fois ou non substitué ; ou un groupe C (O) O- (alkyle en $C_1$ à $C_6$), saturé ou non saturé, ramifié ou non ramifié, substitué une ou plusieurs fois ou non substitué ;
et $R^{12}$ étant choisi entre
H ; un reste cycloalkyle en $C_3$ à $C_8$, aryle ou hétéroaryle, chacun non substitué ou substitué une ou plusieurs fois,
ou bien $R^4$ et $R^5$ forment ensemble un hétérocycle ayant entre 3 et 8 atomes dans le noyau, saturé ou non saturé ; substitué une ou plusieurs fois ou non substitué, qui peut éventuellement être condensé avec d'autres noyaux,
éventuellement sous forme de son racémate, des stéréo-isomères purs, en particulier des énantiomères ou des diastéréo-isomères, ou sous forme de mélanges des stéréo-isomères, en particulier des énantiomères ou des

diastéréo-isomères, dans un rapport de mélange quelconque ;
sous la forme représentée ou sous forme de ses acides ou de ses bases ou sous forme de ses sels, en particulier des sels physiologiquement acceptables ou de sels d'acides ou de cations physiologiquement acceptables ; ou sous forme de ses produits de solvatation, en particulier des hydrates; pour la préparation d'un médicament destiné au traitement de la douleur, en particulier d'une douleur aiguë, neuropathique ou chronique, ou destiné à la coadministration en cas de traitement avec un analgésique opioïde.

**40.** Utilisation d'un dérivé substitué de cyclohexane-1,4-diamine suivant la revendication 1,
dans lequel
$R^1$ et $R^2$ forment ensemble un noyau et représentent $CH_2CH_2OCH_2CH_2$, $CH_2CH_2NR^6CH_2CH_2$ ou $(CH_2)_{3-6}$,
$R^6$ étant choisi entre H ; un reste alkyle en $C_1$ à $C_8$ ou cycloalkyle en $C_3$ à $C_8$, chacun saturé ou non saturé, ramifié ou non ramifié; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;
$R^3$ est choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste lié par l'intermédiaire d'un groupe alkyle en $C_1$ à $C_4$ saturé ou non saturé, ramifié ou non ramifié, substitué ou non substitué, aryle, cyclolalkyle en $C_3$ à $C_8$ ou hétéroaryle, chacun non substitué ou substitué une ou plusieurs fois ;
$R^4$ est choisi entre H, un reste alkyle en $C_1$ à $C_8$, saturé ou non saturé, ramifié ou non ramifié, substitué une ou plusieurs fois ou non substitué, ou un groupe $C(X)R^7$, $C(X)NR^7R^8$, $C(X)OR^9$, $C(X)SR^9$ ou $S(O_2)R^9$,
avec X = O ou S,
$R^7$ étant choisi entre H, un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué, une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;
$R^8$ étant choisi entre H, un reste alkyle en $C_1$ à $C_4$, saturé ou non saturé, ramifié ou non ramifié, substitué une ou plusieurs fois ou non substitué, ou bien
les restes $R^7$ et $R^8$ forment ensemble un noyau et représentent $CH_2CH_2OCH_2CH_2$, $CH_2CH_2NR^6CH_2CH_2$ ou $(CH_2)_{3-6}$,
$R^{10}$ étant choisi entre H; un reste alkyle en $C_1$ à $C_8$ ou cycloalkyle en $C_3$ à $C_8$, saturé ou non saturé, ramifié ou non ramifié, substitué une ou plusieurs fois ou non substitué ; un reste aryle ou hétéroaryle, chacun substitué une ou plusieurs fois ou non substitué; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;
$R^9$ étant choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;
$R^5$ est choisi entre un reste cycloalkyle en $C_3$ à $C_8$, aryle ou hétéroaryle, chacun non substitué ou substitué une ou plusieurs fois ; un groupe $-CHR^{11}R^{12}$, $-CHR^{11}-CH_2R^{12}$, $-CHR^{11}-CH_2CH_2R^{12}$, $-CHR^{11}-CH_2CH_2-CH_2R^{12}$, $-C(Y)R^{12}$, $-C(Y)-CH_2R^{12}$, $-C(Y)-CH_2-CH_2R^{12}$ ou $-C(Y)-CH_2-CH_2-CH_2R^{12}$,
avec Y = O, S ou $H_2$,
$R^{11}$ étant choisi entre
H, un reste alkyle en $C_1$ à $C_7$, saturé ou non saturé, ramifié ou non ramifié, substitué une ou plusieurs fois ou non substitué ; ou un groupe C (O) O-alkyle en $C_1$ à $C_6$, saturé ou non saturé, ramifié ou non ramifié, substitué une ou plusieurs fois ou non substitué ;
et $R^{12}$ étant choisi entre
H ; un reste cycloalkyle en $C_3$ à $C_8$, aryle ou hétéroaryle, chacun non substitué ou substitué une ou plusieurs fois,
ou $R^4$ et $R^5$ forment conjointement un hétérocycle ayant entre 3 et 8 atomes dans le noyau, saturé ou non saturé ; substitué une ou plusieurs fois ou non substitué et pouvant éventuellement être condensé avec d'autres noyaux,
le cas échéant sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ;
sous la forme représentée ou sous forme de ses acides ou de ses bases ou sous forme de ses sels, en particulier des sels physiologiquement acceptables ou de sels d'acides ou de cations physiologiquement acceptables ; ou sous forme de ses produits de solvatation, en particulier des hydrates ;

pour la préparation d'un médicament destiné au traitement de la douleur, en particulier d'une douleur aiguë, neuropathique ou chronique, ou destiné à la coadministration en cas de traitement avec un analgésique opioïde.

**41.** Utilisation d'un dérivé substitué de cyclohexane-1,4-diamine suivant la revendication 1, dans lequel
$R^1$ et $R^2$ sont choisis, indépendamment l'un de l'autre, entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ; $R^1$ et $R^2$ ne pouvant pas représenter tous deux H,
ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,
$R^6$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;
$R^3$ est choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;
$R^4$ est choisi entre H, un groupe C (X) $R^7$, C (X) $NR^7R^8$, C (X) $OR^9$, C (X) $SR^9$ ou S $(O_2)$ $R^9$,
avec X = O ou S,
$R^7$ étant choisi entre H, un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;
$R^8$ étant choisi entre H, un reste alkyle en $C_1$ à $C_4$, saturé ou non saturé, ramifié ou non ramifié, substitué une ou plusieurs fois ou non substitué, ou bien
les restes $R^7$ et $R^8$ forment ensemble un noyau et représentent $CH_2CH_2OCH_2CH_2$, $CH_2CH_2NR^{10}CH_2CH_2$ ou $(CH_2)_{3-6}$,
$R^{10}$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;
$R^9$ étant choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;
$R^5$ est choisi entre un reste cycloalkyle en $C_3$ à $C_8$, aryle ou hétéroaryle, chacun non substitué ou substitué une ou plusieurs fois ; un groupe $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, -C (Y) $R^{12}$, -C (Y) $-CH_2R^{12}$, -C (Y) $-CH_2CH_2R^{12}$ ou -C (Y)-$CH_2$-CH2-$CH_2R^{12}$,
avec Y = O, S ou $H_2$,
$R^{11}$ étant choisi entre
H, un reste alkyle en $C_1$ à $C_7$, saturé ou non saturé, ramifié ou non ramifié, substitué une ou plusieurs fois ou non substitué ; ou un groupe C (O) O- (alkyle en $C_1$ à $C_6$), saturé ou non saturé, ramifié ou non ramifié, substitué une ou plusieurs fois ou non substitué ;
et $R^{12}$ étant choisi entre
H ; un reste cycloalkyle en $C_3$ à $C_8$, aryle ou hétéroaryle, chacun non substitué ou substitué une ou plusieurs fois,
le cas échéant sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ;
sous la forme représentée ou sous forme de ses acides ou de ses bases ou sous forme de ses sels, en particulier des sels physiologiquement acceptables ou de sels d'acides ou de cations physiologiquement acceptables ; ou sous forme de ses produits de solvatation, en particulier des hydrates ; pour la préparation d'un médicament destiné

au traitement de la douleur, en particulier d'une douleur aiguë, neuropatique ou chronique, ou destiné à la coadministration en cas de traitement avec un analgésique opioïde.

**42.** Utilisation d'un dérivé substitué de cyclohexane-1,4-diamine suivant la revendication 1,
dans lequel
$R^1$ et $R^2$ sont choisis, indépendamment l'un de l'autre, entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ; $R^1$ et $R^2$ ne pouvant pas représenter tous deux H,
ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,
$R^6$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;
$R^3$ est choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ; $R^4$ est choisi entre H, un reste alkyle en $C_1$ à $C_8$, saturé ou non saturé, ramifié ou non ramifié, substitué une ou plusieurs fois ou non substitué, ou un groupe $C(X)R^7$, $C(X)NR^7R^8$, $C(X)OR^9$, $C(X)SR^9$, $S(O_2)R^9$,
avec X = O ou S,
$R^7$ étant choisi entre H, un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;
$R^8$ étant choisi entre H, un reste alkyle en $C_1$ à $C_4$, saturé ou non saturé, ramifié ou non ramifié, substitué une ou plusieurs fois ou non substitué,
ou bien
les restes $R^7$ et $R^8$ forment ensemble un noyau et représentent $CH_2CH_2OCH_2CH_2$, $CH_2CH_2NR^{10}CH_2CH_2$ ou $(CH_2)_{3-6}$,
$R^{10}$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou bien un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;
$R^9$ étant choisi entre un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste, lié par l'intermédiaire d'un groupe alkyle en $C_1$ à $C_4$ saturé ou non saturé, ramifié ou non ramifié, substitué ou non substitué, aryle, cycloalkyle en $C_3$ à $C_8$ ou hétéroaryle, chacun non substitué ou substitué une ou plusieurs fois ;
$R^5$ est choisi entre un reste cycloalkyle en $C_3$ à $C_8$, aryle ou hétéroaryle, chacun non substitué ou substitué une ou plusieurs fois ; un groupe $-CHR^{11}R^{12}$, $-CHR^{11}-CH_2R^{12}$, $-CHR^{11}CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ ou $-C(Y)-CH_2-CH_2-CH_2R^{12}$,
avec Y = O, S ou $H_2$,
$R^{11}$ étant choisi entre
H, un reste alkyle en $C_1$ à $C_7$, saturé ou non saturé, ramifié ou non ramifié, substitué une ou plusieurs fois ou non substitué ; ou un groupe C (O) O- (alkyle en $C_1$ à $C_6$), saturé ou non saturé, ramifié ou non ramifié, substitué une ou plusieurs fois ou non substitué ;
et $R^{12}$ étant choisi entre
H ; un reste cycloalkyle en $C_3$ à $C_8$, aryle ou hétéroaryle, chacun non substitué ou substitué une ou plusieurs fois,
ou bien $R^4$ et $R^5$ forment ensemble un hétérocycle ayant entre 3 et 8 atomes dans le noyau, saturé ou non saturé ; substitué une ou plusieurs fois ou non substitué, et pouvant éventuellement être condensé avec d'autres noyaux,
le cas échéant sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ;

sous la forme représentée ou sous forme de ses acides ou de ses bases ou sous forme de ses sels, en particulier des sels physiologiquement acceptables ou de sels d'acides ou de cations physiologiquement acceptables ; ou sous forme de ses produits de solvatation, en particulier des hydrates ; pour la préparation d'un médicament destiné au traitement d'états d'angoisse, du stress ou de syndromes liés à un stress, de dépressions, de l'épilepsie, de la maladie d'Alzheimer, de la démence sénile, de troubles cognitifs généraux, de difficultés d'apprentissage et de mémoire (comme nootrope), de phénomènes de privation, d'abus d'alcool et/ou de drogues et/ou de médicaments et d'alcoolodépendance, et/ou de toxicomanie et/ou de pharmacodépendance, de dysfonctionnements sexuels, de maladies cardio-vasculaires, de l'hypotension, de l'hypertension, des acouphènes, du prurit, de la migraine, de la surdité, de l'insuffisance de motilité intestinale, de troubles de l'ingestion de nourriture, de l'anorexie, de l'obésité, de troubles moteurs, de la diarrhée, de la cachexie, de l'incontinence d'urine ou comme myorelaxant, anticonvulsif ou anesthésique, ou en vue de la coadministration en cas de traitement avec un anesthésique, en vue de la diurèse ou de l'antinatriurèse et/ou en vue de l'anxiolyse.

**43.** Utilisation suivant l'une des revendications 38, 39 ou 42, **caractérisée en ce que**, dans les dérivés substitués de cyclohexane-1,4-diamine utilisés,
$R^1$ et $R^2$ sont choisis, indépendamment l'un de l'autre, entre H ; un reste alkyle en $C_1$ à $C_8$, saturé ou non saturé, ramifié ou non ramifié, substitué une ou plusieurs fois ou non substitué ; $R^1$ et $R^2$ ne pouvant pas être tous deux H, ou bien les restres $R^1$ et $R^2$ forment ensemble un noyau et représentent $CH_2CH_2OCH_2CH_2$, $CH_2CH_2NR^6CH_2CH_2$ ou $(CH_2)_{3-6}$,
$R^6$ étant choisi entre H ; un reste alkyle en $C_1$ à $C_8$, saturé ou non saturé, ramifié ou non ramifié, substitué une ou plusieurs fois ou non substitué,
avantageusement
$R^1$ et $R^2$ sont choisis indépendamment l'un de l'autre entre H ; un reste alkyle en $C_1$ à $C_4$, saturé ou non saturé, ramifié ou non ramifié, substitué une ou plusieurs fois ou non substitué, $R^1$ et $R^2$ ne pouvant pas représenter tous deux H,
ou bien les restes $R^1$ et $R^2$ forment ensemble un noyau et représentent $(CH_2)_{4-5}$,
en particulier
$R^1$ et $R^2$ sont choisis indépendamment l'un de l'autre entre un reste méthyle ou éthyle ou bien les restes $R^1$ et $R^2$ forment ensemble un noyau et représentent $(CH_2)_5$.

**44.** Utilisation suivant la revendication 40, **caractérisée en ce que**, dans les dérivés substitués de cyclohexane-1,4-diamine utilisés,
$R^1$ et $R^2$ forment ensemble un noyau et représentent $CH_2CH_2OCH_2CH_2$, $CH_2CH_2NR^6CH_2CH_2$ ou $(CH_2)_{3-6}$,
$R^6$ étant choisi entre H; un reste alkyle en $C_1$ à $C_8$, saturé ou non saturé, ramifié ou non ramifié, substitué une ou plusieurs fois ou non substitué,
avantageusement
$R^1$ et $R^2$ forment ensemble un noyau et représentent $(CH_2)_{4-5}$,
en particulier
$R^1$ et $R^2$ forment ensemble un noyau et représentent $(CH_2)_5$.

**45.** Utilsation suivant la revendication 41, **caractérisée en ce que**, dans les dérivés substitués de cyclohexane-1,4-diamine utilisés,
$R^1$ et $R^2$ sont choisis indépendamment l'un de l'autre entre H ; un reste alkyle en $C_1$ à $C_8$ saturé ou non saturé, ramifié ou non ramifié, substitué une ou plusieurs fois ou non substitué ; $R^1$ et $R^2$ ne pouvant pas représenter tous deux H,
avantageusement,
$R^1$ et $R^2$ sont choisis indépendamment l'un de l'autre entre H ; un reste alkyle en $C_1$ à $C_4$, saturé ou non saturé, ramifié ou non ramifié, substitué une ou plusieurs fois ou non substitué ; $R^1$ et $R^2$ ne pouvant pas représenter tous deux H,
en particulier
$R^1$ et $R^2$ sont choisis indépendamment l'un de l'autre entre un reste méthyle ou éthyle;
ou bien
$R^1$ et $R^2$ forment ensemble un noyau et représentent $CH_2CH_2OCH_2CH_2$, $CH_2CH_2NR^6CH_2CH_2$ ou $(CH_2)_{3-6}$,
$R^6$ étant choisi entre H ; un reste alkyle en $C_1$ à $C_8$, saturé ou non saturé, ramifié ou non ramifié, substitué une ou plusieurs fois ou non substitué,
avantageusement
$R^1$ et $R^2$ forment ensemble un noyau et représentent $(CH_2)_{4-5}$,
en particulier

$R^1$ et $R^2$ forment ensemble un noyau et représentent $(CH_2)_5$.

**46.** Utilisation suivant l'une des revendications 38 ou 40 à 42, **caractérisée en ce que**, dans les dérivés substitués de cyclohexane-1,4-diamine utilisés,
$R^3$ est choisi entre un reste cycloalkyle en $C_3$ à $C_8$, aryle ou hétéroaryle, chacun non substitué ou substitué une ou plusieurs fois ; un reste, lié par l'intermédiaire d'un groupe alkyle en $C_1$ ou $C_2$ saturé ou non saturé, non ramifié, substitué ou non substitué, aryle, cycloalkyle en $C_3$ à $C_8$ ou hétéroaryle, chacun non substitué ou substitué une ou plusieurs fois ;
avantageusement
$R^3$ est choisi entre un reste cycloalkyle en $C_5$ ou $C_6$, phényle, naphtyle, anthracényle, thiophényle, benzothiophényle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois ; un reste, lié par l'intermédiaire d'un groupe alkyle en $C_1$ ou $C_2$ saturé non ramifié, cycloalkyle en $C_5$ ou $C_6$, phényle, naphtyle, anthracényle, thiophényle, benzothiophényle, pyridyle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois ;
en particulier,
$R^3$ est choisi entre un reste phényle, furyle, thiophényle, cyclohexanyle, naphtyle, benzofurannyle, indolyle, indanyle, benzodioxannyle, benzodioxolannyle, pyrrolyle, pyrimidyle, pyrazinyle ou benzothiophényle, chacun non substitué ou substitué une ou plusieurs fois ; un reste, lié par l'intermédiaire d'un groupe alkyle en $C_1$ ou $C_2$ saturé non ramifié, phényle, furyle ou thiophényle, chacun non substitué ou substitué une ou plusieurs fois.

**47.** Utilisation suivant la revendication 39, **caractérisée en ce que**, dans les dérivés substitués de cyclohexane-1,4-diamine utilisés,
$R^3$ est choisi entre un reste cycloalkyle en $C_3$ à $C_8$, ou hétéroaryle, chacun non substitué ou substitué une ou plusieurs fois ; un reste, lié par l'intermédiaire d'un groupe alkyle en $C_1$ à $C_4$ saturé ou non saturé, non ramifié, substitué ou non substitué, aryle, cycloalkyle en $C_3$ à $C_8$ ou hétéroaryle, chacun non substitué ou substitué une ou plusieurs fois;
avantageusement
$R^3$ est choisi entre un reste cycloalkyle en $C_5$ ou $C_6$, thiophényle, benzothiophényle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois ; un reste, lié par l'intermédiaire d'un groupe alkyle en $C_1$ ou $C_2$ saturé non ramifié, cycloalkyle en $C_5$ ou $C_6$, phényle, naphtyle, anthracényle, thiophényle, benzothiophényle, pyridyle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois;
en particulier,
$R^3$ est choisi entre un reste furyle, thiophényle, cyclohexanyle, benzofurannyle, indolyle, indanyle, benzodioxannyle, benzodioxolannyle, pyrrolyle, pyrimidyle, pyrazinyle ou benzothiophényle, chacun non substitué ou substitué une ou plusieurs fois ; un reste, lié par l'intermédiaire d'un groupe alkyle en $C_1$ ou $C_2$ saturé non ramifié, phényle, furyle ou thiophényle, chacun non substitué ou substitué une ou plusieurs fois.

**48.** Utilisation suivant l'une des revendications 38 à 47, **caractérisée en ce que**, dans les dérivés substitués de cyclohexane-1,4-diamine utilisés, $R^4$ représente H.

**49.** Utilisation suivant l'une des revendications 38 à 47, **caractérisée en ce que**, dans les dérivés substitués de cyclohexane-1,4-diamine utilisés,
$R^4$ est choisi entre H, un groupe C (X) $R^7$, C (X) $NR^7R^8$, C (X) $OR^9$, C (X) $SR^9$ ou $S(O_2)R^9$, avec X = O ou S,
avantageusement,
$R^4$ est choisi entre H, un groupe $C(X)R^7$, $C(X)NR^7R^8$ ou $C(X)OR^9$ avec X = O,
en particulier
$R^4$ est choisi entre H ou un groupe $C(O)R^7$; $R^7$ étant avantageusement choisi entre
H; ou un reste alkyle en $C_1$ à $C_8$ saturé ou non saturé, ramifié ou non ramifié, substitué une ou plusieurs fois ou non substitué ;
avantageusement
H ; ou un reste alkyle en $C_1$ à $C_3$, saturé, non substitué, ramifié ou non ramifié ; en particulier $CH_3$.

**50.** Utilisation suivant l'une des revendications 38 à 40 ou 42, **caractérisée en ce que**, dans les dérivés substitués de cyclohexane-1,4-diamine utilisés,
$R^4$ et $R^5$ forment ensemble un hétérocycle ayant entre 3 et 8 atomes dans le noyau, saturé ou non saturé ; substitué

une ou plusieurs fois ou non substitué, ayant avantageusement entre 5 et 7 atomes dans le noyau dont, à côté de l'atome N obligatoire, 0 à 1 autre hétéroatome choisi entre N, S ou O est présent ; l'hétérocycle formé conjointement par $R^4$ et $R^5$ pouvant éventuellement être condensé avec d'autres noyaux, avantageusement avec des noyaux aromatiques et/ou hétéroaromatiques, lesquels peuvent être condensés avec d'autres noyaux aromatiques et/ou hétéroaromatiques,
en particulier l'hétérocycle formé conjointement par $R^4$ et $R^5$ est condensé avec un ou deux autres noyaux, l'hétérocycle formé conjointement par $R^4$ et $R^5$ étant avantageusement condensé avec deux autres noyaux de façon telle que $R^4$ et $R^5$ représentent ensemble

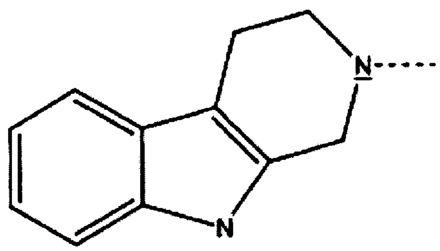

**51.** Utilisation suivant l'une des revendications 38 à 40 ou 42, **caractérisée en ce que**, dans les dérivés substitués de cyclohexane-1,4-diamine utilisés,
$R^4$ est choisi entre H, un reste alkyle en $C_1$ à $C_8$, saturé ou non saturé, ramifié ou non ramifié, substitué une ou plusieurs fois ou non substitué,
avantageusement
H, un reste alkyle en $C_1$ à $C_6$, saturé ou non saturé, ramifié ou non ramifié, substitué une ou plusieurs fois ou non substitué,
en particulier
H, un reste alkyle en $C_1$ à $C_3$, saturé, non ramifié et non substitué.

**52.** Utilisation suivant l'une des revendications 38 à 51, **caractérisée en ce que**, dans les dérivés substitués de cyclohexane-1,4-diamine utilisés,
$R^5$ est choisi entre un reste cycloalkyle en $C_3$ à $C_8$, aryle ou hétéroaryle, chacun non substitué ou substitué une ou plusieurs fois ;
avantageusement
$R^5$ est choisi entre un reste cyclobutyle, cyclopropyle, cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou substitué une ou plusieurs fois ;
en particulier
$R^5$ est choisi entre un reste cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

**53.** Utilisation suivant l'une des revendications 38 à 51, **caractérisée en ce que**, dans les dérivés substitués de cyclohexane-1,4-diamine utilisés,
$R^5$ est choisi entre $-CHR^{11}R^{12}$, $-CHR^{11}-CH^2R^{12}$, $-CHR^{11}-CH^2-CH_2R^{12}$, $-CHR^{11}-CH_2-CH_2{}_-CH_2R^{12}$, $-C(Y) R^{12}$, $-C (Y) -CH_2R^{12}$, $-C (Y) -CH_2-CH_2R^{12}$ ou $-C (Y) -CH_2-CH_2-CH_2R^{12}$,
avec Y = O, S ou $H_2$,
avantageusement
$R^5$ est choisi entre $-CHR^{11}R^{12}$, $-CHR^{11}-CH_2R^{12}$, $-CHR^{11}-CH_2-CH_2R^{12}$, $-C (Y) R^{12}$, $- C (Y) - -CH_2R^{12}$ ou $-C (Y) -CH2-CH_2R^{12}$,
avec Y = O ou S,
en particulier
$R^5$ est choisi entre $-CHR^{11}R^{12}$, $-CHR^{11}-CH_2R^{12}$, $-CHR^{11}-CH_2-CH_2R^{12}$, $-C (Y) R^{12}$ ou $-C(Y) -CH_2R^{12}$,

avec Y = O.

**54.** Utilisation suivant la revendication 53, **caractérisée en ce que**, dans les dérivés substitués de cyclohexane-1,4-diamine utilisés,
$R^{11}$ est choisi entre
H, un reste alkyle en $C_1$ à $C_4$, saturé ou non saturé, ramifié ou non ramifié, substitué une ou plusieurs fois ou non substitué ; ou un groupe C (O) O-(alkyle en $C_1$ à $C_4$), saturé ou non saturé, ramifié ou non ramifié, substitué une ou plusieurs fois ou non substitué ;
avantageusement
H, un reste alkyle en $C_1$ à $C_4$, saturé ou non saturé, ramifié ou non ramifié, substitué une ou plusieurs fois ou non substitué; ou un groupe C(O)O-(alkyle en $C_1$ ou $C_2$), saturé, non ramifié, substitué une ou plusieurs fois ou non substitué;
en particulier
H, $CH_3$, $C_2H_5$ et C(O)O-$CH_3$.

**55.** Utilisation suivant la revendication 53, **caractérisée en ce que**, dans les dérivés substitués de cyclohexane-1,4-diamine utilisés,
$R^{12}$ est choisi entre un reste cycloalkyle en $C_3$ à $C_8$, aryle ou hétéroaryle, chacun non substitué ou substitué une ou plusieurs fois ;
avantageusement
$R^{12}$ est choisi entre un reste cyclobutyle, cyclopropyle, cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]-thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou substitué une ou plusieurs fois;
en particulier
$R^{12}$ est choisi entre un reste cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

**56.** Dérivés substitués de cyclohexane-1,4-diamine, médicament ou procédé selon l'une des revendicatons précédentes, **caractérisés en ce que** $R^4$ et $R^5$ ne forment pas ensemble un hétérocycle.

**57.** Procédé de production d'un dérivé substitué de cyclohexane-1,4-diamine suivant l'une des revendications 1 à 19, comprenant les étapes suivantes :

a. une cyclohexane-1,4-dione de formule II, protégée avec les groupes $S^1$ et $S^2$, est amenée à réagir en présence d'un composé de formule $HNR^{01}R^{02}$, avec un cyanure, avantageusement le cyanure de potassium, pour former un dérivé de 1-amino-4-oxocyclohexane-carbonitrile N-substitué protégé de formule III ;

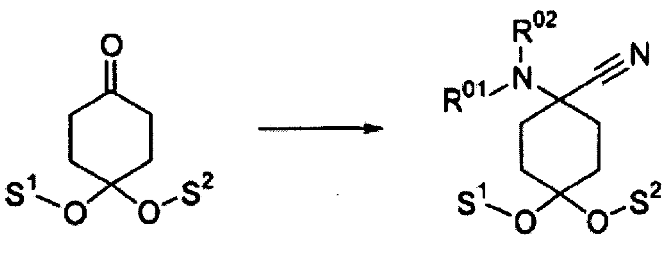

II III

qui, éventuellement, est ensuite acylé, alkylé ou sulfoné dans un ordre quelconque et de façon éventuellement répétée et/ou dans le cas de composés dans lesquels $R^{01}$ et/ou $R^{02}$ et/ou $R^{06}$ représentent H protégé par un groupe protecteur, un groupe protecteur est éliminé au moins une fois et, le cas échéant, les composés sont acylés, alkylés ou sulfonés, et/ou dans le cas de composés dans lesquels $R^{01}$ et/ou $R^{02}$ et/ou $R^{06}$ représentent

H, un groupe protecteur est introduit au moins une fois et les composés sont, le cas échéant, acylés, alkylés ou sulfonés,

b. l'aminonitrile de formule III est amené à réagir avec des réactifs organométalliques, avantageusement des réactifs de Grignard ou des réactifs organiques de lithium de formule métal-$R^3$, de manière à produire un composé de formule IVa;

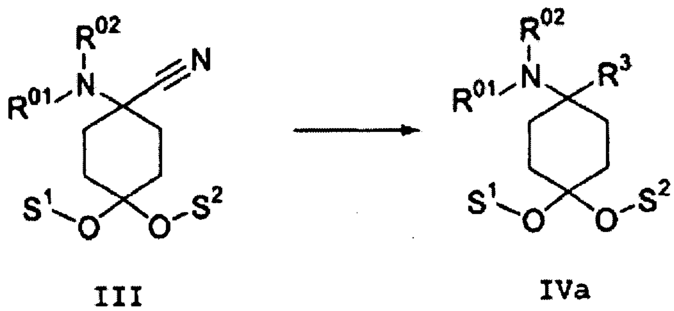

III IVa

qui est ensuite, le cas échéant, acylé, alkylé ou sulfoné dans un ordre quelconque et de façon éventuellement répétée et/ou dans le cas de composés dans lesquels $R^{01}$ et/ou $R^{02}$ et/ou $R^{06}$ représentent H protégé par un groupe protecteur, un groupe protecteur est éliminé au moins une fois et les composés sont éventuellement acylés, alkylés ou sulfonés et/ou dans le cas d'un composé dans lequel $R^{01}$ et/ou $R^{02}$ et/ou $R^{06}$ représentent H, un groupe protecteur est introduit au moins une fois et le composé est éventuellement acylé, alkylé ou sulfoné,

c. les groupes protecteurs $S^1$ et $S^2$ sur le composé répondant à la formule IVa sont éliminés, de manière à former un dérivé de 4-aminocyclohexanone 4-substitué répondant à la formule IV ;

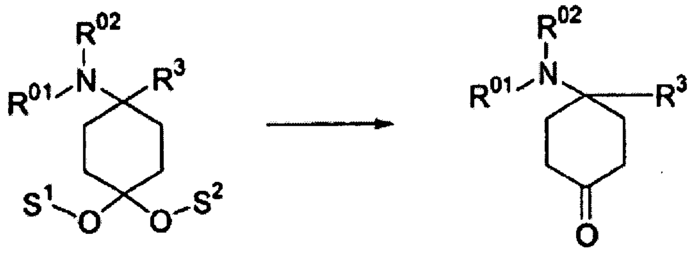

IVa IV

qui est ensuite, le cas échéant, acylé, alkylé ou sulfoné ensuite dans un ordre quelconque et de façon éventuellement répétée et/ou dans le cas de composés dans lesquels $R^{01}$ et/ou $R^{02}$ et/ou $R^{06}$ représentent H protégé par un groupe protecteur, un groupe protecteur est éliminé au moins une fois et les composés sont éventuellement acylés, alkylés ou sulfonés et/ou dans le cas d'un composé dans lequel $R^{01}$ et/ou $R^{02}$ et/ou $R^{06}$ représentent H, au moins un groupe protecteur est introduit et le composé est éventuellement acylé, alkylé ou sulfoné,

d. le dérivé 4-substitué de 4-aminocyclohexanone selon la formule IVa est aminé par voie de réduction avec un composé de formule $HNR^{04}R^{05}$, de manière à former un dérivé de cyclohexane-1,4-diamine de formule V,

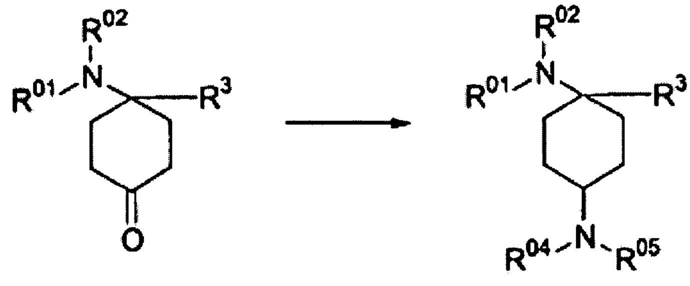

IV

V

qui est ensuite, le cas échéant, acylé, alkylé ou sulfoné dans un ordre quelconque et de façon éventuellement répétée et/ou dans le cas de composés

dans lesquels $R^{01}$ et/ou $R^{02}$ et/ou $R^{04}$ et/ou $R^{05}$ et/ou $R^{06}$ représentent H protégé avec un groupe protecteur, un groupe protecteur est éliminé au moins une fois et les composés sont éventuellement acylés, alkylés, ou sulfonés et/ou dans le cas d'un composé dans lequel $R^{01}$ et/ou $R^{02}$ et/ou $R^{04}$ et/ou $R^{05}$ et/ou $R^{06}$ représentent H, un groupe protecteur est introduit au moins une fois et le composé est éventuellement acylé, alkylé ou sulfoné jusqu'à ce qu'un composé de formule I soit formé,
$R^1$, $R^2$ $R^3$, $R^4$ et $R^5$ ayant la définition indiquée dans la revendication 1
et
$R^{01}$ et $R^{02}$ sont choisis indépendamment l'un de l'autre entre H ; H portant un groupe protecteur; un reste alkyle en $C_1$ à $C_8$ ou cycloalkyle en $C_3$ à $C_8$, chacun saturé ou non saturé, ramifié ou non ramifié, substitué une ou plusieurs fois ou non substitué; un reste aryle ou hétéroaryle, chacun substitué une ou plusieurs fois ou non substitué ; ou bien un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué;
ou bien les restes $R^{01}$ et $R^{02}$ forment ensemble un noyau et représentent $CH_2CH_2OCH_2CH_2$, $CH_2CH_2NR^{06}CH_2CH_2$ ou $(CH_2)_{3-6}$,
$R^{06}$ étant choisi entre H; H pourvu d'un groupe protecteur; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué;
$R^{04}$ est choisi entre H, H pourvu d'un groupe protecteur; un reste alkyle en $C_1$ à $C_8$, saturé ou non saturé, ramifié ou non ramifié, substitué une ou plusieurs fois ou non substitué ;
$R^{05}$ est choisi entre H, H pourvu d'un groupe protecteur ; un reste cycloalkyle en $C_3$ à $C_8$, aryle ou hétéroaryle, chacun non substitué ou substitué une ou plusieurs fois ; un groupe $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C (Y) - CH_2R^{12}$, $-C (Y) -CH_2-CH_2R^{12}$ ou $-C (Y) -CH_2-CH_2-CH_2R^{12}$,
avec $Y = H_2$,
$R^{11}$ étant choisi entre
H, un reste alkyle en $C_1$ à $C_7$, saturé ou non saturé, ramifié ou non ramifié, substitué une ou plusieurs fois ou non substitué ;
et $R^{12}$ étant choisi entre
H ; un reste cycloalkyle en $C_3$ à $C_8$, aryle ou hétéroaryle, chacun non substitué ou substitué une ou plusieurs fois, ou bien $R^{04}$ et $R^{05}$ forment ensemble un hétérocycle ayant entre 3 et 8 atomes dans le noyau, saturé ou non saturé ; substitué une ou plusieurs fois ou non substitué,
et $S^1$ et $S^2$ sont choisis indépendamment l'un de l'autre entre des groupes protecteurs ou forment ensemble un groupe protecteur, avantageusement un groupe monoacétal.

**58.** Procédé de production d'un dérivé substitué de cyclohexane-1,4-diamine suivant l'une des revendications 1 à 19, comprenant les étapes suivantes :

a. une cyclohexane-1,4-dione, protégée avec les groupes $S^1$ et $S^2$, répondant à la formule II est aminée par voie de réduction avec un composé de formule $HNR^{04}R^{05}$, de manière à former un dérivé de 4-aminocyclohexanone de formule VI ;

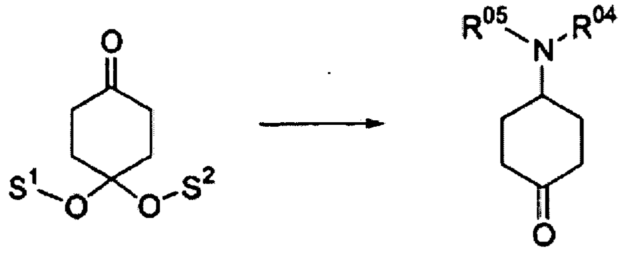

II VI

qui est ensuite, le cas échéant, acylé, alkylé ou sulfoné dans un ordre quelconque et de façon éventuellement répétée et/ou dans le cas de composés dans lesquels $R^{04}$ et/ou $R^{05}$ représentent H protégé par un groupe protecteur, un groupe protecteur est éliminé au moins une fois et le composé est éventuellement acylé, alkylé ou sulfoné et/ou dans le cas d'un composé dans lequel $R^{04}$ et/ou $R^{05}$ représentent H, un groupe protecteur est introduit au moins une fois et le composé est éventuellement acylé, alkylé ou sulfoné,

b. le dérivé de 4-aminocyclohexanone de formule VI est amené à réagir en présence d'un composé de formule $HNR^{01}R^{02}$ avec un cyanure, avantageusement le cyanure de potassium, pour former un dérivé de cyclohexanonenitrile de formule VII,

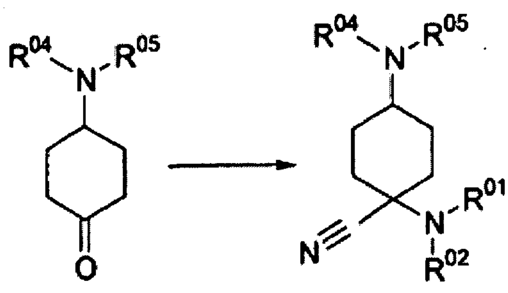

VI VII

qui est ensuite, le cas échéant, acylé, alkylé ou sulfoné dans un ordre quelconque et de façon éventuellement répétée et/ou dans le cas de composés dans lesquels $R^{01}$ et/ou $R^{02}$ et/ou $R^{04}$ et/ou $R^{05}$ et/ou $R^{06}$ représentent H protégé avec un groupe protecteur, un groupe protecteur est éliminé au moins une fois et le composé est éventuellement acylé, alkylé ou sulfoné et/ou dans le cas d'un composé dans lequel $R^{01}$ et/ou $R^{02}$ et/ou $R^{04}$ et/ou $R^{05}$ et/ou $R^{06}$ représentent H, un groupe protecteur est introduit au moins une fois et le composé est éventuellement acylé, alkylé ou sulfoné,

c. le dérivé de cyclohexanonenitrile de formule VII est amené à réagir avec des réactifs organométalliques, avantageusement des réactifs de Grignard ou des réactifs organiques de lithium, de formule métal-$R^3$ et finalement les groupes protecteurs $S^1$ et $S^2$ sont éliminés, de manière à former un dérivé de cyclohexane-1,4-diamine de formule V,

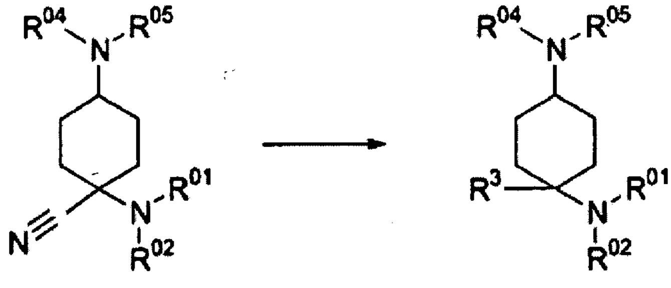

VII V

qui, le cas échéant, est ensuite acylé, alkylé ou sulfoné dans un ordre quelconque et de façon éventuellement répétée et/ou dans le cas de composés

dans lesquels $R^{01}$ et/ou $R^{02}$ et/ou $R^{04}$ et/ou $R^{05}$ et/ou $R^{06}$ représentent H protégé par un groupe protecteur, un groupe protecteur est éliminé au moins une fois et le composé est éventuellement acylé, alkylé ou sulfoné et/ou dans le cas d'un composé dans lequel $R^{01}$ et/ou $R^{02}$ et/ou $R^{04}$ et/ou $R^{05}$ et/ou $R^{06}$ représentent H, un groupe protecteur est introduit au moins une fois et le composé est éventuellement acylé, alkylé ou sulfoné jusqu'à ce qu'un composé de formule I soit produit,
$R^1$, $R^2$, $R^3$, $R^4$ et $R^5$ ayant la définition indiquée dans la revendication 1 et
$R^{01}$ et $R^{02}$ sont choisis, indépendamment l'un de l'autre, entre H ; H pourvu d'un groupe protecteur ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ; ou bien les restes $R^{01}$ et $R^{02}$ forment ensemble un noyau et représentent $CH_2CH_2OCH_2CH_2$, $CH_2CH_2NR^{06}$ $CH_2CH_2$ ou $(CH_2)_{3-6}$,
$R^{06}$ étant choisi entre H; H pourvu d'un groupe protecteur; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;
$R^{04}$ est choisi entre H, H pourvu d'un groupe protecteur ; un reste alkyle en $C_1$ à $C_8$, saturé ou non saturé, ramifié ou non ramifié, substitué une ou plusieurs fois ou non substitué;
$R^{05}$ est choisi entre H pourvu d'un groupe protecteur; un reste cycloalkyle en $C_3$ à $C_8$, aryle ou hétéroaryle, chacun non substitué ou substitué une ou plusieurs fois ; un groupe $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C (Y) -CH_2R^{12}$, $-C (Y) -CH_2CH_2R^{12}$ ou $-C (Y) -CH_2CH_2-CH_2R^{12}$,
avec $Y = H_2$,
$R^{11}$ étant choisi entre
H, un reste alkyle en $C_1$ à $C_7$, saturé ou non saturé, ramifié ou non ramifié, substitué une ou plusieurs fois ou non substitué ;
et $R^{12}$ étant choisi entre
H ; un reste cycloalkyle en $C_3$ à $C_8$, aryle ou hétéroaryle, chacun non substitué ou substitué une ou plusieurs fois, ou bien $R^{04}$ et $R^{05}$ forment ensemble un hétérocycle ayant entre 3 et 8 atomes dans le noyau, saturé ou non saturé ; substitué une ou plusieurs fois ou non substitué,
et $S^1$ et $S^2$ sont choisis indépendamment l'un de l'autre entre des groupes protecteurs ou forment ensemble un groupe protecteur, avantageusement un groupe monoacétal.

**59.** Procédé de production d'un dérivé substitué de cyclohexane-1,4-diamine suivant l'une des revendications 57 ou 58, **caractérisé en ce que** les groupes protecteurs portés par H dans le cas de $R^{01}$, $R^{02}$, $R^{04}$, $R^{05}$ et/ou $R^{06}$ sont choisis entre des groupes alkyle, benzyle ou carbamate, en particulier FMOC, Z ou Boc.

**60.** Procédé de production d'un dérivé substitué de cyclohexane-1,4-diamine suivant la revendication 57, **caractérisé en ce que** l'amination par voie de réduction dans l'étape d est conduite en présence de formiate d'ammonium, d'acétate d'ammonium ou de $NaCNBH_3$.

**61.** Procédé de production d'un dérivé substitué de cyclohexane-1,4-diamine suivant la revendication 57, **caractérisé en ce que**, au lieu de l'amination par voie de réduction avec $HNR^{04}R^{05}$ dans l'étape d, le composé IV est amené à réagir avec de l'hydroxylamine et le produit est réduit après formation de l'oxime.

**62.** Procédé de production d'un dérivé substitué de cyclohexane-1,4-diamine suivant la revendication 58, **caractérisé en ce que**, dans l'étape b, le reste $R^{01}$ représente H dans la formule $HNR^{01}R^{02}$, la réaction avec le cyanure a lieu avec TMSCN et un groupe protecteur est ensuite éventuellement introduit sur $R^{01}$.